# EUROPEAN PATENT APPLICATION

(11) **EP 1 797 897 A1**
(43) Date of publication of application: **20.06.2007**
(21) Application number: 05768466.4
(22) Date of filing: 03.08.2005
(51) Int. Cl.: A61K 45/00, A61K 31/7105, A61K 31/711, A61K 39/395, A61K 31/4725, A61K 48/00, A61P 3/04, A61P 3/06, A61P 3/10, A61P 43/00, C07K 16/18, C12N 15/00

(54) **MEDICINAL COMPOSITION CONTAINING MELTRIN ANTAGONIST**

(30) Priority: 03.08.2004 JP 2004227319; 24.11.2004 JP 2004339661
(71) Applicant: MOCHIDA PHARMACEUTICAL CO., LTD., Shinjuku-ku Tokyo 160-8515 (JP)
(72) Inventor: SEHARA, Atsuko, Sakaimachi-dori Takeyamachi-agaru Nakagyo-ku, Kyoto-shi, Kyoto 604-0802 (JP); KURISAKI, Tomohiro, Kyoto-shi, Kyoto 606-0855 (JP); MASAKI, Megumi, Kyoto-shi, Kyoto 6068202 (JP); SHIRAKAWA, Kamon, MOCHIDA PHARMACEUTICAL CO., LTD., Tokyo 160-8515 (JP); SHIMIZU, Yusuke, MOCHIDA PHARMACEUTICAL CO., LTD., Tokyo 160-8515 (JP); ARAKI, Yasuko, MOCHIDA PHARMACEUTICAL CO., LTD., Tokyo 160-8515 (JP); HOSHIDA, Keiko, MOCHIDA PHARMACEUTICAL CO., LTD., Tokyo 160-8515 (JP)
(74) Representative: Grünecker, Kinkeldey, Stockmair & Schwanhäusser Anwaltssozietät
(86) International application number: PCT/JP2005/014235
(87) International publication number: WO 2006/013904

(57) **Abstract**

Mainly concerning the pharmaceutical field, the present invention has particular features in that an inhibitory effect is exerted on the function of meltrin α of promoting the differentiation of adipocytes by preparing a compound serving as an antagonist to meltrin α, for example, an antisense suppressing the expression of a meltrin α gene or an anti-meltrin α antibody capable of inhibiting the physiological activities of meltrin α, and administering the compound. By employing such a meltrin α antagonist as described above as the effective component, a preventive/therapeutic agent is provided which is efficacious against obesity, adiposis, or a disease caused by or related to obesity such as diabetes.

SEQUENCE LISTING

<110>  MOCHIDA PHARMACEUTICAL CO., LTD.

<120>  Pharmaceutical composition containing meltrin antagonist

<130>  EP49749FZ163pau

<140>  EP 05 768 466.4
<141>  2005-08-03

<150>  PCT/JP2005/014235
<151>  2005-08-03

<150>  JP 2004-227319
<151>  2004-08-03

<150>  JP2004-339661
<151>  2004-11-24

<160>  496

<170>  PatentIn version 3.1

<210>  1
<211>  3328
<212>  DNA
<213>  human

<400>  1
   cactaacgct cttcctagtc cccgggccaa ctcggacagt ttgctcattt attgcaacgg      60
   tcaaggctgg cttgtgccag aacggcgcgc gcgcgacgca cgcacacaca cggggggaaa     120
   cttttttaaa aatgaaaggc tagaagagct cagcggcggc gcgggccgtg cgcgagggct     180
   ccggagctga ctcgccgagg caggaaatcc ctccggtcgc gacgcccggc cccgctcggc     240
   gcccgcgtgg gatggtgcag cgctcgccgc cgggcccgag agctgctgca ctgaaggccg     300
   gcgacgatgg cagcgcgccc gctgcccgtg tcccccgccc gcgccctcct gctcgccctg     360
   gccggtgctc tgctcgcgcc ctgcgaggcc cgaggggtga gcttatggaa cgaaggaaga     420
   gctgatgaag ttgtcagtgc ctctgttcgg agtggggacc tctggatccc agtgaagagc     480
   ttcgactcca agaatcatcc agaagtgctg aatattcgac tacaacggga aagcaaagaa     540
   ctgatcataa atctggaaag aaatgaaggt ctcattgcca gcagtttcac ggaaacccac     600
   tatctgcaag acggtactga tgtctccctc gctcgaaatt acacggtaat tctgggtcac     660
   tgttactacc atggacatgt acggggatat tctgattcag cagtcagtct cagcacgtgt     720
   tctggtctca ggggacttat tgtgtttgaa aatgaaagct atgtcttaga accaatgaaa     780
   agtgcaacca acagatacaa actcttccca gcgaagaagc tgaaaagcgt ccggggatca     840
   tgtggatcac atcacaacac accaaacctc gctgcaaaga atgtgtttcc accaccctct     900
   cagacatggg caagaaggca taaaagagag accctcaagg caactaagta tgtggagctg     960
   gtgatcgtgg cagacaaccg agagtttcag aggcaaggaa aagatctgga aaaagttaag    1020
   cagcgattaa tagagattgc taatcacgtt gacaagtttt acagaccact gaacattcgg    1080
   atcgtgttgg taggcgtgga agtgtggaat gacatggaca aatgctctgt aagtcaggac    1140
   ccattcacca gcctccatga atttctggac tggaggaaga tgaagcttct acctcgcaaa    1200
   tcccatgaca atgcgcagct tgtcagtggg gtttatttcc aagggaccac catcggcatg 1260
   gccccaatca tgagcatgtg cacggcagac cagtctgggg gaattgtcat ggaccattca 1320
   gacaatcccc ttggtgcagc cgtgaccctg gcacatgagc tgggccacaa tttcgggatg 1380
   aatcatgaca cactggacag gggctgtagc tgtcaaatgg cggttgagaa aggaggctgc 1440
   atcatgaacg cttccaccgg gtacccattt cccatggtgt tcagcagttg cagcaggaag 1500
   gacttggaga ccagcctgga gaaaggaatg ggggtgtgcc tgtttaacct gccggaagtc 1560
   agggagtctt tcgggggcca gaagtgtggg aacagatttg tggaagaagg agaggagtgt 1620
   gactgtgggg agccagagga atgtatgaat cgctgctgca atgccaccac ctgtaccctg 1680
   aagccggacg ctgtgtgcgc acatgggctg tgctgtgaag actgccagct gaagcctgca 1740
   ggaacagcgt gcagggactc cagcaactcc tgtgacctcc cagagttctg cacaggggcc 1800
   agccctcact gcccagccaa cgtgtacctg cacgatgggc actcatgtca ggatgtggac 1860
   ggctactgct acaatggcat ctgccagact cacgagcagc agtgtgtcac actctgggga 1920
   ccaggtgcta aacctgcccc tgggatctgc tttgagagag tcaattctgc aggtgatcct 1980
   tatggcaact gtggcaaagt ctcgaagagt tcctttgcca aatgcgagat gagagatgct 2040
   aaatgtggaa aaatccagtg tcaaggaggt gccagccggc cagtcattgg taccaatgcc 2100
   gtttccatag aaacaaacat ccccctgcag caaggaggcc ggattctgtg ccgggggacc 2160
   cacgtgtact tgggcgatga catgccggac ccagggcttg tgcttgcagg cacaaagtgt 2220
   gcagatggaa aaatctgcct gaatcgtcaa tgtcaaaata ttagtgtctt tggggttcac 2280
   gagtgtgcaa tgcagtgcca cggcagaggg gtgtgcaaca acaggaagaa ctgccactgc 2340
   gaggcccact gggcacctcc cttctgtgac aagtttggct ttggaggaag cacagacagc 2400
   ggccccatcc ggcaagcaga agcaaggcag gaagctgcag agtccaacag ggagcgcggc 2460
   cagggccagg agcccgtggg atcgcaggag catgcgtcta ctgcctcact gacactcatc 2520
   tgagccctcc catgacatgg agaccgtgac cagtgctgct gcagaggagg tcacgcgtcc 2580
   ccaaggcctc ctgtgactgg cagcattgac tctgtggctt tgccatcgtt tccatgacaa 2640
   cagacacaac acagttctcg gggctcagga ggggaagtcc agcctaccag gcaggtctgc 2700
   agaaacagtg caaggaaggg cagcgacttc ctggttgagc ttctgctaaa acatggacat 2760
   gcttcagtgc tgctcctgag agagtagcag gttaccactc tggcaggccc cagccctgca 2820
   gcaaggagga agaggactca aaagtctggc ctttcactga gcctccacag cagtggggga 2880
   gaagcaaggg ttgggcccag tgtccccttt ccccagtgac acctccgcct tggcagccct 2940
   gatgactggt ctctggctgc aacttaatgc tctgatatgg cttttagcat ttattatatg 3000
   aaaatagcag ggttttagtt tttaatttat cagagaccct gccacccatt ccatctccat 3060
   ccaagcaaac tgaatggcat tgaaacaaac tggagaagaa ggtaggagaa agggcggtga 3120
   actctggctc tttgctgtgg acatgcgtga ccagcagtac tcaggtttga gggtttgcag 3180
   aaagccaggg aacccacaga gtcaccaacc cttcatttaa caagtaagaa tgttaaaaag 3240
   tgaaaacaat gtaagagcct aactccatcc cccgtggcca ttactgcata aaaatagagt 3300
   gcattttgaa ataaaaaaaa aaaaaaaa 3328

<210> 2
<211> 738
<212> PRT
<213> human

<400> 2

Met Ala Ala Arg Pro Leu Pro Val Ser Pro Ala Arg Ala Leu Leu Leu
1               5                   10                  15

Ala Leu Ala Gly Ala Leu Leu Ala Pro Cys Glu Ala Arg Gly Val Ser
            20                  25                  30

Leu Trp Asn Glu Gly Arg Ala Asp Glu Val Val Ser Ala Ser Val Arg
        35                  40                  45

Ser Gly Asp Leu Trp Ile Pro Val Lys Ser Phe Asp Ser Lys Asn His
    50                  55                  60

Pro Glu Val Leu Asn Ile Arg Leu Gln Arg Glu Ser Lys Glu Leu Ile
65                  70                  75                  80

Ile Asn Leu Glu Arg Asn Glu Gly Leu Ile Ala Ser Ser Phe Thr Glu
                85                  90                  95

Thr His Tyr Leu Gln Asp Gly Thr Asp Val Ser Leu Ala Arg Asn Tyr
            100                 105                 110

Thr Val Ile Leu Gly His Cys Tyr Tyr His Gly His Val Arg Gly Tyr
        115                 120                 125

Ser Asp Ser Ala Val Ser Leu Ser Thr Cys Ser Gly Leu Arg Gly Leu
    130                 135                 140

Ile Val Phe Glu Asn Glu Ser Tyr Val Leu Glu Pro Met Lys Ser Ala
145                 150                 155                 160

Thr Asn Arg Tyr Lys Leu Phe Pro Ala Lys Lys Leu Lys Ser Val Arg
                165                 170                 175

Gly Ser Cys Gly Ser His His Asn Thr Pro Asn Leu Ala Ala Lys Asn
            180                 185                 190

Val Phe Pro Pro Pro Ser Gln Thr Trp Ala Arg Arg His Lys Arg Glu
        195                 200                 205

Thr Leu Lys Ala Thr Lys Tyr Val Glu Leu Val Ile Val Ala Asp Asn
    210                 215                 220

Arg Glu Phe Gln Arg Gln Gly Lys Asp Leu Glu Lys Val Lys Gln Arg

225                 230                 235                 240

Leu Ile Glu Ile Ala Asn His Val Asp Lys Phe Tyr Arg Pro Leu Asn
                245                 250                 255

Ile Arg Ile Val Leu Val Gly Val Glu Val Trp Asn Asp Met Asp Lys
            260                 265                 270

Cys Ser Val Ser Gln Asp Pro Phe Thr Ser Leu His Glu Phe Leu Asp
        275                 280                 285

Trp Arg Lys Met Lys Leu Leu Pro Arg Lys Ser His Asp Asn Ala Gln
    290                 295                 300

Leu Val Ser Gly Val Tyr Phe Gln Gly Thr Thr Ile Gly Met Ala Pro
305                 310                 315                 320

Ile Met Ser Met Cys Thr Ala Asp Gln Ser Gly Gly Ile Val Met Asp
                325                 330                 335

His Ser Asp Asn Pro Leu Gly Ala Ala Val Thr Leu Ala His Glu Leu
            340                 345                 350

Gly His Asn Phe Gly Met Asn His Asp Thr Leu Asp Arg Gly Cys Ser
        355                 360                 365

Cys Gln Met Ala Val Glu Lys Gly Gly Cys Ile Met Asn Ala Ser Thr
    370                 375                 380

Gly Tyr Pro Phe Pro Met Val Phe Ser Ser Cys Ser Arg Lys Asp Leu
385                 390                 395                 400

Glu Thr Ser Leu Glu Lys Gly Met Gly Val Cys Leu Phe Asn Leu Pro
                405                 410                 415

Glu Val Arg Glu Ser Phe Gly Gly Gln Lys Cys Gly Asn Arg Phe Val
            420                 425                 430

Glu Glu Gly Glu Glu Cys Asp Cys Gly Glu Pro Glu Glu Cys Met Asn
        435                 440                 445

Arg Cys Cys Asn Ala Thr Thr Cys Thr Leu Lys Pro Asp Ala Val Cys
    450                 455                 460

Ala His Gly Leu Cys Cys Glu Asp Cys Gln Leu Lys Pro Ala Gly Thr
465                 470                 475                 480

Ala Cys Arg Asp Ser Ser Asn Ser Cys Asp Leu Pro Glu Phe Cys Thr
                485                 490                 495

Gly Ala Ser Pro His Cys Pro Ala Asn Val Tyr Leu His Asp Gly His
            500                 505                 510

Ser Cys Gln Asp Val Asp Gly Tyr Cys Tyr Asn Gly Ile Cys Gln Thr
        515                 520                 525

His Glu Gln Gln Cys Val Thr Leu Trp Gly Pro Gly Ala Lys Pro Ala
    530                 535                 540

Pro Gly Ile Cys Phe Glu Arg Val Asn Ser Ala Gly Asp Pro Tyr Gly
545                 550                 555                 560

Asn Cys Gly Lys Val Ser Lys Ser Ser Phe Ala Lys Cys Glu Met Arg
                565                 570                 575

Asp Ala Lys Cys Gly Lys Ile Gln Cys Gln Gly Gly Ala Ser Arg Pro
            580                 585                 590

Val Ile Gly Thr Asn Ala Val Ser Ile Glu Thr Asn Ile Pro Leu Gln
        595                 600                 605

Gln Gly Gly Arg Ile Leu Cys Arg Gly Thr His Val Tyr Leu Gly Asp
    610                 615                 620

Asp Met Pro Asp Pro Gly Leu Val Leu Ala Gly Thr Lys Cys Ala Asp
625                 630                 635                 640

Gly Lys Ile Cys Leu Asn Arg Gln Cys Gln Asn Ile Ser Val Phe Gly
                645                 650                 655

Val His Glu Cys Ala Met Gln Cys His Gly Arg Gly Val Cys Asn Asn
            660                 665                 670

Arg Lys Asn Cys His Cys Glu Ala His Trp Ala Pro Pro Phe Cys Asp
        675                 680                 685

Lys Phe Gly Phe Gly Gly Ser Thr Asp Ser Gly Pro Ile Arg Gln Ala
    690                 695                 700

Glu Ala Arg Gln Glu Ala Ala Glu Ser Asn Arg Glu Arg Gly Gln Gly
705                 710                 715                 720

Gln Glu Pro Val Gly Ser Gln Glu His Ala Ser Thr Ala Ser Leu Thr
                725                 730                 735

Leu Ile

<210>  3
<211>  5062
<212>  DNA
<213>  human

<400>  3
cactaacgct cttcctagtc cccgggccaa ctcggacagt ttgctcattt attgcaacgg      60

tcaaggctgg cttgtgccag aacggcgcgc gcgcgacgca cgcacacaca cggggggaaa 120
cttttttaaa aatgaaaggc tagaagagct cagcggcggc gcgggccgtg cgcgagggct 180
ccggagctga ctcgccgagg caggaaatcc ctccggtcgc gacgcccggc cccgctcggc 240
gcccgcgtgg gatggtgcag cgctcgccgc cgggcccgag agctgctgca ctgaaggccg 300
gcgacgatgg cagcgcgccc gctgcccgtg tcccccgccc gcgccctcct gctcgccctg 360
gccggtgctc tgctcgcgcc ctgcgaggcc cgaggggtga gcttatggaa ccaaggaaga 420
gctgatgaag ttgtcagtgc ctctgttcgg agtggggacc tctggatccc agtgaagagc 480
ttcgactcca agaatcatcc agaagtgctg aatattcgac tacaacggga aagcaaagaa 540
ctgatcataa atctggaaag aaatgaaggt ctcattgcca gcagtttcac ggaaacccac 600
tatctgcaag acggtactga tgtctccctc gctcgaaatt acacggtaat tctgggtcac 660
tgttactacc atggacatgt acggggatat tctgattcag cagtcagtct cagcacgtgt 720
tctggtctca ggggacttat tgtgtttgaa aatgaaagct atgtcttaga accaatgaaa 780
agtgcaacca acagatacaa actcttccca gcgaagaagc tgaaaagcgt ccggggatca 840
tgtggatcac atcacaacac accaaacctc gctgcaaaga atgtgtttcc accaccctct 900
cagacatggg caagaaggca taaaagagag accctcaagg caactaagta tgtggagctg 960
gtgatcgtgg cagacaaccg agagtttcag aggcaaggaa aagatctgga aaaagttaag 1020
cagcgattaa tagagattgc taatcacgtt gacaagtttt acagaccact gaacattcgg 1080
atcgtgttgg taggcgtgga agtgtggaat gacatggaca aatgctctgt aagtcaggac 1140
ccattcacca gcctccatga atttctggac tggaggaaga tgaagcttct acctcgcaaa 1200
tcccatgaca atgcgcagct tgtcagtggg gtttatttcc aagggaccac catcggcatg 1260
gccccaatca tgagcatgtg cacggcagac cagtctgggg gaattgtcat ggaccattca 1320
gacaatcccc ttggtgcagc cgtgaccctg gcacatgagc tgggccacaa tttcgggatg 1380
aatcatgaca cactggacag gggctgtagc tgtcaaatgg cggttgagaa aggaggctgc 1440
atcatgaacg cttccaccgg gtacccattt cccatggtgt tcagcagttg cagcaggaag 1500
gacttggaga ccagcctgga gaaaggaatg ggggtgtgcc tgtttaacct gccggaagtc 1560
agggagtctt tcgggggcca gaagtgtggg aacagatttg tggaagaagg agaggagtgt 1620
gactgtgggg agccagagga atgtatgaat cgctgctgca atgccaccac ctgtaccctg 1680
aagccggacg ctgtgtgcgc acatgggctg tgctgtgaag actgccagct gaagcctgca 1740
ggaacagcgt gcagggactc cagcaactcc tgtgacctcc cagagttctg cacaggggcc 1800
agccctcact gcccagccaa cgtgtacctg cacgatgggc actcatgtca ggatgtggac 1860
ggctactgct acaatggcat ctgccagact cacgagcagc agtgtgtcac actctgggga 1920
ccaggtgcta aacctgcccc tgggatctgc tttgagagag tcaattctgc aggtgatcct 1980
tatggcaact gtggcaaagt ctcgaagagt tcctttgcca aatgcgagat gagagatgct 2040
aaatgtggaa aaatccagtg tcaaggaggt gccagccggc cagtcattgg taccaatgcc 2100

gtttccatag aaacaaacat ccccctgcag caaggaggcc ggattctgtg ccgggggacc 2160
cacgtgtact tgggcgatga catgccggac ccagggcttg tgcttgcagg cacaaagtgt 2220
gcagatggaa aaatctgcct gaatcgtcaa tgtcaaaata ttagtgtctt tggggttcac 2280
gagtgtgcaa tgcagtgcca cggcagaggg gtgtgcaaca acaggaagaa ctgccactgc 2340
gaggcccact gggcacctcc cttctgtgac aagtttggct ttggaggaag cacagacagc 2400
ggccccatcc ggcaagcaga taaccaaggt ttaaccatag gaattctggt gaccatcctg 2460
tgtcttcttg ctgccggatt tgtggtttat ctcaaaagga agaccttgat acgactgctg 2520
tttacaaata agaagaccac cattgaaaaa ctaaggtgtg tgcgcccttc ccggccaccc 2580
cgtggcttcc aaccctgtca ggctcacctc ggccaccttg gaaaaggcct gatgaggaag 2640
ccgccagatt cctacccacc gaaggacaat cccaggagat tgctgcagtg tcagaatgtt 2700
gacatcagca gacccctcaa cggcctgaat gtccctcagc cccagtcaac tcagcgagtg 2760
cttcctcccc tccaccgggc cccacgtgca cctagcgtcc ctgccagacc cctgccagcc 2820
aagcctgcac ttaggcaggc ccaggggacc tgtaagccaa acccccctca gaagcctctg 2880
cctgcagatc ctctggccag aacaactcgg ctcactcatg ccttggccag gaccccagga 2940
caatgggaga ctgggctccg cctggcaccc ctcagacctg ctccacaata tccacaccaa 3000
gtgcccagat ccacccacac cgcctatatt aagtgagaag ccgacacctt ttttcaacag 3060
tgaagacaga agtttgcact atctttcagc tccagttgga gttttttgta ccaactttta 3120
ggattttttt taatgtttaa aacatcatta ctataagaac tttgagctac tgccgtcagt 3180
gctgtgctgt gctatggtgc tctgtctact tgcacaggta cttgtaaatt attaatttat 3240
gcagaatgtt gattacagtg cagtgcgctg tagtaggcat ttttaccatc actgagtttt 3300
ccatggcagg aaggcttgtt gtgcttttag tattttagtg aacttgaaat atcctgcttg 3360
atgggattct ggacaggatg tgtttgcttt ctgatcaagg ccttattgga aagcagtccc 3420
ccaactaccc ccagctgtgc ttatggtacc agatgcagct caagagatcc caagtagaat 3480
ctcagttgat tttctggatt ccccatctca ggccagagcc aaggggcttc aggtccaggc 3540
tgtgtttggc tttcagggag gccctgtgcc ccttgacaac tggcaggcag gctcccaggg 3600
acacctggga gaaatctggc ttctggccag gaagctttgg tgagaacctg ggttgcagac 3660
aggaatctta aggtgtagcc acaccaggat agagactgga acactagaca agccagaact 3720
tgaccctgag ctgaccagcc gtgagcatgt ttggaagggg tctgtagtgt cactcaaggc 3780
ggtgcttgat agaaatgcca agcacttctt tttctcgctg tcctttctag agcactgcca 3840
ccagtaggtt atttagcttg ggaaaggtgg tgtttctgta agaaacctac tgcccaggca 3900
ctgcaaaccg ccacctccct atactgcttg gagctgagca aatcaccaca aactgtaata 3960
caatgatcct gtattcagac agatgaggac tttccatggg accacaacta ttttcagatg 4020
tgaaccatta accagatcta gtcaatcaag tctgtttact gcaaggttca acttattaac 4080
aattaggcag actctttatg cttgcaaaaa ctacaaccaa tggaatgtga tgttcatggg 4140
tatagttcat gtctgctatc attattcgta gatattggac aaagaacctt ctctatgggg 4200

catcctcttt ttccaacttg gctgcaggaa tctttaaaag atgcttttaa cagagtctga 4260

acctatttct taaacacttg caacctacct gttgagcatc acagaatgtg ataaggaaat 4320

caacttgctt atcaacttcc taaatattat gagatgtggc ttgggcagca tccccttgaa 4380

ctcttcactc ttcaaatgcc tgactaggga gccatgtttc acaaggtctt taaagtgact 4440

aatggcatga gaaatacaaa aatactcaga taaggtaaaa tgccatgatg cctctgtctt 4500

ctggactggt tttcacatta gaagacaatt gacaacagtt acataattca ctctgagtgt 4560

tttatgagaa agccttcttt tggggtcaac agttttccta tgctttgaaa cagaaaaata 4620

tgtaccaaga atcttggttt gccttccaga aaacaaaact gcatttcact ttcccggtgt 4680

tccccactgt atctaggcaa catagtattc atgactatgg ataaactaaa cacgtgacac 4740

aaacacacac aaaagggaac ccagctctaa tacattccaa ctcgtatagc atgcatctgt 4800

ttattctata gttattaagt tctttaaaat gtaaagccat gctggaaaat aatactgctg 4860

agatacatac agaattactg taactgatta cacttggtaa ttgtactaaa gccaaacata 4920

tatatactat taaaaaggtt tacagaattt tatggtgcat tacgtgggca ttgtcttttt 4980

agatgcccaa atccttagat ctggcatgtt agcccttcct ccaattataa gaggatatga 5040

accaaaaaaa aaaaaaaaaa aa 5062

<210> 4
<211> 909
<212> PRT
<213> human

<400> 4

Met Ala Ala Arg Pro Leu Pro Val Ser Pro Ala Arg Ala Leu Leu Leu
1               5                   10                  15

Ala Leu Ala Gly Ala Leu Leu Ala Pro Cys Glu Ala Arg Gly Val Ser
            20                  25                  30

Leu Trp Asn Gln Gly Arg Ala Asp Glu Val Val Ser Ala Ser Val Arg
        35                  40                  45

Ser Gly Asp Leu Trp Ile Pro Val Lys Ser Phe Asp Ser Lys Asn His
    50                  55                  60

Pro Glu Val Leu Asn Ile Arg Leu Gln Arg Glu Ser Lys Glu Leu Ile
65                  70                  75                  80

Ile Asn Leu Glu Arg Asn Glu Gly Leu Ile Ala Ser Ser Phe Thr Glu
                85                  90                  95

Thr His Tyr Leu Gln Asp Gly Thr Asp Val Ser Leu Ala Arg Asn Tyr
            100                 105                 110

Thr Val Ile Leu Gly His Cys Tyr Tyr His Gly His Val Arg Gly Tyr
        115                 120                 125

Ser Asp Ser Ala Val Ser Leu Ser Thr Cys Ser Gly Leu Arg Gly Leu
    130                 135                 140

Ile Val Phe Glu Asn Glu Ser Tyr Val Leu Glu Pro Met Lys Ser Ala
145                 150                 155                 160

Thr Asn Arg Tyr Lys Leu Phe Pro Ala Lys Lys Leu Lys Ser Val Arg
                165                 170                 175

Gly Ser Cys Gly Ser His His Asn Thr Pro Asn Leu Ala Ala Lys Asn
            180                 185                 190

Val Phe Pro Pro Pro Ser Gln Thr Trp Ala Arg Arg His Lys Arg Glu
        195                 200                 205

Thr Leu Lys Ala Thr Lys Tyr Val Glu Leu Val Ile Val Ala Asp Asn
    210                 215                 220

Arg Glu Phe Gln Arg Gln Gly Lys Asp Leu Glu Lys Val Lys Gln Arg
225                 230                 235                 240

Leu Ile Glu Ile Ala Asn His Val Asp Lys Phe Tyr Arg Pro Leu Asn
                245                 250                 255

Ile Arg Ile Val Leu Val Gly Val Glu Val Trp Asn Asp Met Asp Lys
            260                 265                 270

Cys Ser Val Ser Gln Asp Pro Phe Thr Ser Leu His Glu Phe Leu Asp
        275                 280                 285

Trp Arg Lys Met Lys Leu Leu Pro Arg Lys Ser His Asp Asn Ala Gln
    290                 295                 300

Leu Val Ser Gly Val Tyr Phe Gln Gly Thr Thr Ile Gly Met Ala Pro
305                 310                 315                 320

Ile Met Ser Met Cys Thr Ala Asp Gln Ser Gly Gly Ile Val Met Asp
                325                 330                 335

His Ser Asp Asn Pro Leu Gly Ala Ala Val Thr Leu Ala His Glu Leu
            340                 345                 350

Gly His Asn Phe Gly Met Asn His Asp Thr Leu Asp Arg Gly Cys Ser
        355                 360                 365

Cys Gln Met Ala Val Glu Lys Gly Gly Cys Ile Met Asn Ala Ser Thr
    370                 375                 380

Gly Tyr Pro Phe Pro Met Val Phe Ser Ser Cys Ser Arg Lys Asp Leu
385                 390                 395                 400

Glu Thr Ser Leu Glu Lys Gly Met Gly Val Cys Leu Phe Asn Leu Pro
                405                 410                 415

Glu Val Arg Glu Ser Phe Gly Gly Gln Lys Cys Gly Asn Arg Phe Val
            420                 425                 430

Glu Glu Gly Glu Glu Cys Asp Cys Gly Glu Pro Glu Glu Cys Met Asn
        435                 440                 445

Arg Cys Cys Asn Ala Thr Thr Cys Thr Leu Lys Pro Asp Ala Val Cys
    450                 455                 460

Ala His Gly Leu Cys Cys Glu Asp Cys Gln Leu Lys Pro Ala Gly Thr
465                 470                 475                 480

Ala Cys Arg Asp Ser Ser Asn Ser Cys Asp Leu Pro Glu Phe Cys Thr
                485                 490                 495

Gly Ala Ser Pro His Cys Pro Ala Asn Val Tyr Leu His Asp Gly His
            500                 505                 510

Ser Cys Gln Asp Val Asp Gly Tyr Cys Tyr Asn Gly Ile Cys Gln Thr
        515                 520                 525

His Glu Gln Gln Cys Val Thr Leu Trp Gly Pro Gly Ala Lys Pro Ala
    530                 535                 540

Pro Gly Ile Cys Phe Glu Arg Val Asn Ser Ala Gly Asp Pro Tyr Gly
545                 550                 555                 560

Asn Cys Gly Lys Val Ser Lys Ser Ser Phe Ala Lys Cys Glu Met Arg
                565                 570                 575

Asp Ala Lys Cys Gly Lys Ile Gln Cys Gln Gly Gly Ala Ser Arg Pro
            580                 585                 590

Val Ile Gly Thr Asn Ala Val Ser Ile Glu Thr Asn Ile Pro Leu Gln
        595                 600                 605

Gln Gly Gly Arg Ile Leu Cys Arg Gly Thr His Val Tyr Leu Gly Asp
    610                 615                 620

Asp Met Pro Asp Pro Gly Leu Val Leu Ala Gly Thr Lys Cys Ala Asp
625                 630                 635                 640

Gly Lys Ile Cys Leu Asn Arg Gln Cys Gln Asn Ile Ser Val Phe Gly
                645                 650                 655

Val His Glu Cys Ala Met Gln Cys His Gly Arg Gly Val Cys Asn Asn
            660                 665                 670

Arg Lys Asn Cys His Cys Glu Ala His Trp Ala Pro Pro Phe Cys Asp
        675                 680                 685

Lys Phe Gly Phe Gly Gly Ser Thr Asp Ser Gly Pro Ile Arg Gln Ala
    690                 695                 700

Asp Asn Gln Gly Leu Thr Ile Gly Ile Leu Val Thr Ile Leu Cys Leu
705                 710                 715                 720

Leu Ala Ala Gly Phe Val Val Tyr Leu Lys Arg Lys Thr Leu Ile Arg
                725                 730                 735

Leu Leu Phe Thr Asn Lys Lys Thr Thr Ile Glu Lys Leu Arg Cys Val
            740                 745                 750

Arg Pro Ser Arg Pro Pro Arg Gly Phe Gln Pro Cys Gln Ala His Leu
        755                 760                 765

Gly His Leu Gly Lys Gly Leu Met Arg Lys Pro Pro Asp Ser Tyr Pro
    770                 775                 780

Pro Lys Asp Asn Pro Arg Arg Leu Leu Gln Cys Gln Asn Val Asp Ile
785                 790                 795                 800

Ser Arg Pro Leu Asn Gly Leu Asn Val Pro Gln Pro Gln Ser Thr Gln
                805                 810                 815

Arg Val Leu Pro Pro Leu His Arg Ala Pro Arg Ala Pro Ser Val Pro
            820                 825                 830

Ala Arg Pro Leu Pro Ala Lys Pro Ala Leu Arg Gln Ala Gln Gly Thr
        835                 840                 845

Cys Lys Pro Asn Pro Pro Gln Lys Pro Leu Pro Ala Asp Pro Leu Ala
    850                 855                 860

Arg Thr Thr Arg Leu Thr His Ala Leu Ala Arg Thr Pro Gly Gln Trp
865                 870                 875                 880

Glu Thr Gly Leu Arg Leu Ala Pro Leu Arg Pro Ala Pro Gln Tyr Pro
                885                 890                 895

His Gln Val Pro Arg Ser Thr His Thr Ala Tyr Ile Lys
            900                 905

<210>  5
<211>  24
<212>  DNA
<213>  human

<400>  5
gaggcaggaa atccctccgg tcgc                                              24

<210>  6
<211>  21
<212>  DNA
<213>  human

<400>  6
cactgaaggc cggcgacgat g                                                21


<210>  7
<211>  24
<212>  DNA
<213>  human

<400>  7
gctgagactg actgctgaat caga                                             24


<210>  8
<211>  24
<212>  DNA
<213>  human

<400>  8
tctgattcag cagtcagtct cagc                                             24


<210>  9
<211>  25
<212>  DNA
<213>  human

<400>  9
ctgacttaca gagcatttgt ccatg                                            25


<210>  10
<211>  25
<212>  DNA
<213>  human

<400>  10
catggacaaa tgctctgtaa gtcag                                            25


<210>  11
<211>  24
<212>  DNA
<213>  human

<400>  11
cttcgagact ttgccacagt tgcc                                             24


<210>  12
<211>  24
<212>  DNA
<213>  human

<400>  12
ggcaactgtg gcaaagtctc gaag                                             24


<210>  13
<211>  23
<212>  DNA
<213>  human

<400>  13

tcagatgagt gtcagtgagg cag                                                23

<210>  14
<211>  25
<212>  DNA
<213>  human

<400>  14
cactggtcac ggtctccatg tcatg                                              25

<210>  15
<211>  25
<212>  DNA
<213>  human

<400>  15
tgtcggcttc tcacttaata taggc                                              25

<210>  16
<211>  30
<212>  DNA
<213>  human

<400>  16
tccataagct cacccctcgg gcctcgcagg                                         30

<210>  17
<211>  30
<212>  DNA
<213>  human

<400>  17
cctgcgaggc ccgaggggtg agcttatgga                                         30

<210>  18
<211>  24
<212>  DNA
<213>  human

<400>  18
cttggagtcg aagctcttca ctgg                                               24

<210>  19
<211>  21
<212>  DNA
<213>  human

<400>  19
ggatcccagt gaagagcttc g                                                  21

<210>  20
<211>  24
<212>  DNA
<213>  human

<400>  20
ctcgaggatg agtgtcagtg aggc                                               24

<210>  21
<211>  33
<212>  DNA

<213>  human

<400>  21
tcgaggacta caaggacgac gacgacaagt aat                                    33


<210>  22
<211>  33
<212>  DNA
<213>  human

<400>  22
ctagattact tgtcgtcgtc gtccttgtag tcc                                    33


<210>  23
<211>  27
<212>  DNA
<213>  human

<400>  23
tcgagcatca tcatcatcat cattaat                                           27


<210>  24
<211>  27
<212>  DNA
<213>  human

<400>  24
ctagattaat gatgatgatg atgatgc                                           27


<210>  25
<211>  30
<212>  DNA
<213>  human

<400>  25
ctggttccgc gtggatccct caaggcaact                                        30


<210>  26
<211>  30
<212>  DNA
<213>  human

<400>  26
ggatccacgc ggaaccagtc ttgcccatgt                                        30


<210>  27
<211>  24
<212>  DNA
<213>  human

<400>  27
cacgggcagc gggcgcgctg ccat                                              24


<210>  28
<211>  24
<212>  DNA
<213>  human

<400>  28
atggcagcgc gcccgctgcc cgtg                                              24

<210>  29
<211>  19
<212>  RNA
<213>  human

<400>  29
augcgagaug agagaugcu                                                    19


<210>  30
<211>  19
<212>  RNA
<213>  human

<400>  30
guguggaaug acauggaca                                                    19


<210>  31
<211>  19
<212>  RNA
<213>  human

<400>  31
gaaucaucca gaagugcug                                                    19


<210>  32
<211>  19
<212>  RNA
<213>  human

<400>  32
agcaucucuc aucucgcau                                                    19


<210>  33
<211>  19
<212>  RNA
<213>  human

<400>  33
uguccauguc auuccacac                                                    19


<210>  34
<211>  19
<212>  RNA
<213>  human

<400>  34
cagcacuucu ggaugauuc                                                    19


<210>  35
<211>  18
<212>  DNA
<213>  human

<400>  35
caccgaagga caatccca                                                     18


<210>  36
<211>  18
<212>  DNA
<213>  human

<400>  36

ggaagccgcc agattcct                                                    18

<210>  37
<211>  21
<212>  DNA
<213>  human

<400>  37
aggaagcact cgctgagttg a                                               21

<210>  38
<211>  21
<212>  DNA
<213>  human

<400>  38
ctccaggctg gtctccaagt c                                               21

<210>  39
<211>  378
<212>  DNA
<213>  human

<400>  39
gaggtgaagc ttctcgagtc tggaggtggc ctggtgcagc ctggaggatc cctgaaactc      60

tcctgtgcag cctcaggatt cgattttagt agatactgga tgagttgggt ccggcaggct     120

ccagggaaag ggctagaatg gattggagaa attaatccag atagcagtac gataaactat     180

acgccatctc taaaggataa attcatcatc tccagagaca acgccaaaaa tacgctgtac     240

ctgcacatga gcaaagtgag atctgaggac acagcccttt attactgtgc aagaccggat     300

agttactata ggtactatgc tatggactac tggggtcaag gaacctcagt caccgtctcc     360

tcagccaaaa caacagcc                                                   378

<210>  40
<211>  375
<212>  DNA
<213>  human

<400>  40
cagatccagt tggtacagtc tggacctgag ctgaagaagc ctggagagac agtcaagatc      60

tcctgcaagg cttctgggta taccttcaca acctatggaa tgagctgggt gaaacaggct     120

ccaggaaagg gtttaaagtg gatgggctgg ataaacacct actctggagt gccaacatat     180

gctgatgact tcaagggacg gtttgccttc tctttggaaa cctctgccag cactgcctat     240

ttgcagatca acaacctctc aaatgaggac acggctacat atttctgtgc aagctcctat     300

tactacggta gtacctgggt ggactactgg ggccaaggca ccactctcac agtctcctca     360

gccaaaacaa caccc                                                      375

<210>  41
<211>  378
<212>  DNA
<213>  human

<400>  41

caggttcagc tgcaacagtc tgacgctgag ttggtgaaac ctggagcttc agtgaagata 60

tcctgcaagg tttctggcta caccttcact gaccatacta ttcactggat gaaacagagg 120

cctgaacagg gcctggaatg gattggatat attcatccta gagatggtag tactaagtac 180

aatgagaagt tcaagggcaa ggccacattg actgcagaca aatcctccag cacagcctac 240

atgcagctca acagcctgac atctgaggac tctgcagtct atttctgtgc aagagggggt 300

ttctacggca gtagtccctg gtttgcttac tggggccaag ggactctggt cactgtctct 360

gcagccaaaa caacaccc 378

<210> 42
<211> 324
<212> DNA
<213> human

<400> 42
gatatccaga tgacacagac tacatcctcc ctgtctgcct ctctgggaga cagagtcacc 60

atcagttgca gggcaagtca ggacattagc aattatttaa actggtatca gcagaaacca 120

gatggaactg ttaaactcct gatctactac acatcaagat tacactcagg agtcccatca 180

aggttcagtg gcagtgggtc tggaacagat tattctctca ccattagtaa ccaggagcaa 240

gaagatattg ccacttactt ttgccaacag ggtaatacgc ttccgtacac gttcggaggg 300

gggaccaagc tggaaataaa acgg 324

<210> 43
<211> 339
<212> DNA
<213> human

<400> 43
gatgttttga tgacccaaac tccactctcc ctgcctgtca gtcttggaga tcaagcctcc 60

atctcttgca gatctagtca gagcattgta catagtaatg gaaacaccta tttagaatgg 120

tacctgcaga aaccaggcca gtctccaaag ctcctgatct acaaagtttc caaccgattt 180

tctggggtcc cagacaggtt cagtggcagt ggatcaggga cagatttcac actcaagatc 240

agcagagtgg aggctgagga tctgggagtt tattactgct ttcaaggttc acatgttccg 300

tggacgttcg gtggaggcac caagctggaa atcaaacgg 339

<210> 44
<211> 333
<212> DNA
<213> human

<220>
<221> misc_feature
<222> (1)..(54)
<223> n stands for any base

<400> 44
nnnnnnnnnn nnnnnnnnnn nnnnnnnnnn nnnnnnnnnn nnnnnnnnnn nnnngtatcc 60

atctcctgca ggtctagtaa gagtctcctg catagtaatg gcaacactta cttgtattgg 120

ttcctgcaga ggccaggcca gtctcctcag ctcctgatat atcggatgtc caaccttgcc 180

tcaggagtcc cagacaggtt cagtggcagt gggtcaggaa ctgctttcac actgagaatc 240

agtagagtgg aggctgagga tgtgggtgtt tattactgta tgcaacatct agaatatccg 300

tacacgttcg gaggggggac caagctggaa ata 333

<210> 45
<211> 126
<212> PRT
<213> human

<400> 45

Glu Val Lys Leu Leu Glu Ser Gly Gly Gly Leu Val Gln Pro Gly Gly
1               5                   10                  15

Ser Leu Lys Leu Ser Cys Ala Ala Ser Gly Phe Asp Phe Ser Arg Tyr
            20                  25                  30

Trp Met Ser Trp Val Arg Gln Ala Pro Gly Lys Gly Leu Glu Trp Ile
        35                  40                  45

Gly Glu Ile Asn Pro Asp Ser Ser Thr Ile Asn Tyr Thr Pro Ser Leu
    50                  55                  60

Lys Asp Lys Phe Ile Ile Ser Arg Asp Asn Ala Lys Asn Thr Leu Tyr
65                  70                  75                  80

Leu His Met Ser Lys Val Arg Ser Glu Asp Thr Ala Leu Tyr Tyr Cys
                85                  90                  95

Ala Arg Pro Asp Ser Tyr Tyr Arg Tyr Tyr Ala Met Asp Tyr Trp Gly
            100                 105                 110

Gln Gly Thr Ser Val Thr Val Ser Ser Ala Lys Thr Thr Ala
        115                 120                 125

<210> 46
<211> 125
<212> PRT
<213> human

<400> 46

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Thr Tyr
            20                  25                  30

Gly Met Ser Trp Val Lys Gln Ala Pro Gly Lys Gly Leu Lys Trp Met
        35                  40                  45

Gly Trp Ile Asn Thr Tyr Ser Gly Val Pro Thr Tyr Ala Asp Asp Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Ser Asn Glu Asp Thr Ala Thr Tyr Phe Cys
                85                  90                  95

Ala Ser Ser Tyr Tyr Tyr Gly Ser Thr Trp Val Asp Tyr Trp Gly Gln
            100                 105                 110

Gly Thr Thr Leu Thr Val Ser Ser Ala Lys Thr Thr Pro
        115                 120                 125

<210>  47
<211>  126
<212>  PRT
<213>  human

<400>  47

Gln Val Gln Leu Gln Gln Ser Asp Ala Glu Leu Val Lys Pro Gly Ala
1               5                   10                  15

Ser Val Lys Ile Ser Cys Lys Val Ser Gly Tyr Thr Phe Thr Asp His
            20                  25                  30

Thr Ile His Trp Met Lys Gln Arg Pro Glu Gln Gly Leu Glu Trp Ile
        35                  40                  45

Gly Tyr Ile His Pro Arg Asp Gly Ser Thr Lys Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Gly Gly Phe Tyr Gly Ser Ser Pro Trp Phe Ala Tyr Trp Gly
            100                 105                 110

Gln Gly Thr Leu Val Thr Val Ser Ala Ala Lys Thr Thr Pro
        115                 120                 125

<210>  48
<211>  108
<212>  PRT
<213>  human

<400>  48

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr

20                          25                          30

Leu Asn Trp Tyr Gln Gln Lys Pro Asp Gly Thr Val Lys Leu Leu Ile
            35                          40                          45

Tyr Tyr Thr Ser Arg Leu His Ser Gly Val Pro Ser Arg Phe Ser Gly
    50                          55                          60

Ser Gly Ser Gly Thr Asp Tyr Ser Leu Thr Ile Ser Asn Gln Glu Gln
65                          70                          75                          80

Glu Asp Ile Ala Thr Tyr Phe Cys Gln Gln Gly Asn Thr Leu Pro Tyr
                        85                          90                          95

Thr Phe Gly Gly Gly Thr Lys Leu Glu Ile Lys Arg
                100                         105

<210>  49
<211>  113
<212>  PRT
<213>  human

<400>  49

Asp Val Leu Met Thr Gln Thr Pro Leu Ser Leu Pro Val Ser Leu Gly
1                       5                           10                          15

Asp Gln Ala Ser Ile Ser Cys Arg Ser Ser Gln Ser Ile Val His Ser
                20                          25                          30

Asn Gly Asn Thr Tyr Leu Glu Trp Tyr Leu Gln Lys Pro Gly Gln Ser
            35                          40                          45

Pro Lys Leu Leu Ile Tyr Lys Val Ser Asn Arg Phe Ser Gly Val Pro
    50                          55                          60

Asp Arg Phe Ser Gly Ser Gly Ser Gly Thr Asp Phe Thr Leu Lys Ile
65                          70                          75                          80

Ser Arg Val Glu Ala Glu Asp Leu Gly Val Tyr Tyr Cys Phe Gln Gly
                        85                          90                          95

Ser His Val Pro Trp Thr Phe Gly Gly Gly Thr Lys Leu Glu Ile Lys
                100                         105                         110

Arg

<210>  50
<211>  111
<212>  PRT
<213>  human

<220>
<221>  MISC_FEATURE

<222>  (1)..(18)
<223>  X stands for any amino acids

<400>  50

Xaa Xaa Xaa Xaa Xaa Xaa Xaa Xaa Xaa Xaa Xaa Xaa Xaa Xaa Xaa Xaa
1               5                   10                  15

Xaa Xaa Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Leu Glu Ile
            100                 105                 110

<210>  51
<211>  5
<212>  PRT
<213>  human

<400>  51

Arg Tyr Trp Met Ser
1               5

<210>  52
<211>  15
<212>  DNA
<213>  human

<400>  52
agatactgga tgagt                                                        15

<210>  53
<211>  17
<212>  PRT
<213>  human

<400>  53

Glu Ile Asn Pro Asp Ser Ser Thr Ile Asn Tyr Thr Pro Ser Leu Lys
1               5                   10                  15

Asp

<210>  54
<211>  51
<212>  DNA
<213>  human

<400>  54
gaaattaatc cagatagcag tacgataaac tatacgccat ctctaaagga t                51


<210>  55
<211>  12
<212>  PRT
<213>  human

<400>  55

Pro Asp Ser Tyr Tyr Arg Tyr Tyr Ala Met Asp Tyr
1               5                   10


<210>  56
<211>  36
<212>  DNA
<213>  human

<400>  56
ccggatagtt actataggta ctatgctatg gactac                                 36


<210>  57
<211>  11
<212>  PRT
<213>  human

<400>  57

Arg Ala Ser Gln Asp Ile Ser Asn Tyr Leu Asn
1               5                   10


<210>  58
<211>  33
<212>  DNA
<213>  human

<400>  58
agggcaagtc aggacattag caattattta aac                                    33


<210>  59
<211>  7
<212>  PRT
<213>  human

<400>  59

Tyr Thr Ser Arg Leu His Ser
1               5


<210>  60
<211>  21
<212>  DNA
<213>  human

<400>  60
tacacatcaa gattacactc a                                                 21

<210>  61
<211>  9
<212>  PRT
<213>  human

<400>  61

Gln Gln Gly Asn Thr Leu Pro Tyr Thr
1               5


<210>  62
<211>  27
<212>  DNA
<213>  human

<400>  62
caacagggta atacgcttcc gtacacg                                          27


<210>  63
<211>  5
<212>  PRT
<213>  human

<400>  63

Thr Tyr Gly Met Ser
1               5


<210>  64
<211>  15
<212>  DNA
<213>  human

<400>  64
acctatggaa tgagc                                                       15


<210>  65
<211>  17
<212>  PRT
<213>  human

<400>  65

Trp Ile Asn Thr Tyr Ser Gly Val Pro Thr Tyr Ala Asp Asp Phe Lys
1               5                   10                  15


Gly



<210>  66
<211>  51
<212>  DNA
<213>  human

<400>  66
tggataaaca cctactctgg agtgccaaca tatgctgatg acttcaaggg a               51


<210>  67
<211>  11
<212>  PRT
<213>  human

<400>  67

Ser Tyr Tyr Tyr Gly Ser Thr Trp Val Asp Tyr
1               5                   10


<210>  68
<211>  33
<212>  DNA
<213>  human

<400>  68
tcctattact acggtagtac ctgggtggac tac                                    33


<210>  69
<211>  16
<212>  PRT
<213>  human

<400>  69

Arg Ser Ser Gln Ser Ile Val His Ser Asn Gly Asn Thr Tyr Leu Glu
1               5                   10                  15


<210>  70
<211>  48
<212>  DNA
<213>  human

<400>  70
agatctagtc agagcattgt acatagtaat ggaaacacct atttagaa                    48


<210>  71
<211>  7
<212>  PRT
<213>  human

<400>  71

Lys Val Ser Asn Arg Phe Ser
1               5


<210>  72
<211>  21
<212>  DNA
<213>  human

<400>  72
aaagtttcca accgattttc t                                                 21


<210>  73
<211>  9
<212>  PRT
<213>  human

<400>  73

Phe Gln Gly Ser His Val Pro Trp Thr
1               5


<210>  74
<211>  27
<212>  DNA

<213>  human

<400>  74
tttcaaggtt cacatgttcc gtggacg                                          27


<210>  75
<211>  5
<212>  PRT
<213>  human

<400>  75

Asp His Thr Ile His
1               5


<210>  76
<211>  15
<212>  DNA
<213>  human

<400>  76
gaccatacta ttcac                                                       15


<210>  77
<211>  17
<212>  PRT
<213>  human

<400>  77

Tyr Ile His Pro Arg Asp Gly Ser Thr Lys Tyr Asn Glu Lys Phe Lys
1               5                   10                  15


Gly



<210>  78
<211>  51
<212>  DNA
<213>  human

<400>  78
tatattcatc ctagagatgg tagtactaag tacaatgaga agttcaaggg c               51


<210>  79
<211>  12
<212>  PRT
<213>  human

<400>  79

Gly Gly Phe Tyr Gly Ser Ser Pro Trp Phe Ala Tyr
1               5                   10


<210>  80
<211>  36
<212>  DNA
<213>  human

<400>  80
gggggtttct acggcagtag tccctggttt gcttac                                36

<210>  81
<211>  16
<212>  PRT
<213>  human

<400>  81

Arg Ser Ser Lys Ser Leu Leu His Ser Asn Gly Asn Thr Tyr Leu Tyr
1               5                   10                  15


<210>  82
<211>  48
<212>  DNA
<213>  human

<400>  82
aggtctagta agagtctcct gcatagtaat ggcaacactt acttgtat                    48


<210>  83
<211>  7
<212>  PRT
<213>  human

<400>  83

Arg Met Ser Asn Leu Ala Ser
1               5


<210>  84
<211>  21
<212>  DNA
<213>  human

<400>  84
cggatgtcca accttgcctc a                                                 21


<210>  85
<211>  9
<212>  PRT
<213>  human

<400>  85

Met Gln His Leu Glu Tyr Pro Tyr Thr
1               5


<210>  86
<211>  27
<212>  DNA
<213>  human

<400>  86
atgcaacatc tagaatatcc gtacacg                                           27


<210>  87
<211>  20
<212>  DNA
<213>  Artificial Sequence

<220>
<223>  Phosphorothioate Antisense Oligonucleotide

<400>  87
gactaggaag agcgttagtg                                                    20


<210>  88
<211>  20
<212>  DNA
<213>  Artificial Sequence

<220>
<223>  Phosphorothioate Antisense Oligonucleotide

<400>  88
actgtccgag ttggcccggg                                                    20


<210>  89
<211>  20
<212>  DNA
<213>  Artificial Sequence

<220>
<223>  Phosphorothioate Antisense Oligonucleotide

<400>  89
ccgttgcaat aaatgagcaa                                                    20


<210>  90
<211>  20
<212>  DNA
<213>  Artificial Sequence

<220>
<223>  Phosphorothioate Antisense Oligonucleotide

<400>  90
ctggcacaag ccagccttga                                                    20


<210>  91
<211>  20
<212>  DNA
<213>  Artificial Sequence

<220>
<223>  Phosphorothioate Antisense Oligonucleotide

<400>  91
tgcgtcgcgc gcgcgccgtt                                                    20


<210>  92
<211>  20
<212>  DNA
<213>  Artificial Sequence

<220>
<223>  Phosphorothioate Antisense Oligonucleotide

<400>  92
   tttccccccg tgtgtgtgcg                                                     20


<210>  93
<211>  20
<212>  DNA
<213>  Artificial Sequence

<220>
<223>  Phosphorothioate Antisense Oligonucleotide

<400>  93
gcctttcatt tttaaaaaag                                                    20


<210>  94
<211>  20
<212>  DNA
<213>  Artificial Sequence

<220>
<223>  Phosphorothioate Antisense Oligonucleotide

<400>  94
gccgccgctg agctcttcta                                                    20


<210>  95
<211>  20
<212>  DNA
<213>  Artificial Sequence

<220>
<223>  Phosphorothioate Antisense Oligonucleotide

<400>  95
agccctcgcg cacggcccgc                                                    20


<210>  96
<211>  20
<212>  DNA
<213>  Artificial Sequence

<220>
<223>  Phosphorothioate Antisense Oligonucleotide

<400>  96
cctcggcgag tcagctccgg                                                    20


<210>  97
<211>  20
<212>  DNA
<213>  Artificial Sequence

<220>
<223>  Phosphorothioate Antisense Oligonucleotide

<400>  97
gcgaccggag ggatttcctg                                                    20


<210>  98
<211>  20
<212>  DNA
<213>  Artificial Sequence

<220>
<223>  Phosphorothioate Antisense Oligonucleotide

<400>  98
gccgagcggg gccgggcgtc                                                    20


<210>  99
<211>  20

<212>  DNA
<213>  Artificial Sequence

<220>
<223>  Phosphorothioate Antisense Oligonucleotide

<400>  99
ctgcaccatc ccacgcgggc                                                    20


<210>  100
<211>  20
<212>  DNA
<213>  Artificial Sequence

<220>
<223>  Phosphorothioate Antisense Oligonucleotide

<400>  100
ctcgggcccg gcggcgagcg                                                    20


<210>  101
<211>  20
<212>  DNA
<213>  Artificial Sequence

<220>
<223>  Phosphorothioate Antisense Oligonucleotide

<400>  101
cggccttcag tgcagcagct                                                    20


<210>  102
<211>  20
<212>  DNA
<213>  Artificial Sequence

<220>
<223>  Phosphorothioate Antisense Oligonucleotide

<400>  102
gggcgcgctg ccatcgtcgc                                                    20


<210>  103
<211>  20
<212>  DNA
<213>  Artificial Sequence

<220>
<223>  Phosphorothioate Antisense Oligonucleotide

<400>  103
gggcggggga cacgggcagc                                                    20


<210>  104
<211>  20
<212>  DNA
<213>  Artificial Sequence

<220>
<223>  Phosphorothioate Antisense Oligonucleotide

<400>  104
cagggcgagc aggagggcgc                                                    20

<210>  105
<211>  20
<212>  DNA
<213>  Artificial Sequence

<220>
<223>  Phosphorothioate Antisense Oligonucleotide

<400>  105
ggcgcgagca gagcaccggc                                                    20


<210>  106
<211>  20
<212>  DNA
<213>  Artificial Sequence

<220>
<223>  Phosphorothioate Antisense Oligonucleotide

<400>  106
tcacccctcg ggcctcgcag                                                    20


<210>  107
<211>  20
<212>  DNA
<213>  Artificial Sequence

<220>
<223>  Phosphorothioate Antisense Oligonucleotide

<400>  107
tcttccttgg ttccataagc                                                    20


<210>  108
<211>  20
<212>  DNA
<213>  Artificial Sequence

<220>
<223>  Phosphorothioate Antisense Oligonucleotide

<400>  108
gcactgacaa cttcatcagc                                                    20


<210>  109
<211>  20
<212>  DNA
<213>  Artificial Sequence

<220>
<223>  Phosphorothioate Antisense Oligonucleotide

<400>  109
ggtccccact ccgaacagag                                                    20


<210>  110
<211>  20
<212>  DNA
<213>  Artificial Sequence

<220>
<223>  Phosphorothioate Antisense Oligonucleotide

<400>  110
gctcttcact gggatccaga                                                    20


<210>  111
<211>  20
<212>  DNA
<213>  Artificial Sequence

<220>
<223>  Phosphorothioate Antisense Oligonucleotide

<400>  111
ggatgattct tggagtcgaa                                                    20


<210>  112
<211>  20
<212>  DNA
<213>  Artificial Sequence

<220>
<223>  Phosphorothioate Antisense Oligonucleotide

<400>  112
gtcgaatatt cagcacttct                                                    20


<210>  113
<211>  20
<212>  DNA
<213>  Artificial Sequence

<220>
<223>  Phosphorothioate Antisense Oligonucleotide

<400>  113
ttctttgctt tcccgttgta                                                    20


<210>  114
<211>  20
<212>  DNA
<213>  Artificial Sequence

<220>
<223>  Phosphorothioate Antisense Oligonucleotide

<400>  114
ctttccagat ttatgatcag                                                    20


<210>  115
<211>  20
<212>  DNA
<213>  Artificial Sequence

<220>
<223>  Phosphorothioate Antisense Oligonucleotide

<400>  115
tggcaatgag accttcattt                                                    20


<210>  116
<211>  20
<212>  DNA
<213>  Artificial Sequence

<220>
<223>  Phosphorothioate Antisense Oligonucleotide

<400>  116
gtgggtttcc gtgaaactgc                                                    20


<210>  117
<211>  20
<212>  DNA
<213>  Artificial Sequence

<220>
<223>  Phosphorothioate Antisense Oligonucleotide

<400>  117
tcagtaccgt cttgcagata                                                    20


<210>  118
<211>  20
<212>  DNA
<213>  Artificial Sequence

<220>
<223>  Phosphorothioate Antisense Oligonucleotide

<400>  118
aatttcgagc gagggagaca                                                    20


<210>  119
<211>  20
<212>  DNA
<213>  Artificial Sequence

<220>
<223>  Phosphorothioate Antisense Oligonucleotide

<400>  119
gtgacccaga attaccgtgt                                                    20


<210>  120
<211>  20
<212>  DNA
<213>  Artificial Sequence

<220>
<223>  Phosphorothioate Antisense Oligonucleotide

<400>  120
acatgtccat ggtagtaaca                                                    20


<210>  121
<211>  20
<212>  DNA
<213>  Artificial Sequence

<220>
<223>  Phosphorothioate Antisense Oligonucleotide

<400>  121
ctgaatcaga atatccccgt                                                    20


<210>  122
<211>  20

<212>  DNA
<213>  Artificial Sequence

<220>
<223>  Phosphorothioate Antisense Oligonucleotide

<400>  122
acacgtgctg agactgactg                                                    20


<210>  123
<211>  20
<212>  DNA
<213>  Artificial Sequence

<220>
<223>  Phosphorothioate Antisense Oligonucleotide

<400>  123
ataagtcccc tgagaccaga                                                    20


<210>  124
<211>  20
<212>  DNA
<213>  Artificial Sequence

<220>
<223>  Phosphorothioate Antisense Oligonucleotide

<400>  124
agctttcatt ttcaaacaca                                                    20


<210>  125
<211>  20
<212>  DNA
<213>  Artificial Sequence

<220>
<223>  Phosphorothioate Antisense Oligonucleotide

<400>  125
tttcattggt tctaagacat                                                    20


<210>  126
<211>  20
<212>  DNA
<213>  Artificial Sequence

<220>
<223>  Phosphorothioate Antisense Oligonucleotide

<400>  126
ttgtatctgt tggttgcact                                                    20


<210>  127
<211>  20
<212>  DNA
<213>  Artificial Sequence

<220>
<223>  Phosphorothioate Antisense Oligonucleotide

<400>  127
gcttcttcgc tgggaagagt                                                    20

<210>  128
<211>  20
<212>  DNA
<213>  Artificial Sequence

<220>
<223>  Phosphorothioate Antisense Oligonucleotide

<400>  128
tgatccccgg acgcttttca                                                    20


<210>  129
<211>  20
<212>  DNA
<213>  Artificial Sequence

<220>
<223>  Phosphorothioate Antisense Oligonucleotide

<400>  129
gtgttgtgat gtgatccaca                                                    20


<210>  130
<211>  20
<212>  DNA
<213>  Artificial Sequence

<220>
<223>  Phosphorothioate Antisense Oligonucleotide

<400>  130
tctttgcagc gaggtttggt                                                    20


<210>  131
<211>  20
<212>  DNA
<213>  Artificial Sequence

<220>
<223>  Phosphorothioate Antisense Oligonucleotide

<400>  131
agagggtggt ggaaacacat                                                    20


<210>  132
<211>  20
<212>  DNA
<213>  Artificial Sequence

<220>
<223>  Phosphorothioate Antisense Oligonucleotide

<400>  132
tgccttcttg cccatgtctg                                                    20


<210>  133
<211>  20
<212>  DNA
<213>  Artificial Sequence

<220>
<223>  Phosphorothioate Antisense Oligonucleotide

<400>  133
ccttgagggt ctctctttta                                                    20


<210>  134
<211>  20
<212>  DNA
<213>  Artificial Sequence

<220>
<223>  Phosphorothioate Antisense Oligonucleotide

<400>  134
cagctccaca tacttagttg                                                    20


<210>  135
<211>  20
<212>  DNA
<213>  Artificial Sequence

<220>
<223>  Phosphorothioate Antisense Oligonucleotide

<400>  135
cggttgtctg ccacgatcac                                                    20


<210>  136
<211>  20
<212>  DNA
<213>  Artificial Sequence

<220>
<223>  Phosphorothioate Antisense Oligonucleotide

<400>  136
ttccttgcct ctgaaactct                                                    20


<210>  137
<211>  20
<212>  DNA
<213>  Artificial Sequence

<220>
<223>  Phosphorothioate Antisense Oligonucleotide

<400>  137
cttaactttt tccagatctt                                                    20


<210>  138
<211>  20
<212>  DNA
<213>  Artificial Sequence

<220>
<223>  Phosphorothioate Antisense Oligonucleotide

<400>  138
gcaatctcta ttaatcgctg                                                    20


<210>  139
<211>  20
<212>  DNA
<213>  Artificial Sequence

<220>
<223>  Phosphorothioate Antisense Oligonucleotide

<400>  139
aaaacttgtc aacgtgatta                                                    20


<210>  140
<211>  20
<212>  DNA
<213>  Artificial Sequence

<220>
<223>  Phosphorothioate Antisense Oligonucleotide

<400>  140
ccgaatgttc agtggtctgt                                                    20


<210>  141
<211>  20
<212>  DNA
<213>  Artificial Sequence

<220>
<223>  Phosphorothioate Antisense Oligonucleotide

<400>  141
tccacgccta ccaacacgat                                                    20


<210>  142
<211>  20
<212>  DNA
<213>  Artificial Sequence

<220>
<223>  Phosphorothioate Antisense Oligonucleotide

<400>  142
tgtccatgtc attccacact                                                    20


<210>  143
<211>  20
<212>  DNA
<213>  Artificial Sequence

<220>
<223>  Phosphorothioate Antisense Oligonucleotide

<400>  143
gtcctgactt acagagcatt                                                    20


<210>  144
<211>  20
<212>  DNA
<213>  Artificial Sequence

<220>
<223>  Phosphorothioate Antisense Oligonucleotide

<400>  144
tcatggaggc tggtgaatgg                                                    20


<210>  145
<211>  20

<212>  DNA
<213>  Artificial Sequence

<220>
<223>  Phosphorothioate Antisense Oligonucleotide

<400>  145
tcttcctcca gtccagaaat                                                    20


<210>  146
<211>  20
<212>  DNA
<213>  Artificial Sequence

<220>
<223>  Phosphorothioate Antisense Oligonucleotide

<400>  146
tttgcgaggt agaagcttca                                                    20


<210>  147
<211>  20
<212>  DNA
<213>  Artificial Sequence

<220>
<223>  Phosphorothioate Antisense Oligonucleotide

<400>  147
agctgcgcat tgtcatggga                                                    20


<210>  148
<211>  20
<212>  DNA
<213>  Artificial Sequence

<220>
<223>  Phosphorothioate Antisense Oligonucleotide

<400>  148
ggaaataaac cccactgaca                                                    20


<210>  149
<211>  20
<212>  DNA
<213>  Artificial Sequence

<220>
<223>  Phosphorothioate Antisense Oligonucleotide

<400>  149
catgccgatg gtggtccctt                                                    20


<210>  150
<211>  20
<212>  DNA
<213>  Artificial Sequence

<220>
<223>  Phosphorothioate Antisense Oligonucleotide

<400>  150
cacatgctca tgattggggc                                                    20

<210>  151
<211>  20
<212>  DNA
<213>  Artificial Sequence

<220>
<223>  Phosphorothioate Antisense Oligonucleotide

<400>  151
ccccagactg gtctgccgtg                                                    20


<210>  152
<211>  20
<212>  DNA
<213>  Artificial Sequence

<220>
<223>  Phosphorothioate Antisense Oligonucleotide

<400>  152
tgaatggtcc atgacaattc                                                    20


<210>  153
<211>  20
<212>  DNA
<213>  Artificial Sequence

<220>
<223>  Phosphorothioate Antisense Oligonucleotide

<400>  153
gctgcaccaa ggggattgtc                                                    20


<210>  154
<211>  20
<212>  DNA
<213>  Artificial Sequence

<220>
<223>  Phosphorothioate Antisense Oligonucleotide

<400>  154
gctcatgtgc cagggtcacg                                                    20


<210>  155
<211>  20
<212>  DNA
<213>  Artificial Sequence

<220>
<223>  Phosphorothioate Antisense Oligonucleotide

<400>  155
catcccgaaa ttgtggccca                                                    20


<210>  156
<211>  20
<212>  DNA
<213>  Artificial Sequence

<220>
<223>  Phosphorothioate Antisense Oligonucleotide

<400>  156
ctgtccagtg tgtcatgatt                                                   20


<210>  157
<211>  20
<212>  DNA
<213>  Artificial Sequence

<220>
<223>  Phosphorothioate Antisense Oligonucleotide

<400>  157
ccatttgaca gctacagccc                                                   20


<210>  158
<211>  20
<212>  DNA
<213>  Artificial Sequence

<220>
<223>  Phosphorothioate Antisense Oligonucleotide

<400>  158
gcagcctcct ttctcaaccg                                                   20


<210>  159
<211>  20
<212>  DNA
<213>  Artificial Sequence

<220>
<223>  Phosphorothioate Antisense Oligonucleotide

<400>  159
ccggtggaag cgttcatgat                                                   20


<210>  160
<211>  20
<212>  DNA
<213>  Artificial Sequence

<220>
<223>  Phosphorothioate Antisense Oligonucleotide

<400>  160
acaccatggg aaatgggtac                                                   20


<210>  161
<211>  20
<212>  DNA
<213>  Artificial Sequence

<220>
<223>  Phosphorothioate Antisense Oligonucleotide

<400>  161
cttcctgctg caactgctga                                                   20


<210>  162
<211>  20
<212>  DNA
<213>  Artificial Sequence

<220>
<223>  Phosphorothioate Antisense Oligonucleotide

<400>  162
tccaggctgg tctccaagtc                                                   20


<210>  163
<211>  20
<212>  DNA
<213>  Artificial Sequence

<220>
<223>  Phosphorothioate Antisense Oligonucleotide

<400>  163
ggcacacccc cattcctttc                                                   20


<210>  164
<211>  20
<212>  DNA
<213>  Artificial Sequence

<220>
<223>  Phosphorothioate Antisense Oligonucleotide

<400>  164
gacttccggc aggttaaaca                                                   20


<210>  165
<211>  20
<212>  DNA
<213>  Artificial Sequence

<220>
<223>  Phosphorothioate Antisense Oligonucleotide

<400>  165
tggcccccga aagactccct                                                   20


<210>  166
<211>  20
<212>  DNA
<213>  Artificial Sequence

<220>
<223>  Phosphorothioate Antisense Oligonucleotide

<400>  166
caaatctgtt cccacacttc                                                   20


<210>  167
<211>  20
<212>  DNA
<213>  Artificial Sequence

<220>
<223>  Phosphorothioate Antisense Oligonucleotide

<400>  167
acactcctct ccttcttcca                                                   20


<210>  168
<211>  20

<212>  DNA
<213>  Artificial Sequence

<220>
<223>  Phosphorothioate Antisense Oligonucleotide

<400>  168
tcctctggct ccccacagtc                                                    20


<210>  169
<211>  20
<212>  DNA
<213>  Artificial Sequence

<220>
<223>  Phosphorothioate Antisense Oligonucleotide

<400>  169
tgcagcagcg attcatacat                                                    20


<210>  170
<211>  20
<212>  DNA
<213>  Artificial Sequence

<220>
<223>  Phosphorothioate Antisense Oligonucleotide

<400>  170
cagggtacag gtggtggcat                                                    20


<210>  171
<211>  20
<212>  DNA
<213>  Artificial Sequence

<220>
<223>  Phosphorothioate Antisense Oligonucleotide

<400>  171
gcgcacacag cgtccggctt                                                    20


<210>  172
<211>  20
<212>  DNA
<213>  Artificial Sequence

<220>
<223>  Phosphorothioate Antisense Oligonucleotide

<400>  172
cttcacagca cagcccatgt                                                    20


<210>  173
<211>  20
<212>  DNA
<213>  Artificial Sequence

<220>
<223>  Phosphorothioate Antisense Oligonucleotide

<400>  173
tgcaggcttc agctggcagt                                                    20

<210>  174
<211>  20
<212>  DNA
<213>  Artificial Sequence

<220>
<223>  Phosphorothioate Antisense Oligonucleotide

<400>  174
gagtccctgc acgctgttcc                                                    20


<210>  175
<211>  20
<212>  DNA
<213>  Artificial Sequence

<220>
<223>  Phosphorothioate Antisense Oligonucleotide

<400>  175
ggaggtcaca ggagttgctg                                                    20


<210>  176
<211>  20
<212>  DNA
<213>  Artificial Sequence

<220>
<223>  Phosphorothioate Antisense Oligonucleotide

<400>  176
ggcccctgtg cagaactctg                                                    20


<210>  177
<211>  20
<212>  DNA
<213>  Artificial Sequence

<220>
<223>  Phosphorothioate Antisense Oligonucleotide

<400>  177
ttggctgggc agtgagggct                                                    20


<210>  178
<211>  20
<212>  DNA
<213>  Artificial Sequence

<220>
<223>  Phosphorothioate Antisense Oligonucleotide

<400>  178
gcccatcgtg caggtacacg                                                    20


<210>  179
<211>  20
<212>  DNA
<213>  Artificial Sequence

<220>
<223>  Phosphorothioate Antisense Oligonucleotide

<400>  179
gtccacatcc tgacatgagt                                                    20


<210>  180
<211>  20
<212>  DNA
<213>  Artificial Sequence

<220>
<223>  Phosphorothioate Antisense Oligonucleotide

<400>  180
atgccattgt agcagtagcc                                                    20


<210>  181
<211>  20
<212>  DNA
<213>  Artificial Sequence

<220>
<223>  Phosphorothioate Antisense Oligonucleotide

<400>  181
gctgctcgtg agtctggcag                                                    20


<210>  182
<211>  20
<212>  DNA
<213>  Artificial Sequence

<220>
<223>  Phosphorothioate Antisense Oligonucleotide

<400>  182
tccccagagt gtgacacact                                                    20


<210>  183
<211>  20
<212>  DNA
<213>  Artificial Sequence

<220>
<223>  Phosphorothioate Antisense Oligonucleotide

<400>  183
ggggcaggtt tagcacctgg                                                    20


<210>  184
<211>  20
<212>  DNA
<213>  Artificial Sequence

<220>
<223>  Phosphorothioate Antisense Oligonucleotide

<400>  184
ctctctcaaa gcagatccca                                                    20


<210>  185
<211>  20
<212>  DNA
<213>  Artificial Sequence

<220>
<223>  Phosphorothioate Antisense Oligonucleotide

<400>  185
aggatcacct gcagaattga                                                    20


<210>  186
<211>  20
<212>  DNA
<213>  Artificial Sequence

<220>
<223>  Phosphorothioate Antisense Oligonucleotide

<400>  186
actttgccac agttgccata                                                    20


<210>  187
<211>  20
<212>  DNA
<213>  Artificial Sequence

<220>
<223>  Phosphorothioate Antisense Oligonucleotide

<400>  187
tggcaaagga actcttcgag                                                    20


<210>  188
<211>  20
<212>  DNA
<213>  Artificial Sequence

<220>
<223>  Phosphorothioate Antisense Oligonucleotide

<400>  188
agcatctctc atctcgcatt                                                    20


<210>  189
<211>  20
<212>  DNA
<213>  Artificial Sequence

<220>
<223>  Phosphorothioate Antisense Oligonucleotide

<400>  189
cactggattt ttccacattt                                                    20


<210>  190
<211>  20
<212>  DNA
<213>  Artificial Sequence

<220>
<223>  Phosphorothioate Antisense Oligonucleotide

<400>  190
gccggctggc acctccttga                                                    20


<210>  191
<211>  20

<212>  DNA
<213>  Artificial Sequence

<220>
<223>  Phosphorothioate Antisense Oligonucleotide

<400>  191
ggcattggta ccaatgactg                                                    20


<210>  192
<211>  20
<212>  DNA
<213>  Artificial Sequence

<220>
<223>  Phosphorothioate Antisense Oligonucleotide

<400>  192
atgtttgttt ctatggaaac                                                    20


<210>  193
<211>  20
<212>  DNA
<213>  Artificial Sequence

<220>
<223>  Phosphorothioate Antisense Oligonucleotide

<400>  193
ggcctccttg ctgcaggggg                                                    20


<210>  194
<211>  20
<212>  DNA
<213>  Artificial Sequence

<220>
<223>  Phosphorothioate Antisense Oligonucleotide

<400>  194
ggtcccccgg cacagaatcc                                                    20


<210>  195
<211>  20
<212>  DNA
<213>  Artificial Sequence

<220>
<223>  Phosphorothioate Antisense Oligonucleotide

<400>  195
tcatcgccca agtacacgtg                                                    20


<210>  196
<211>  20
<212>  DNA
<213>  Artificial Sequence

<220>
<223>  Phosphorothioate Antisense Oligonucleotide

<400>  196
caagccctgg gtccggcatg                                                    20

<210>  197
<211>  20
<212>  DNA
<213>  Artificial Sequence

<220>
<223>  Phosphorothioate Antisense Oligonucleotide

<400>  197
acactttgtg cctgcaagca                                                    20


<210>  198
<211>  20
<212>  DNA
<213>  Artificial Sequence

<220>
<223>  Phosphorothioate Antisense Oligonucleotide

<400>  198
aggcagattt ttccatctgc                                                    20


<210>  199
<211>  20
<212>  DNA
<213>  Artificial Sequence

<220>
<223>  Phosphorothioate Antisense Oligonucleotide

<400>  199
tattttgaca ttgacgattc                                                    20


<210>  200
<211>  20
<212>  DNA
<213>  Artificial Sequence

<220>
<223>  Phosphorothioate Antisense Oligonucleotide

<400>  200
gtgaacccca aagacactaa                                                    20


<210>  201
<211>  20
<212>  DNA
<213>  Artificial Sequence

<220>
<223>  Phosphorothioate Antisense Oligonucleotide

<400>  201
tggcactgca ttgcacactc                                                    20


<210>  202
<211>  20
<212>  DNA
<213>  Artificial Sequence

<220>
<223>  Phosphorothioate Antisense Oligonucleotide

<400>  202
tgttgcacac ccctctgccg                                                    20


<210>  203
<211>  20
<212>  DNA
<213>  Artificial Sequence

<220>
<223>  Phosphorothioate Antisense Oligonucleotide

<400>  203
gcagtggcag ttcttcctgt                                                    20


<210>  204
<211>  20
<212>  DNA
<213>  Artificial Sequence

<220>
<223>  Phosphorothioate Antisense Oligonucleotide

<400>  204
ggaggtgccc agtgggcctc                                                    20


<210>  205
<211>  20
<212>  DNA
<213>  Artificial Sequence

<220>
<223>  Phosphorothioate Antisense Oligonucleotide

<400>  205
agccaaactt gtcacagaag                                                    20


<210>  206
<211>  20
<212>  DNA
<213>  Artificial Sequence

<220>
<223>  Phosphorothioate Antisense Oligonucleotide

<400>  206
gctgtctgtg cttcctccaa                                                    20


<210>  207
<211>  20
<212>  DNA
<213>  Artificial Sequence

<220>
<223>  Phosphorothioate Antisense Oligonucleotide

<400>  207
tctgcttgcc ggatggggcc                                                    20


<210>  208
<211>  20
<212>  DNA
<213>  Artificial Sequence

<220>
<223>  Phosphorothioate Antisense Oligonucleotide

<400>  208
ctatggttaa accttggtta                                                    20


<210>  209
<211>  20
<212>  DNA
<213>  Artificial Sequence

<220>
<223>  Phosphorothioate Antisense Oligonucleotide

<400>  209
caggatggtc accagaattc                                                    20


<210>  210
<211>  20
<212>  DNA
<213>  Artificial Sequence

<220>
<223>  Phosphorothioate Antisense Oligonucleotide

<400>  210
aatccggcag caagaagaca                                                    20


<210>  211
<211>  20
<212>  DNA
<213>  Artificial Sequence

<220>
<223>  Phosphorothioate Antisense Oligonucleotide

<400>  211
tccttttgag ataaaccaca                                                    20


<210>  212
<211>  20
<212>  DNA
<213>  Artificial Sequence

<220>
<223>  Phosphorothioate Antisense Oligonucleotide

<400>  212
cagcagtcgt atcaaggtct                                                    20


<210>  213
<211>  20
<212>  DNA
<213>  Artificial Sequence

<220>
<223>  Phosphorothioate Antisense Oligonucleotide

<400>  213
gtggtcttct tatttgtaaa                                                    20


<210>  214
<211>  20

<212>  DNA
<213>  Artificial Sequence

<220>
<223>  Phosphorothioate Antisense Oligonucleotide

<400>  214
cacaccttag tttttcaatg                                                    20


<210>  215
<211>  20
<212>  DNA
<213>  Artificial Sequence

<220>
<223>  Phosphorothioate Antisense Oligonucleotide

<400>  215
gggtggccgg gaagggcgca                                                    20


<210>  216
<211>  20
<212>  DNA
<213>  Artificial Sequence

<220>
<223>  Phosphorothioate Antisense Oligonucleotide

<400>  216
tgacagggtt ggaagccacg                                                    20


<210>  217
<211>  20
<212>  DNA
<213>  Artificial Sequence

<220>
<223>  Phosphorothioate Antisense Oligonucleotide

<400>  217
gagcctgaca gggttggaag                                                    20


<210>  218
<211>  20
<212>  DNA
<213>  Artificial Sequence

<220>
<223>  Phosphorothioate Antisense Oligonucleotide

<400>  218
gaggtgagcc tgacagggtt                                                    20


<210>  219
<211>  20
<212>  DNA
<213>  Artificial Sequence

<220>
<223>  Phosphorothioate Antisense Oligonucleotide

<400>  219
tggccgaggt gagcctgaca                                                    20

<210>  220
<211>  20
<212>  DNA
<213>  Artificial Sequence

<220>
<223>  Phosphorothioate Antisense Oligonucleotide

<400>  220
caaggtggcc gaggtgagcc                                                    20


<210>  221
<211>  20
<212>  DNA
<213>  Artificial Sequence

<220>
<223>  Phosphorothioate Antisense Oligonucleotide

<400>  221
ttttccaagg tggccgaggt                                                    20


<210>  222
<211>  20
<212>  DNA
<213>  Artificial Sequence

<220>
<223>  Phosphorothioate Antisense Oligonucleotide

<400>  222
aggccttttc caaggtggcc                                                    20


<210>  223
<211>  20
<212>  DNA
<213>  Artificial Sequence

<220>
<223>  Phosphorothioate Antisense Oligonucleotide

<400>  223
tcatcaggcc ttttccaagg                                                    20


<210>  224
<211>  20
<212>  DNA
<213>  Artificial Sequence

<220>
<223>  Phosphorothioate Antisense Oligonucleotide

<400>  224
cttcctcatc aggccttttc                                                    20


<210>  225
<211>  20
<212>  DNA
<213>  Artificial Sequence

<220>
<223>  Phosphorothioate Antisense Oligonucleotide

<400>  225
ggcggcttcc tcatcaggcc                                                   20


<210>  226
<211>  20
<212>  DNA
<213>  Artificial Sequence

<220>
<223>  Phosphorothioate Antisense Oligonucleotide

<400>  226
aatctggcgg cttcctcatc                                                   20


<210>  227
<211>  20
<212>  DNA
<213>  Artificial Sequence

<220>
<223>  Phosphorothioate Antisense Oligonucleotide

<400>  227
gtaggaatct ggcggcttcc                                                   20


<210>  228
<211>  20
<212>  DNA
<213>  Artificial Sequence

<220>
<223>  Phosphorothioate Antisense Oligonucleotide

<400>  228
ggtaggaatc tggcggcttc                                                   20


<210>  229
<211>  20
<212>  DNA
<213>  Artificial Sequence

<220>
<223>  Phosphorothioate Antisense Oligonucleotide

<400>  229
gggtaggaat ctggcggctt                                                   20


<210>  230
<211>  20
<212>  DNA
<213>  Artificial Sequence

<220>
<223>  Phosphorothioate Antisense Oligonucleotide

<400>  230
tgggtaggaa tctggcggct                                                   20


<210>  231
<211>  20
<212>  DNA
<213>  Artificial Sequence

<220>
<223>  Phosphorothioate Antisense Oligonucleotide

<400>  231
gtgggtagga atctggcggc                                                    20


<210>  232
<211>  20
<212>  DNA
<213>  Artificial Sequence

<220>
<223>  Phosphorothioate Antisense Oligonucleotide

<400>  232
ggtgggtagg aatctggcgg                                                    20


<210>  233
<211>  20
<212>  DNA
<213>  Artificial Sequence

<220>
<223>  Phosphorothioate Antisense Oligonucleotide

<400>  233
cggtgggtag gaatctggcg                                                    20


<210>  234
<211>  20
<212>  DNA
<213>  Artificial Sequence

<220>
<223>  Phosphorothioate Antisense Oligonucleotide

<400>  234
tcggtgggta ggaatctggc                                                    20


<210>  235
<211>  20
<212>  DNA
<213>  Artificial Sequence

<220>
<223>  Phosphorothioate Antisense Oligonucleotide

<400>  235
ttcggtgggt aggaatctgg                                                    20


<210>  236
<211>  20
<212>  DNA
<213>  Artificial Sequence

<220>
<223>  Phosphorothioate Antisense Oligonucleotide

<400>  236
cttcggtggg taggaatctg                                                    20


<210>  237
<211>  20

<212>  DNA
<213>  Artificial Sequence

<220>
<223>  Phosphorothioate Antisense Oligonucleotide

<400>  237
ccttcggtgg gtaggaatct                                                    20


<210>  238
<211>  20
<212>  DNA
<213>  Artificial Sequence

<220>
<223>  Phosphorothioate Antisense Oligonucleotide

<400>  238
attgtccttc ggtgggtagg                                                    20


<210>  239
<211>  20
<212>  DNA
<213>  Artificial Sequence

<220>
<223>  Phosphorothioate Antisense Oligonucleotide

<400>  239
ctgggattgt ccttcggtgg                                                    20


<210>  240
<211>  20
<212>  DNA
<213>  Artificial Sequence

<220>
<223>  Phosphorothioate Antisense Oligonucleotide

<400>  240
atctcctggg attgtccttc                                                    20


<210>  241
<211>  20
<212>  DNA
<213>  Artificial Sequence

<220>
<223>  Phosphorothioate Antisense Oligonucleotide

<400>  241
cagcaatctc ctgggattgt                                                    20


<210>  242
<211>  20
<212>  DNA
<213>  Artificial Sequence

<220>
<223>  Phosphorothioate Antisense Oligonucleotide

<400>  242
cactgcagca atctcctggg                                                    20

<210>  243
<211>  20
<212>  DNA
<213>  Artificial Sequence

<220>
<223>  Phosphorothioate Antisense Oligonucleotide

<400>  243
tctgacactg cagcaatctc                                                    20


<210>  244
<211>  20
<212>  DNA
<213>  Artificial Sequence

<220>
<223>  Phosphorothioate Antisense Oligonucleotide

<400>  244
aacattctga cactgcagca                                                    20


<210>  245
<211>  20
<212>  DNA
<213>  Artificial Sequence

<220>
<223>  Phosphorothioate Antisense Oligonucleotide

<400>  245
atgtcaacat tctgacactg                                                    20


<210>  246
<211>  20
<212>  DNA
<213>  Artificial Sequence

<220>
<223>  Phosphorothioate Antisense Oligonucleotide

<400>  246
tgctgatgtc aacattctga                                                    20


<210>  247
<211>  20
<212>  DNA
<213>  Artificial Sequence

<220>
<223>  Phosphorothioate Antisense Oligonucleotide

<400>  247
gggtctgctg atgtcaacat                                                    20


<210>  248
<211>  20
<212>  DNA
<213>  Artificial Sequence

<220>
<223>  Phosphorothioate Antisense Oligonucleotide

<400>  248
ttgaggggtc tgctgatgtc                                                   20


<210>  249
<211>  20
<212>  DNA
<213>  Artificial Sequence

<220>
<223>  Phosphorothioate Antisense Oligonucleotide

<400>  249
ggccgttgag gggtctgctg                                                   20


<210>  250
<211>  20
<212>  DNA
<213>  Artificial Sequence

<220>
<223>  Phosphorothioate Antisense Oligonucleotide

<400>  250
attcaggccg ttgaggggtc                                                   20


<210>  251
<211>  20
<212>  DNA
<213>  Artificial Sequence

<220>
<223>  Phosphorothioate Antisense Oligonucleotide

<400>  251
gggacattca ggccgttgag                                                   20


<210>  252
<211>  20
<212>  DNA
<213>  Artificial Sequence

<220>
<223>  Phosphorothioate Antisense Oligonucleotide

<400>  252
agggacattc aggccgttga                                                   20


<210>  253
<211>  20
<212>  DNA
<213>  Artificial Sequence

<220>
<223>  Phosphorothioate Antisense Oligonucleotide

<400>  253
gagggacatt caggccgttg                                                   20


<210>  254
<211>  20
<212>  DNA
<213>  Artificial Sequence

<220>
<223>  Phosphorothioate Antisense Oligonucleotide

<400>  254
tgagggacat tcaggccgtt                                                    20


<210>  255
<211>  20
<212>  DNA
<213>  Artificial Sequence

<220>
<223>  Phosphorothioate Antisense Oligonucleotide

<400>  255
ctgagggaca ttcaggccgt                                                    20


<210>  256
<211>  20
<212>  DNA
<213>  Artificial Sequence

<220>
<223>  Phosphorothioate Antisense Oligonucleotide

<400>  256
gctgagggac attcaggccg                                                    20


<210>  257
<211>  20
<212>  DNA
<213>  Artificial Sequence

<220>
<223>  Phosphorothioate Antisense Oligonucleotide

<400>  257
ggctgaggga cattcaggcc                                                    20


<210>  258
<211>  20
<212>  DNA
<213>  Artificial Sequence

<220>
<223>  Phosphorothioate Antisense Oligonucleotide

<400>  258
gggctgaggg acattcaggc                                                    20


<210>  259
<211>  20
<212>  DNA
<213>  Artificial Sequence

<220>
<223>  Phosphorothioate Antisense Oligonucleotide

<400>  259
ggggctgagg gacattcagg                                                    20


<210>  260
<211>  20

<212>  DNA
<213>  Artificial Sequence

<220>
<223>  Phosphorothioate Antisense Oligonucleotide

<400>  260
tggggctgag ggacattcag                                                    20


<210>  261
<211>  20
<212>  DNA
<213>  Artificial Sequence

<220>
<223>  Phosphorothioate Antisense Oligonucleotide

<400>  261
ctggggctga gggacattca                                                    20


<210>  262
<211>  20
<212>  DNA
<213>  Artificial Sequence

<220>
<223>  Phosphorothioate Antisense Oligonucleotide

<400>  262
gttgactggg gctgagggac                                                    20


<210>  263
<211>  20
<212>  DNA
<213>  Artificial Sequence

<220>
<223>  Phosphorothioate Antisense Oligonucleotide

<400>  263
gctgagttga ctggggctga                                                    20


<210>  264
<211>  20
<212>  DNA
<213>  Artificial Sequence

<220>
<223>  Phosphorothioate Antisense Oligonucleotide

<400>  264
cactcgctga gttgactggg                                                    20


<210>  265
<211>  20
<212>  DNA
<213>  Artificial Sequence

<220>
<223>  Phosphorothioate Antisense Oligonucleotide

<400>  265
ggaagcactc gctgagttga                                                    20

<210>  266
<211>  20
<212>  DNA
<213>  Artificial Sequence

<220>
<223>  Phosphorothioate Antisense Oligonucleotide

<400>  266
ggggaggaag cactcgctga                                                    20


<210>  267
<211>  20
<212>  DNA
<213>  Artificial Sequence

<220>
<223>  Phosphorothioate Antisense Oligonucleotide

<400>  267
gtggagggga ggaagcactc                                                    20


<210>  268
<211>  20
<212>  DNA
<213>  Artificial Sequence

<220>
<223>  Phosphorothioate Antisense Oligonucleotide

<400>  268
gcccggtgga ggggaggaag                                                    20


<210>  269
<211>  20
<212>  DNA
<213>  Artificial Sequence

<220>
<223>  Phosphorothioate Antisense Oligonucleotide

<400>  269
gtggggcccg gtggagggga                                                    20


<210>  270
<211>  20
<212>  DNA
<213>  Artificial Sequence

<220>
<223>  Phosphorothioate Antisense Oligonucleotide

<400>  270
tgcacgtggg gcccggtgga                                                    20


<210>  271
<211>  20
<212>  DNA
<213>  Artificial Sequence

<220>
<223>  Phosphorothioate Antisense Oligonucleotide

<400>  271
gtgcacgtgg ggcccggtgg                                                   20


<210>  272
<211>  20
<212>  DNA
<213>  Artificial Sequence

<220>
<223>  Phosphorothioate Antisense Oligonucleotide

<400>  272
ggtgcacgtg gggcccggtg                                                   20


<210>  273
<211>  20
<212>  DNA
<213>  Artificial Sequence

<220>
<223>  Phosphorothioate Antisense Oligonucleotide

<400>  273
aggtgcacgt ggggcccggt                                                   20


<210>  274
<211>  20
<212>  DNA
<213>  Artificial Sequence

<220>
<223>  Phosphorothioate Antisense Oligonucleotide

<400>  274
taggtgcacg tggggcccgg                                                   20


<210>  275
<211>  20
<212>  DNA
<213>  Artificial Sequence

<220>
<223>  Phosphorothioate Antisense Oligonucleotide

<400>  275
ctaggtgcac gtggggcccg                                                   20


<210>  276
<211>  20
<212>  DNA
<213>  Artificial Sequence

<220>
<223>  Phosphorothioate Antisense Oligonucleotide

<400>  276
gctaggtgca cgtggggccc                                                   20


<210>  277
<211>  20
<212>  DNA
<213>  Artificial Sequence

<220>
<223>  Phosphorothioate Antisense Oligonucleotide

<400>  277
cgctaggtgc acgtggggcc                                                    20


<210>  278
<211>  20
<212>  DNA
<213>  Artificial Sequence

<220>
<223>  Phosphorothioate Antisense Oligonucleotide

<400>  278
acgctaggtg cacgtggggc                                                    20


<210>  279
<211>  20
<212>  DNA
<213>  Artificial Sequence

<220>
<223>  Phosphorothioate Antisense Oligonucleotide

<400>  279
gacgctaggt gcacgtgggg                                                    20


<210>  280
<211>  20
<212>  DNA
<213>  Artificial Sequence

<220>
<223>  Phosphorothioate Antisense Oligonucleotide

<400>  280
ggacgctagg tgcacgtggg                                                    20


<210>  281
<211>  20
<212>  DNA
<213>  Artificial Sequence

<220>
<223>  Phosphorothioate Antisense Oligonucleotide

<400>  281
ggcagggacg ctaggtgcac                                                    20


<210>  282
<211>  20
<212>  DNA
<213>  Artificial Sequence

<220>
<223>  Phosphorothioate Antisense Oligonucleotide

<400>  282
ggtctggcag ggacgctagg                                                    20


<210>  283
<211>  20

<212>  DNA
<213>  Artificial Sequence

<220>
<223>  Phosphorothioate Antisense Oligonucleotide

<400>  283
gcaggggtct ggcagggacg                                                    20


<210>  284
<211>  20
<212>  DNA
<213>  Artificial Sequence

<220>
<223>  Phosphorothioate Antisense Oligonucleotide

<400>  284
ggctggcagg ggtctggcag                                                    20


<210>  285
<211>  20
<212>  DNA
<213>  Artificial Sequence

<220>
<223>  Phosphorothioate Antisense Oligonucleotide

<400>  285
ggcttggctg gcaggggtct                                                    20


<210>  286
<211>  20
<212>  DNA
<213>  Artificial Sequence

<220>
<223>  Phosphorothioate Antisense Oligonucleotide

<400>  286
gtgcaggctt ggctggcagg                                                    20


<210>  287
<211>  20
<212>  DNA
<213>  Artificial Sequence

<220>
<223>  Phosphorothioate Antisense Oligonucleotide

<400>  287
cctaagtgca ggcttggctg                                                    20


<210>  288
<211>  20
<212>  DNA
<213>  Artificial Sequence

<220>
<223>  Phosphorothioate Antisense Oligonucleotide

<400>  288
gcctgcctaa gtgcaggctt                                                    20

<210>  289
<211>  20
<212>  DNA
<213>  Artificial Sequence

<220>
<223>  Phosphorothioate Antisense Oligonucleotide

<400>  289
cctgggcctg cctaagtgca                                                    20


<210>  290
<211>  20
<212>  DNA
<213>  Artificial Sequence

<220>
<223>  Phosphorothioate Antisense Oligonucleotide

<400>  290
ggtcccctgg gcctgcctaa                                                    20


<210>  291
<211>  20
<212>  DNA
<213>  Artificial Sequence

<220>
<223>  Phosphorothioate Antisense Oligonucleotide

<400>  291
ttacaggtcc cctgggcctg                                                    20


<210>  292
<211>  20
<212>  DNA
<213>  Artificial Sequence

<220>
<223>  Phosphorothioate Antisense Oligonucleotide

<400>  292
ttggcttaca ggtcccctgg                                                    20


<210>  293
<211>  20
<212>  DNA
<213>  Artificial Sequence

<220>
<223>  Phosphorothioate Antisense Oligonucleotide

<400>  293
ggggtttggc ttacaggtcc                                                    20


<210>  294
<211>  20
<212>  DNA
<213>  Artificial Sequence

<220>
<223>  Phosphorothioate Antisense Oligonucleotide

<400>  294
gggggtttgg cttacaggtc                                                   20


<210>  295
<211>  20
<212>  DNA
<213>  Artificial Sequence

<220>
<223>  Phosphorothioate Antisense Oligonucleotide

<400>  295
ggggggtttg gcttacaggt                                                   20


<210>  296
<211>  20
<212>  DNA
<213>  Artificial Sequence

<220>
<223>  Phosphorothioate Antisense Oligonucleotide

<400>  296
aggggggttt ggcttacagg                                                   20


<210>  297
<211>  20
<212>  DNA
<213>  Artificial Sequence

<220>
<223>  Phosphorothioate Antisense Oligonucleotide

<400>  297
gaggggggtt tggcttacag                                                   20


<210>  298
<211>  20
<212>  DNA
<213>  Artificial Sequence

<220>
<223>  Phosphorothioate Antisense Oligonucleotide

<400>  298
tgaggggggt ttggcttaca                                                   20


<210>  299
<211>  20
<212>  DNA
<213>  Artificial Sequence

<220>
<223>  Phosphorothioate Antisense Oligonucleotide

<400>  299
ctgagggggg tttggcttac                                                   20


<210>  300
<211>  20
<212>  DNA
<213>  Artificial Sequence

<220>
<223>  Phosphorothioate Antisense Oligonucleotide

<400>  300
tctgaggggg gtttggctta                                                   20


<210>  301
<211>  20
<212>  DNA
<213>  Artificial Sequence

<220>
<223>  Phosphorothioate Antisense Oligonucleotide

<400>  301
ttctgagggg ggtttggctt                                                   20


<210>  302
<211>  20
<212>  DNA
<213>  Artificial Sequence

<220>
<223>  Phosphorothioate Antisense Oligonucleotide

<400>  302
cttctgaggg gggtttggct                                                   20


<210>  303
<211>  20
<212>  DNA
<213>  Artificial Sequence

<220>
<223>  Phosphorothioate Antisense Oligonucleotide

<400>  303
gcttctgagg ggggtttggc                                                   20


<210>  304
<211>  20
<212>  DNA
<213>  Artificial Sequence

<220>
<223>  Phosphorothioate Antisense Oligonucleotide

<400>  304
cagaggcttc tgaggggggt                                                   20


<210>  305
<211>  20
<212>  DNA
<213>  Artificial Sequence

<220>
<223>  Phosphorothioate Antisense Oligonucleotide

<400>  305
gcaggcagag gcttctgagg                                                   20


<210>  306
<211>  20

<212>  DNA
<213>  Artificial Sequence

<220>
<223>  Phosphorothioate Antisense Oligonucleotide

<400>  306
tgcaggcaga ggcttctgag                                                    20


<210>  307
<211>  20
<212>  DNA
<213>  Artificial Sequence

<220>
<223>  Phosphorothioate Antisense Oligonucleotide

<400>  307
ctgcaggcag aggcttctga                                                    20


<210>  308
<211>  20
<212>  DNA
<213>  Artificial Sequence

<220>
<223>  Phosphorothioate Antisense Oligonucleotide

<400>  308
tctgcaggca gaggcttctg                                                    20


<210>  309
<211>  20
<212>  DNA
<213>  Artificial Sequence

<220>
<223>  Phosphorothioate Antisense Oligonucleotide

<400>  309
atctgcaggc agaggcttct                                                    20


<210>  310
<211>  20
<212>  DNA
<213>  Artificial Sequence

<220>
<223>  Phosphorothioate Antisense Oligonucleotide

<400>  310
gatctgcagg cagaggcttc                                                    20


<210>  311
<211>  20
<212>  DNA
<213>  Artificial Sequence

<220>
<223>  Phosphorothioate Antisense Oligonucleotide

<400>  311
ggatctgcag gcagaggctt                                                    20

<210>  312
<211>  20
<212>  DNA
<213>  Artificial Sequence

<220>
<223>  Phosphorothioate Antisense Oligonucleotide

<400>  312
aggatctgca ggcagaggct                                                    20


<210>  313
<211>  20
<212>  DNA
<213>  Artificial Sequence

<220>
<223>  Phosphorothioate Antisense Oligonucleotide

<400>  313
gaggatctgc aggcagaggc                                                    20


<210>  314
<211>  20
<212>  DNA
<213>  Artificial Sequence

<220>
<223>  Phosphorothioate Antisense Oligonucleotide

<400>  314
agaggatctg caggcagagg                                                    20


<210>  315
<211>  20
<212>  DNA
<213>  Artificial Sequence

<220>
<223>  Phosphorothioate Antisense Oligonucleotide

<400>  315
cagaggatct gcaggcagag                                                    20


<210>  316
<211>  20
<212>  DNA
<213>  Artificial Sequence

<220>
<223>  Phosphorothioate Antisense Oligonucleotide

<400>  316
ccagaggatc tgcaggcaga                                                    20


<210>  317
<211>  20
<212>  DNA
<213>  Artificial Sequence

<220>
<223>  Phosphorothioate Antisense Oligonucleotide

<400>  317
gccagaggat ctgcaggcag                                                    20


<210>  318
<211>  20
<212>  DNA
<213>  Artificial Sequence

<220>
<223>  Phosphorothioate Antisense Oligonucleotide

<400>  318
ggccagagga tctgcaggca                                                    20


<210>  319
<211>  20
<212>  DNA
<213>  Artificial Sequence

<220>
<223>  Phosphorothioate Antisense Oligonucleotide

<400>  319
tggccagagg atctgcaggc                                                    20


<210>  320
<211>  20
<212>  DNA
<213>  Artificial Sequence

<220>
<223>  Phosphorothioate Antisense Oligonucleotide

<400>  320
ctggccagag gatctgcagg                                                    20


<210>  321
<211>  20
<212>  DNA
<213>  Artificial Sequence

<220>
<223>  Phosphorothioate Antisense Oligonucleotide

<400>  321
tctggccaga ggatctgcag                                                    20


<210>  322
<211>  20
<212>  DNA
<213>  Artificial Sequence

<220>
<223>  Phosphorothioate Antisense Oligonucleotide

<400>  322
ttctggccag aggatctgca                                                    20


<210>  323
<211>  20
<212>  DNA
<213>  Artificial Sequence

<220>
<223>  Phosphorothioate Antisense Oligonucleotide

<400>  323
gttctggcca gaggatctgc                                                    20


<210>  324
<211>  20
<212>  DNA
<213>  Artificial Sequence

<220>
<223>  Phosphorothioate Antisense Oligonucleotide

<400>  324
tgttctggcc agaggatctg                                                    20


<210>  325
<211>  20
<212>  DNA
<213>  Artificial Sequence

<220>
<223>  Phosphorothioate Antisense Oligonucleotide

<400>  325
ttgttctggc cagaggatct                                                    20


<210>  326
<211>  20
<212>  DNA
<213>  Artificial Sequence

<220>
<223>  Phosphorothioate Antisense Oligonucleotide

<400>  326
gttgttctgg ccagaggatc                                                    20


<210>  327
<211>  20
<212>  DNA
<213>  Artificial Sequence

<220>
<223>  Phosphorothioate Antisense Oligonucleotide

<400>  327
agttgttctg gccagaggat                                                    20


<210>  328
<211>  20
<212>  DNA
<213>  Artificial Sequence

<220>
<223>  Phosphorothioate Antisense Oligonucleotide

<400>  328
gagttgttct ggccagagga                                                    20


<210>  329
<211>  20

<212>  DNA
<213>  Artificial Sequence

<220>
<223>  Phosphorothioate Antisense Oligonucleotide

<400>  329
cgagttgttc tggccagagg                                                    20


<210>  330
<211>  20
<212>  DNA
<213>  Artificial Sequence

<220>
<223>  Phosphorothioate Antisense Oligonucleotide

<400>  330
ccgagttgtt ctggccagag                                                    20


<210>  331
<211>  20
<212>  DNA
<213>  Artificial Sequence

<220>
<223>  Phosphorothioate Antisense Oligonucleotide

<400>  331
gtgagccgag ttgttctggc                                                    20


<210>  332
<211>  20
<212>  DNA
<213>  Artificial Sequence

<220>
<223>  Phosphorothioate Antisense Oligonucleotide

<400>  332
catgagtgag ccgagttgtt                                                    20


<210>  333
<211>  20
<212>  DNA
<213>  Artificial Sequence

<220>
<223>  Phosphorothioate Antisense Oligonucleotide

<400>  333
caaggcatga gtgagccgag                                                    20


<210>  334
<211>  20
<212>  DNA
<213>  Artificial Sequence

<220>
<223>  Phosphorothioate Antisense Oligonucleotide

<400>  334
ctggccaagg catgagtgag                                                    20

<210> 335
<211> 20
<212> DNA
<213> Artificial Sequence

<220>
<223> Phosphorothioate Antisense Oligonucleotide

<400> 335
gggtcctggc caaggcatga 20


<210> 336
<211> 20
<212> DNA
<213> Artificial Sequence

<220>
<223> Phosphorothioate Antisense Oligonucleotide

<400> 336
tcctggggtc ctggccaagg 20


<210> 337
<211> 20
<212> DNA
<213> Artificial Sequence

<220>
<223> Phosphorothioate Antisense Oligonucleotide

<400> 337
cattgtcctg gggtcctggc 20


<210> 338
<211> 20
<212> DNA
<213> Artificial Sequence

<220>
<223> Phosphorothioate Antisense Oligonucleotide

<400> 338
tctcccattg tcctggggtc 20


<210> 339
<211> 20
<212> DNA
<213> Artificial Sequence

<220>
<223> Phosphorothioate Antisense Oligonucleotide

<400> 339
cccagtctcc cattgtcctg 20


<210> 340
<211> 20
<212> DNA
<213> Artificial Sequence

<220>
<223> Phosphorothioate Antisense Oligonucleotide

<400>  340
cggagcccag tctcccattg                                                   20


<210>  341
<211>  20
<212>  DNA
<213>  Artificial Sequence

<220>
<223>  Phosphorothioate Antisense Oligonucleotide

<400>  341
ccaggcggag cccagtctcc                                                   20


<210>  342
<211>  20
<212>  DNA
<213>  Artificial Sequence

<220>
<223>  Phosphorothioate Antisense Oligonucleotide

<400>  342
gggtgccagg cggagcccag                                                   20


<210>  343
<211>  20
<212>  DNA
<213>  Artificial Sequence

<220>
<223>  Phosphorothioate Antisense Oligonucleotide

<400>  343
ctgaggggtg ccaggcggag                                                   20


<210>  344
<211>  20
<212>  DNA
<213>  Artificial Sequence

<220>
<223>  Phosphorothioate Antisense Oligonucleotide

<400>  344
caggtctgag gggtgccagg                                                   20


<210>  345
<211>  20
<212>  DNA
<213>  Artificial Sequence

<220>
<223>  Phosphorothioate Antisense Oligonucleotide

<400>  345
tggagcaggt ctgaggggtg                                                   20


<210>  346
<211>  20
<212>  DNA
<213>  Artificial Sequence

<220>
<223>  Phosphorothioate Antisense Oligonucleotide

<400>  346
tattgtggag caggtctgag                                                    20


<210>  347
<211>  20
<212>  DNA
<213>  Artificial Sequence

<220>
<223>  Phosphorothioate Antisense Oligonucleotide

<400>  347
gtggatattg tggagcaggt                                                    20


<210>  348
<211>  20
<212>  DNA
<213>  Artificial Sequence

<220>
<223>  Phosphorothioate Antisense Oligonucleotide

<400>  348
ttggtgtgga tattgtggag                                                    20


<210>  349
<211>  20
<212>  DNA
<213>  Artificial Sequence

<220>
<223>  Phosphorothioate Antisense Oligonucleotide

<400>  349
ggcacttggt gtggatattg                                                    20


<210>  350
<211>  20
<212>  DNA
<213>  Artificial Sequence

<220>
<223>  Phosphorothioate Antisense Oligonucleotide

<400>  350
atctgggcac ttggtgtgga                                                    20


<210>  351
<211>  20
<212>  DNA
<213>  Artificial Sequence

<220>
<223>  Phosphorothioate Antisense Oligonucleotide

<400>  351
ggtggatctg ggcacttggt                                                    20


<210>  352
<211>  20

<212>  DNA
<213>  Artificial Sequence

<220>
<223>  Phosphorothioate Antisense Oligonucleotide

<400>  352
gtgtgggtgg atctgggcac                                                    20


<210>  353
<211>  20
<212>  DNA
<213>  Artificial Sequence

<220>
<223>  Phosphorothioate Antisense Oligonucleotide

<400>  353
cttctcactt aatataggcg                                                    20


<210>  354
<211>  20
<212>  DNA
<213>  Artificial Sequence

<220>
<223>  Phosphorothioate Antisense Oligonucleotide

<400>  354
ctgttgaaaa aaggtgtcgg                                                    20


<210>  355
<211>  20
<212>  DNA
<213>  Artificial Sequence

<220>
<223>  Phosphorothioate Antisense Oligonucleotide

<400>  355
agtgcaaact tctgtcttca                                                    20


<210>  356
<211>  20
<212>  DNA
<213>  Artificial Sequence

<220>
<223>  Phosphorothioate Antisense Oligonucleotide

<400>  356
tccaactgga gctgaaagat                                                    20


<210>  357
<211>  20
<212>  DNA
<213>  Artificial Sequence

<220>
<223>  Phosphorothioate Antisense Oligonucleotide

<400>  357
taaaagttgg tacaaaaaac                                                    20

<210>  358
<211>  20
<212>  DNA
<213>  Artificial Sequence

<220>
<223>  Phosphorothioate Antisense Oligonucleotide

<400>  358
ttaaacatta aaaaaaatcc                                                    20


<210>  359
<211>  20
<212>  DNA
<213>  Artificial Sequence

<220>
<223>  Phosphorothioate Antisense Oligonucleotide

<400>  359
gttcttatag taatgatgtt                                                    20


<210>  360
<211>  20
<212>  DNA
<213>  Artificial Sequence

<220>
<223>  Phosphorothioate Antisense Oligonucleotide

<400>  360
actgacggca gtagctcaaa                                                    20


<210>  361
<211>  20
<212>  DNA
<213>  Artificial Sequence

<220>
<223>  Phosphorothioate Antisense Oligonucleotide

<400>  361
gcaccatagc acagcacagc                                                    20


<210>  362
<211>  20
<212>  DNA
<213>  Artificial Sequence

<220>
<223>  Phosphorothioate Antisense Oligonucleotide

<400>  362
tacctgtgca agtagacaga                                                    20


<210>  363
<211>  20
<212>  DNA
<213>  Artificial Sequence

<220>
<223>  Phosphorothioate Antisense Oligonucleotide

<400>  363
ataaattaat aatttacaag                                                    20


<210>  364
<211>  20
<212>  DNA
<213>  Artificial Sequence

<220>
<223>  Phosphorothioate Antisense Oligonucleotide

<400>  364
cactgtaatc aacattctgc                                                    20


<210>  365
<211>  20
<212>  DNA
<213>  Artificial Sequence

<220>
<223>  Phosphorothioate Antisense Oligonucleotide

<400>  365
atgcctacta cagcgcactg                                                    20


<210>  366
<211>  20
<212>  DNA
<213>  Artificial Sequence

<220>
<223>  Phosphorothioate Antisense Oligonucleotide

<400>  366
aaaactcagt gatggtaaaa                                                    20


<210>  367
<211>  20
<212>  DNA
<213>  Artificial Sequence

<220>
<223>  Phosphorothioate Antisense Oligonucleotide

<400>  367
aacaagcctt cctgccatgg                                                    20


<210>  368
<211>  20
<212>  DNA
<213>  Artificial Sequence

<220>
<223>  Phosphorothioate Antisense Oligonucleotide

<400>  368
   cactaaaata ctaaaagcac                                                     20


<210>  369
<211>  20
<212>  DNA
<213>  Artificial Sequence

<220>
<223>  Phosphorothioate Antisense Oligonucleotide

<400>  369
caagcaggat atttcaagtt                                                    20


<210>  370
<211>  20
<212>  DNA
<213>  Artificial Sequence

<220>
<223>  Phosphorothioate Antisense Oligonucleotide

<400>  370
catcctgtcc agaatcccat                                                    20


<210>  371
<211>  20
<212>  DNA
<213>  Artificial Sequence

<220>
<223>  Phosphorothioate Antisense Oligonucleotide

<400>  371
ccttgatcag aaagcaaaca                                                    20


<210>  372
<211>  20
<212>  DNA
<213>  Artificial Sequence

<220>
<223>  Phosphorothioate Antisense Oligonucleotide

<400>  372
gggactgctt tccaataagg                                                    20


<210>  373
<211>  20
<212>  DNA
<213>  Artificial Sequence

<220>
<223>  Phosphorothioate Antisense Oligonucleotide

<400>  373
gcacagctgg gggtagttgg                                                    20


<210>  374
<211>  20
<212>  DNA
<213>  Artificial Sequence

<220>
<223>  Phosphorothioate Antisense Oligonucleotide

<400>  374
agctgcatct ggtaccataa                                                    20


<210>  375
<211>  20

<212>  DNA
<213>  Artificial Sequence

<220>
<223>  Phosphorothioate Antisense Oligonucleotide

<400>  375
attctacttg ggatctcttg                                                    20


<210>  376
<211>  20
<212>  DNA
<213>  Artificial Sequence

<220>
<223>  Phosphorothioate Antisense Oligonucleotide

<400>  376
aatccagaaa atcaactgag                                                    20


<210>  377
<211>  20
<212>  DNA
<213>  Artificial Sequence

<220>
<223>  Phosphorothioate Antisense Oligonucleotide

<400>  377
ggctctggcc tgagatgggg                                                    20


<210>  378
<211>  20
<212>  DNA
<213>  Artificial Sequence

<220>
<223>  Phosphorothioate Antisense Oligonucleotide

<400>  378
gcctggacct gaagcccctt                                                    20


<210>  379
<211>  20
<212>  DNA
<213>  Artificial Sequence

<220>
<223>  Phosphorothioate Antisense Oligonucleotide

<400>  379
ctccctgaaa gccaaacaca                                                    20


<210>  380
<211>  20
<212>  DNA
<213>  Artificial Sequence

<220>
<223>  Phosphorothioate Antisense Oligonucleotide

<400>  380
gttgtcaagg ggcacagggc                                                    20

<210> 381
<211> 20
<212> DNA
<213> Artificial Sequence

<220>
<223> Phosphorothioate Antisense Oligonucleotide

<400> 381
ccctgggagc ctgcctgcca 20


<210> 382
<211> 20
<212> DNA
<213> Artificial Sequence

<220>
<223> Phosphorothioate Antisense Oligonucleotide

<400> 382
gccagatttc tcccaggtgt 20


<210> 383
<211> 20
<212> DNA
<213> Artificial Sequence

<220>
<223> Phosphorothioate Antisense Oligonucleotide

<400> 383
ccaaagcttc ctggccagaa 20


<210> 384
<211> 20
<212> DNA
<213> Artificial Sequence

<220>
<223> Phosphorothioate Antisense Oligonucleotide

<400> 384
gtctgcaacc caggttctca 20


<210> 385
<211> 20
<212> DNA
<213> Artificial Sequence

<220>
<223> Phosphorothioate Antisense Oligonucleotide

<400> 385
ggctacacct taagattcct 20


<210> 386
<211> 20
<212> DNA
<213> Artificial Sequence

<220>
<223> Phosphorothioate Antisense Oligonucleotide

<400>  386
tccagtctct atcctggtgt                                                    20

<210>  387
<211>  20
<212>  DNA
<213>  Artificial Sequence

<220>
<223>  Phosphorothioate Antisense Oligonucleotide

<400>  387
agttctggct tgtctagtgt                                                    20

<210>  388
<211>  20
<212>  DNA
<213>  Artificial Sequence

<220>
<223>  Phosphorothioate Antisense Oligonucleotide

<400>  388
ggctggtcag ctcagggtca                                                    20

<210>  389
<211>  20
<212>  DNA
<213>  Artificial Sequence

<220>
<223>  Phosphorothioate Antisense Oligonucleotide

<400>  389
ccccttccaa acatgctcac                                                    20

<210>  390
<211>  20
<212>  DNA
<213>  Artificial Sequence

<220>
<223>  Phosphorothioate Antisense Oligonucleotide

<400>  390
gccttgagtg acactacaga                                                    20

<210>  391
<211>  20
<212>  DNA
<213>  Artificial Sequence

<220>
<223>  Phosphorothioate Antisense Oligonucleotide

<400>  391
tggcatttct atcaagcacc                                                    20

<210>  392
<211>  20
<212>  DNA
<213>  Artificial Sequence

<220>
<223>  Phosphorothioate Antisense Oligonucleotide

<400>  392
cagcgagaaa aagaagtgct                                                    20


<210>  393
<211>  20
<212>  DNA
<213>  Artificial Sequence

<220>
<223>  Phosphorothioate Antisense Oligonucleotide

<400>  393
tggcagtgct ctagaaagga                                                    20


<210>  394
<211>  20
<212>  DNA
<213>  Artificial Sequence

<220>
<223>  Phosphorothioate Antisense Oligonucleotide

<400>  394
caagctaaat aacctactgg                                                    20


<210>  395
<211>  20
<212>  DNA
<213>  Artificial Sequence

<220>
<223>  Phosphorothioate Antisense Oligonucleotide

<400>  395
tacagaaaca ccacctttcc                                                    20


<210>  396
<211>  20
<212>  DNA
<213>  Artificial Sequence

<220>
<223>  Phosphorothioate Antisense Oligonucleotide

<400>  396
tgcctgggca gtaggtttct                                                    20


<210>  397
<211>  20
<212>  DNA
<213>  Artificial Sequence

<220>
<223>  Phosphorothioate Antisense Oligonucleotide

<400>  397
agggaggtgg cggtttgcag                                                    20


<210>  398
<211>  20

<212>  DNA
<213>  Artificial Sequence

<220>
<223>  Phosphorothioate Antisense Oligonucleotide

<400>  398
tgctcagctc caagcagtat                                                    20


<210>  399
<211>  20
<212>  DNA
<213>  Artificial Sequence

<220>
<223>  Phosphorothioate Antisense Oligonucleotide

<400>  399
tattacagtt tgtggtgatt                                                    20


<210>  400
<211>  20
<212>  DNA
<213>  Artificial Sequence

<220>
<223>  Phosphorothioate Antisense Oligonucleotide

<400>  400
gtctgaatac aggatcattg                                                    20


<210>  401
<211>  20
<212>  DNA
<213>  Artificial Sequence

<220>
<223>  Phosphorothioate Antisense Oligonucleotide

<400>  401
cccatggaaa gtcctcatct                                                    20


<210>  402
<211>  20
<212>  DNA
<213>  Artificial Sequence

<220>
<223>  Phosphorothioate Antisense Oligonucleotide

<400>  402
catctgaaaa tagttgtggt                                                    20


<210>  403
<211>  20
<212>  DNA
<213>  Artificial Sequence

<220>
<223>  Phosphorothioate Antisense Oligonucleotide

<400>  403
tagatctggt taatggttca                                                    20

<210>  404
<211>  20
<212>  DNA
<213>  Artificial Sequence

<220>
<223>  Phosphorothioate Antisense Oligonucleotide

<400>  404
agtaaacaga cttgattgac                                                    20


<210>  405
<211>  20
<212>  DNA
<213>  Artificial Sequence

<220>
<223>  Phosphorothioate Antisense Oligonucleotide

<400>  405
gttaataagt tgaaccttgc                                                    20


<210>  406
<211>  20
<212>  DNA
<213>  Artificial Sequence

<220>
<223>  Phosphorothioate Antisense Oligonucleotide

<400>  406
cataaagagt ctgcctaatt                                                    20


<210>  407
<211>  20
<212>  DNA
<213>  Artificial Sequence

<220>
<223>  Phosphorothioate Antisense Oligonucleotide

<400>  407
ttggttgtag tttttgcaag                                                    20


<210>  408
<211>  20
<212>  DNA
<213>  Artificial Sequence

<220>
<223>  Phosphorothioate Antisense Oligonucleotide

<400>  408
cccatgaaca tcacattcca                                                    20


<210>  409
<211>  20
<212>  DNA
<213>  Artificial Sequence

<220>
<223>  Phosphorothioate Antisense Oligonucleotide

<400>  409
gatagcagac atgaactata                                                    20

<210>  410
<211>  20
<212>  DNA
<213>  Artificial Sequence

<220>
<223>  Phosphorothioate Antisense Oligonucleotide

<400>  410
gtccaatatc tacgaataat                                                    20

<210>  411
<211>  20
<212>  DNA
<213>  Artificial Sequence

<220>
<223>  Phosphorothioate Antisense Oligonucleotide

<400>  411
ccccatagag aaggttcttt                                                    20

<210>  412
<211>  20
<212>  DNA
<213>  Artificial Sequence

<220>
<223>  Phosphorothioate Antisense Oligonucleotide

<400>  412
caagttggaa aaagaggatg                                                    20

<210>  413
<211>  20
<212>  DNA
<213>  Artificial Sequence

<220>
<223>  Phosphorothioate Antisense Oligonucleotide

<400>  413
cttttaaaga ttcctgcagc                                                    20

<210>  414
<211>  20
<212>  DNA
<213>  Artificial Sequence

<220>
<223>  Phosphorothioate Antisense Oligonucleotide

<400>  414
tcagactctg ttaaaagcat                                                    20

<210>  415
<211>  20
<212>  DNA
<213>  Artificial Sequence

<220>
<223>  Phosphorothioate Antisense Oligonucleotide

<400>  415
caagtgttta agaaataggt                                                   20


<210>  416
<211>  20
<212>  DNA
<213>  Artificial Sequence

<220>
<223>  Phosphorothioate Antisense Oligonucleotide

<400>  416
gatgctcaac aggtaggttg                                                   20


<210>  417
<211>  20
<212>  DNA
<213>  Artificial Sequence

<220>
<223>  Phosphorothioate Antisense Oligonucleotide

<400>  417
atttccttat cacattctgt                                                   20


<210>  418
<211>  20
<212>  DNA
<213>  Artificial Sequence

<220>
<223>  Phosphorothioate Antisense Oligonucleotide

<400>  418
ggaagttgat aagcaagttg                                                   20


<210>  419
<211>  20
<212>  DNA
<213>  Artificial Sequence

<220>
<223>  Phosphorothioate Antisense Oligonucleotide

<400>  419
gccacatctc ataatattta                                                   20


<210>  420
<211>  20
<212>  DNA
<213>  Artificial Sequence

<220>
<223>  Phosphorothioate Antisense Oligonucleotide

<400>  420
ttcaagggga tgctgcccaa                                                   20


<210>  421
<211>  20

<212>  DNA
<213>  Artificial Sequence

<220>
<223>  Phosphorothioate Antisense Oligonucleotide

<400>  421
ggcatttgaa gagtgaagag                                                    20


<210>  422
<211>  20
<212>  DNA
<213>  Artificial Sequence

<220>
<223>  Phosphorothioate Antisense Oligonucleotide

<400>  422
gaaacatggc tccctagtca                                                    20


<210>  423
<211>  20
<212>  DNA
<213>  Artificial Sequence

<220>
<223>  Phosphorothioate Antisense Oligonucleotide

<400>  423
agtcacttta aagaccttgt                                                    20


<210>  424
<211>  20
<212>  DNA
<213>  Artificial Sequence

<220>
<223>  Phosphorothioate Antisense Oligonucleotide

<400>  424
tttgtatttc tcatgccatt                                                    20


<210>  425
<211>  20
<212>  DNA
<213>  Artificial Sequence

<220>
<223>  Phosphorothioate Antisense Oligonucleotide

<400>  425
ttttacctta tctgagtatt                                                    20


<210>  426
<211>  20
<212>  DNA
<213>  Artificial Sequence

<220>
<223>  Phosphorothioate Antisense Oligonucleotide

<400>  426
aagacagagg catcatggca                                                    20

<210>  427
<211>  20
<212>  DNA
<213>  Artificial Sequence

<220>
<223>  Phosphorothioate Antisense Oligonucleotide

<400>  427
taatgtgaaa accagtccag                                                    20


<210>  428
<211>  20
<212>  DNA
<213>  Artificial Sequence

<220>
<223>  Phosphorothioate Antisense Oligonucleotide

<400>  428
aactgttgtc aattgtcttc                                                    20


<210>  429
<211>  20
<212>  DNA
<213>  Artificial Sequence

<220>
<223>  Phosphorothioate Antisense Oligonucleotide

<400>  429
acactcagag tgaattatgt                                                    20


<210>  430
<211>  20
<212>  DNA
<213>  Artificial Sequence

<220>
<223>  Phosphorothioate Antisense Oligonucleotide

<400>  430
aaagaaggct ttctcataaa                                                    20


<210>  431
<211>  20
<212>  DNA
<213>  Artificial Sequence

<220>
<223>  Phosphorothioate Antisense Oligonucleotide

<400>  431
taggaaaact gttgacccca                                                    20


<210>  432
<211>  20
<212>  DNA
<213>  Artificial Sequence

<220>
<223>  Phosphorothioate Antisense Oligonucleotide

<400>  432
tatttttctg tttcaaagca                                                    20

<210>  433
<211>  20
<212>  DNA
<213>  Artificial Sequence

<220>
<223>  Phosphorothioate Antisense Oligonucleotide

<400>  433
aaaccaagat tcttggtaca                                                    20

<210>  434
<211>  20
<212>  DNA
<213>  Artificial Sequence

<220>
<223>  Phosphorothioate Antisense Oligonucleotide

<400>  434
agttttgttt tctggaaggc                                                    20

<210>  435
<211>  20
<212>  DNA
<213>  Artificial Sequence

<220>
<223>  Phosphorothioate Antisense Oligonucleotide

<400>  435
acaccgggaa agtgaaatgc                                                    20

<210>  436
<211>  20
<212>  DNA
<213>  Artificial Sequence

<220>
<223>  Phosphorothioate Antisense Oligonucleotide

<400>  436
ttgcctagat acagtgggga                                                    20

<210>  437
<211>  20
<212>  DNA
<213>  Artificial Sequence

<220>
<223>  Phosphorothioate Antisense Oligonucleotide

<400>  437
ccatagtcat gaatactatg                                                    20

<210>  438
<211>  20
<212>  DNA
<213>  Artificial Sequence

<220>
<223>  Phosphorothioate Antisense Oligonucleotide

<400>  438
gtgtcacgtg tttagtttat                                                   20


<210>  439
<211>  20
<212>  DNA
<213>  Artificial Sequence

<220>
<223>  Phosphorothioate Antisense Oligonucleotide

<400>  439
gttccctttt gtgtgtgttt                                                   20


<210>  440
<211>  20
<212>  DNA
<213>  Artificial Sequence

<220>
<223>  Phosphorothioate Antisense Oligonucleotide

<400>  440
ttggaatgta ttagagctgg                                                   20


<210>  441
<211>  20
<212>  DNA
<213>  Artificial Sequence

<220>
<223>  Phosphorothioate Antisense Oligonucleotide

<400>  441
acagatgcat gctatacgag                                                   20


<210>  442
<211>  20
<212>  DNA
<213>  Artificial Sequence

<220>
<223>  Phosphorothioate Antisense Oligonucleotide

<400>  442
acttaataac tatagaataa                                                   20


<210>  443
<211>  20
<212>  DNA
<213>  Artificial Sequence

<220>
<223>  Phosphorothioate Antisense Oligonucleotide

<400>  443
atggctttac attttaaaga                                                   20


<210>  444
<211>  20

<212>  DNA
<213>  Artificial Sequence

<220>
<223>  Phosphorothioate Antisense Oligonucleotide

<400>  444
cagcagtatt attttccagc                                                    20


<210>  445
<211>  20
<212>  DNA
<213>  Artificial Sequence

<220>
<223>  Phosphorothioate Antisense Oligonucleotide

<400>  445
cagtaattct gtatgtatct                                                    20


<210>  446
<211>  20
<212>  DNA
<213>  Artificial Sequence

<220>
<223>  Phosphorothioate Antisense Oligonucleotide

<400>  446
ttaccaagtg taatcagtta                                                    20


<210>  447
<211>  20
<212>  DNA
<213>  Artificial Sequence

<220>
<223>  Phosphorothioate Antisense Oligonucleotide

<400>  447
tatgtttggc tttagtacaa                                                    20


<210>  448
<211>  20
<212>  DNA
<213>  Artificial Sequence

<220>
<223>  Phosphorothioate Antisense Oligonucleotide

<400>  448
   aaccttttta atagtatata                                                    20


<210>  449
<211>  20
<212>  DNA
<213>  Artificial Sequence

<220>
<223>  Phosphorothioate Antisense Oligonucleotide

<400>  449
atgcaccata aaattctgta                                                    20

<210>  450
<211>  20
<212>  DNA
<213>  Artificial Sequence

<220>
<223>  Phosphorothioate Antisense Oligonucleotide

<400>  450
aaaaagacaa tgcccacgta                                                    20


<210>  451
<211>  20
<212>  DNA
<213>  Artificial Sequence

<220>
<223>  Phosphorothioate Antisense Oligonucleotide

<400>  451
atctaaggat ttgggcatct                                                    20


<210>  452
<211>  20
<212>  DNA
<213>  Artificial Sequence

<220>
<223>  Phosphorothioate Antisense Oligonucleotide

<400>  452
aggaagggct aacatgccag                                                    20


<210>  453
<211>  20
<212>  DNA
<213>  Artificial Sequence

<220>
<223>  Phosphorothioate Antisense Oligonucleotide

<400>  453
tcatatcctc ttataattgg                                                    20


<210>  454
<211>  20
<212>  DNA
<213>  Artificial Sequence

<220>
<223>  Phosphorothioate Antisense Oligonucleotide

<400>  454
gttgcaagtg tttaagaaat                                                    20


<210>  455
<211>  20
<212>  DNA
<213>  Artificial Sequence

<220>
<223>  Phosphorothioate Antisense Oligonucleotide

<400>  455
ctggttaatg gttcacatct                                                    20


<210>  456
<211>  20
<212>  DNA
<213>  Artificial Sequence

<220>
<223>  Phosphorothioate Antisense Oligonucleotide

<400>  456
aaacatgctc acggctggtc                                                    20


<210>  457
<211>  20
<212>  DNA
<213>  Artificial Sequence

<220>
<223>  Phosphorothioate Antisense Oligonucleotide

<400>  457
ggtcctggcc aaggcatgag                                                    20


<210>  458
<211>  20
<212>  DNA
<213>  Artificial Sequence

<220>
<223>  Phosphorothioate Antisense Oligonucleotide

<400>  458
gtttggctta caggtcccct                                                    20


<210>  459
<211>  20
<212>  DNA
<213>  Artificial Sequence

<220>
<223>  Phosphorothioate Antisense Oligonucleotide

<400>  459
gctttacatt ttaaagaact                                                    20


<210>  460
<211>  20
<212>  DNA
<213>  Artificial Sequence

<220>
<223>  Phosphorothioate Antisense Oligonucleotide

<400>  460
tagaataaac agatgcatgc                                                    20


<210>  461
<211>  20
<212>  DNA
<213>  Artificial Sequence

<220>
<223>  Phosphorothioate Antisense Oligonucleotide

<400>  461
acactcagag tgaattatgt                                                    20


<210>  462
<211>  20
<212>  DNA
<213>  Artificial Sequence

<220>
<223>  Phosphorothioate Antisense Oligonucleotide

<400>  462
ctgtctgcaa cccaggttct                                                    20


<210>  463
<211>  20
<212>  DNA
<213>  Artificial Sequence

<220>
<223>  Phosphorothioate Antisense Oligonucleotide

<400>  463
ttgaccccaa aagaaggctt                                                    20


<210>  464
<211>  20
<212>  DNA
<213>  Artificial Sequence

<220>
<223>  Phosphorothioate Antisense Oligonucleotide

<400>  464
ctacgaataa tgatagcaga                                                    20


<210>  465
<211>  20
<212>  DNA
<213>  Artificial Sequence

<220>
<223>  Phosphorothioate Antisense Oligonucleotide

<400>  465
tgctctagaa aggacagcga                                                    20


<210>  466
<211>  20
<212>  DNA
<213>  Artificial Sequence

<220>
<223>  Phosphorothioate Antisense Oligonucleotide

<400>  466
atactatgtt gcctagatac                                                    20


<210>  467
<211>  20

<212>  DNA
<213>  Artificial Sequence

<220>
<223>  Phosphorothioate Antisense Oligonucleotide

<400>  467
caagattctt ggtacatatt                                                    20


<210>  468
<211>  20
<212>  DNA
<213>  Artificial Sequence

<220>
<223>  Phosphorothioate Antisense Oligonucleotide

<400>  468
gattctactt gggatctctt                                                    20


<210>  469
<211>  20
<212>  DNA
<213>  Artificial Sequence

<220>
<223>  Phosphorothioate Antisense Oligonucleotide

<400>  469
aatcagttac agtaattctg                                                    20


<210>  470
<211>  20
<212>  DNA
<213>  Artificial Sequence

<220>
<223>  Phosphorothioate Antisense Oligonucleotide

<400>  470
ggttcacatc tgaaaatagt                                                    20


<210>  471
<211>  20
<212>  DNA
<213>  Artificial Sequence

<220>
<223>  Phosphorothioate Antisense Oligonucleotide

<400>  471
ttccaaacat gctcacggct                                                    20


<210>  472
<211>  20
<212>  DNA
<213>  Artificial Sequence

<220>
<223>  Phosphorothioate Antisense Oligonucleotide

<400>  472
tgttgcctag atacagtggg                                                    20

<210>  473
<211>  20
<212>  DNA
<213>  Artificial Sequence

<220>
<223>  Phosphorothioate Antisense Oligonucleotide

<400>  473
agcacagcac agcactgacg                                                    20


<210>  474
<211>  20
<212>  DNA
<213>  Artificial Sequence

<220>
<223>  Phosphorothioate Antisense Oligonucleotide

<400>  474
tcaaagcata ggaaaactgt                                                    20


<210>  475
<211>  20
<212>  DNA
<213>  Artificial Sequence

<220>
<223>  Phosphorothioate Antisense Oligonucleotide

<400>  475
gactgctttc caataaggcc                                                    20


<210>  476
<211>  20
<212>  DNA
<213>  Artificial Sequence

<220>
<223>  Phosphorothioate Antisense Oligonucleotide

<400>  476
gaattatgta actgttgtca                                                    20


<210>  477
<211>  20
<212>  DNA
<213>  Artificial Sequence

<220>
<223>  Phosphorothioate Antisense Oligonucleotide

<400>  477
tcttatttgt aaacagcagt                                                    20


<210>  478
<211>  20
<212>  DNA
<213>  Artificial Sequence

<220>
<223>  Phosphorothioate Antisense Oligonucleotide

<400>  478
atgccagatc taaggatttg                                                    20


<210>  479
<211>  20
<212>  DNA
<213>  Artificial Sequence

<220>
<223>  Phosphorothioate Antisense Oligonucleotide

<400>  479
atggcatttt accttatctg                                                    20


<210>  480
<211>  20
<212>  DNA
<213>  Artificial Sequence

<220>
<223>  Phosphorothioate Antisense Oligonucleotide

<400>  480
ttgactagat ctggttaatg                                                    20


<210>  481
<211>  20
<212>  DNA
<213>  Artificial Sequence

<220>
<223>  Phosphorothioate Antisense Oligonucleotide

<400>  481
tcccaagcta aataacctac                                                    20


<210>  482
<211>  20
<212>  DNA
<213>  Artificial Sequence

<220>
<223>  Phosphorothioate Antisense Oligonucleotide

<400>  482
taaaacactc agagtgaatt                                                    20


<210>  483
<211>  20
<212>  DNA
<213>  Artificial Sequence

<220>
<223>  Phosphorothioate Antisense Oligonucleotide

<400>  483
agcaggatat ttcaagttca                                                    20


<210>  484
<211>  20
<212>  DNA
<213>  Artificial Sequence

<220>
<223>  Phosphorothioate Antisense Oligonucleotide

<400>  484
tttctatcaa gcaccgcctt                                                    20


<210>  485
<211>  20
<212>  DNA
<213>  Artificial Sequence

<220>
<223>  Phosphorothioate Antisense Oligonucleotide

<400>  485
tcactttaaa gaccttgtga                                                    20


<210>  486
<211>  20
<212>  DNA
<213>  Artificial Sequence

<220>
<223>  Phosphorothioate Antisense Oligonucleotide

<400>  486
tggctctggc ctgagatggg                                                    20


<210>  487
<211>  20
<212>  DNA
<213>  Artificial Sequence

<220>
<223>  Phosphorothioate Antisense Oligonucleotide

<400>  487
acattctgca taaattaata                                                    20


<210>  488
<211>  20
<212>  DNA
<213>  Artificial Sequence

<220>
<223>  Phosphorothioate Antisense Oligonucleotide

<400>  488
gcacacacct tagtttttca                                                    20


<210>  489
<211>  20
<212>  DNA
<213>  Artificial Sequence

<220>
<223>  Phosphorothioate Antisense Oligonucleotide

<400>  489
tcaagttcac taaaatacta                                                    20


<210>  490
<211>  20

<212>  DNA
<213>  Artificial Sequence

<220>
<223>  Phosphorothioate Antisense Oligonucleotide

<400>  490
ccacatctca taatatttag                                                    20


<210>  491
<211>  20
<212>  DNA
<213>  Artificial Sequence

<220>
<223>  Chimera Antisense Oligonucleotide

<400>  491
tgctgcaact gctgaacacc                                                    20


<210>  492
<211>  20
<212>  DNA
<213>  Artificial Sequence

<220>
<223>  Chimera Antisense Oligonucleotide

<400>  492
aagtccttcc tgctgcaact                                                    20


<210>  493
<211>  20
<212>  DNA
<213>  Artificial Sequence

<220>
<223>  Chimera Antisense Oligonucleotide

<400>  493
gctggtctcc aagtccttcc                                                    20


<210>  494
<211>  20
<212>  DNA
<213>  Artificial Sequence

<220>
<223>  Chimera Antisense Oligonucleotide

<400>  494
ctttctccag gctggtctcc                                                    20


<210>  495
<211>  20
<212>  DNA
<213>  Artificial Sequence

<220>
<223>  Chimera Antisense Oligonucleotide

<400>  495
acccccattc ctttctccag                                                    20

<210> 496
<211> 20
<212> DNA
<213> Artificial Sequence

<220>
<223> Chimera Antisense Oligonucleotide

<400> 496
aaacaggcac acccccattc 20

## Description

### TECHNICAL FIELD

This invention relates to a preventive or therapeutic agent for obesity or a disease caused by or related to obesity, and an antisense compound or an antibody for human meltrin α.

### BACKGROUND ART

Obesity is the state of excessive accumulation of adipose tissue, and this condition has a significant influence on the onset of lifestyle diseases such as diabetes, hypertension, hyperlipidemia, coronary artery disease, and ischemic brain disease. The need for active prevention of obesity has been pointed out in view of preventing such lifestyle diseases.
The drugs which are under development for obesity/adiposity can be roughly categorized into drugs which affects the action of the neurotransmitter of the central nervous system, drugs which suppress absorption from the digestive tract, drugs which promote thermogenesis, and the drugs which have the action of promoting lipolysis and inhibiting liposynthesis. Exemplary drugs which act on the central nervous system include adrenergic and noradrenergic agent, serotonergic agent, adrenergic and serotonergic agent, and central feeding-related peptides such as leptin. Exemplary drugs which suppress absorption include inhibitors for catabolic enzyme of lipid and sugar. A typical drug which promotes lipolysis and thermogenesis is a drug which stimulates β3 adrenaline receptor. However, most of such drugs have failed to exhibit sufficient clinical effect, and identification of a new drug target has become an urgent challenge.

For example, many researches have been conducted focusing on adipocyte, and in these researches, factors such as C/EBP, PPAR family, and UCP have been identified as factors playing central role in the proliferation and differentiation of the adipocyte and energy metabolism.

Also known is association of remodeling of extracellular matrix with the differentiation of adipocyte, and influence of matrix metalloprotease (abbreviated as MMP) on the adipocyte differentiation has been reported. The action, however, is different by the type of the MMP, and for example, adipocyte differentiation has been reported to be suppressed when MMP-2 and MMP-9 are inhibited by using an antibody (see Non-Patent Document 1). On the other hand, there are also reports that, in the knockout mice of MMP-3 or MMP-11, high fat diet resulted in the increase in body weight and adipose tissue weight compared to the wild mice (see Non-Patent Documents 2 and 3). As described above, much are yet to be found out about the effect of the MMP and the remodeling of the extracellular matrix on the adipocyte, and their relation with the obesity are ambiguous and far from clear.

Meltrin α (ADAM 12) is a protein which has been cloned by Sehara(one of the inventors of the present invention) et al. from a cDNA library of mouse myoblast as a substance which is related to the fusion and aggregation of muscle cells (see Non-Patent Document 4). Since it has a structure including disintegrin domain and metalloprotease domain within its molecule, meltrin α has been classified to be a member of ADAM (a disintegrin and metalloprotease) family.
Recently, Kawaguchi et al. reported emergence of fat in skeletal muscle in the transgenic mouse of human meltrin α (see Non-Patent Document 5). According to Kawaguchi et al., of the mice exhibiting excessive expression of meltrin α, 1 year old female mice exhibited increase in the body weight and significant increase in the total body fat mass. Since this effect was not observed in the transgenic mouse of the meltrin lacking the metalloprotease domain and the prodomain of the meltrin α, Kawaguchi et al. point out that adipogenesis is induced by protease site of the meltrin α. However, such increases in the body weight and in the fat mass in a transgenic mouse were not clearly observed in young female animals or male animals, and there is a description that no change was observed in these animals for serum insulin, cholesterol, or triglycerides. Accordingly, it is yet to be found out whether the meltrin is essentially related to the increase in the body weight and the fat mass.
Kawaguchi et al. also report that excessive expression of the meltrin α in preadipocyte 3T3-L1 cell results in the disappearance of actin stress network in the cell, and this causes the shape of the cell to become more rounded. On the other hand, when an siRNA for the meltrin α is introduced in 3T3-L1 cell, change in the cell shape does not occur even if differentiation were induced, and the stress fibers are also maintained. Kawaguchi et al., therefore, point out that meltrin α is related to the cytoskeleton reorganization, which in turn relates to differentiation and maturation of the preadipocyte and mesenchymal precursor cell (see Non-Patent Document 6).

In the meanwhile, the inventors of the present invention have reported the results obtained by analyzing meltrin α knockout mice (see Non-Patent Document 7). In the case of meltrin α knockout mice, 30% of the pups born died at an early stage, and in such animals, impaired formation of the brown adipose tissue as well as impaired formation of interscapular muscles were noted. While these observations suggested the possibility that meltrin α may be involved in the regulation of adipogenesis and myogenesis, generation of both brown adipose tissue and white adipose tissue was comparable to wild-type in adult mice despite the absence of the meltrin α expression, and failure in the adipogenesis was also absent. These findings are not consistent with the findings of Kawaguchi et al. obtained by using the siRNA.
As described above, while involvement of the meltrin α in the adipogenesis at least at some stage of the development has been indicated, role of the meltrin α in the adipogenesis in the adult mice is not yet found, and its relation with the increase in the body weight, accumulation of the fat, and the obesity is not at all clear.

As described above, in spite of the progress in elucidating factors of the obesity such as adipogenesis, adipose accumulation, and energy metabolism, the factors essentially modulating such factors are far from being clear. In addition, it is not clear whether the substance whose association has been indicated with the adipogenesis and the adipose accumulation can modulate such adipogenesis and adipose accumulation in vivo, and in particular, under a pathological stress, and light should be cast on the finding of factors that can serve as the target molecule of an antiobesity drug.
NON-PATENT DOCUMENT 1: Anne, B. et al., Diabetes, 2001, 50, pp. 2080-2086.
NON-PATENT DOCUMENT 2: Lijnen, H.R. et al., Thromb. Heamost., 2002, 87, pp. 530-535.
NON-PATENT DOCUMENT 3: Erik, M. et al., Thromb. Heamost., 2003, 89, pp. 696-704.
NON-PATENT DOCUMENT 4: Nature, 1995, Vol.377, No. 19, pp. 652-656 .
NON-PATENT DOCUMENT 5: Am. J. Pathol., 1985, 160(5).
NON-PATENT DOCUMENT 6: J. Cell Science, 2003, 116(19), 3893-3904.
NON-PATENT DOCUMENT 7: Kurisaki, T. et al., Mol. Cell Biol., 2003, 23, pp. 55-61.

### DISCLOSURE OF THE INVENTION

### PROBLEMS TO BE SOLVED BY THE INVENTION

Accordingly, an object of the present invention is to identify a novel target factor which is associated with the obesity, and another object of the present invention is to provide a novel preventive or therapeutic agent for the obesity, a novel human meltrin α antisense compound, and a novel anti-human meltrin α antibody.

### MEANS TO SOLVE THE PROBLEMS

The inventors of the present invention have made extensive study in order to find a novel target factor associated with the obesity to thereby develop a preventive or therapeutic agent for the obesity. Based on the premise that factors modulating the adipogenesis and the adipose accumulation and the role of such factors may be different between the adipogenesis in normal condition and the adipose accumulation under the conditions of so-called pathological stress, the inventors of the present invention conducted the investigation by focusing on the function of physiologically active protein found when the animals are fed a high fat diet. Surprisingly, it was then found that the meltrin α knockout mice which showed no significant difference with the wild-type mice in the case of adult mice show characters markedly different from wild-type mice under the conditions of high fat diet. More specifically, while the wild-type mouse exhibits body weight increase and adipose accumulation, namely, the conditions of obesity by the high fat diet, such obesity conditions were not observed in the meltrin α knockout mice. Moreover, the meltrin α knockout mice exhibited enhancement of sugar and lipid metabolism as well as improvement of lipid level in the blood. Furthermore, while adipose accumulation in liver, namely, the fatty liver was noted in the wild-type mice on the high fat diet, no such tendency was noted in the meltrin α knockout mice. In view of the fact that obesity is a cause of the lifestyle diseases such as diabetes and hyperlipidemia, the phenomena observed in the meltrin α knockout mice indicate that not only obesity, but also conditions of lifestyle disease associated with the obesity may be improved or prevented by the inhibition of the meltrin α. In addition, no particular harmful condition or abnormal observation compared to the wild-type mice was found in the meltrin α knockout mice. Based on these findings, the inventors of the present invention found that a drug which inhibits meltrin α will be usable to a safe preventive or therapeutic agent for the obesity. The present invention has been completed on such a finding.

Accordingly, the first aspect of the present invention is a preventive or therapeutic agent for obesity, adiposity, or a disease caused by or related to obesity, which contains meltrin α antagonist.
The second aspect of the present invention is a novel antisense compound for human meltrin α.
The third aspect of the present invention is an anti-human meltrin α antibody, its active fragment, or a derivative thereof which exhibits inhibitory activity for proliferation and differentiation of the adipocyte in an assay system of adipocyte proliferation and differentiation.

### EFFECTS OF THE INVENTION

The present invention provides a novel preventive or therapeutic agent for obesity, adiposity, or a disease caused by or related to obesity. A novel antisense compound for meltrin α and a novel anti-meltrin α antibody which can be used as an effective component in the preventive or therapeutic agent are also provided. Such preventive or therapeutic agent is capable of not only suppressing the increase in the body weight and the fat mass associated with the obesity, but also capable of enhancing energy metabolism, suppressing visceral fat, and generally preventing or treating diseases such as hyperlipidemia.

### BRIEF DESCRIPTION OF THE DRAWINGS

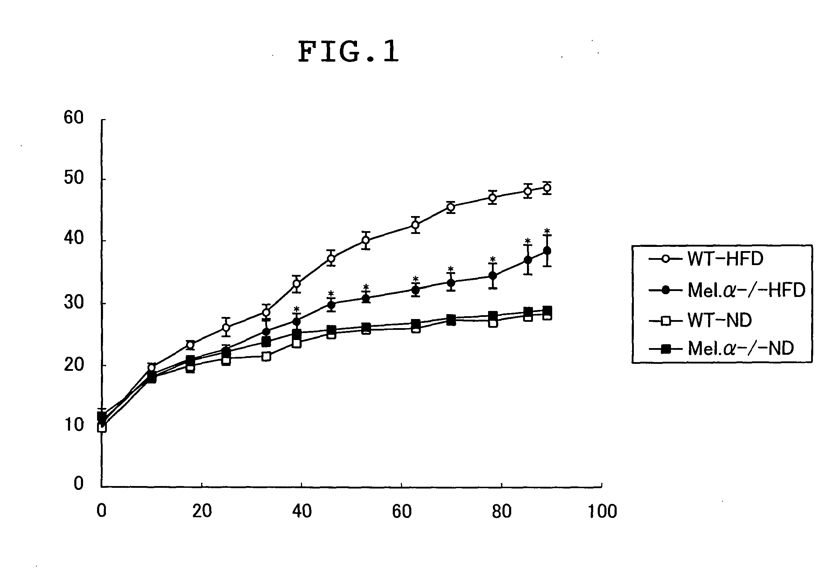

[FIG. 1] FIG. 1 shows change in body weight of wild-type mice and meltrin α knockout mice during investigation of action of the meltrin α on the high fat diet. Y axis indicates the body weight (g), and the x axis indicates the number of days of breeding. In FIG. 1, WT represents wild-type mice, Melα-/- represents meltrin α knockout mice, HFD represents high fat diet, and ND represents low fat diet.

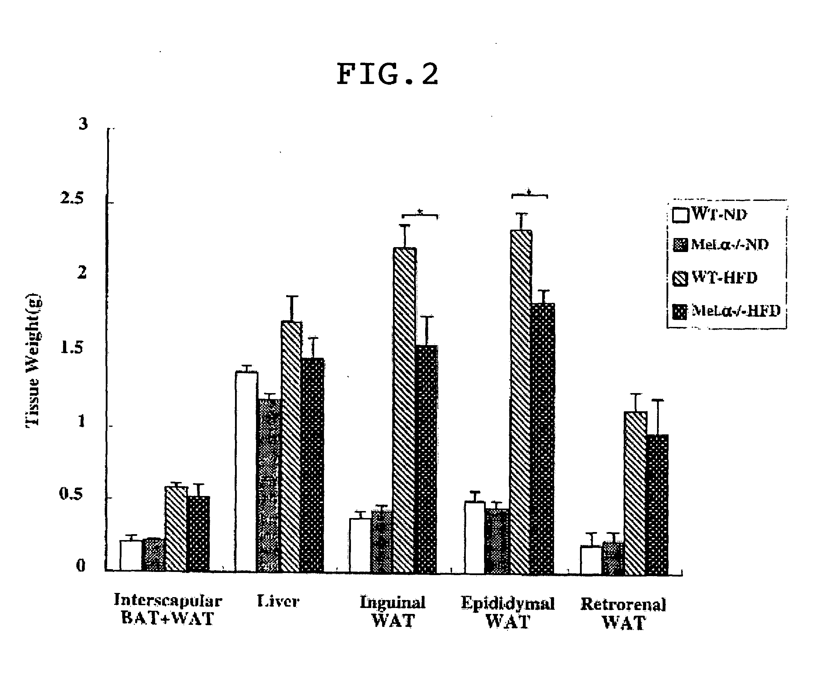

[FIG. 2] FIG. 2 shows weight of various organs in wild-type mice and meltrin α knockout mice in the investigation of effects of the meltrin α on the weight of the adipose tissues and liver. In FIG. 2, WT represents wild-type mice, Melα-/- represents meltrin α knockout mice, HFD represents high fat diet, and ND represents low fat diet.

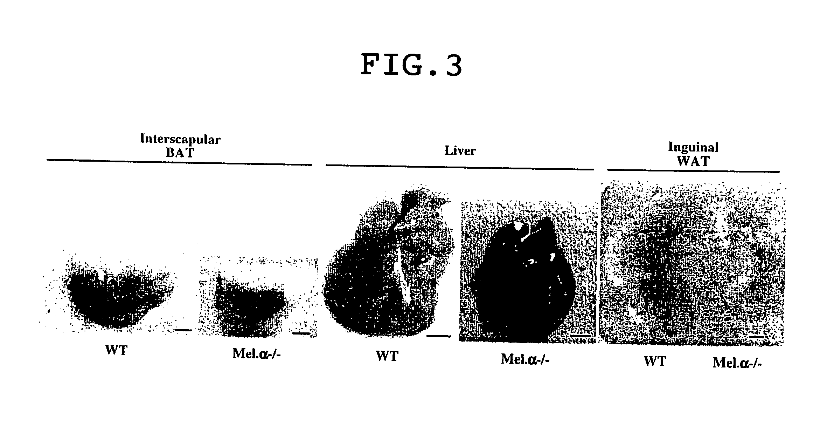

[FIG. 3] FIG. 3 shows images of adipose tissues and liver tissue in wild-type mice and meltrin α knockout mice in the investigation of the action of meltrin α on the adipose tissues and the liver when the mouse is under the high fat diet stress. In FIG. 3, WT represents wild-type mice, and Melα-/- represents meltrin α knockout mice.

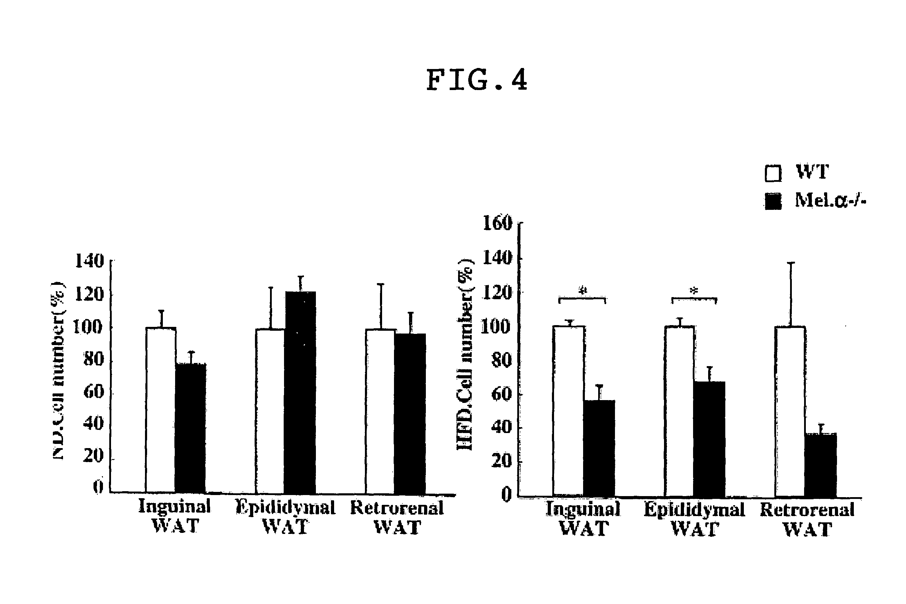

[FIG. 4] FIG. 4 shows the effects of meltrin α on the number of adipocytes, and more specifically, shows the number of adipocytes in the adipose tissue of meltrin α knockout mice in relation to the number of adipocytes in wild-type mice which is shown as 100%. In FIG. 4, WT represents wild-type mice, Melα-/- represents meltrin α knockout mice, HFD represents high fat diet, and ND represents low fat diet.

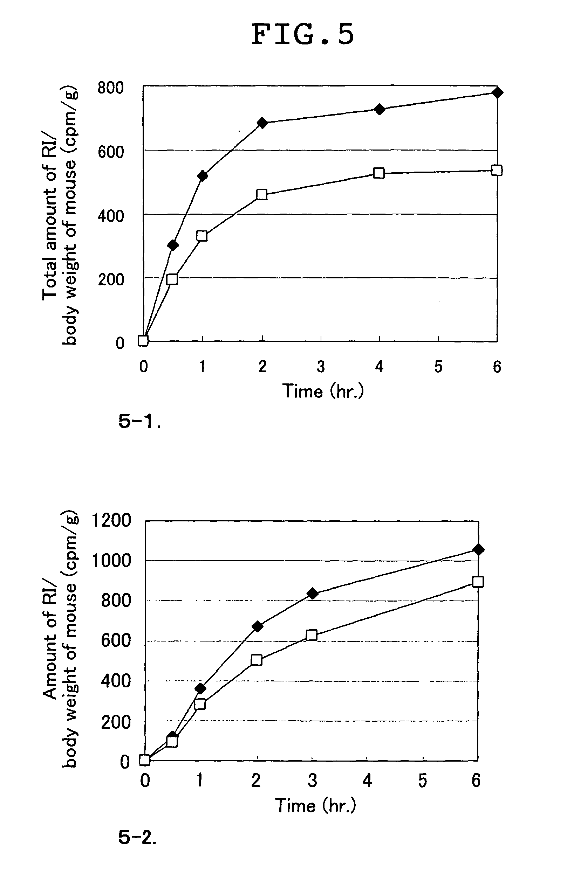

[FIG. 5] FIG. 5 shows glucose and lipid metabolism in meltrin α knockout mice and wild-type mice after feeding on a high fat diet in terms of transfer of the administered RI-labeled agent to the exhalation. FIG. 5-1 shows amount of RI in the exhalation after administering ¹⁴C palmitic acid, and FIG. 5-2 shows amount of RI in the exhalation after administering ¹⁴C glucose. In FIG. 5, □ represents the wild-type mice, and ◆ represents the meltrin knockout mice.

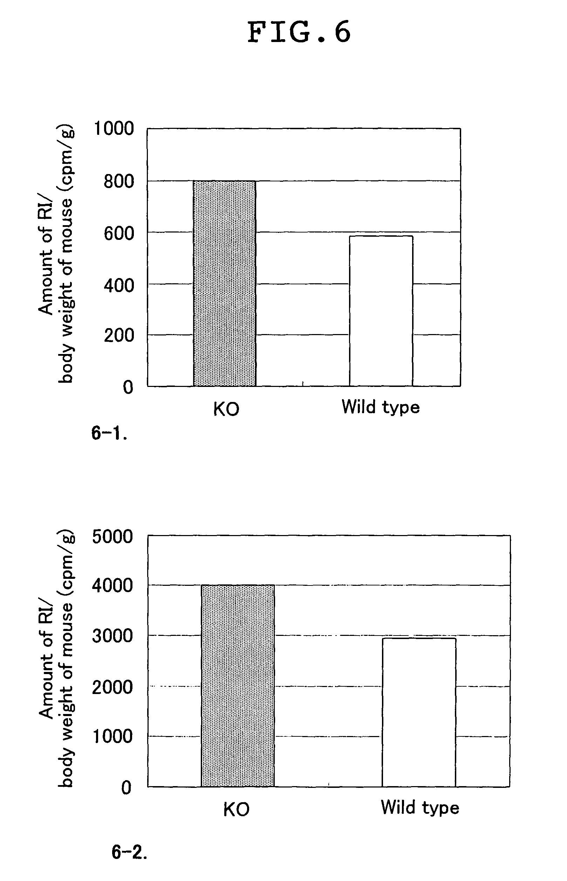

[FIG. 6] FIG. 6 shows excretion of the RI-labeled agent that had been administered to the meltrin α knockout mice and the wild-type mice after feeding on a high fat diet. FIG. 6-1 shows amount of the RI in feces after administering ¹⁴C palmitic acid, and FIG. 6-2 shows amount of the RI in urine after administering ¹⁴C glucose.

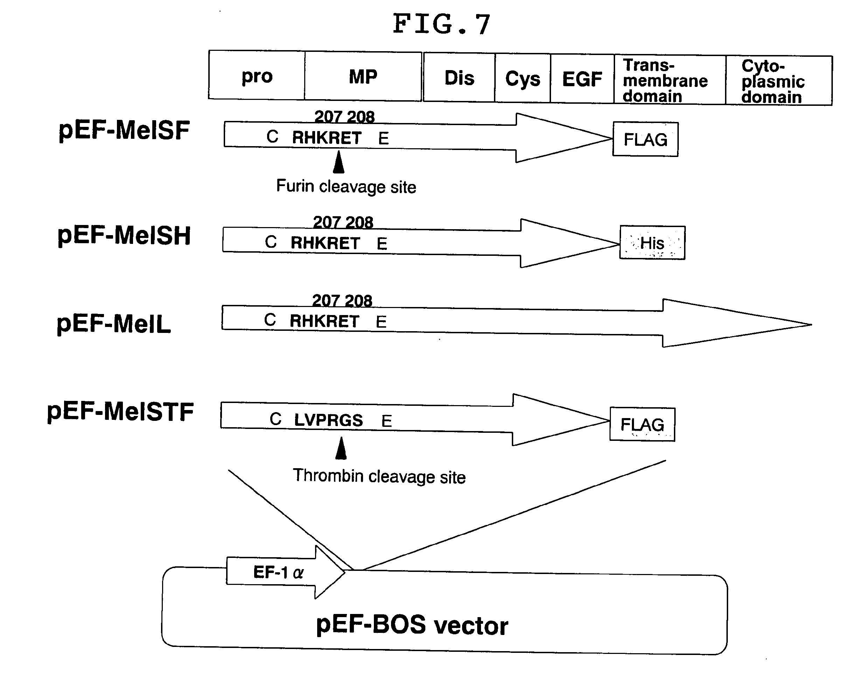

[FIG. 7] FIG. 7 is a diagram showing construction of a plasmid expressing the meltrin α.

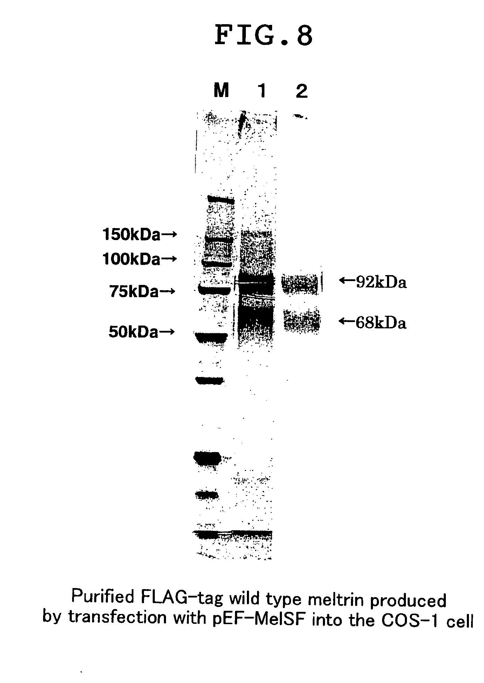

[FIG. 8] FIG. 8 shows silver staining image and western blotting image of purified meltrin α-FLAG produced by introducing pEF-MelSF in COS-1 cell.
   In FIG. 8, M represents silver stained molecular weight marker. Lane 1 was subjected to silver staining, and Lane 2 was subjected to western analysis using anti-FLAG antibody. 92 kDa corresponds to full length meltrin α, and 68 kDa corresponds to meltrin α from which the prodomain has been deleted.
[FIG. 9] FIG. 9 is a diagram showing expression plasmids for various domains of the meltrin α.
   Structure of the constructed domains of the meltrin α is shown. The meltrin α proteins expressed are schematically shown next to the names of the plasmids.
[FIG. 10] FIG. 10 shows western blotting image of the proteins of various domains stained with anti-FLAG antibody. The image shows expression of different domains of meltrin α.
   Lane 1: Pro domain, Lane 2: Pro-MP domain, Lane 3: MP domain, Lane 4: Dis domain, and Lane 5: Cys-rich domain; Pro: prosequence, MP: metalloprotease, Dis: disintegrin, and Cys: cysteine.
[FIG. 11] FIG. 11 shows western blotting image obtained by digesting recombinant human IGF-BP3 with meltrin α. The image shows activity of the anti-human meltrin α antibodies for inhibiting metalloprotease activity of the meltrin α.
   Lane 1: no antibody, Lane 2: antibody No. 20, Lane 3: antibody No. 21, Lane 4: antibody No. 107, and Lane 5: antibody No. 129. The antibodies were used at 300 µg/mL each.
[FIG. 12] FIG. 12 shows western blotting image obtained by digesting recombinant human IGF-BP3 with meltrin α. The image shows activity of the low molecular weight compounds for inhibiting metalloprotease activity of the meltrin α.
   Lane 1: no sample, 2: IGF-BP3, 3: IGF-BP3 + meltrin having Pro domain, 4: IGF-BP3 + active meltrin, and 5: 4 + 10 µg/mL of M82463.
[FIG. 13] FIG. 13 is a diagram showing effect of meltrin α siRNA in suppression of the expression of the meltrin α in adipocyte.
   The value of the group with no administration of the siRNA is shown as 100%.
[FIG. 14] FIG. 14 is a diagram showing effect of antisense of the meltrin α in suppression of the expression of the meltrin α in adipocyte.
   The value of the group having the sense sequence DNA introduced is shown as 100%.
[FIG. 15] FIG. 15 is a diagram showing suppression of the adipocyte proliferation and differentiation by M82463 in terms of fluorescence intensity of the adipocyte stained by AdioRed.
[FIG. 16] FIG. 16 is a diagram showing suppression of the adipocyte proliferation and differentiation by antisense oligonucleotide of the meltrin α in terms of fluorescence intensity of the adipocyte stained by AdioRed.
[FIG. 17] FIG. 17 is a diagram showing suppression of the adipocyte proliferation and differentiation by siRNA of the meltrin α in terms of fluorescence intensity of the adipocyte stained by AdioRed.
[FIG. 18] FIG. 18 shows body weight of the mice fed a high fat diet. The body weight of wild-type mice, meltrin α heterozygous mice, and meltrin α knockout mice was measured after 24 weeks on high fat diet.
[FIG. 19] FIG. 19 shows leptin concentration of the mice fed a high fat diet. The leptin concentration was measured by ELISA for the serum separated from the blood which had been collected from wild-type mice, meltrin α heterozygous mice, and meltrin α knockout mice after 24 weeks on high fat diet.
[FIG. 20] FIG. 20 shows insulin concentration of the mice fed a high fat diet. The insulin concentration was measured by ELISA for the serum separated from the blood which had been collected from wild-type mice, meltrin α heterozygous mice, and meltrin α knockout mice after 24 weeks on high fat diet.
[FIG. 21] FIG. 21 shows blood glucose level of the mice fed a high fat diet. The glucose level was measured for the serum separated from the blood which had been collected from wild-type mice, meltrin α heterozygous mice, and meltrin α knockout mice after 24 weeks on high fat diet.

### BEST MODE FOR CARRYING OUT THE INVENTION

The preventive or therapeutic agent according to the first aspect of the present invention has the characteristic feature that it contains a meltrin α antagonist.
The "meltrin α antagonist" used herein is a substance which inhibits human meltrin α, and the modes of the inhibition include inhibition of the expression of the meltrin α and inhibition of the activity of the meltrin α. The "inhibition of the expression of the meltrin α" means inhibition of production of the meltrin α protein in the meltrin producing cell in the living body, and preferably, supression of the level of the transcription to mRNA of the meltrin α. The "inhibition of the activity of the meltrin α" means suppression of the increase in the body weight in animals on a high fat diet, and preferably, realization of at least one action selected from suppression of increase in the fat mass, enhancement of sugar and lipid metabolism, decrease of blood cholesterol value, and suppression of fatty liver formation. As will be described in the Examples, the inventors of the present invention confirmed that the inhibition of the meltrin α results in the suppression of the proliferation of the adipocyte and suppression of the neutral fat intake into the adipocyte after differentiation stimulation, and therefore, the substance which suppresses the meltrin α activity may also be a substance having such a suppressive activity. The inventors of the present invention will also disclose the method of assaying such inhibitory actions in the Examples, and the substance which suppresses the meltrin α activity can be evaluated by using such system. The system used for evaluation, however, is not limited to the one disclosed in the Examples.

The degree of the suppression of the expression or the activity by the meltrin α antagonist is not necessarily needed to be 100%, even though higher degree of suppression near 100% is expected to realize a higher effect, and any degree of suppression is acceptable as long as the expression or the activity is suppressed distinctively as compared with the control group. However, a substance which suppresses the expression or the activity of the meltrin to a degree of at least 50%, and preferably at least 70% particularly in an in vitro assay system is desirable as an effective component in the preventive or therapeutic agent of the present invention.
Exemplary antagonists which suppress the expression of the meltrin α include antisense compounds and siRNAs for the meltrin α, and exemplary substances which suppress the activity of the meltrin α include antibodies and low molecular weight compounds against the meltrin α. Each of these substances will be described later in further detail.

The preventive or therapeutic agent of the present invention is administered to the patient at a dose of 0.1 mg/kg/day to 100 mg/kg/day in terms of the amount of the effective component when the effective component is an antisense compound or siRNA; 0.1 mg/kg/day to 100 mg/kg/day in terms of the amount of the effective component when the effective component is an antibody; and 0.1 mg/kg/day to 20 mg/kg/day in terms of the amount of the effective component when the effective component is a low molecular weight compound.
With regard to the dose and route of the administration as well as the frequency and interval of the administration, the most suitable method can be adequately selected by taking conditions, age, sex and body weight of the patientas well as complications, if any, into consideration. In the cases of the antisense compound, the siRNA, and the antibody, the agent may be administered to the patient once or twice daily for 2 to 15 weeks, or alternatively, a daily dose may be administered every one or two weeks for 2 to 15 weeks. In the case of the low molecular weight compound, the agent may be administered to the patient once or twice daily.

The preventive or therapeutic agent of the present invention is administered to a patient suffering from obesity, adiposity, or a disease caused by or related to the obesity. An index used for the obesity is value of BMI. For example, WHO and NIH (U.S.) take a BMI value of 30 or more as the indication of obesity while, in Japan, a value of 25 or more is to be considered as the indication. The definition of the obesity varies with the race and dietary habits. According to the definition of Japan Society for the Study of Obesity, "adiposity is a pathological condition which is associated with, or expected to be associated with a complication of health problem caused by or related to the obesity, and which requires weight loss in medical point of view, and this condition is regarded as a disease entity". More illustratively, there is a stipulation that adiposity is to be diagnosed in the case of obesity with the BMI being 25 or higher when either of 1) suffering from a health problem caused by or related to the obesity and requiring weight loss, and 2) suffering from visceral fat-type obesity with definite diagnosis by CT after being suspected of upper body obesity is the case (Obesity Research, vol. 6, pages 18-28, 2000; Adiposcience, vol. 1, pages 56-61, 2004). In view of such situation, the term "obesity" used in the present invention includes the typical case of the patient having the BMI of 25 or higher, and also, the case in which the patient him- or herself or the health professional thinks that reduction of the visceral fat or body weight is desirable.

The disease caused by or related to the obesity is a disease which results from the obesity as one of the causes, and exemplary such diseases include diabetes, in particular, type 2 diabetes or impared glucose tolerance, lipid metabolism abnormality, fatty liver, hyperlipidemia, hypertension, hyperuricemia or gout, ischemic heart disease (for example, coronary artery disease such as myocardial infarction or angina), cerebrovascular disorder (for example, cerebral infarction, cerebral thrombosis, or transient ischemic attack), arteriosclerosis, sleep apnea syndrome, Pickwickian syndrome, orthopedic disease such as osteoarthritis or lumbar disorder, and menstrual disorder. While the patients are often diagnosed in the practice of clinical medicine with the particular name of the disease under clear criteria, the disease caused by or related to the obesity also includes the case in which at least one of the glucose level, lipid level, and cholesterol level of the blood exceeds the normal range, and the case in which a lesion is recognized in an organ or blood vessel by at least one of the diagnostic imaging using CT, echo, and the like, biopsy, and the like with a simultaneous suspicion of the diabetes, fatty liver, hyperlipidemia, ischemic heart disease, cerebrovascular disorder, arteriosclerosis, or the like.
The preventive or therapeutic agent of the present invention can be used for treating the obesity or the obesity-related diseases, and also, for preventive purposes when a health professional has judged generally from dietary habit, lifestyle, hereditary predisposition, and the like that there is a high probability of such a disease. The use of the preventive or therapeutic agent of the present invention is not limited to medical purposes, and it may be used by incorporating in a health food or functional food aiming at prevention and treatment of the obesity or the obesity-related diseases.
The method for using the preventive or therapeutic agent of the present invention will be described later in further detail.

The present invention also provides a preventive or therapeutic agent for diabetes (for example, type 1 diabetes, type 2 diabetes, or pregnancy diabetes), a preventive or therapeutic agent for impaired glucose tolerance (IGT), an agent for promoting secretion of insulin, an agent for suppressing transfer from impared glucose tolerance to diabetes, and an agent for improving glucose metabolism, all of which contain a meltrin α antagonist.
In accordance with the new criteria of diabetes reported by Japanese Diabetes Society in 1999, diabetes is defined to be a condition showing any one of a fasting blood glucose level (glucose concentration in venous plasma) of 126 mg/dl or higher, a 75 g oral glucose tolerance test (75 g OGTT) two-hour value (glucose concentration in venous plasma) of 200 mg/dl or higher, and a casual blood glucose level (glucose concentration in venous plasma) of 200 mg/dl or higher. The condition which does not fit the criteria as described above and is not the "condition showing a fasting blood glucose level (glucose concentration in venous plasma) of less than 110 mg/dl or a 75 g oral glucose tolerance test (75 g OGTT) two-hour value (glucose concentration in venous plasma) of less than 140 mg/dl" (namely, the normal condition) is called "borderline type". New criteria for the diabetes have also been reported by ADA (American Diabetes Association) in 1997 and by WHO in 1998. According to these reports, diabetes is the condition in which the fasting blood glucose level (glucose concentration in venous plasma) is 126 mg/dl or higher, and the 75 g oral glucose tolerance test two-hour value (glucose concentration in venous plasma) is 200 mg/dl or higher.

According to these reports, impaired glucose tolerance is a condition in which the fasting blood glucose level (glucose concentration in venous plasma) is less than 126 mg/dl and the 75 g oral glucose tolerance test two-hour value (glucose concentration in venous plasma) is 140 mg/dl or more but less than 200 mg/dl. In addition, according to the report of the ADA, the condition showing a fasting blood glucose level (glucose concentration in venous plasma) of 110 mg/dl or more but less than 126 mg/dl is called IFG (Impaired Fasting Glucose). In the meanwhile, according to the report of the WHO, among the IFG (Impaired Fasting Glucose) conditions, the condition showing a 75 g oral glucose tolerance test two-hour value (glucose concentration in venous plasma) of less than 140 mg/dl is called IFG (Impaired Fasting Glycemia).
The preventive or therapeutic agent of the present invention (meltrin α antagonist) may also be used as a preventive or therapeutic agent for diabetes, borderline type, impaired glucose tolerance, IFG (Impaired Fasting Glucose), and IFG (Impaired Fasting Glycemia) as determined on the new criteria as described above. The preventive or therapeutic agent of the present invention is also capable of preventing progress of borderline type, impaired glucose tolerance, IFG (Impaired Fasting Glucose), or IFG (Impaired Fasting Glycemia) to the diabetes.

The preventive or therapeutic agent of the present invention may also be used for preventing or treating, for example, diabetic complications [such as neuropathy, nephropathy, retinopathy, cataract, macroangiopathy, osteopenia, diabetic hyperosmotic coma, infections (such as respiratory infection, urinary tract infection, digestive infection, skin soft tissue infection, and inferior limb infection), diabetic gangrene, xerostomia, impaired auditory sense, cerebrovascular disorder, and disruption of peripheral blood circulation], diabetic cachexia, renal diseases (for example, diabetic nephropathy, glomerulonephritis, glomerulosclerosis, nephrotic syndrome, hypertensive nephrosclerosis, and terminal renal disease), insulin resistant syndrome, syndrome X, metabolic syndrome (a condition having at least one of type 2 diabetes, impaired glucose tolerance, and insulin resistance; and also having at least two of obesity, lipid metabolism abnormality, hypertension, and microalbuminuria), Cushing's syndrome, hyperinsulinemia, and sensory disturbance in hyperinsulinemia. The preventive or therapeutic agent of the present invention is also used for secondary prevention of the diseases as described above and for suppressing the progress thereof.
The anti-diabetic action of the preventive or therapeutic agent of the present invention can be confirmed by using an appropriate disease animal model, for example, a spontaneous diabetes model, including a spontaneous type 2 diabetes mouse model such as db/db mouse, ob/ob mouse, or KK-Ay mouse, a mouse or rat having drug-induced diabetes, or a mouse or rat having diabetes induced by a special feed (high fat diet, high sugar diet, or the like) to measure the blood glucose level, serum insulin, and the like, or test the glucose tolerance by fasting the animal overnight, applying oral glucose load to the animal, and then measuring the blood glucose level over time. Such test may be conducted, for example, by referring to the method described in Endocrinology 144(11), 4755-4762, 2003.
The method for using the preventive or therapeutic agent of the present invention will be described later in further detail.

### (Preventive or therapeutic agent containing meltrin α antisense compound or modified product thereof as its effective component)

### (1) Antisense compound

The antisense compound which is preferable for use as the effective component of the preventive or therapeutic agent of the present invention is the one which binds at least to the gene, namely, the DNA or the RNA, more specifically, the DNA or the RNA transcribed from the DNA, and in particular, the mRNA coding for the human meltrin α, and suppresses its expression, transcription, translation, or the like. Typically, the antisense compound is produced so that it may target 5' terminal hairpin loop, 5' terminal 6-base pair repeat, 5' terminal untranslated region, polypeptide translation initiation codon, protein coding region, ORF translation termination codon, 3' terminal untranslated region, 3' terminal palindrome region, or 3' terminal hairpin loop of the target gene, and have a nucleotide sequence which is complementary to the target sequence. More specifically, a site which serves as such target is selected from the sequences of human meltrin α shown in SEQ ID NOS: 1 and 3, and a compound is chemically synthesized by the method commonly used in the art to have a nucleotide sequence complementary to the selected site. Alternatively, the target may be the part extending over the intron and the exon in the sequence of the region coding for the meltrin α in the genomic DNA containing the meltrin α gene registered in sequence database provided by NCBI under the registration number NT_035040.

The antisense compound against the human meltrin α is not limited in length or binding site as long as the compound binds to the nucleic acid coding for the human meltrin α to thereby suppress its expression, transcription, or translation. The antisense compound, however, preferably comprises an oligonucleotide comprising 10 to 40 nucleotides, and, more preferably, the antisense compound has a length of 15 to 30 nucleotides, even more preferably a length of 18 to 25 nucleotides, since an excessively long compound is inadequate for intake in the cell. Since 5' upstream region of the DNA coding for the human meltrin α is relatively short, it is preferable to use a region other than such region for the target in view of enabling the selection of a more suitable molecule with a greater variation in the specificity, the binding ability, and the expression suppressing ability. In view of the merit of confirming the effect not only in the human but also in other animals such as mouse or rat, the target used is preferably a region where a high homology is found between the human and such animals. Exemplary such region is the region of 1 to 2420 in SEQ ID NOS: 1 and 3, and an antisense compound comprising 10 to 40 nucleotides which has a complementary sequence may be chemically synthesized from such region.

The antisense compound incorporated in the preventive or therapeutic agent of the present invention may be the one which suppresses the expression of transmembrane human meltrin α, or the one which suppresses the expression of soluble human meltrin α, and may be prepared by using any sequence coding for the human meltrin α as the target. However, in view of efficiently inhibiting the action of the meltrin in the living body, the antisense compound is preferably the one which inhibits the expression of both the transmembrane meltrin α and the soluble meltrin α. Such antisense compound may be prepared by using as the target a sequence which both the transmembrane meltrin α and the soluble meltrin α have in the downstream of the initiation codon including the 5' upstream untranslated region.

More specifically, the antisense compound of the second aspect of the present invention can be used for the effective component of the preventive or therapeutic agent of the present invention, and exemplary antisense compounds include the one comprising or containing the nucleotide sequence of Table 6 or Table 8. The antisense compound may also be the one comprising a known sequence such as the one comprising or containing the nucleotide sequence of Table 7.
Among these, the one exhibiting a high suppression rate (score) is more useful. The activity of the antisense compound to suppress the expression of the meltrin α may be confirmed by measuring the amount of meltrin α protein in the cell extract or the culture medium of the cell having the antisense compound introduced therein by western blotting or the like.

The antisense compound may be oligonucleotide containing 2'-deoxy-_{D}-ribose, oligonucleotide containing _{D}-ribose, or other type of oligonucleotide which is N-glycoside of purine or pyridine base, or other polymer containing non-nucleotide backbone. The antisense compound may be unmodified oligonucleotide or modified oligonucleotide.

Examples of the modified oligonucleotide include the most commonly used nuclease resistant phosphorothioated derivatives; and also, hexitol nucleic acids (HNAs) as pyranose ring conversion bodies, morpholino DNA, peptide nucleic acids (PNAs), 3'-amino-DNA, 2',4'-ethylene bridged nucleic acid (ENA), and 2',4'-methylene bridged nucleic acid (2',4'-BNA), each of which is nuclease resistant and has a high affinity for RNA. Other examples include capped oligonucleotide; methylated oligonucleotide; the one containing methylphosphonate, phosphotriester, phosphoramidate, or carbamate in the oligonucleotide; the one containing phosphorothioate or phosphorodithioate in the oligonucleotide; and the one containing a polypeptide such as poly-_{L}-lysine, sugar, intercalation compound (for example, acridine or psoralen), chelete compound, or alkylating agent.

Such modified nucleic acids are capable of providing an antisense compound with a high nuclease resistance and a high affinity for the target RNA. More illustratively, the effectiveness of the antisense compound which acts by complementarily hybridizing to the nucleotide sequence of the particular site in the target RNA to inhibit translation to the protein or to digest the target RNA in an RNaseH-dependent manner has been dependent largely on how a sequence complementary to the target RNA sequence which is capable of undergoing specific and effective hybridizing could be selected. For example, to accomplish an effective design of the antisense compound, the loop which is likely to take single stranded structure should be selected and the selection of stem structure which is the part with a double stranded structure should be avoided.

However, accurate estimation of the multi-dimensional structure of the RNA is difficult, and therefore, a more effective antisense compound having a sequence not limited to particular sequence can be designed by providing the antisense compound with a hybridizing ability higher than that of the double stranded RNA. For this reason, modified nucleic acids with a higher affinity for RNA have been developed. Examples of those developed at an earlier stage include PNA, which has a flexibility in binding to the target RNA.

Recently, there is a demand for chemically modified compounds having a more rigid structure, for example, bridged antisense compounds (Protein, Nucleic Acid and Enzyme, vol. 48, 1616-1624, 2003). In such modified antisense compounds, the binding ability to the ssRNA or the dsDNA can be improved by fixing the puckering mode of the furanose ring of the sugar part of the nucleic acid to N type conformation, so that the compounds are not only used as an antisense compound but their use for a ribozyme, antigene, RNA interference (RNAi), and decoy nucleic acid method is under study. Typical examples of such bridged antisense compounds include 2',4'-ethylene bridged nucleic acid (ENA), 2',4'-methylene bridged nucleic acid (2',4'-BNA), and 3'-amino-2',3'-BNP, and such modified oligonucleotides have a high sequence recognition specificity and a sufficient nuclease resistance despite a very high hybridizing ability to the complimentary RNA chain.

In addition to the fixing of the conformation of the franose ring, the degree of conformational freedom of the sugar part of the nucleic acid may be restricted by the use of a 2'-O-alkyl nucleotide [(containing approximately 1 to 4 carbon atoms, for example, -CH₃, -CH₂CH₂OCH₃ (2'-O-methoxyethyl, MOE), or -CH₂CH₂CH₂NH₂ (AP)), a 2'-F substitution product, or a 3'-NH₂ substitution product] having the chemically modified 2'-OH group of the RNA or the chemically modified 3'-OH group of the DNA.

Of the modified oligonucleotides having high hybridizing ability and nuclease resistance, many have lost their ability to recognize RNaseH which is important to the expression of the action of an antisense compound.
However, it is known that by using a structure called "gapmer", such modifications as above can be easily introduced while retaining the RNaseH dependent action mechanism. More specifically, an antisense compound having high hybridizing ability and nuclease resistance which is capable of operating by the RNaseH dependent action mechanism can be obtained by designing an antisense compound using phosphorothioate or unmodified nucleic acid which can serve as the substrate for the RNaseH as a part of the antisense oligonucleotide, and more often, as the central part of the antisense oligonucleotide, and arranging the modified nucleotide sequence on opposite sides thereof.
The modification of such oligonucleotides can similarly be applied to the antisense compound according to the second aspect of the present invention as will be described later.
Oligonucleotides and their derivatives may be produced by the method commonly used in the art (see, for example, Stanley T. Crooke and Bernald Lebleu ed., in Antisense Research and Applications, CRC Press, Florida, 1993; Methods In Enzymology, vol. 313, vol. 314, Academic Press, 2000).

### (2) Preventive or therapeutic agent containing the antisense compound

The preventive or therapeutic agent containing the antisense compound as described above may be produced by a method known in the art by referring to, for example, Applied Antisense Oligonucleotide Technology (1998, Wiley-Liss, Inc.). The antisense molecule may be introduced in the cell by microinjection, by incorporating in the liposome capsule, or by incorporating in an appropriate vector. Exemplary vectors include plasmid and viral vector which are commonly used in the art for expression of an antisense sequence, and also, vectors such as peptide vector and nonviral vector.

The preventive or therapeutic agent of the present invention contains an antisense compound as its effective component, and also, optional additives such as pharmacologically acceptable excipient, lubricant, binder, disintegrant, emulsifier, stabilizer, flavouring agent, and diluent.

Examples of the excipient are organic excipients including sugar derivatives such as lactose, sucrose, glucose, mannitol, and sorbitol; starch derivatives such as starch and dextrin; cellulose derivatives such as crystalline cellulose; gum arabic; and dextran; and inorganic excipients including silicate, carbonate, and sulfate. Exemplary lubricants include metal stearate, wax, lauryl sulfate, silicic acid compounds, and starch derivative; and exemplary binders include hydroxypropylcellulose, polyvinylpyrrolidone, and macrogol. Exemplary disintegrants include cellulose derivative; exemplary emulsifiers include colloidal clays such as bentonite and Beegum, metal hydroxide, anionic surfactant, cationic surfactant, and nonionic surfactant; and exemplary stabilizers include parahydroxybenzoates, alcohols, phenols, and sorbic acid. Exemplary flavouring agents include those commonly used in the art such as sweetener, acidulant, and flavor.

The preventive or therapeutic agent containing the antisense compound is preferably used as an injection which is administered by subcutaneous administration, intradermal administration, intravenous administration, intramuscular administration, or intraperitoneal administration, or as a microcapsule implanted subcutaneously. The injection is administered either daily or, for example, once every one to two weeks. The administration is not limited in its route or interval, and the agent may also be administered by oral administration, transdermal administration, rectal administration, or intranasal administration at an adequate interval according to the age and the conditions of the patient.

### (Preventive or therapeutic agent containing siRNA of meltrin α as its effective component)

### (1) siRNA

Ribonucleic acid siRNA contains at least an RNA having a sequence complementary to the mRNA coding for the human meltrin α, and this RNA is incorporated in a protein complex called RISC which has RNase activity, and thereby digests specifically the mRNA of the meltrin α. The sequence of the siRNA is prepared by selecting a site which serves as the target by a method known in the art, and chemically synthesizing a sequence which has a nucleotide sequence complementary to the sequence comprising approximately 1 to 25 nucleotides of the selected sequence by a method known in the art. The site used for the target may be the region which serves as the target for the antisense compound as described above.

The DNAs shown in the SEQ ID NOS: 1 and 3 code for the soluble human meltrin α and the transmembrane human meltrin α, respectively. SEQ ID NOS: 2 and 4 are amino acid sequences of the soluble human meltrin α and the transmembrane human meltrin α to be produced by the expression of the DNAs of SEQ ID NOS: 1 and 3, respectively. Human meltrin α is known to have a soluble form of meltrin α with no transmembrane domain which is generated by alternative splicing. The DNA coding for this soluble meltrin α is different from the DNA coding for the transmembrane meltrin α in the downstream region of the 2113rd nucleotide counting from the initiation codon. The siRNA contained in the preventive or therapeutic agent of the present invention may be the one which suppresses the expression of the transmembrane human meltrin α, or the one which suppresses the expression of the soluble human meltrin α, and in such a case, the siRNA may be produced by using any sequence of the human meltrin α for the target. However, in view of efficiently inhibiting the in vivo action of the meltrin α, the siRNA is preferably the one which inhibits the expression of both the transmembrane meltrin α and the soluble meltrin α. Such siRNA may be prepared by using for the target the consensus sequence which the transmembrane meltrin α and the soluble meltrin α have in the downstream of the initiation codon as well as the 5' upstream untranslated region.
In addition to the designing and synthesis as described above, the siRNA may also be obtained by using a plasmid or a viral vector which is designed and constructed so that the siRNA sequence may be expressed under the control of tRNA promoter or a conventional polymerase II promoter which induces specific expression depending on the tissue or stimulation.

### (2) Production of preventive or therapeutic agent containing siRNA

The preventive or therapeutic agent of the present invention contains an siRNA as its effective component, and also, optional additives such as pharmacologically acceptable excipient, lubricant, binder, disintegrant, emulsifier, stabilizer, flavouring agent, and diluent. The siRNA may be administered to the patient in a similar manner to the antisense compound as described above.

### (Preventive or therapeutic agent containing an antibody which recognizes meltrin α as its effective component)

### Anti-meltrin α antibody

The anti-meltrin α antibody contained in the preventive or therapeutic agent of the present invention is an antibody which binds to the human meltrin α to suppress the meltrin α activity, and preferably, an antibody which suppresses increase in the body weight upon ingestion of high fat diet, an antibody which reduces the fat mass, an antibody which has the activity of enhancing energy metabolism, an antibody which reduces lipid in the blood, an antibody which suppresses fatty liver, an antibody which suppresses increase of adipocyte on feeding of high fat diet or high nutrition diet, or an antibody which has the activity of suppressing accumulation of lipid droplet into the adipocyte after the differentiation stimulation. Since human meltrin α and mouse meltrin α have a high homology in their protein primary sequences, meltrin α from an animal other than mouse may also be highly homologous to the human meltrin α. Accordingly, an antibody obtained by using the meltrin α from a non-human animal species for the antigen may bind to the human meltrin α to inhibit its activity, and if so, any such antibody can serve as the effective component of the preventive or therapeutic agent of the present invention. However, the antibody which is used for the effective component is preferably an antibody obtained by using the human meltrin α for the antigen.

In this regard, an antibody which has cross-reactivity with the meltrin α from human and that from other animal such as mouse or rat, and in particular mouse, is useful in view of its applicability to the in vivo experiment using such non-human animal. The antibody to be used is preferably an antibody whose reactivity with other ADAM family proteins having structural homology with the meltrin α, and in particular meltrin β and meltrin γ, has been made clear, and an antibody which reacts with meltrin α without reacting with meltrin P and meltrin γ is more preferable in view of the specificity.

Immunoglobulin has a structure comprising heavy chains (H chains) and light chains (L chains), and the immunoglobulin is divided into 5 isotypes (IgG, IgA, IgM, IgD, and IgE) in accordance with the class of the heavy chain (γ, α, µ, δ, and ε). Among these, IgG and IgA are divided into subclasses (for example, in the case of human, IgG1, IgG2, IgG3, IgG4, IgA1, and IgA2) based on the difference in the heavy chain (for example, in the case of human, γ1, γ2, γ3, γ4, α1, and α2). The light chain is divided into types κ and λ. In the present invention, the antibody used as a meltrin α antagonist is not limited to any particular class, subtype, or isotype, and the one belonging to any of such categories may be used. The antibody, however, is preferably the one whose isotype is IgG, and more preferably, the one which belongs to the subclass IgG4 in view of the absence of the complement fixing ability.

### (2) Method for preparing the antibody

### (2-1) Immunization of animal

The antibody may be prepared typically by referring to a known book such as "Method of Immunological Experiments" edited by and published from Japanese Society for Immunology. First, human meltrin α or its fragment, or meltrin α of a non-human animal is inoculated as an immunogen to an animal optionally with an adequate adjuvant such as Freund's complete adjuvant (FCA) or Freund's incomplete adjuvant (FIA), and if necessary, the animal is boosted at an interval of 2 to 4 weeks. However, when the antigen used is partial fragment of human meltrin α, or even in the case of human meltrin α, antibody titer will not increase if the mouse is immunized by the procedure normally used in the art. This is believed to be the result of the high sequence homology among different animals. Therefore, the combination of the immunized animal and the antigen, the type of the adjuvant, and the method of treating the antigen greatly affect the production efficiency of the antibody.

In order to efficiently produce the antibody, some measures, for example, use of a strong adjuvant or increase in the antigenicity by somehow denaturing the administered antigen should be taken.
For example, when a mouse is immunized by using human meltrin α for the antigen, use of CpG for the adjuvant is preferable. Also preferred is treatment of the solution containing the human meltrin α as the antigen with at least one denaturing agent selected from dinitrofluorobenzene (DNP), glutaraldehyde (GA), and the like at 25°C for 10 to 60 minutes, or thermal denaturing at 95°C or higher for 5 minutes of the same solution with the same denaturing agent. Immunization efficiency is remarkably increased and the expected antibody is readily obtained when CpG is used for the adjuvant with the antigen that has been treated with the denaturing agent.

Also preferred is use of an animal having the meltrin α gene knocked out for the immunization. With regard to the meltrin α knockout animal, knockout mouse is known (Kurisaki, T. et al., Mol. Cell Biol., 23, pp. 55-61, 2003), and the knockout animal of other species may be produced in a similar manner. By using a knockout animal, an antibody can be produced at a high efficiency even if the human meltrin α used for the antigen has not been treated with the denaturing agent or the like, and in such a case, an antibody can be produced at a high efficiency even if the antibody produced is the one against a highly homologous region.
As shown in Examples, a high antibody titer is readily realized in the case of using a knockout mouse compared to the case of using the CpG adjuvant, and the use of a knockout mouse is more adapted for the preparation of the antibody which binds to a main domain of the meltrin α such as metalloprotease domain or cysteine rich domain and for the preparation of the neutralizing antibody. Therefore, use of a knockout animal is more preferable in producing an antibody which recognizes human meltrin α, in particular, a neutralizing antibody or an antibody which is specific to the metalloprotease domain or the cysteine rich domain.

### (2-2) Production of polyclonal antibody and monoclonal antibody

After the immunization (or optional boosting) of the animal, blood was collected to obtain an antiserum. A polyclonal antibody can be produced by purifying the resulting antiserum. The purification may be conducted by an adequate combination of known methods such as salting out, ion exchange chromatography, and affinity chromatography.

A monoclonal antibody can be produced by a method commonly used in the art (for example, Kohler et al., Nature 256, pp. 495-497, 1975). First, antibody producing cell such as spleen cell or lymphocyte is collected from the immunized animal, and the collected cell is fused with myeloma cell line or the like by the method known in the art using polyethylene glycol, Sendai virus, electric pulse, or the like to produce a hybridoma. Then, western blotting or the like for the human meltrin α is conducted to select the clone secreting the expected antibody in the culture supernatant, and the culture supernatant of the selected clone is subjected to an adequate combination of known methods such as salting out, ion exchange chromatography, and affinity chromatography to produce the monoclonal antibody.

The antibody can also be made by a genetic engineering means, for example, by collecting mRNA from splenocyte or lymphocyte of the immunized animal, or the monoclonal antibody-producing hybridoma, preparing cDNA library, screening the clone producing the antibody which reacts with the antigen, and thereafter incubating the screened clone for purification of the expected antibody from the culture mixture.

### (2-3) Preparation of chimeric antibody

When a pharmaceutical is to be administered to a human, the antibody used for its effective component is preferably the one that does not exhibit immunogenicity in the human body so that an unwanted immune responce may be avoided. Examples of such antibody include "chimeric antibody", "humanized antibody", and "human antibody". "Chimeric antibody" is an antibody having the variable region derived from the immunoglobulin of a non-human animal and the constant region from human immunoglobulin. Such chimeric antibody can be produced by the procedure as described below. First, the DNA coding for the monoclonal antibody against the human meltrin α is isolated from the hybridoma producing the monoclonal antibody against human meltrin α, and active VH gene (rearranged VDJ gene coding for the H chain variable region) and VL gene (rearranged VJ gene coding for the L chain variable region) are isolated from this DNA. An expression vector is then prepared by operatively incorporating the CH gene (C gene coding for H chain constant region) from the DNA coding for the human immunoglobulin in the downstream of the thus obtained VH gene, and also operatively incorporating the CL gene (C gene coding for L chain constant region) from DNA coding for the human immunoglobulin in the downstream of the VL gene, and the host cell is transformed with this expression vector. Finally, the chimeric antibody is isolated and purified from the culture of the resulting transformant host cell by the purification method known in the art.

### (2-4) Preparation of humanized antibody

"Humanized antibody" is an antibody in which all or a part of the complementarily determining region (also abbreviated as CDR) in the variable region is the one derived from a monoclonal antibody of a non-human animal, most or all of the region other than the CDR in the variable region, namely, framework region (hereinafter abbreviated as FR) is from human immunoglobulin, and the constant region is the one from human immunoglobulin. An exemplary humanized antibody is an antibody in which the CDR is the one from mouse monoclonal antibody, and the FR and the constant region are each derived from human immunoglobulin.
Such humanized antibody can be produced by the procedure as described below. First, a mouse monoclonal antibody against the human meltrin α is prepared by using a mouse or a meltrin α knockout mouse. Next, CDR-grafting (see Winter and Milstein, Nature 349, pp. 293-299, 1991) is conducted to transplant CDR-region (CDR-1, 2, and 3) of the resulting mouse monoclonal antibody in the CDR region of human IgG so as to produce the expected antibody.

A plurality of definitions for the complementarity determining region (CDR) as well as the methods of determining its location have been reported, and any of such definitions and methods may be adopted. Typical definitions are the one by Kabat ("Sequences of proteins of immunological interest", 5th ed., U.S. Department of Health and Human Services, 1991) and the one by Chothia (Chothia and Lesk, J. Mol. Biol., 1987; 196: 901-917). In the present invention, while the CDR used in the preferred embodiment is the one by the definition of Kabat, the CDR is not limited to such CDR. In some cases, the CDR used may be determined by taking both definitions of Kabat and Chothia into consideration, that is to say, the CDR used may be an overlapping part of the CDRs which fit the two definitions, respectively, or a region which includes both of the CDRs by the two definitions. An exemplary such method is the method of Martin et al. (Proc. Natl. Acad. Sci. USA, 1989; 86: 9268-9272) which is carried out based on a compromise between the definition of Kabat and the definition of Chothia, and using Oxford Molecular's AbM antibody modeling software.

### (2-5) Preparation of human antibody

"Human antibody" is an antibody in which the entire region constituting the immunoglobulin is from the gene coding for human immunoglobulin. The human antibody may be produced by a known method such as the method using a transgenic animal prepared by incorporating the human immunoglobulin gene in the gene of a non-human mammal (for example, WO94/25585), and the method in which lymphocytes from human peripheral blood are transplanted in a severe combined immune deficiency (SCID) mouse and this SCID mouse is used for the production of the human antibody (Mosier, D.E. et al., Nature 335, pp. 256-259, 1988; Duchosal, M.A. et al., Nature 355, pp. 258-262, 1992). A polyclonal human antibody or a monoclonal human antibody recognizing the human meltrin α can be produced by administering the human meltrin α to such animal or mouse.

### (2-6) Production of active fragment

In addition to the antibody molecule itself (an antibody comprising 2 heavy chains and 2 light chains), active fragments of the antibody or the like can also be used for the effective component of the preventive or therapeutic agent of the present invention. Exemplary active fragments of the antibody include Fab (fragment of antigen binding), Fab', and F(ab')2, and examples of the antibody active fragments bonded by a linker include single chain antibody (single chain Fv: scFv) and disulfide-stabilized antibody (disulfide stabilized Fv: dsFv), and examples of the peptides containing active fragment of the antibody include peptides containing CDR. These products may be produced by a method known in the art using the antibody against the human meltrin α.

Fab can be produced by treating the antibody with the protease pepsin when the antibody is IgM, or by treating with the protease papain in the case of IgG, or alternatively, by inserting the DNA coding for the Fab of the antibody in an expression vector for prokaryote or an expression vector for eukaryote, and introducing the vector in a prokaryote or a eukaryote for expression.
F(ab')2 can be produced by treating the antibody with the protease pepsin, or alternatively, by binding the Fab' as described below by thioether bond or disulfide bond.
Fab' can be produced by treating F(ab')2 with the reducing agent dithiothreitol.

scFv can be produced by collecting the cDNAs coding for the VH and the VL of the antibody from the hybridoma as described above, constructing the DNA coding for the scFv, inserting the DNA in an expression vector for prokaryote or an expression vector for eukaryote, and introducing the expression vector in a prokaryote or a eukaryote for expression.
dsFv is produced by substituting 1 amino acid residue in each of the polypeptides as VH and VL with cysteine residue, and linking the polypeptides by disulfide bond between the cysteine residues. The cDNAs coding for the VH and the VL can be obtained from the hybridoma as described above. The amino acid residue to be substituted with the cysteine residue may be selected based on the conformation estimation of the antibody according to the method of Reiter et al. [Protein Engineering, 7, 697 (1994)], and the substitution of the selected amino acid may be carried out by the method like site-directed mutagenesis. The resulting DNAs are then inserted in an expression vector for prokaryote or an expression vector for eukaryote, and the expression vector is introduced in a prokaryote or a eukaryote for expression.

### (2-7) Screening of the expected antibody

Whether or not the antiserum or antibody thus prepared recognizes the human meltrin α can be confirmed by ELISA or western blotting using human meltrin α as will be described later in Examples.
It is also possible to perform western blotting using extract of the cell expressing the transmembrane meltrin α or analysis under an FACS using the cell expressing the transemembrane meltrin α to confirm the reactivity of the antibody against the membrane-bound meltrin α.

The effect of the resulting antiserum or antibody on suppressing the activity of the human meltrin α, and preferably the activity of the antibody may be confirmed by at least one effect on the animal on a high fat diet selected from suppression of the body weight increase, decrease in the fat mass, enhancement of energy metabolism, decrease of lipid in the blood, and inhibition of the fatty liver.
Alternatively, the effect of the resulting antiserum or antibody may be confirmed by the suppressive effect on the adipocyte proliferation on a high fat or high nutrition diet, for example, by the suppressive effect on the differentiation and maturing of the adipocyte which is measued by suppressive activity on the intake of the neutral fat into the adiposcyte after the differentiation stimulation.

As will be described later in Examples, some antibodies exhibit both metalloprotease inhibitory activity and inhibitory activity for adipocyte differentiation, and in the case of such an antibody, first screening may be conducted for the metalloprotease inhibitory activity before conducting the screening for suppression of the body weight increase or activity for the adipocyte proliferation and differentiation.

### (2-8) Purification of the antibody and the pharmaceutical composition containing the antibody as its effective component

The antibodies capable of recognizing the human meltrin α can be purified to the degree allowing their use in a pharmaceutical by combining the methods commonly used in the purification of an antibody (such as antibody affinity chromatography using protein A or the human meltrin α for the ligand, hydrophobic chromatography, and HPLCN).

The preventive or therapeutic agent of the present invention contains 0.1 to 100 mg of the antibody, and if necessary, at least one pharmacologically acceptable additive such as excipient, lubricant, binder, disintegrant, emulsifier, stabilizer, flavouring agent, and diluent. Typical examples of the additives are the same as those described in the section of the preventive or therapeutic agent containing the antisense oligonucleotide. The preventive or therapeutic agent containing the antibody is preferably used as an injection which is administered by subcutaneous administration, intradermal administration, intravenous administration, intramuscular administration, or intraperitoneal administration, or as a microcapsule implanted subcutaneouly. The injection is administered either daily or once every one to two weeks. The administration is not limited in its route or interval, and the agent may also be administered by oral administration, transdermal administration, rectal administration, or intranasal administration at an adequate interval depending on the age and the condition of the patient.

### (Preventive or therapeutic agent containing low molecular weight compound as its effective component)

The low molecular weight compound contained in the preventive or therapeutic agent of the present invention as the effective component is not particularly limited in its structure or molecular weight. The low molecular weight compound used may be selected by choosing from a compound library or the like the low molecular weight compounds each of which binds to the human meltrin α, and then choosing from them the one which inhibits the meltrin α activity. The human meltrin α inhibitory activity may be evaluated on the basis of an effect on an animal on a high fat diet, such as suppression of the body weight increase, suppression of the fat mass, improvement of formation of fatty liver, increase in serum lipid, or energy metabolism, as in the case of the screening of the antibody as described above. The evaluation can also be made by using suppression of adipocyte proliferation on high fat diet or high nutrition diet, or suppression of neutral fat intake into the adipocyte after differentiation stimulation. In view of a higher efficiency, the low molecular weight compound is preferably screened for the suppression of the in vitro intake of neutral fat into the adipocyte. Alternatively, the expected compound may be obtained efficiently by conducting primary screening for choosing the compounds which inhibit the metalloprotease activity of the meltrin α, and then conducing screening for any of those effects as mentioned above.

The preventive or therapeutic agent of the present invention contains 0.1 to 20 mg of the low molecular weight compound, and if necessary, at least one pharmacologically acceptable additive such as excipient, lubricant, binder, disintegrant, emulsifier, stabilizer, flavouring agent, and diluent. Typical examples of the additives are the same as those described in the section of the preventive or therapeutic agent containing the antisense compound. The preventive or therapeutic agent of the present invention is used through subcutaneous administration, intradermal administration, intravenous administration, intramuscular administration, intraperitoneal administration, or administration by another route, such as oral administration, transdermal administratllion, rectal administration, and intranasal administration. The preventive or therapeutic agent of the present invention is preferably administered daily at a frequency of once or twice a day. The route and the interval of the administration, however, may be selected adequately according to the age and the condition of the patient.

### (Characteristics of the preventive or therapeutic agent of the present invention)

The preventive or therapeutic agent of the present invention is administered to a patient suffering from or having the risk of developing obesity, adiposity, or a disease caused by or related to the obesity, or a disease like diabetes.

The way how preventive and therapeutic effects develop may vary according to the degree of obesity, presence of complication, and dietary habit of the patient to whom the agent is administered, and the preventive or therapeutic agent of the present invention is a pharmaceutical composition which is administered in the expectation of improving at least one of the various symptoms, test values, and test images of the patient. The preventive or therapeutic agent of the present invention, however, preferably exhibits at least one of the following actions of:
(1) suppressing accumulation of white adipocytes;
(2) suppressing proliferation of white adipocytes;
(3) suppressing accumulation of adipocytes in the internal organs;
(4) reducing blood cholesterol;
(5) enhancing energy metabolism;
(6) suppressing formation and progress of fatty liver and onset of hepatopathy;
(7) suppressing blood glucose level;
(8) suppressing blood insulin concentration; and
(9) improving glucose tolerance.

The enhancement of the energy metabolism (5) is an important and novel mechanism of the antiobesity effect of the meltrin α antagonist, and because of this action, the pharmaceutical composition of the present invention can be administered to a patient suffering from or under the risk of diabetes as an antiobesity agent enhancing the metabolism. (4) is also an important character, and because of this effect, the agent can be administered to a patient suffering from or under the risk of hyperlipidemia. Furthermore, (3) is also an important character, and because of this effect, the agent can be administered to a patient with or under the risk of accumulation of visceral fat such as fatty liver, and in particular, because of the effect as shown in (6), to a patient with fatty liver, non-alcoholic steatohepatitis (NASH), or loss of liver function associated therewith for preventive or therapeutic purpose.

### (Method for using the preventive or therapeutic agent of the present invention)

The preventive or therapeutic agent of the present invention may be used for prevention or treatment of a obesity, adiposity, or a disease caused by or related to obesity, or a disease like diabetes, alone or in combination with other antiobesity agent, antidiabetic agent, or other drugs.
The preventive or therapeutic agent of the present invention is expected to show high safety as well as high therapeutic effects particularly when combined with an antiobesity agent or an antidiabetic agent having a different action mechanism. A pharmaceutical composition further comprising such antiobesity agent or antidiabetic agent having a different action mechanism, and a pharmaceutical composition in which such antiobesity agent or antidiabetic agent having a different action mechanism and the pharmaceutical composition of the present invention are incorporated in a single package are also within the scope of the pharmaceutical composition of the present invention.
The preventive or therapeutic agent of the present invention is based on new action mechanism, and therefore, it is expected to show the antiobesity or antidiabetic effect even on the patient who showed resistance to other antiobesity agent or antidiabetic agent.

The second aspect of the present invention is directed to an antisense compound against human meltrin α. The preferable target region for the antisense compound of the present invention is the region corresponding to nucleotide Nos. 61 to 360 or 2581 to 3020, and more preferably nucleotide Nos. 2581 to 2680, 2701 to 2760, 2771 to 2800, 2849 to 2905, or 2971 to 3020 in SEQ ID NO: 3 of Sequence Listing. The antisense compounds provided in the present invention typically include those comprising the nucleotide sequence shown in Table 6 or 8 in Examples or containing such sequence. Among these, more useful are those having a higher suppression rate (score). The antisense compound against human meltrin α according to the second aspect of the present invention can be used in the preventive or therapeutic agent according to the first aspect as its effective component. The description of the antisense compound in the preventive or therapeutic agent according to the first aspect also serves as the description of the antisense compound according to the second aspect.
The antisense compound against human meltrin α is described below in further detail.

The present invention uses an oligomer antisense compound, and in particular, an oligonucleotide for the purpose of modulating function of the nucleic acid molecule coding for the meltrin α, and ultimately, for modulating the amount of the meltrin α produced. This purpose is accomplished by providing an antisense compound which specifically hybridizes with 1 or more nucleic acids coding for the meltrin α.
In the present invention, the term "target nucleic acid" and the term "nucleic acid coding for meltrin α" are to be considered to bear the meaning covering a DNA coding for the meltrin α, an RNA (including pre-mRNA and mRNA) transcribed from such DNA, and also, a cDNA derived from such RNA.
Specific hybridization of the oligomer compound to the target nucleic acid inhibits normal functions of the nucleic acid. Such modulation of the functions of the target nucleic acid by the compound which specifically hybridizes to the target nucleic acid is generally called "antisense". The functions of DNA to be inhibited include replication and transcription. The functions of RNA to be inhibited include all the functions required for the survival including transfer of RNA to the site of protein translation, translation of RNA into a protein, RNA splicing to obtain 1 or more mRNA species, and catalytic activity inherent in or promoted by RNA. The overall effect of the inhibition of the functions of the target nucleic acid is modulation of the meltrin α expression. In the present invention, "modulation" means either an increase (stimulation) or a decrease (inhibition) in the expression of a gene. In the present invention, inhibition is the preferred form of the modulation of gene expression and mRNA is the preferred target.

It is preferred to target specific nucleic acids for antisense. "Targeting" an antisense compound to a particular nucleic acid, in the present invention, is a multistep process. The process usually begins with the identification of a nucleic acid sequence whose function is to be modulated. This may be, for example, a cellular gene (or mRNA transcribed from the gene) whose expression is associated with a particular disorder or disease state, or a nucleic acid molecule from an infectious agent. In the present invention, the target is a nucleic acid molecule coding for the meltrin α. The targeting process also includes determination of a site or sites within this gene for the antisense interaction to occur such that the desired effect, for example, detection or modulation of expression of the protein, will result. In the present invention, a preferred intragenic site is the region encompassing the translation initiation or termination codon of the open reading frame (ORF) of the gene. Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function in vivo. Thus, the terms "translation initiation codon" and "start codon" can encompass many codon sequences, even though the initiator amino acid in each instance is typically methionine (in eukaryotes) or formylmethionine (in prokaryotes). It is also known in the art that eukaryotic and prokaryotic genes may have two or more alternative start codons, any one of which can be utilized for translation initiation preferably in a particular cell type or tissue, or under a particular set of conditions. In the present invention, "start codon" and "translation initiation codon" refer to the codon or codons that are used in vivo to initiate translation of an mRNA molecule transcribed from a gene encoding the meltrin α, regardless of the sequence(s) of such codon(s).

It is also known in the art that the translation termination codon (or "stop codon") of a gene may have one of three sequences, that is, 5'-UAA, 5'-UAG and 5'-UGA (the corresponding DNA sequences being 5'-TAA, 5'-TAG and 5'-TGA, respectively). The terms "start codon region" and "translation initiation codon region" refer to such a portion of an mRNA or gene that comprises from about 25 to about 50 contiguous nucleotides in either direction (that is, toward 5' or 3') from the translation initiation codon. Similarly, the terms "stop codon region" and "translation termination codon region" refer to such a portion of an mRNA or gene that comprises from about 25 to about 50 contiguous nucleotides in either direction (that is, toward 5' or 3') from the translation termination codon.

The open reading frame (ORF) or "coding region," which is known in the art to refer to the region between the translation initiation codon and the translation termination codon, is also a region which may be used as an effective target. Other target regions include the 5' untranslated region (5'UTR), known in the art to refer to the portion of an mRNA in the 5' direction from the translation initiation codon, and thus including nucleotides between the 5' cap site and the translation initiation codon of an mRNA or corresponding nucleotides on the gene, and the 3' untranslated region (3'UTR), known in the art to refer to the portion of an mRNA in the 3' direction from the translation termination codon, and thus including nucleotides between the translation termination codon and 3' end of an mRNA or corresponding nucleotides on the gene. The 5' cap of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap. The 5' cap region may also be a preferred target region.

Although some eukaryotic mRNA transcripts are directly translated, many contain one or more regions known as "introns", which are excised from a transcript before it is translated. The remaining (and therefore translated) regions are known as "exons" and are spliced together to form a continuous mRNA sequence. mRNA splice sites, i.e., intron-exon junctions, may also be preferred target regions, and are particularly useful in situations where aberrant splicing is implicated in disease, or where an overproduction of a particular mRNA splice product is implicated in disease. Aberrant fusion junctions due to rearrangements or deletions are also preferred targets. It has also been found that introns can also be effective, and therefore can be preferred target regions for antisense compounds targeted, for example, to DNA or pre-mRNA.

Once one or more target sites have been identified, oligonucleotides are chosen which are sufficiently complementary to the target, that is, hybridize sufficiently well and with sufficient specificity, to give the desired effect.

In the present invention, "hybridization" means hydrogen bonding between complementary nucleoside or nucleotide bases, which may be Watson-Crick, Hoogsteen, or reversed Hoogsteen hydrogen bonding. For example, adenine and thymine are complementary nucleobases, which pair through the formation of hydrogen bonds. The term "complementary," as used herein, refers to the capacity for precise pairing between two nucleotides. For example, if a nucleotide at a certain position in an oligonucleotide is capable of hydrogen bonding with a nucleotide at the same position in a DNA or RNA molecule, then the oligonucleotide and the DNA or RNA are considered to be complementary to each other at that position. The oligonucleotide and the DNA or RNA are complementary to each other when a sufficient number of positions in one molecule are occupied by the nucleotides which can hydrogen bond with those at the corresponding positions in the other molecule. Thus, the terms "specifically hybridizable" and "complementary" are terms which are used to indicate a sufficient degree of complementarity or precise pairing such that stable and specific binding occurs between the oligonucleotide and the DNA or RNA target. It is understood in the art that an antisense compound does not need to have a sequence 100% complementary or homologous to that of its target nucleic acid in order to be specifically hybridizable with the target. An antisense compound is specifically hybridizable when binding of the compound to the target DNA or RNA molecule interferes with a normal function of the target DNA or RNA to cause a loss of utility, and there is a sufficient degree of complementarity to avoid non-specific binding of the antisense compound to non-target sequences under conditions in which specific binding is desired, that is, under physiological conditions in the case of in vivo assays or therapeutic treatment, and in the case of in vitro assays, under conditions for performing the assays.

Antisense compounds are commonly used as research reagents and diagnostics. For example, antisense oligonucleotides, which are able to inhibit gene expression with exquisite specificity, are often used by those of ordinary skill in the art to elucidate the function of particular genes. Antisense compounds are also used, for example, to distinguish among functions of various members of a biological pathway.

Antisense modulation as well as the specificity and sensitivity of antisense are utilized for therapeutic uses. Antisense oligonucleotides have been employed as therapeutic moieties in the treatment of disease states in animals and man. Antisense oligonucleotides have been safely and effectively administered to humans, and numerous clinical trials are underway. It is thus established that oligonucleotides can be useful therapeutic modalities that can be configured to be useful in treatment regimens for treatment of cells, tissues and animals, especially humans. In the present invention, the term "oligonucleotide" refers to an oligomer or polymer of ribonucleic acid (RNA) or deoxyribonucleic acid (DNA) or mimetics thereof. This term encompasses oligonucleotides composed of oligonucleotides having naturally occurring nucleobases, sugars and covalent internucleoside (backbone) linkages as well as non-naturally occurring portions which function similarly.
Such modified or substituted oligonucleotides are often preferred over native forms because of desirable properties such as, for example, enhanced cellular uptake, enhanced affinity for nucleic acid target and increased stability in the presence of nucleases.

While antisense oligonucleotides are a preferred form of antisense compound, the present invention comprehends other oligomeric antisense compounds, including but not limited to oligonucleotide mimetics such as are described below. The antisense compound according to the present invention preferably comprises from 10 to 40 nucleobases (that is, from 10 to 40 linked nucleosides). Particularly preferred antisense compound is an antisense oligonucleotide, even more preferably that comprising from 15 to 30 nucleobases. As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3', or 5' hydroxyl moiety of the sugar. In forming oligonucleotides, the phosphate groups covalently link adjacent nucleosides to one another to form a linear polymeric compound. In turn the two ends of this linear polymeric structure can be joined together to form a circular structure, however, open linear structures are generally preferred. Within the oligonucleotide structure, the phosphate groups are commonly thought to form the internucleoside backbone of the oligonucleotide. The normal linkage or backbone of RNA and DNA is a 3' to 5' phosphodiester linkage.

Specific examples of preferred antisense compounds useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. In this specification, modified oligonucleotides that do not have a phosphorus atom in their internucleoside backbone can also be considered to be oligonucleosides.

Preferable modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, as well as boranophosphonates having normal 3'-5' linkages, 2'-5' linked analogs of these, and those containing adjacent pairs of nucleoside units with inverted polarities having 3'-5' linkages for 5'-3' linkages, or having 2'-5' linkages for 5'-2' linkages. Various salts, mixed salts and free acid forms are also included.

Typical examples that teach the preparation of the above phosphorus-containing linkages include, but are not limited to, USP 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which is herein incorporated by reference.

Preferred modified oligonucleotide backbones that do not contain a phosphorus atom therein include backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. Examples of such backbones include those comprising morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and other backbones having mixed N, O, S and CH₂ component parts.

Typical examples that teach the preparation of the above oligonucleosides include, but are not limited to, USP 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, each of which is herein incorporated by reference.

In other preferred oligonucleotide mimetics, both the sugar and the internucleoside linkage, i.e., the backbone, of the nucleotide units are replaced with novel groups. The base units are maintained for an appropriate hybridization with the nucleic acid target compound. One such oligomeric compound, or oligonucleotide mimetic, that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Exemplary documents that teach the preparation of PNA compounds include, but are not limited to, USP 5,539,082; 5,714,331; and 5,719,262, each of which is herein incorporated by reference. Further teaching of PNA compounds can be found in Nielsen et al. (Science, 1991, 254, 1497-1500).

Most preferred embodiments of the invention are oligonucleotides with phosphorothioate backbones and oligonucleosides with heteroatom backbones, and in particular those with the backbones -CH₂NH-O-CH₂-, - CH₂N(CH₃)-O-CH₂-[methylene (methylimino) or MMI backbone], - CH₂O-N(CH₃)-CH₂-, -CH₂N(CH₃)-N(CH₃)-CH₂- and -O-N(CH₃)-CH₂CH₂-[wherein the native phosphodiester backbone is represented as -O-P-O-CH₂-] of the above referenced USP 5,489,677, and the amide backbones of the above referenced USP 5,602,240. Also preferred are oligonucleotides having the morpholino backbone structures of the above-referenced USP 5,034,506.

Modified oligonucleotides may also contain one or more substituted sugar moieties. Preferable oligonucleotides have one of the following substituents at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be C₁ to C₁₀ alkyl, C₂ to C₁₀ alkenyl and C₂ to C₁₀ alkynyl, respectively, which are each substituted or unsubstituted. Examples of particularly preferable groups include: O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃]₂, wherein n and m are each from 1 to about 10. Another preferable oligonucleotides have one of the following substituents at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacological properties of an oligonucleotide, and other substituents having similar properties. Preferable modifications include 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504), that is, an alkoxyalkoxy group. Further preferable modifications include 2'-dimethylaminooxyethoxy, that is, an O(CH₂)₂ON(CH₃) group also known as 2'-DMAOE, as described in Examples, and 2'-dimethylaminoethoxyethoxy (also known in the art as 2'-O-dimethyl-amino-ethoxy-ethyl or 2'-DMAEOE), that is, 2'-O-CH₂-O-CH₂-N(CH₂)₂, as described in Examples.

Other preferred modifications include 2'-methoxy (2'-OCH₃), 2'-aminopropoxy (2'-OCH₂CH₂CH₂NH₂), and 2'-fluoro (2'-F). Similar modifications may also be made at other positions on the oligonucleotide, particularly the 3' position of the sugar in a 3' terminal nucleotide or in a 2'-5' linked oligonucleotide, and the 5' position of a 5' terminal nucleotide. Oligonucleotides may also have sugar mimetics such as cyclobutyl moieties in place of the pentofuranosyl sugar. Exemplary documents that teach the preparation of such modified sugar structures include, but are not limited to, USP 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which is herein incorporated by reference.

The oligonucleotide may also include nucleobase (often referred to in the art simply as "base") modifications or substitutions. The "unmodified" or "natural" nucleobases as mentioned herein include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and -cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine, and 3-deazaguanine and 3-deazaadenine. Further nucleobases include those disclosed in USP 3,687,808, those disclosed in The Concise Encyclopedia Of Polymer Science And Engineering, pages 858-859, Kroschwitz, J. I. ed., John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B. ed., CRC Press, 1993. Certain ones among these nucleobases are particularly useful for increasing the binding affinity of the oligomeric compounds of the invention. Such nucleobases include 2-aminopropyladenine, 5-substituted pyrimidines including 5-propynyluracil and 5-propynylcytosine, 6-azapyrimidine, and N-2, N-6 and O-6 substituted purines. 5-methylcytosine substitutions have been shown to increase nucleic acid duplex stability by 0.6 to 1.2°C (Sanghvi, Y. S., Crooke, S. T. and Lebleu, B. eds., Antisense Research and Applications, CRC Press, Boca Raton, 1993, pp. 276-278) and are presently suitable base substitutions, and even more suitable particularly when combined with 2'-O-methoxyethyl sugar modifications.

Typical examples that teach the preparation of certain ones among the above noted modified nucleobases as well as other modified nucleobases include, but are not limited to, the above noted USP 3,687,808, as well as USP 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121; 5,596,091; 5,614,617; 5,681,941; and USP 5,750,692, each of which is herein incorporated by reference.

Another modification of the oligonucleotides of the invention involves chemically linking to the oligonucleotide one or more moieties or conjugates which enhance the activity, cellular distribution, or cellular uptake of the oligonucleotide. Such moieties include, but are not limited to, lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, for example, hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, for example, dodecandiol or undecyl residue (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, for example, di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Mancharan et al., Nucleosides & Nucleotides, 1995, 14, 969-973), adamantane acetic acid (Manoharan et al., Tetrahedron Lett., 1995, 36, 3651-3654), a palmityl moiety (Mishra et al., Biochim. Biophys. Acta, 1995, 1264, 229-237), and an octadecylamine or hexylamino-carbonyl-oxycholesterol moiety (Crooke et al., J. Pharmacol. Exp. Ther., 1996, 277, 923-937).

Typical examples that teach the preparation of such oligonucleotide conjugates include, but are not limited to, USP 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, each of which is herein incorporated by reference.

It is not necessary to modify all positions in a given compound uniformly, and in fact, one or more of the aforementioned modifications may be incorporated in a single compound or even at a single nucleoside within an oligonucleotide. The present invention also includes antisense compounds, which are chimeric compounds. "Chimeric" antisense compounds or "chimeras," in the present invention, are antisense compounds, particularly oligonucleotides, which contain two or more chemically distinct regions, each made up of at least one monomer unit, that is, nucleotide in the case of an oligonucleotide compound. These oligonucleotides typically contain at least one region where the oligonucleotide is modified so as to confer upon the oligonucleotide increased resistance to nuclease digestion, increased cellular uptake, and/or increased binding affinity for the target nucleic acid. An additional region of the oligonucleotide may serve as the substrate for an enzyme capable of cleaving RNA:DNA or RNA:RNA hybrids. By way of example, RNase H is a cellular endonuclease, which cleaves the RNA strand of RNA:DNA duplex. Activation of RNase H, therefore, results in cleavage of the RNA target, thereby greatly enhancing the efficiency of oligonucleotide inhibition of gene expression. Consequently, when chimeric oligonucleotides are used, comparable results can often be obtained with shorter oligonucleotides compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds of the present invention may be formed as composite structures of two or more oligonucleotides, modified oligonucleotides, oligonucleosides and/or oligonucleotide mimetics as described above. Such compounds have also been referred to in the art as hybrids or gapmers. Representative United States Patents that teach the preparation of such hybrid structures include, but are not limited to, USP 5,013,830; 5,149,797; 5,220,007; 5,256,775; 5,366,878; 5,403,711; 5,491,133; 5,565,350; 5,623,065; 5,652,355; 5,652,356; and 5,700,922, certain of which are commonly owned with the instant application, and each of which is herein incorporated by reference in its entirety.

The antisense compounds used in accordance with the present invention can be conveniently, and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

The compounds of the present invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Typical examples that teach the preparation of such uptake, distribution and/or absorption assisting formulations include, but are not limited to, USP 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756, each of which is herein incorporated by reference.

The antisense compound of the present invention encompasses any pharmaceutically acceptable salt, ester, or salt of such ester, or any other compound which, upon administration to an animal including a human, is capable of providing (directly or indirectly) the biologically active metabolite or residue thereof. Accordingly, for example, the disclosure is also drawn to prodrugs, pharmaceutically acceptable salts of the compounds of the invention, pharmaceutically acceptable salts of such prodrugs, and other bioequivalents.

The term "prodrug" indicates a therapeutic agent that is prepared in an inactive form and is converted to an active agent (that is, drug) within the living body or cells by the action of endogenous enzymes or other chemicals and/or conditions. In particular, prodrug versions of the oligonucleotides of the invention are prepared as SATE [(S-acetyl-2-thioethyl)phosphate] derivatives according to the methods disclosed in WO 93/24510 to Gosselin et al. (published Dec. 9, 1993) or in WO 94/26764 to Imbach et al.

The term "pharmaceutically acceptable salt" refers to a physiologically and pharmaceutically acceptable salt of the compound of the present invention: that is, a salt that retains the desired biological activity of the parent compound and does not impart undesired toxicological effects thereto.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge et al., "Pharmaceutical Salts," J. of Pharma Sci., 1977, 66, 1-19). The base addition salts of the above acidic compounds are prepared by bringing the free acid form into contact with a sufficient amount of desired base to produce a salt in a covalent manner. The free acid form may be regenerated by bringing the salt form into contact with an acid and isolating the free acid in the conventional manner. The free acid forms differ from their respective salt forms somewhat in certain physiological properties such as solubility in polar solvents, but otherwise the salts are equivalent to their respective free acids for purposes of the present invention. As used herein, the term "pharmaceutical addition salt" encompasses a pharmaceutically acceptable salt of the acid form of one of the components of the compositions of the invention. Exemplary salts include organic or inorganic acid salts of the amines. Preferred acid salts are the hydrochlorides, acetates, salicylates, nitrates, and phosphates. Other suitable pharmaceutically acceptable salts are well known to those skilled in the art and include basic salts of a variety of inorganic and organic acids, such as with inorganic acids, for example, hydrochloric acid, hydrobromic acid, sulfuric acid or phosphoric acid; with organic acids such as carboxylic, sulfonic, sulfo or phospho acids or N-substituted sulfamic acids, for example, acetic acid, propionic acid, glycolic acid, succinic acid, maleic acid, hydroxymaleic acid, methylmaleic acid, fumaric acid, malic acid, tartaric acid, lactic acid, oxalic acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid; and with amino acids such as the 20 α-amino acids involved in the synthesis of proteins in nature, for example, glutamic acid or aspartic acid; and also with phenylacetic acid, methanesulfonic acid, ethanesulfonic acid, 2-hydroxyethanesulfonic acid, ethane-1,2-disulfonic acid, benzenesulfonic acid, 4-methylbenzenesulfoic acid, naphthalene-2-sulfonic acid, naphthalene-1,5-disulfonic acid, 2- or 3-phosphoglycerate, glucose-6-phosphate or N-cyclohexylsulfamic acid (with the formation of cyclamates), or with other acid organic compounds, such as ascorbic acid. Pharmaceutically acceptable salts of compounds may also be prepared with a pharmaceutically acceptable cation. Suitable pharmaceutically acceptable cations are well known to those skilled in the art and include alkaline, alkaline earth, ammonium, and quaternary ammonium cations. Carbonates or hydrogen carbonates are also possible.

For oligonucleotides, preferred examples of pharmaceutically acceptable salts include, but are not limited to, (a) salts formed with cations such as sodium, potassium, ammonium, magnesium, calcium, and polyamines including spermine and spermidine; (b) acid addition salts formed with inorganic acids such as hydrochloric acid, hydrobromic acid, sulfuric acid, phosphoric acid, and nitric acid; (c) salts formed with organic acids such as acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, and polygalacturonic acid; and (d) salts formed from elemental anions such as chlorine, bromine, and iodine.

The present invention also provides a preventive or therapeutic method for obesity, adiposity or a disease caused by or related to the obesity, which is characterized by the use of a meltrin α antisense compound. The meltrin α antisense compound used in the preventive or therapeutic method according to the present invention is the same as the one described in the sections on the first and second aspects of the present invention. The preventive or therapeutic method according to the present invention is preferably a method using an antisense compound which is capable of suppressing the expression of meltrin α, and more preferably, a method using an siRNA as the antisense compound against the meltrin α.
The preventive or therapeutic method according to the present invention is applied to patients diagnosed by a health professional as having or potentially having obesity, adiposity or a disease caused by or related to the obesity. More specifically, the preventive or therapeutic method according to the present invention is applied to patients exhibiting the value of BMI, blood glucose level, lipid in blood, value of cholesterol in blood, or the like beyond the normal range, and patients who have been found to have adipose accumulation or pathological manifestation by diagnostic imaging such as CT or echo, biopsy, or the like, and the method of the invention may be applied even to the patients exhibiting the above-mentioned values within the normal range if it is recognized as necessary for them by taking other test values and lifestyle such as dietary habits totally into consideration. In practice, the preventive or therapeutic method according to the present invention is carried out by administering the pharmaceutical composition described before as the first aspect of the present invention to a patient while making the administration route, dose, and administeration interval adequate for the age, sex, body weight, and condition of the patient. For example, the pharmaceutical composition may be administered at an effective dose once to several times daily for 2 to 15 weeks, or once every 1 to 4 weeks. The preventive or therapeutic method according to the present invention may be combined with diet therapy, exercise therapy, or other drugs as required.

The third aspect of the present invention is an antibody, its active fragment, or a derivative thereof which recognizes the human meltrin α, and which has at least one activity selected from enhancement of energy metabolism, inhibition of preadipocyte proliferation, inhibition of adipocyte differentiation, and inhibition of the metalloprotease activity of the human meltrin α. The antibody may be a polyclonal antibody or a monoclonal antibody, and may be any of the chimeric antibody, humanized antibody, and human antibody. The antibody, its active fragment, or a derivative thereof according to the present invention may be prepared and screened by the procedures described for the first aspect of the present invention (with respect to the anti-meltrin α antibody to be contained).
The antibody of the present invention has at least one of the activities as described above. For inhibitory activities, the degree of inhibition does not have to be 100%, and the antibody is preferably the one which has at least one of the inhibitory activities as above with an inhibition degree of 30% or higher, more preferably 50% or higher, and still more preferably 70% or higher.

As will be described in Examples relating to the antibodies which inhibit metalloprotease activity of the meltrin α, it has been estimated that inhibition of the metalloprotease is partly associated with the inhibition of the adipocyte differentiation activity of the meltrin α, or the structure in the vicinity of the metalloprotease active domain is involved in the inhibition of the adipocyte differentiation activity of the meltrin α. With regard to the proteins having protease activity including the meltrin α, it is known that a protein having protease activity activates the physiologically active substance in the living body which is inert and, reversely, inactivates the physiologically active substance which is active, by cleavage in both cases. Accordingly, an antibody having the metalloprotease inhibitory activity is expected to have a wide variety of other physiological functions. In view of the situation as described above, the antibody of the present invention is more preferably an antibody which has at least the metalloprotease inhibitory activity among the activities as mentioned above. The activity of inhibiting the metalloprotease activity of the meltrin α can be confirmed, for example, as an activity of inhibiting cleavage of IGF-BP3 by the meltrin α as will be described later in Examples.

The present invention also provides a preventive or therapeutic method for obesity or a disease related to the obesity which is characterized by the use of a meltrin α antagonist.
The meltrin α antagonist used in the preventive or therapeutic method according to the present invention may be either the one which suppresses the meltrin expression or the one which suppresses the meltrin activity, and both of these cases have been described in detail for the first aspect of the present invention. The preventive or therapeutic method according to the present invention is preferably a method which uses an antagonist capable of suppressing the expression of meltrin α, and more preferably, a method which uses an antisense for the meltrin α, an siRNA for the meltrin α, or an antibody which recognizes the meltrin α as antagonist.
The preventive or therapeutic method according to the present invention is applied to patients diagnosed by a health professional as having or potentially having obesity or a disease related to the obesity. More specifically, the preventive or therapeutic method according to the present invention is applied to patients exhibiting the value of BMI, blood glucose level, lipid in blood, value of cholesterol in blood, or the like beyond the normal range, and patients who have been found to have adipose accumulation or pathological manifestation by diagnostic imaging such as CT or echo, biopsy, or the like, and the method of the invention may be applied even to the patients exhibiting the above-mentioned values within the normal range if it is recognized as necessary for them by taking other test values and lifestyle such as dietary habits totally into consideration. In practice, the preventive or therapeutic method according to the present invention is carried out by administering the pharmaceutical composition described before as the first aspect of the present invention to a patient while making the administration route, dose, and administeration interval adequate for the age, sex, body weight, and condition of the patient. For example, the pharmaceutical composition may be administered at an effective dose once to several times daily for 2 to 15 weeks, or once every 1 to 4 weeks. The preventive or therapeutic method according to the present invention may be combined with diet therapy, exercise therapy, or other drugs as required.

### EXAMPLES

Next, the present invention is further described by referring to Examples, which by no means limit the scope of the present invention.

### (Example 1) Evaluation of antiobesity action of the meltrin α using a knockout mouse

In order to confirm antiobesity action of the meltrin α, meltrin α gene knockout mice (hereinafter sometimes referred to as knockout mice) and wild-type mice (hereinafter sometimes referred to as WT mice) were fed on a high fat diet, and change in their body weight was measured over time. The mice were also sacrificed after feeding on a high fat diet to measure the weight of various organs and conduct pathological analysis.
The mice used meltrin α knockout were produced by Kurisaki et al. (Mol. Cell Biol. 23: 55-61; 2003) and housed in 12-hour light 12-hour dark cycles in a facility at a regulated temperature. The used wild-type mice were C57BL/6J mice which are the same strain as the knockout mice (male 6 week old, CLEA Japan Inc.). The high fat diet was the 60% fat diet prepared by Oriental Yeast Co., Ltd. The control diet was the 10% fat diet (hereinafter sometimes referred to as low fat diet).

4 week old male mice were divided into high fat diet group and low fat diet group, and their body weight was measured every week. Food intake was also periodically measured. As a consequence, as shown in FIG. 1, both the meltrin α knockout mice and the WT mice of the low fat diet group showed ordinary body weight increase. On the other hand, the WT mice fed on the high fat diet showed the body weight increase about twice greater than that of the low fat diet group, while the meltrin α knockout mice showed significantly suppressed increase in the body weight. These results revealed that the suppression of the meltrin α expression had an antiobesity effect.

Since no difference in food intake was observed, the possibility that the antiobesity action found in the meltrin α knockout mice is due to the decrease in food intake was denied. The mice fed high fat diet for 15 weeks were sacrificed to measure the weight of various organs. As shown in FIG. 2, for brown adipose tissue and liver, difference in the weight was not so large for both the low fat diet group and the high fat diet group. On the other hand, for the meltrin α knockout mice of the high fat diet group, significant decrease in the weight of the white adipose tissue (WAT) was observed for inguinal adipose tissue and epididymal adipose tissue, revealing that inhibition of the meltrin α had the effect of reducing the adipose tissue weight.

Furthermore, histological observation of the liver revealed, as shown FIG. 3, that the fatty liver was formed in the WT mice and not in the meltrin α knockout mice. This demonstrated that inhibition of the meltrin α had the effect of suppressing the fatty liver formation.

Next, whether the decrease in the adipose tissue weight had been caused by the difference in the size of the adipocyte or difference in the number of adipocyte was examined. Namely, the adipose tissue was immobilized with 4% paraformaldehyde solution, dehydrated with ethanol, and embeded in paraffin, and its sections having a thickness of 4 µm were prepared. After deparaffinization, the section was stained with hematoxylin and eosin, and the adipocyte number per unit area was measured according to the procedure of Picard et al. (Cell 111: 931-41; 2002), and the adipocyte number in the tissue was compared between the meltrin α knockout mouse and the WT mouse. As shown in FIG. 4 in which the adipocyte number of the WT mouse is shown as 100%, no significant difference was found between the meltrin α knockout mouse and the WT mouse in the case of the low fat diet group, while adipocyte number was significantly smaller in the meltrin α knockout mouse compared to the WT mouse in the high fat diet group. Since no difference was found in the size of the adipocyte between these mice, it was for the first time revealed that the suppression of the meltrin α expression results in the antiobesity action associated with the suppression of the proliferation of the adipocyte.

### (Example 2) Enhancement of lipid and glucose metabolism in meltrin α knockout mouse after feeding on high fat diet

In order to assay energy metabolism of lipid and glucose in the meltrin α knockout mouse after feeding on a high fat diet, the mouse was administered ¹⁴C labeled palmitic acid or glucose from tail vein, and amount of radioactivity that transferred to various organs, urine, feces, and exhaled CO₂ was measured. More specifically, meltrin α knockout mice (male, 6 week old) and WT mice C57BL/6J (male, 6 week old) were fed a high fat diet containing 60% fat (Oriental Yeast) for 7 to 10 weeks. After such feeding, blood was collected, and the mice were transferred to a metabolic cage for mouse (Metabolica, Sugiyama-Gen Iriki Co., Ltd.). [U-¹⁴C] palmitic acid (Daiichi Pure Chemicals Co., Ltd.) or D-[U-¹⁴C] glucose (Amersham Biosciences) was administered to the mice at 1.85 MBq/kg from their tail vein. After the administration, the mice were housed in Metabolica, and the entire amounts of urine and feces were collected after 24 hours to measure the amount of RI that had been excreted. Amount of RI that had transferred to the exhalated CO₂ was also measured by absorbing the exhalation in 20% aqueous solution of monoethanolamine. More specifically, 1 mL of aqueous solution of monoethanolamine was collected over time after the administration to measure the RI.

RI was measured as described below. In the case of urine, 50 µL of urine was mixed with Soluene-350 (Packard), and then, mixed with Hionic-Fluor (Packard), and the RI of the mixture was measured by scintillation counter (LS6000TA, Beckman). In the case of feces, 10 mg of feces was dissolved in water, and after adding Soluene-350 and treating at 50°C for 2 hours, isopropylalcohol was added, and the solution was further treated at 50°C for 2 hours. And then hydrogen peroxide solution was added to the mixture, and RI was measured in a similar manner to the case of urine after adding Hionic-Fluor. Amount of RI in the exhalation was measured by mixing the sampled aqueous solution of monoethanolamine with Hionic-Fluor. As a consequence, as shown in FIGS. 5-1 and 5-2, for both cases of the palmitic acid administration and the glucose administration, amount of RI excreted to the exhalation increased in the knockout mice, confirming the enhancement of glucose and lipid metabolism in the meltrin α knockout mice. Furthermore, as shown in FIGS. 6-1 and 6-2, in the case of the palmitic acid administration, amount of the RI transferring to the feces was higher in the meltrin α knockout mice compared to the WT mice, and in the case of the glucose administration, amount of the RI transferring to the urine was higher in the meltrin α knockout mice compared to the WT mice. Accordingly, it was estimated that the glucose and the lipid were swiftly metabolized and excreted in the meltrin α knockout mice.

### (Example 3) Lipid concentration in blood of meltrin α knockout mouse after feeding on high fat diet

Triglyceride (TG), cholesterol (TC), non-esterified fatty acid (NEFA), and leptin (leptin) in blood during fasting were measured for the meltrin α knockout mice and WT mice described in Example 2 after feeding on a high fat diet. The TG, TC, and NEFA values were measured by using reagents from Wako Pure Chemical Industries, Ltd. and COBAS MIRA PLUS (Roche). The leptin value was measured by mouse leptin kit (Morinaga). Table 1 shows the average values obtained from the meltrin α knockout mice and the WT mice. As shown in the results, decrease in the cholesterol value and the leptin value was significant in the meltrin α knockout mice compared to the WT mice, and this indicates that the meltrin α inhibition is effective in suppressing cholesterol in the case of obesity. It was estimated that the decrease in the leptin value is associated with the decrease in the weight of the adipose tissue.

[Table 1]

**Table 1: Effect of meltrin α on lipid and leptin concentrations in serum (Lipid and leptin concentrations in mouse serum)**

| | TG mg/dl | TC mg/dl | NEFA meq/l | Leptin ng/ml |
|---|---|---|---|---|
| WT | 106 | 203 | 2.38 | 14971 |
| KO | 101 | 145 | 2.09 | 5700 |

| | | | | |
|---|---|---|---|---|
| TG: triglyceride, TC: total cholesterol, NEFA: non-esterified fatty acid | | | | |

### (Example 4) Cloning of human meltrin α gene and construction of expression plasmid

### (1) Cloning of soluble (free) and transmembrane (membrane-bound) human meltrin α genes

(a-q) fragment was amplified by the first PCR using HeLa gDNA for the template with primers (meltrin α-a (SEQ ID NO: 5) and meltrin α-q (SEQ ID NO: 16)), and similarly, (r-s) fragment was amplified by using primers (meltrin α-r (SEQ ID NO: 17) and meltrin α-s (SEQ ID NO: 18)). The thus amplified fragments were mixed, and by using this mixture for the template, (b-s) fragment was amplified by the second PCR using primers (meltrin α-b (SEQ ID NO: 6) and meltrin α-s (SEQ ID NO: 18)). This (b-s) fragment was introduced in pT7-Blue(T) vector by TA cloning to construct pT7-Meltrinbs having the 5' side region of the soluble and transmembrane meltrin α genes.

### (2) Cloning of cDNA coding for soluble or transmembrane human meltrin α

(t-c) fragment was amplified by the first PCR using placenta cDNA library for the template with primers (meltrin α-t (SEQ ID NO: 19) and meltrin α-c (SEQ ID NO: 7)). Similarly, (d-e) fragment was amplified by using primers (meltrin α-d (SEQ ID NO: 8) and meltrin α-e (SEQ ID NO: 9)), (f-i) fragment was amplified by using primers (meltrin α-f (SEQ ID NO: 10) and meltrin α-i (SEQ ID NO: 11)), and (j-1) fragment was amplified by using primers (meltrin α-j (SEQ ID NO: 12) and meltrin α-1 (SEQ ID NO: 14)).
These 4 amplified fragments were mixed, and by using this mixture for the template, (t-k) fragment was amplified by the second PCR using primers (meltrin α-t (SEQ ID NO: 19) and meltrin α-k (SEQ ID NO: 13)).
This (t-k) fragment was introduced in pT7-Blue(T) vector by TA cloning to construct pT7-Meltrintk having the 3' side region of the soluble meltrin α gene. The above pT7-Meltrinbs was cleaved with restriction enzyme BamHI, and the resulting fragment was ligated in pT7-Meltrintk which had been cleaved with BamHI to obtain pT7-Meltrinbk. This pT7-Meltrinbk was cleaved with restriction enzymes SalI and EcoRI, and the resulting fragment was inserted in SalI and EcoRI site of pBluescriptII(SK+) to construct pTK-2169 having the full length soluble meltrin α gene (SEQ ID NO: 1).

(j-o) fragment was amplified by the PCR using placenta cDNA library for the template with primers (meltrin α-j (SEQ ID NO: 12) and meltrin α-o (SEQ ID NO: 15)). This (j-o) fragment was mixed with the (d-e) fragment and the (f-i) fragment as described above, and by using this mixture for the template, (t-o) fragment was amplified by the second PCR using primers (meltrin α-t (SEQ ID NO: 19) and meltrin α-o (SEQ ID NO: 15)). This (t-o) fragment was introduced in pT7-Blue(T) vector by TA cloning to obtain pT7-Meltrinto having the 3' side region of the trancemembrane meltrin α gene.
Next, pT7-Melrtrinbk was cleaved with restriction enzymes HindIII and EcoRI to prepare fragment A, and pT7-Meltrinto was cleaved with HindIII and SalI to prepare fragment B. These fragments were inserted in SalI and EcoRI site of pBluescriptII(SK+) so that they may form fragment A + fragment B, to thereby construct pEF-MelL having the full length transmembrane meltrin α gene (SEQ ID NO: 3) (FIG. 7).

### (3) Construction of plasmid expressing soluble meltrin α having Flag tag at its C terminus

(j-v) fragment was amplified by the PCR using pTK-2169 for the template with primers (meltrin α-j (SEQ ID NO: 12) and meltrin α-v (SEQ ID NO: 20)). This (j-v) fragment was introduced in pT7-Blue(T) vector by TA cloning to construct pT7-Meltrinjv having cleavage site of restriction enzyme XhoI at the 3' end of the soluble meltrin α gene. This pT7-Meltrinjv was cleaved with restriction enzymes SphI and XhoI to prepare fragment C.
pTK-2169 was cleaved with restriction enzymes XbaI and SphI to prepare fragment D.
Oligo DNA fragments (FLAG-S (SEQ ID NO: 21) and FLAG-A (SEQ ID NO: 22)) coding for Flag tag peptides were phosphorylated, and annealed to prepare fragment E. This fragment E had been designed to have the sticky end of XhoI on the 5' side and the sticky end of XbaI on the 3' side.
pEF-BOS which is a mammalian cell-specified expression vector was cleaved with restriction enzyme XbaI, and fragments C, D and E were ligated in the downstream of EF-1α promoter so that these fragments may form fragment D + fragment C + fragment E, to thereby construct expression plasmid pEF-MelSF (FIG. 7).

### (4) Construction of plasmid expressing soluble meltrin α having His tag at its C terminus

Oligo DNA fragments (HIS-S (SEQ ID NO: 23) and HIS-A (SEQ ID NO: 24)) coding for His tag peptides were phosphorylated, and annealed to prepare fragment F. This fragment F had been designed to have the sticky end of XhoI on the 5' side and the sticky end of XbaI on the 3' side.
pEF-BOS which is a mammalian cell-specified expression vector was cleaved with restriction enzyme XbaI, and fragments C, D and F were ligated in the downstream of EF-1α promoter so that these fragments may form fragment D + fragment C + fragment F, to thereby construct expression plasmid pEF-MelSH (FIG. 7).

### (5) Construction of plasmid expressing soluble meltrin α having Flag tag in which furin cleavage site has been replaced with thrombin cleavage site

Artificial regulation of the production of the active form meltrin α which is formed by cleavage of the prodomain from the proform meltrin α is useful in assaying metalloprotease activity of the meltrin α. As one means for such regulation, the inventors of the present invention considered producing the meltrin α having its furin cleavage site substituted with a known protease cleavage site. More specifically, a mutant meltrin α having the furin cleavage site substituted with thrombin cleavage site was prepared. This mutant can be artificially activated by thrombin as will be demonstrated in Example 9. The plasmid was prepared as described below.

(t-At2) fragment was amplified by the first PCR using pTK-2169 for the template with primers (meltrin α-t (SEQ ID NO: 19) and meltrin α-thrombin 2 (SEQ ID NO: 26)), and similarly, (g-At1) fragment was amplified by using primers (meltrin α-g (SEQ ID NO: 38) and meltrin α-thrombin 1 (SEQ ID NO: 25)). These two amplified fragments were mixed, and by using this mixture for the template, (t-g) fragment was amplified by the second PCR using primers (meltrin α-t (SEQ ID NO: 19) and meltrin α-g). This (t-g) fragment was introduced in pT7-Blue(T) vector by TA cloning to construct pTK-2220.
pTK-2220 was cleaved with restriction enzymes NcoI and HindIII to prepare fragment G.
pTK-2169 was cleaved with restriction enzymes XbaI and NcoI to prepare fragment H.
pTK-2169 was cleaved with restriction enzymes HindIII and SphI to prepare fragment I.
pEF-BOS which is a mammalian cell-specified expression vector was cleaved with restriction enzyme XbaI, and fragments C, E, G, H and I were ligated in the downstream of EF-1α promoter so that these fragments may form fragment H + fragment G + fragment I + fragment C + fragment E, to thereby construct expression plasmid pEF-MelSTF (FIG. 7).
It is to be noted that the primers used in the above (1) to (5) are described in the Sequence Listing.

### (Example 5) Preparation of meltrin α protein

COS-1 cell was transformed with plasmid pEF-MelSF, pEF-MelSTF, or pEF-MelSH prepared in Example 4 by using FuGene6 (Roche). The transformant was incubated at 37°C for 72 hours in the presence of 5% CO₂, and the supernatant was collected after the incubation. The incubation was continued for another 72 hours by adding 48 mL of fresh D-MEM containing 1% inactivated fetal bovine serum, and the supernatant was collected after the incubation. This supernatant was centrifuged at 1000 rpm at 20°C for 5 minutes, and the supernatant was filtered through 0.22 µm Stericup (Nihon Millipore K.K.) for further purification as described below.

The culture supernatants of the transformants introduced pEF-MelSF and pEF-MelSTF respectively, were each purified by using a column filled with anti-FLAG M2 antibody agarose (Sigma) to obtain human meltrin α-FLAG and human meltrin α-STFLAG, respectively. The supernatant of the transformant having pEF-MelSH introduced was purified by using nickel chelete column (Hitrap Chelating HP, Amersham Biosciences) to obtain human meltrin α-His as the purified protein.

Purity of the human meltrin α-FLAG was confirmed by SDS-PAGE, and it was also confirmed by western blotting using anti-FLAG-M2 antibody (SIGMA) that this product was the desired protein. As a consequence, substantially consistent 2 bands were detected by silver staining. The two bands detected by the western blotting and the two bands detected by the silver staining coincided, and from their estimated molecular weight, they were estimated to be the human meltrin α containing the prodomain (molecular weight 92 kDa) and the meltrin α from which the prodomain had been deleted (molecular weight 68 kDa), respectively (FIG. 8). In the meanwhile, for human meltrin α-STFLAG, a single band corresponding to the human meltrin α containing the prodomain (molecular weight 92 kDa) was obtained.

### (Example 6) Preparation of mouse anti-human meltrin α antibody using meltrin α knockout mouse

20 µg of purified human meltrin α-FLAG mixed with an equal amount of FCA (GIBCO) was administered intraperitoneally to the meltrin α knockout mouse prepared by Kurisaki et al. (female, 5 week old). After 2 weeks, 20 µg of the antigen mixed with an equal amount of FIA (GIBCO) was additionally administered. Blood was collected after 1 week to measure the antibody titer. The antibody titer was determined by specific reactivity with the human meltrin α-His produced in Example 5 which has no FLAG sequence.

20 µg of the antigen was further administered intraperitoneally to the mouse having exhibited an increased antibody titer, and after 3 days, spleen was isolated from the mouse for cell fusion. Namely, lymphocytes were separated from the spleen, mixed with myeloma cells (P3U1; P3 x 63-Ag.8.U1), and undergoing the cell fusion by the procedure described in Ando, T. and Chiba, J., "Introduction to Experimental Procedures for Monoclonal Antibody (In Japanese)", page 83, 1991 (Kodansha Ltd.) using polyethylene glycol (SIGMA). Hybridoma was selected by using HAT medium, and wells exhibiting specific reactivity with human meltrin α-His were selected after 10 days. The wells exhibiting the specific reactivity were cloned by limiting dilution procedure (Ando, T. and Chiba, J., "Introduction to Experimental Procedures for Monoclonal Antibody (In Japanese)", page 97, 1991 (Kodansha Ltd.)). After 10 days, screening was conducted by the same procedure to obtain 94 clones producing the anti-meltrin α monoclonal antibody. The clones were each incubated in 10% FCS/RPMI-1640 medium (GIBCO), and then, in Hybridoma-SFM medium (GIBCO) for production of the antibody, and the antibody was purified from the culture supernatant using Prosep-A column (Millipore). Subtype of the thus obtained antibodies was determined by using IsoStrip Mouse Monoclonal antibody Isotyping Kit (Roche). The subtypes are shown in Table 2.

### (Example 7) Preparation of mouse anti-human meltrin α antibody using wild-type mouse

Dinitrofluorobenzene (Wako) was added to the purified human meltrin α-FLAG to a final dinitrofluorobenzene concentration of 1%, and after allowing the mixture to react at room temperature for 20 minutes, the mixture was dialyzed against physiological saline. The thus prepared DNP-ated meltrin α-FLAG was administered to a mouse as an antigen for immunization of the mouse, more specifically, by mixing 20 µg of the DNP-ated meltrin α with 20 µL of CpG adjuvant (ImmunoEasy Mouse Adjubant, QIAGEN), and administering this mixture to ddY mouse (female, 8 week old, SLC) at its foot pad. After 2 weeks, 20 µg of the antigen was administered in a similar manner, and after the final administration, lymphocyte was separated, and the cells were fused by repeating the procedure of Example 6. Hybridoma was selected by using HAT medium, and after 10 days, the hybridoma producing the target antibody was screened. The cell which reacted with the purified human meltrin α-His was cloned by limiting dilution procedure, and after 10 days, the screening was conducted again to obtain 37 clones producing the antibody which reacts with the purified human meltrin α-His. The selected hybridoma was incubated in 10% FCS/RPMI-1640 medium (GIBCO), and then, in Hybridoma-SFM medium (GIBCO) to produce the antibody, and the antibody was purified from the culture supernatant by using Prosep-A column (Millipore). Subtype of the thus obtained antibodies was determined by using IsoStrip Mouse Monoclonal antibody Isotyping Kit (Roche). The subtypes are shown in Table 2.

[Table 2]

**Table 2: Characterization of anti-human meltrin α monoclonal antibody (Subclass of the antibody)**

| Antibody NO. | Subclass | Antibody NO. | Subclass |
|---|---|---|---|
| 3 | IgG2b, κ | 84 | IgG2b, K |
| 6 | IgG2a, κ | 85 | IgG2a, κ |
| 7 | IgG2a, κ | 88 | IgG2b, κ |
| 15 | IgG1, κ | 95 | IgG2a, κ |
| 18 | IgG1, κ | 101 | IgG2a, κ |
| 20 | IgG1, κ | 102 | IgG2a, κ |
| 21 | IgG1, κ | 103 | IgG2a, κ |
| 28 | IgG3, κ | 107 | IgG1, κ |
| 56 | IgG2a, κ | 111 | IgG2b, κ |
| 59 | IgG2a, κ | 117 | IgG2a, κ |
| 66 | IgG1, κ | 119 | IgG2b, κ |
| 83 | IgG2a, κ | 129 | IgG2b, κ |

### (Example 8) Analysis of binding activity of anti-human meltrin α monoclonal antibody

Binding activity of the purified antibodies prepared in Examples 6 and 7 was assayed by the following methods (1) and (2).

### (1) Confirmation of binding activity in antigen immobilized system (Method A)

The purified human meltrin α-FLAG was diluted with PBS to 1 µg/mL, and placed in an immunoplate (Maxisorb, NUNC) at 50 µL/well. After overnight incubation at 4°C, the wells were washed 5 times with ion exchanged water, and 100 µL/well of PBS containing 0.5% BSA was added for blocking. Next, the antibodies obtained in Examples 6 and 7 were diluted with PBS containing 0.1% BSA to 5 µg/mL, and added to the wells, respectively. After allowing to react at 37°C for 1 hour, the wells were washed 3 times with physiological saline containing 0.05% Tween 20 (hereinafter sometimes referred to as washing solution). Peroxidase-labeled anti-mouse immunoglobulin antibody (DAKO) was 1000 fold diluted with 10% solution of rabbit serum in PBS, and added to the wells at 50 µL/well. After allowing to react at 37°C for 1 hour, the wells were washed 5 times in the manner as described above, and TMB solution (BioFix) was added to the wells. After allowing to react at room temperature for 10 minutes, the reaction was terminated by adding 0.5 M sulfuric acid solution, and absorbance at 450 nm was measured by a spectrophotometric microplate reader (E-Max, Molecular Devices Corp.). The results of the measurement are shown in Table 3. The antibody exhibiting the absorbance of 0.5 or higher is indicated by "+".

[Table 3]

**Table 3: Characterization of anti-human meltrin α monoclonal antibody (Binding activity of the antibody evaluated by Method A: the method using an antigen immobilized EIA system)**

| Antibody NO. | Binding activity | Antibody NO. | Binding activity | Antibody NO. | Binding activity |
|---|---|---|---|---|---|
| 3 | + | 46 | + | 92 | + |
| 6 | + | 56 | + | 94 | + |
| 7 | + | 59 | + | 95 | + |
| 8 | + | 62 | + | 96 | + |
| 12 | + | 63 | + | 97 | + |
| 13 | + | 65 | + | 98 | + |
| 14 | + | 66 | + | 99 | + |
| 15 | + | 69 | + | 101 | + |
| 16 | + | 71 | + | 102 | + |
| 19 | + | 72 | + | 103 | + |
| 20 | + | 73 | + | 104 | + |
| 21 | + | 75 | + | 107 | + |
| 23 | + | 76 | + | 108 | + |
| 24 | + | 81 | + | 109 | + |
| 28 | + | 82 | + | 111 | + |
| 32 | + | 83 | + | 114 | + |
| 33 | + | 84 | + | 115 | + |
| 35 | + | 85 | + | 116 | + |
| 37 | + | 86 | + | 117 | + |
| 38 | + | 87 | + | 119 | + |
| 39 | + | 88 | + | 120 | + |
| 41 | + | 89 | + | 127 | + |
| 42 | + | 90 | + | 128 | + |
| 43 | + | 91 | + | 129 | + |

### (2) Confirmation of binding activity by sandwich EIA system (Method B)

According to the method of Nakane et al. (J. Histochem. Cytochem., 22, 1084, 1974), 1 mg of peroxidase (Toyobo) was dissolved in distilled water, and after adding 100 mM periodic acid dissolved in distilled water, the mixture was allowed to react at 25°C for 20 minutes. After completion of the reaction, 1.5% ethylene glycol was added and the mixture was allowed to react at 25°C for 10 minutes. After the reaction, the reaction mixture was dialyzed against 1 mM acetic acid buffer solution (pH 4.4). Next, anti-FLAG-M2 antibody (SIGMA) was dialyzed against 10 mM carbonate buffer solution (pH 9.5), and after mixing 1 mg of peroxidase which had been activated by adding 1 M carbonate buffer solution (pH 9.5) per 1 mg of the antibody, the mixture was reacted at 25°C for 2 hours. 4 mg/mL of sodium borohydride was added, and the mixture was reacted at 4°C for 2 hours. The reaction mixture was dialyzed against PBS to obtain the peroxidase-labeled antibody.

Using the thus prepared labeled antibody, a sandwich ELISA system was constructed by the following procedure. The antibodies obtained in Examples 6 and 7 were diluted with PBS to 10 µg/mL, and added to the wells of immunoplate (Maxisorp, NUNC), respectively, in an amount of 50 µL each. After overnight incubation at 4°C, the wells were washed 5 times with ion exchanged water, and 100 µL of 2% StabilGuard (SurModics) in PBS was added to each well for blocking. The used standard was the purified meltrin α-FLAG diluted with 0.1% BSA/PBS to 1 µg/mL. The used blank was 0.1% BSA/PBS.

The measurement was carried out by discarding the blocking solution in the plate, adding 50 µL of the standard or the blank that had been prepared, and allowing the reaction to take place at 37°C for 1 hour. Subsequently, the wells were washed 3 times with the washing solution, and the peroxidase-labeled anti-FLAG-M2 antibody that had been prepared as described above and diluted with PBS containing 2% rat serum, 1% mouse serum, and 0.05% Tween 20 to 1 µg/mL was added, and the mixture was allowed to react at 37°C for 1 hour. The plate was washed 5 times with the washing solution, and TMB solution (BioFX) was added to the wells. After reacting at room temperature for 10 minutes, the reaction was terminated with 0.5 M sulfuric acid solution, and absorbance at 450 nm was measured by a spectrophotometric microplate reader (E-Max, Molecular Devices Corp.) to select an antibody which showed no increase in the absorbance for the blank but showed increase in the absorbance for the purified meltrin α-FLAG. The results are shown in Table 4. The antibodies exhibiting the absorbance of 0.1 or higher are indicated by "+".

[Table 4]

**Table 4: Characterization of anti-human meltrin α monoclonal antibody (Binding activity of the antibody evaluated by Method B: the method using an antibody immobilized EIA system)**

| Antibody NO. | Binding activity |
|---|---|
| 3 | + |
| 17 | + |
| 18 | + |
| 44 | + |
| 106 | + |
| 113 | + |

### (3) Construction of meltrin α assay system

In order to measure the meltrin α in a specimen, a sandwich ELISA system was constructed by using the thus obtained anti-meltrin α antibody according to the method of Nakane et al. (J. Histochem. Cytochem., 22, 1084, 1974). More specifically, a peroxidase-labeled antibody was prepared, and the combination of antibodies to be used for producing the sandwich ELISA system was selected. Next, an assay system was prepared by using the selected two antibodies, and the serum was assayed. First, the antibody of No. 102 was immobilized on an immunoplate (Maxisorp, NUNC), and the purified meltrin α used for the standard, and various human samples each used for the specimen were added. After the reaction, peroxidase-labeled No. 101 antibody was added for reaction. The plate was washed 5 times with the washing solution, and after adding TMB solution (BioFX), the reaction was terminated with 0.5 M sulfuric acid solution and the absorbance at 450 nm was measured by using a spectrophotometric microplate reader (E-Max, Molecular Devices Corp.). Concentration of the soluble meltrin α in the specimen was calculated by referring to the standard curve. Concentration of the meltrin α detected in the serum from pregnant women was higher than that of the normal donor, and the concentration increased with the number of months of pregnancy.

### (4) Preparation of various human meltrin α domain proteins

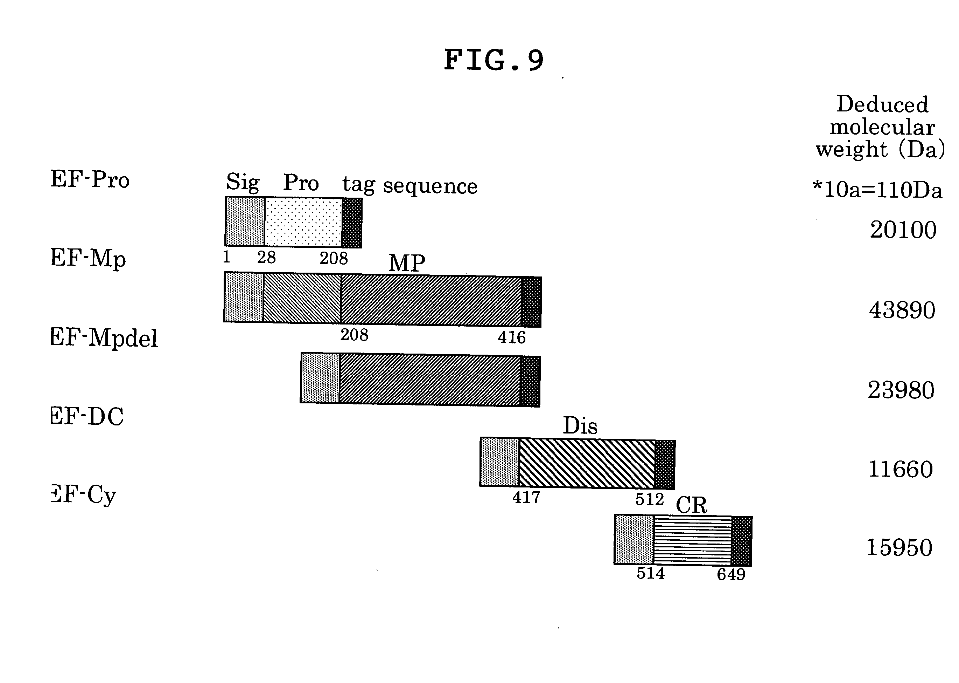

Plasmids pEF-Pro, pEF-Mp, pEF-Mpdel, pEF-DC, and pEF-Cy expressing the peptides containing various domains of the human meltrin α were constructed. Each plasmid was constructed so that the relevant peptide may include FLAG tag sequence at its C terminus, to thereby enable confirmation of the expression. FIG. 9 shows the constructed sequences with the deduced molecular weight. By using FuGENE6 (Roche Diagnostics), each of the plasmids pEF-Pro, pEF-Mp, pEF-Mpdel, pEF-DC and pEF-Cy was transfected into COS-1 cell. The cell was recovered with cell scraper (Costar), and washed with Dulbecco PBS (hereinafter sometimes referred to as D-PBS) to count the cell number. The cells were suspended in an appropriate amount of D-PBS, and after adding equal amount of Tris SDS sample treating solution (Daiichi Pure Chemicals Co., Ltd.), ultrasonicated (5202-P2T, Ohtake Works Company, Ltd., 50 W, 10 sec. x 3) to prepare extracts of the cells expressing the proteins containing various domains of the human meltrin α.

### (5) Confirmation of reactivity of proteins of various domains of human meltrin α with antibody

Expression of the proteins of various domains of the human meltrin α was confirmed by western blotting as described below. The extracts of the cells expressing various domains of the human meltrin α (each corresponding to 200 cells) and the meltrin α-FLAG obtained in Example 5 (200 ng) were electrophoresed by SDS-PAGE (5-20%, ATTO), and after the completion of the electrophoresis, the gels were transferred to PVDF membrane (Millipore). After the transfer, the membrane was blocked with 5% skim milk (Meiji Dairies Corporation)/T-PBS (T-PBS representing phosphate buffer solution containing 0.05% Tween 20, pH 7.4). Next, the membrane was reacted overnight at 4°C with anti-FLAG antibody (M2, SIGMA) or one of the mouse anti-meltrin α monoclonal antibodies prepared in Examples 6 to 7 which had been diluted with T-PBS containing 5% skim milk to 1.0 µg/mL, and washed with T-PBS. Subsequently, the membrane was reacted with peroxidase-labeled anti-mouse immunoglobulin antibody (DAKO) which had been 1000 fold diluted with T-PBS containing 0.5% bovine serum albumin (hereinafter sometimes referred to as BSA) at room temperature for 1 hour, and after washing the membrane as described above, the membrane was reacted with ECL-Plus (Amersham) to detect the resulting chemiluminescence with a cooled CCD camera (LightCapture, ATTO).

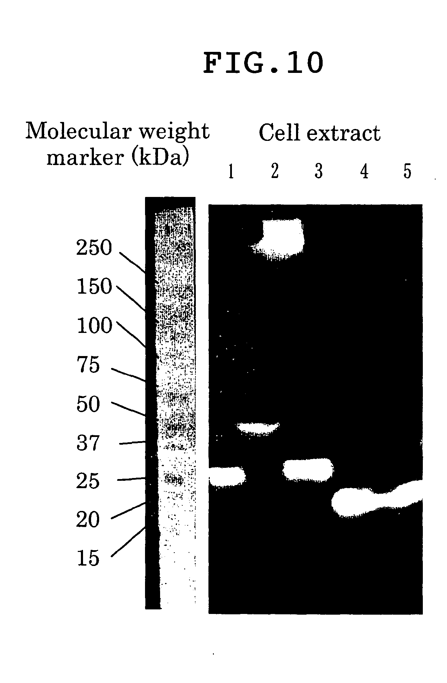

FIG. 10 shows the stained image obtained by using the anti-FLAG antibody. The image confirmed that the proteins containing various domains had been expressed with the deduced molecular weights. Similarly, for each mouse anti-human meltrin α monoclonal antibody, the domain recognized by the relevant antibody was analyzed. It was then revealed that the antibodies of Nos. 20, 101, 103, and 107 specifically bind to MP domain, and also, the antibodies of Nos. 18, 21, 88, and 119 bind to the cysteine rich domain.
It is to be noted that the antibodies whose specific bindings were confirmed had been prepared by using the knockout mouse except for the antibody No. 103.

### (Example 9) Measurement of protease activation ability of meltrin α

### (1) Activation of meltrin α

Thrombin (Amersham Biosciences) was added to the purified meltrin α-STFLAG prepared in Example 5, and mixture was kept at 25°C for 15 hours. When the solution after the reaction was examined by repeating the procedure of Example 5, the human meltrin α containing the prodomain (molecular weight 92 kDa) had been converted to mature meltrin α having the prodomain deleted (molecular weight 68 kDa). For measuring the enzymatic activity of the meltrin α, the solution after the prodomain cleavage with thrombin followed by inactivation of the thrombin by 10 mM APMSF (Wako Pure Chemical Industries, Ltd.) was used. The disappearance of the thrombin activity was confirmed by using the synthetic substrate for the thrombin on the reaction solution.

### (2) Measurement of protease activity of meltrin α

To a buffer solution containing 50 mM HEPES (pH 8.0), 100 mM CaCl₂, 0.05% Brij-35, and 0.01% BSA were added meltrin α from which prodomain had been cleaved by thrombin (final concentration 20 µg/mL) and recombinant human IGF-BP3 (GT2675 produced by GT, hereinafter referred to as rhIGF-BP3) as the IGF-BP3 which had been reported to be one of the substrates for the meltrin α (Frosty Loechel et al., BBRC (2000), 278, pp 511-515) (final concentration 0.5 µg/mL), and the mixture was allowed to react at 37°C for 24 hours. To the reaction solution was added an equal amount of tris SDS sample treating solution (Daiichi Pure Chemicals Co.,Ltd.), and the mixture was electrophoresed by SDS-PAGE(5-20%, ATTO). The gel was then transferred to a PVDF membrane (Millipore). After the transfer, the membrane was blocked with 5% skim milk (Meiji Dairies Corporation)/T-PBS (T-PBS representing phosphate buffer solution containing 0.05% Tween 20, pH 7.4). Next, the membrane was reacted at room temperature for 1 hour with biotinylated goat anti-human IGF-BP3 antibody (GT44675 produced by GT) which had been diluted with 0.5% bovine serum albumin (hereinafter sometimes referred to as BSA)/T-PBS to 0.1 µg/mL, and the membrane was washed with T-PBS. Subsequently, the membrane was reacted with peroxidase-labeled anti-streptavidin (GIBCO BRL) which had been 10000 fold diluted with 0.5% BSA/T-PBS, and after washing the membrane as described above, the membrane was reacted with ECL-Plus (Amersham) to detect the resulting chemiluminescence with a cooled CCD camera (LightCapture, ATTO) .

### (Example 10) Activity of anti-human meltrin α antibody for inhibiting protease activity of meltrin α

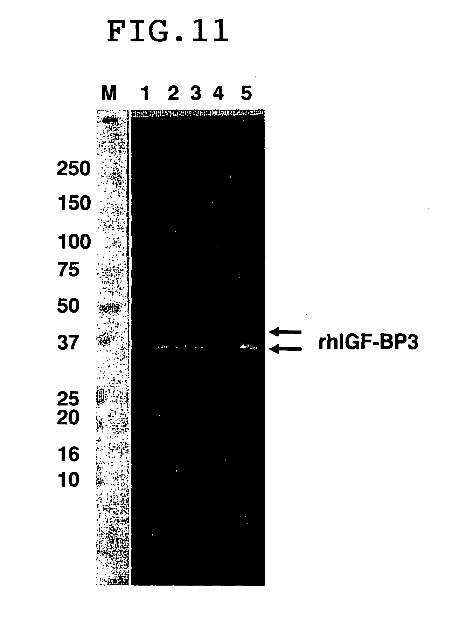

The anti-human meltrin α antibodies produced in Examples 6 and 7 were evaluated on their activity for inhibiting protease activity of meltrin α by using the protease activity assay system of Example 9. In the assay system of Example 9, before adding the rhIGF-BP3, the anti-human meltrin α antibody was added at a concentration of 300 µg/mL, and the reaction was allowed to take place for 30 minutes. rhIGF-BP3 was thereafter added, and the reaction was allowed to take place at 37°C for 24 hours. After the reaction, the reaction solution was electrophoresed by SDS-PAGE, and the rhIGF-BP3 was detected by western blotting. As confirmed in Example 9, the band corresponding to rhIGF-BP3 disappeared in the case of the meltrin α having the prodomain cleaved, while addition of the anti-human meltrin α antibodies of Nos. 20, 21, 107, and 129 suppressed disappearance of the rhIGF-BP3 band (FIG. 11).

These results have demonstrated that the protease activity of meltrin α is suppressed by the antibodies of Nos. 20, 21, 107, and 129. The antibodies were also evaluated on the specificity of their enzymatic inhibitory activity using MMP-1 (manufactured by BIOMOL) and MMP-9 (manufactured by Yagai Co.) with a commercially available fluorescent synthetic substrate (MMP-1/-9substrate (DNP-Pro-Cha-Gly-Cys(Me)-His-Ala-Lys(N-Me-Abz)-NH₂, CALBIOCHEM) according to the method of Bickett et al. (Anal. Biochem. 212, 58, 1993). It was then revealed that none of these antibodies had inhibitory activity for MMP-1 or MMP-9, and that the antibody of the present invention is not the one which non-specifically inhibits the metalloprotease.
It is to be noted that the antibodies which were found to have high neutralizing activity had been prepared by using the knockout mouse.

### (Example 11) Activity of low molecular weight compound for inhibiting protease activity of meltrin α

According to the method of Example 9, activity of the low molecular weight compound for inhibiting protease activity of the meltrin α was assayed as described below. In the protease activity assay system of Example 9, before adding the rhIGF-BP3, low molecular weight compound was added at a concentration of 10 µg/mL, and the reaction was allowed to take place for 30 minutes. rhIGF-BP3 was thereafter added, and the reaction was allowed to take place at 37°C for 24 hours. After the reaction, the reaction solution was electrophoresed by SDS-PAGE, and the rhIGF-BP3 was detected by western blotting.

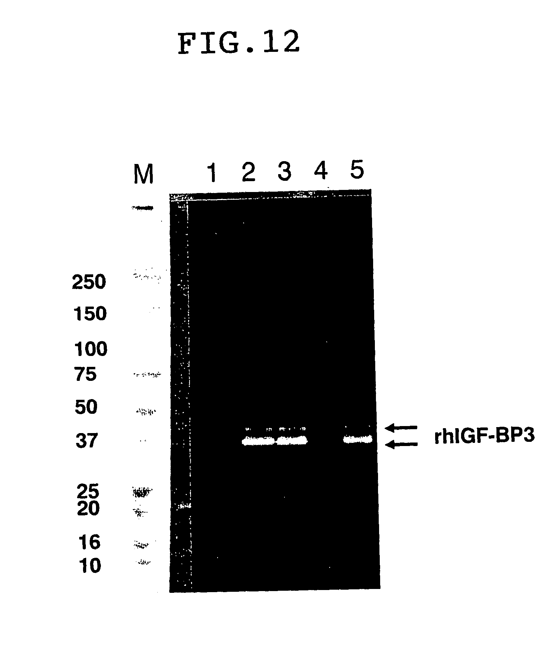

FIG. 12 shows the results for the low molecular weight compound 7-[phenyl(pyridine-2-ylamino)methyl]quinoline-8-ol (BAS0917466 manufactured by ASINEX and called M82463).
In the group added with the meltrin α having the prodomain cleaved, the band corresponding to the rhIGF-BP3 disappeared through the digestion by the protease activity. In contrast, addition of the M82463 suppressed the disappearance of the rhIGF-BP3 (FIG. 12). These results indicate that the low molecular weight compound M82463 suppresses the protease activity of meltrin α.

### (Example 12) Preparation of antisense DNA for meltrin α and its introduction into a cell

Oligo DNA were synthesized was performed (SIGMA Genosys Japan) to obtain a sequence HS0307A (SEQ ID NO: 27): CACGGGCAGCGGGCGCGCTGCCAT which is an antisense sequence for a part near the 5' end of the coding region of human meltrin α, and a sequence HS0307S(24) (SEQ ID NO: 28): ATGGCAGCGCGCCCGCTGCCCGTG which is the sense sequence for the same part and was used as a control, both having been modified with phosphorothioate.

First, the human preadipocyte for use in the assay (human preadipocytes-sq donor10547, POIETICS #PT-5001, Lot.3F0242, Sanko Junyaku Co., Ltd.) which had been adjusted to a cell concentration of 1 x 10⁵ cells/mL was added to a 96 well culture plate at 1 x 10² to 1 x 10⁴ cells/well/100 µL, and cultured overnight. In the meanwhile, the culture medium in which preadipocytes had been suspended at 1 x 10⁵ cells/mL was added to a 6 well plate at 1600 µL/well, and growth medium was also added to the plate at 400 µL/well, and the cells were cultured overnight. 1 µg to 50 µg of synthetic oligo DNA was added to serum free adipocyte growth medium or serum free D-MEM, and after adding 3 µL of FuGENE6 per 1 µg of oligo DNA, the mediums were allowed to stand at room temperature for 15 to 45 minutes. From the 6 well plate described above, 400 µL/well of the medium was removed and 400 µL/well of the culture medium containing the oligo DNA was added, and the plate was incubated at 37°C for 18 hours in the presence of 5% CO₂ to evaluate the level of expression of meltrin in Example 15. From the 96 well plate, 10 µL/well of the medium was removed and 10 µL/well of the culture medium containing the oligo DNA was added to evaluate of the adipocyte proliferation and differentiation in Example 16.

### (Example 13) Preparation of siRNA for meltrin α and its introduction into a cell

Sequence 1 (SEQ ID NO: 29): AUGCGAGAUGAGAGAUGCU(TT),
sequence 2 (SEQ ID NO: 30): GUGUGGAAUGACAUGGACA(TT),
sequence 3 (SEQ ID NO: 31): GAAUCAUCCAGAAGUGCUG(TT), and RNAs complementary to such sequences (SEQ ID NOS: 32 to 34) were synthesized (Takara Bio).
The complementary RNAs were mixed in a solution containing 100 mM potassium acetate, 30 mM HEPES-KOH (pH 7.4), and 2 mM magnesium acetate so that the siRNA concentration was 20 pmol/µL, and this solution was heated at 95°C for 1 minute and at 70°C for 1 minute, and then cooled for 1 hour to 37°C for annealing to thereby produce a double stranded siRNA.

In order to examine adipocyte proliferation and differentiation, 1185.7 µL of culture medium and 72 µL of RNA fect (QIAGEN) were mixed with 12 µg of the siRNA, and the mixture was allowed to stand at room temperature for 15 minutes. As in the case of Example 12, 25 µL/well of the culture medium was removed from the 96 well plate inoculated with the human preadipocyte, and 25 µL/well of the culture medium containing the siRNA was added for use in the examination of adipocyte proliferation and differentiation in Example 15. For the examination of the effect of suppressing the expression, as in the case of Example 12, 400 µL/well of the culture medium was removed from the 6 well plate inoculated with the human preadipocyte, and 400 µL/well of the culture medium containing the siRNA was added, and the plate was incubated at 37°C for 18 hours in the presence of 5% CO₂. RNA was thereafter recovered for use in Example 14.

### (Example 14) Quantitative determination of the human meltrin α expression in cell introduced the siRNA and the antisense oligonucleotide

### (1) Isolation of RNA

Culture medium was removed from the 6 well plates prepared in Examples 12 and 13, and the plates were washed twice with PBS(-).
After removing PBS from the plate, 0.5 mL/well of Trizol Reagent (Invitrogen) was added to the plate. The cells were lyzed by pipetting, and transferred to Eppendorf tube. Next, 100 µL of chloroform was added, and after stirring, the mixture was allowed to stand at room temperature for 2 to 2 minutes, and then, centrifuged at 15000 rpm and 4°C for 10 minutes. The supernatant was discarded, and the precipitate was washed with 75% ethanol and dried at room temperature. After drying, the precipitate was dissolved in water not containing RNase, and absorbance at a wavelength of 260 nm was measured to determine the RNA concentration (10D = 40 µg/mL).

### (2) Synthesis of cDNA

0.2 µg of the isolated RNA, and 2 µL of 10 x TaqMan buffer, 4.4 µL of 25mM MgCl₂, 4 µL of 2.5mM dNTP Mix, 1.0 µL of 50µM Random Hexamers, 0.4 µL of 20 U/µL RNase inhibitor, and 50U/µL MultiScribe Reverse Transcriptase (all purchased from Applied Biosystems) were mixed, and water was added to make up the total volume to 20 µL. This solution was maintained at 25°C for 10 minutes, at 48°C for 30 minutes, and at 95°C for 5 minutes, and reverse transcription was conducted to synthesize cDNA.

### (3) Real time PCR

A solution in total volume of 20 µL containing 10 µL of TaqMan Universal PCR Master Mix, 0.4 µL of 10 µM TaqMan MGB Probe (SEQ ID NO: 35) (sequence 5'FAM-caccgaaggacaatccca-MGB-3'), 0.1 µL each of 50 µM forward primer (SEQ ID NO: 36) (sequence, ggaagccgccagattcct) and reverse primer (SEQ ID NO: 37) (sequence, aggaagcactcgctgagttga) (Applied Biosystems) for detecting meltrin α, 5 µL of the cDNA solution of (2), and 4.4 µL of water was prepared, and after maintaining at 50°C for 2 minutes and at 95°C for 10 minutes, PCR was conducted by repeating 40 cycles of 95°C for 15 seconds and 60°C for 1 minute in ABI Prism 7000. A standard curve was generated by real time PCR using plasmid pEF-MelL containing full length human meltrin α for the template, and RNA copy number of meltrin α in each sample was determined.

### (4) Results

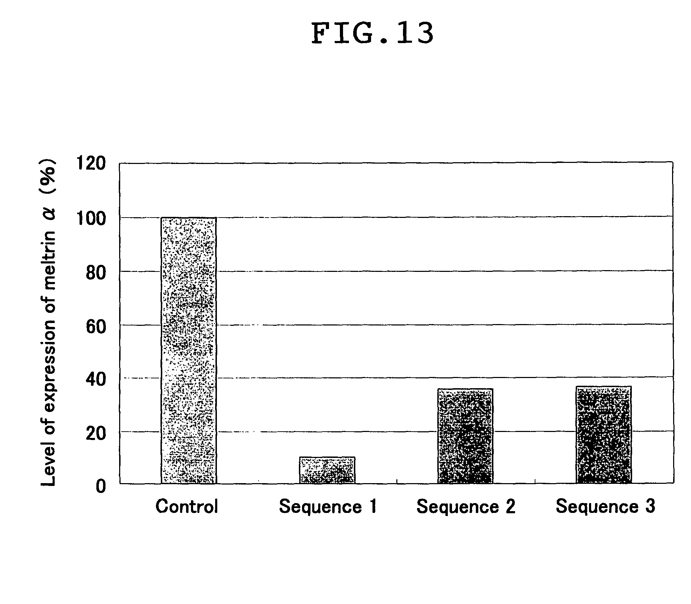

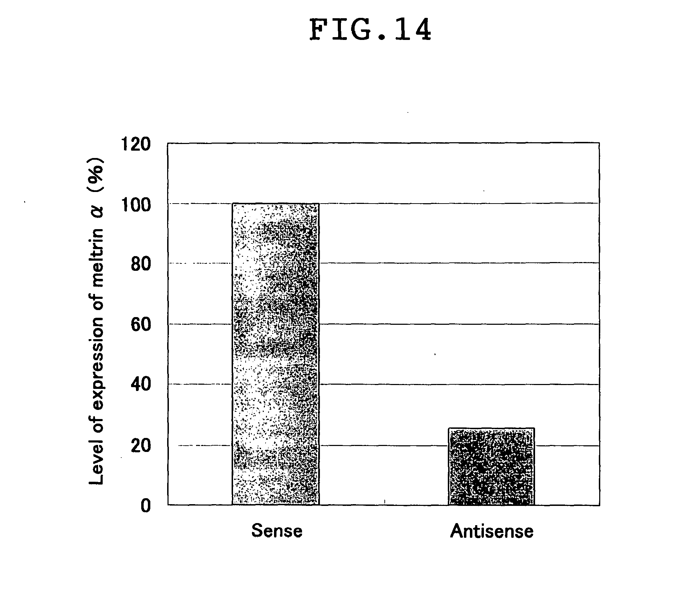

For all of the 3 used siRNAs, the level of expression of the meltrin α mRNA in the RNA extracted from the human preadipocyte transfected with the siRNA was suppressed by 60 to 90% compared to the control group (FIG. 13). The level of expression of the meltrin α in the human preadipocyte transfected with the antisense DNA was suppressed by about 70% when the level of expression of the meltrin α in the group transfected with the sense oligo DNA was 100% (FIG. 14). These results confirm that the siRNA and the antisense DNA used in this examination reduce the level of expression of the meltrin α mRNA in the human adipocyte.

### (Example 15) Examination of the effect of siRNA and antisense oligonucleotide on adipocyte proliferation and differentiation

### (1) Stimulation for differentiation of preadipocyte

The differentiation medium was prepared by adding supplement kit PGM SingleQuots, #PT-9500, Lot.08101163 (Sanko Junyaku Co., Ltd.) to 100 mL of growth medium. 50 µL of the supernatant was removed from the 96 well plate prepared in Example 12, and 50 µL of the siRNA or the antisense oligonucleotide prepared in the differentiation medium was added, and incubation was conducted in a CO₂ incubator for 5 days to 15 days.

### (2) Examination of the proliferation and differentiation

The amount of neutral fat accumulated in the adipocyte was determined by fluorometry using Adipo Red stain.
After taking the 96 well plate out of the CO₂ incubator, the plate was allowed to stand until it cooled to room temperature. The plate was centrifuged at 300 g (1200 rpm) for 10 minutes at room temperature to remove the medium. Next, the plate was gently washed with 200 µL/well of PBS (Sigma), and 200 µL/well of PBS was added to the well.
Next, 5 µL/well of Adipo Red Assay Reagent (Adipo Red, Cat. #PT-7009, Lot.3F0497, A Cambrex Company) was added, and the plate was allowed to stand at room temperature for at least 10 minutes to thereby stain the neutral fat that had accumulated in the adipocyte. The fluorescence intensity was measured in a fluorometer at an excitation wavelength of 485 nm and an emission wavelength of 535 nm to thereby determine the fat accumulation.

### (3) Examination of the toxicity for the adipocyte

Toxicity of the test substance to the adipocyte was examined by WST1 assay. More specifically, Premix WST-1 Cell Proliferation Assay System (Takara Shuzo Co., Ltd.) was added at 10 µL/well, and after incubating at 37°C for 60 minutes in a CO₂ incubator, absorbance (450 nm - 650 nm) was measured to examine influence on the adipocyte.

### (4) Results

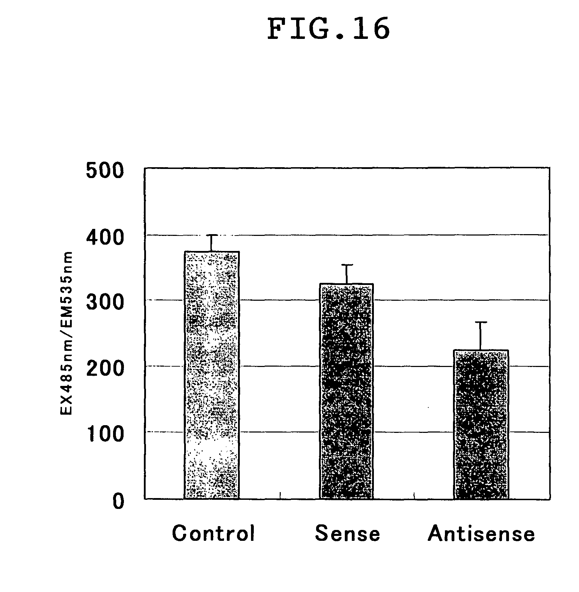

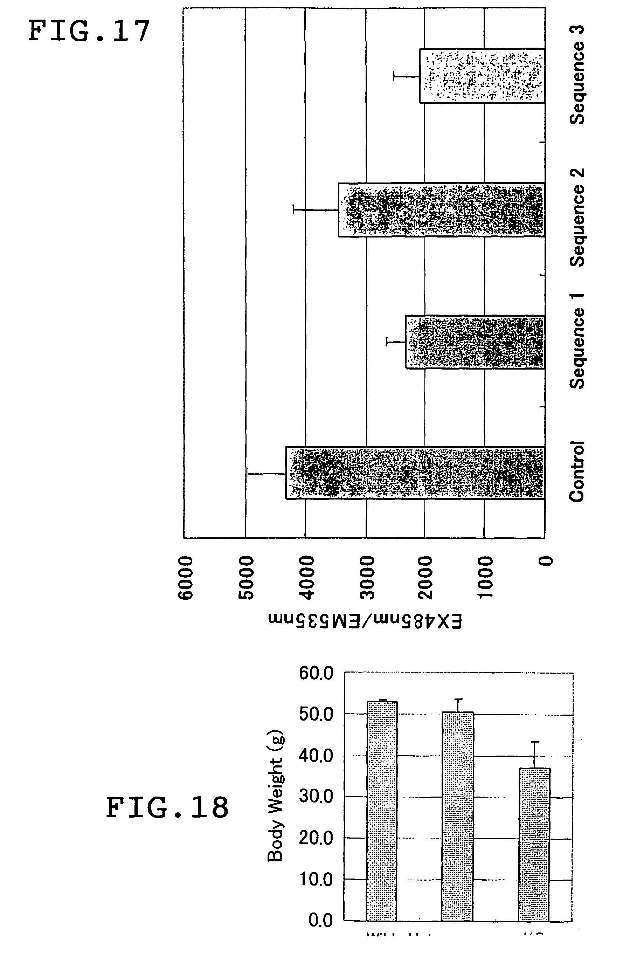

5 days after the differentiation stimulation, the adipocyte was stained with AdipoRed to examine the fat accumulation. It was then found that the accumulation of the neutral fat in the preadipocyte that has been given the differentiation stimulation was suppressed to a greater degree in the group transfected with the antisense oligonucleotide compared to the group transfected with the sense sequence (FIG. 16). In the group transfected with the siRNA, the adipocyte was stained with AdipoRed 12 days after the differentiation stimulation to examine the fat accumulation. It was then found that the accumulation of the neutral fat in the preadipocyte that has been given the differentiation stimulation was suppressed to a greater degree for all of the transfected siRNA sequences 1, 2, and 3 compared to the control group transfected with only the reagent (FIG. 17).

The results as described above revealed that the antisense oligo DNA and the siRNA which suppress the expression of the human meltrin α also suppress the proliferation and differentiation of the human adipocyte as well as the accumulation of the neutral fat in the adipocyte. More specifically, since the acquired suppression of the meltrin α gene expression enabled by the administration of the antisense or the siRNA has been found to realize the inhibition of the adipocyte proliferation and differentiation and suppression of the intracellular fat accumulation in addition to the finding of the obesity effect in the knockout mouse, suppression of the meltrin α expression has been shown to be effective in the antiobesity effect. Drugs such as the antisense oligonucleotide and the siRNA capable of suppressing the meltrin α expression are estimated to be quite effective when used as an antiobesity drug.

### (Example 16) Examination of suppressive effect of the low molecular weight compound on differentiation and proliferation of adipocyte

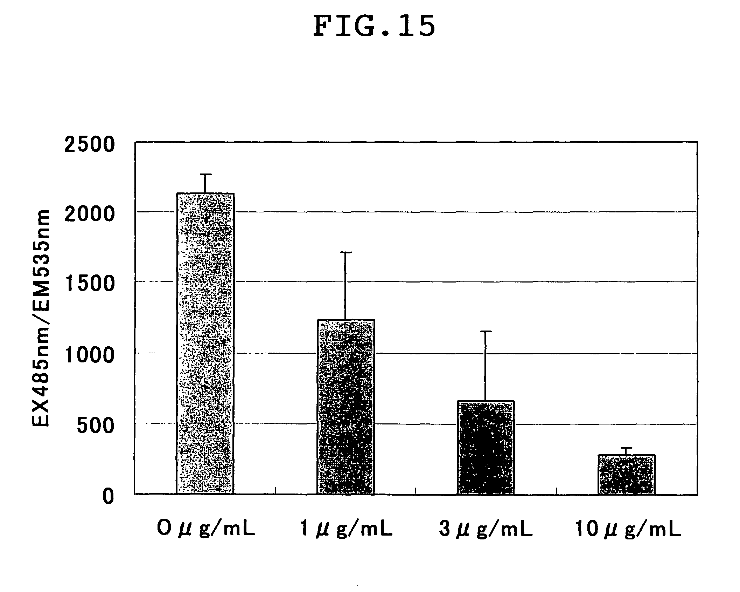

M82463 which was confirmed to have the inhibitory activity of the metalloprotease activity of the meltrin α in Example 11 was examined for its effect on the adipocyte proliferation and differentiation.
According to the method of Example 15, M82463 was added to the human preadipocyte incubation system at a concentration of 0.3 to 10 µg/mL so that the final concentration of the DMSO was 0.2%. As a control, a group having only the DMSO added to a final concentration of 0.2% was used. It was then found that M82463 suppressed the adipocyte proliferation and differentiation in a dose dependent manner (FIG. 15). It should also be noted that no cytotoxicity was found for the M82463 at the concentration used in the assay.

### (Example 17) Examination of suppressive effect of the anti-human meltrin a antibody on differentiation and proliferation of adipocyte

The anti-human meltrin α antibodies prepared in Examples 6 and 7 were dialyzed overnight against physiological saline, and they were quantitated by absorbance measurements at a wavelength of 280 nm (Factor = 1).
After preparation of the antibody to the concentration of 0.100 mg/mL with physiological saline, 9 volumes of adipocyte differentiation medium was added to 1 volume of the antibody solution, and the solution was sterilized by filtering through a filter having a diameter of 0.22 µm (MILLEX-GV 0.22 µm Filter unit, 3slgv013SL, Lot. R2NN83622, MILLIPORE). 50 µL/well of this culture medium containing the antibody was added to a 96 well plate having the human preadipocyte inoculated therein from which 50 µL of the medium had been removed. The final concentration of the added antibody was 5 µL/mL. 15 days after the addition of the medium, the cells were stained with AdipoRed according to the method of Example 15.
As a consequence, antibodies which suppress the accumulation of the neutral fat in the preadipocyte that has been given the differentiation stimulation and which suppress proliferation and differentiation of such cell were found from the anti-human meltrin α antibodies (Table 5). Most of the antibodies found to have the suppressive activity were obtained by using the knockout mouse.

The antibodies which showed clear effect on the adipocyte included antibodies which bonded to the metalloprotease domain or cysteine-rich domain as well as antibodies which had metalloprotease inhibitory activity. On the other hand, some antibodies had no influence on the metalloprotease activity. These results combined with the results for the low molecular weight compounds indicated that the substance which has the meltrin α antagonist effect should be effective as an antiobesity drug due to its suppressive effect on the accumulation of the neutral fat in the preadipocyte and suppressing the proliferation and the differentiation of the adipocyte. It was also indicated that the structure near the metalloprotease active site and the structure near the cysteine-rich domain are partly associated with such antiobesity action. Accordingly, the inventors believe that measurement of the capability of inhibiting the metalloprotease activity of the meltrin α could be used as one step in screening for meltrin α antagonists showing antiobesity activity.

[Table 5]

**Table 5: Effect of anti-meltrin α antibody on differentiation and proliferation system of human adipocyte**

| Antibody NO. | Suppressive effect | Antibody NO. | Suppressive effect | Antibody NO. | Suppressive effect |
|---|---|---|---|---|---|
| 1 | + | 17 | + | 75 | + |
| 2 | ++ | 18 | ++ | 80 | +++ |
| 3 | ++ | 21 | ++ | 84 | ++ |
| 4 | ++ | 22 | ++ | 87 | + |
| 6 | ++ | 23 | + | 96 | + |
| 7 | ++ | 25 | + | 98 | + |
| 8 | + | 34 | + | 101 | + |
| 9 | + | 39 | + | 102 | ++ |
| 10 | ++ | 46 | + | 105 | + |
| 11 | ++ | 56 | ++ | 107 | + |
| 12 | + | 59 | ++ | 110 | + |
| 14 | + | 66 | ++ | 111 | ++ |
| 15 | ++ | 72 | + | 116 | + |
| 16 | + | 73 | + | 118 | ++ |

| | | | | | |
|---|---|---|---|---|---|
| In the table, the antibodies were divided by the following criteria. +++: suppression by 70% or higher ++: suppression by 50% or higher +: suppression by 25-50% | | | | | |

### (Example 18) In vivo antiobesity effect of meltrin α antagonist

According to the procedure of Example 1, a high fat diet containing 60% of fat is prepared, and a low fat diet containing 10% of fat is also prepared as control. The mice are divided into the group feeding on the high fat diet and the group feeding on the low fat diet, and both groups are fed on the corresponding diets. The animals are administered with the meltrin α antagonist in the case of the antisense DNA or the siRNA, at a dose of 0.1 to 10 mg/kg and at a frequency of twice a day to once every two weeks, and in the case of the anti-meltrin α antibody, at a dose of 0.1 to 100 mg/kg at a frequency of once a week to once every two weeks for 2 to 15 weeks. The low molecular weight compound is administered at a dose of 0.1 to 100 mg/kg every day. During the test period, the body weight is measured every week, and the food intake is also periodically measured. As a consequence, in the group fed on the low fat diet, both the mice administered with the meltrin α antagonist and the mice administered with vehicle shows normal body weight. In contrast, in the group fed on the high fat diet, while the mice administered with the vehicle shows more significant increase in the body weight compared to the group fed on the low fat diet, increase in the body weight is suppressed in the mice administered with the meltrin α antagonist. This reveals that the antagonist which has the suppressive effect on the expression or the activity of the meltrin α has the antiobesity effect.
In addition, the animals are sacrificed at 2 to 15 weeks after the start of the administration, and organs are isolated. By analyzing the results of the organ weight measurement and the histological observation, the suppressive effect of the meltrin α antagonist on the visceral fat increase and the fatty liver formation can be confirmed.

### (Example 19) Enhancement by meltrin α antagonist of lipid and glucose metabolism after feeding on high fat diet

The effect of the meltrin α antagonist in enhancing energy metabolism of lipid and glucose after feeding on a high fat diet is evaluated by administering the meltrin antagonist to the mice having the obesity induced by feeding on a low fat diet or a high fat diet at a dose according to Example 18 for 1 to 15 weeks, administering ¹⁴C labeled palmitic acid or glucose from tail vein of the mice, and measuring the amount of the radioactivity transferred to each of the organ, urine, feces, and CO₂ in the exhalation according to the method of Example 2. When the meltrin α antagonist is administered, excretion of the RI to the exhalation after the administration of the palmitic acid and the glucose increases, and enhancement of the sugar and lipid metabolism can be confirmed in terms of such increase of the RI transfer. In the case of the palmitic acid administration, the amount of RI transferred to the feces is high compared to the mice administered with the vehicle, and in the case of the glucose administration, the amount of RI is high in urine, and swift metabolism and excretion of the glucose and lipid in the mice administered with the meltrin α antagonist is thereby confirmed.

### (Example 20) Examination of toxicity of meltrin α antagonist

Mice are administered with the meltrin antagonist at a dose according to Example 18. Significant toxicity is observed in none of the cases including fatal case irrespective of the dose. This demonstrates that the meltrin α antagonist of the present invention can be administered to a living organism as a therapeutic drug.

### Synthesis of antisense compound

Synthesis of the phosphorothioate antisense oligodeoxynucleotides having the nucleotide sequences described in Tables 6 and 7 and the chimeric antisense oligonucleotides having the nucleotide sequences described in Table 8 were subcontracted to PROLIGO Japan K.K., and the chemically synthesized antisense oligonucleotides were used as the samples in the following Example 21 or 22. In Table 8, the 5 nucleotides (described in the uppercase) on the 5' and 3' ends are 2'-methoxy nucleotides, and the intermediate 10 nucleotides (described in the lowercase) are phosphorothioate nucleotides.

### (Example 21) Suppression of human meltrin α expression by antisense compound

### (1) Introduction of antisense oligodeoxynucleotide

First, RD cell (ATCC) was suspended in DMEM containing 10% fetal bovine serum (FBS, SIGMA), and the suspension was inoculated in 6 well culture plate at 2.5 x 10⁵ cells/well/2 mL. After incubating overnight at 37°C in the presence of 5% CO₂, the cells were washed twice with serum-free DMEM, and Opti-MEM medium (GIBCO) was added at 800 µL/well.
Gene transfection reagent Lipofectin (Invitrogen) was diluted with Opti-MEM to 20 µg/mL, and the dilution was allowed to stand at room temperature for 30 minutes. Next, the antisense oligodeoxynucleotide synthesized as described above was heated at 90°C for 3 minutes, and after quenching on ice, diluted with Opti-MEM to a concentration 10 times higher than the final concentration. Equal amounts of the Lipofectin in Opti-MEM and the antisense oligo DNA in Opti-MEM were mixed, and the mixture was incubated at room temperature for 15 minutes and added to 6 well plate at 200 µL/well.
The culture was incubated at 37°C for 4 hours in the presence of 5% CO₂, and the culture supernatant containing the Lipofectin was removed. The cells were washed twice with serum-free DMEM, and after adding 1 mL/well of Opti-MEM and incubating for 18 hours at 37°C in the presence of 5% CO₂, the cells were subjected to the measurement of the level of expression of meltrin α RNA.

### (2) Isolation of RNA

Culture medium was removed from the 6 well plate prepared in the above (1), and the plate was washed twice with PBS(-).
After the washing, RNA was isolated from the cell by using RNA isolation kit (RNeasy Micro Kit, QIAGEN) according to the protocol of the kit, and RNA concentration was determined by measuring absorbance at a wavelength of 260 nm with a spectrophotometer (Nano Drop, LMS Co., Ltd.) (1OD = 40 µg/mL). After the quantitation, the RNA was prepared to a concentration of 0.1 µg/µL by using DEPC-treated water.

### (3) Synthesis of cDNA

0.35 µg of the isolated RNA, and 3.5 µL of 10 x TaqMan buffer, 7.7 µL of 25mM MgCl₂, 7.0 µL of 2.5mM each dNTP Mix, 1.75 µL of 50 µM Random Hexamers, 0.7 µL of 20U/µL RNase inhibitor, and 0.9 µL of 50U/µL MultiScribe Reverse Transcriptase (all purchased from Applied Biosystems) were mixed, and water was added to make up the total volume to 35 µL. This solution was maintained at 25°C for 10 minutes, at 48°C for 30 minutes, and at 95°C for 5 minutes, and reverse transcription was conducted to synthesize cDNA.

### (3) Real time PCR

A solution in total volume of 20 µL containing 10 µL of TaqMan Universal PCR Master Mix, 0.4 µL of 10 µM TaqMan MGB Probe (sequence 5'FAM-caccgaaggacaatccca-MGB-3': Applied Biosystems), 0.1 µL of 50 µM Forward primer (sequence ggaagccgccagattcct) for detecting meltrin α, 0.1 µL of 50 µM Reverse primer (sequence aggaagcactcgctgagttga) for detecting meltrin α (all purchased from Sigma-Genosys), 5 µL of the cDNA solution of (3), and 4.4 µL of water was prepared, and after maintaining the solution at 50°C for 2 minutes and at 95°C for 10 minutes, PCR was conducted by repeating 40 cycles of 95°C for 15 seconds and 60°C for 1 minute in ABI Prism 7000.
The amount of the human meltrin α RNA was measured by conducting real time PCR by using the plasmid containing the cDNA coding for the human meltrin α for the template to generate the standard curve, and calculating RNA copy number of the meltrin α in each sample. The level of expression of GAPDH was also determined by using a primer and probe set for real time PCR of human GAPDH (Pre-Developed TaqMan Assay Reagents Human GAPDH (20 x) (Cat# 4310884E, Applied Biosystems).

### (5) Calculation of suppression rate of expression

The results of the real time PCR of the meltrin α was corrected by the level of expression of the GAPDH to calculate the suppression rate. More specifically, [meltrin α threshold cycle (Ct) value of each sample/(GAPDH Ct value of each sample/GAPDH Ct value without addition of the sample)] was used for the corrected meltrin α Ct value of each sample, and after calculating the copy number from the corrected meltrin α Ct value of each sample, suppression rate of expression upon addition of each antisense oligodeoxynucleotide was determined by assuming the level of expression(copy number) of the meltrin α gene of the cell having no antisense oligodeoxynucleotide added as 100%. The results are shown in Table 6 to 8. The result in each concentration was rated "+" when the suppression rate was at least 30%, "++" when the suppression rate was at least 50%, and "+++" when the suppression rate was at least 70%.

[Table 6]

**Table 6**

| No. | Sequence | 200 nM | 50 nM |
|---|---|---|---|
| H001 | gactaggaagagcgttagtg | + | |
| H002 | actgtccgagttggcccggg | ++ | |
| H003 | ccgttgcaataaatgagcaa | + | |
| H004 | ctggcacaagccagccttga | +++ | + |
| H005 | tgcgtcgcgcgcgcgccgtt | ++ | ++ |
| H006 | tttccccccgtgtgtgtgcg | ++ | ++ |
| H007 | gcctttcatttttaaaaaag | | |
| H008 | gccgccgctgagctcttcta | ++ | ++ |
| H009 | agccctcgcgcacggcccgc | +++ | + |
| H010 | cctcggcgagtcagctccgg | ++ | ++ |
| H011 | gcgaccggagggatttcctg | ++ | + |
| H012 | gccgagcggggccgggcgtc | +++ | |
| H013 | ctgcaccatcccacgcgggc | ++ | + |
| H014 | ctcgggcccggcggcgagcg | +++ | |
| H015 | cggccttcagtgcagcagct | + | ++ |
| H016 | gggcgcgctgccatcgtcgc | ++ | + |
| H017 | gggcgggggacacgggcagc | ++ | |
| H018 | cagggcgagcaggagggcgc | ++ | + |
| H019 | ggcgcgagcagagcaccggc | ++ | |
| H020 | tcacccctcgggcctcgcag | ++ | |
| H021 | tcttccttggttccataagc | + | |
| H022 | gcactgacaacttcatcagc | | |
| H023 | ggtccccactccgaacagag | + | |
| H024 | gctcttcactgggatccaga | | |
| H025 | ggatgattcttggagtcgaa | | |
| H026 | gtcgaatattcagcacttct | + | |
| H027 | ttctttgctttcccgttgta | + | |
| H028 | ctttccagatttatgatcag | + | |
| H029 | tggcaatgagaccttcattt | + | |
| H030 | gtgggtttccgtgaaactgc | + | |
| H031 | tcagtaccgtcttgcagata | + | |
| H032 | aatttcgagcgagggagaca | + | |
| H033 | gtgacccagaattaccgtgt | + | |
| H034 | acatgtccatggtagtaaca | ++ | |
| H035 | ctgaatcagaatatccccgt | ++ | |
| H036 | acacgtgctgagactgactg | ++ | |
| H037 | ataagtcccctgagaccaga | + | |
| H038 | agctttcattttcaaacaca | | |
| H039 | tttcattggttctaagacat | | |
| H040 | ttgtatctgttggttgcact | + | |
| H041 | gcttcttcgctgggaagagt | + | |

**[Table 6-2]**

| | | | |
|---|---|---|---|
| H042 | tgatccccggacgcttttca | | |
| H043 | gtgttgtgatgtgatccaca | + | |
| H044 | tctttgcagcgaggtttggt | ++ | |
| H045 | agagggtggtggaaacacat | ++ | |
| H046 | tgccttcttgcccatgtctg | ++ | |
| H047 | ccttgagggtctctctttta | | |
| H048 | cagctccacatacttagttg | ++ | |
| H049 | cggttgtctgccacgatcac | + | |
| H050 | ttccttgcctctgaaactct | | |
| H051 | cttaactttttccagatctt | | |
| H052 | gcaatctctattaatcgctg | | |
| H053 | aaaacttgtcaacgtgatta | | |
| H054 | ccgaatgttcagtggtctgt | | |
| H055 | tccacgcctaccaacacgat | ++ | |
| H056 | tgtccatgtcattccacact | | |
| H057 | gtcctgacttacagagcatt | | |
| H058 | tcatggaggctggtgaatgg | ++ | |
| H059 | tcttcctccagtccagaaat | | |
| H060 | tttgcgaggtagaagcttca | | |
| H061 | agctgcgcattgtcatggga | + | |
| H062 | ggaaataaaccccactgaca | | |
| H063 | catgccgatggtggtccctt | ++ | |
| H064 | cacatgctcatgattggggc | | |
| H065 | ccccagactggtctgccgtg | ++ | |
| H066 | tgaatggtccatgacaattc | | |
| H067 | gctgcaccaaggggattgtc | | |
| H068 | gctcatgtgccagggtcacg | + | |
| H069 | catcccgaaattgtggccca | + | |
| H070 | ctgtccagtgtgtcatgatt | + | |
| H071 | ccatttgacagctacagccc | + | |
| H072 | gcagcctcctttctcaaccg | + | |
| H073 | ccggtggaagcgttcatgat | | |
| H074 | acaccatgggaaatgggtac | + | |
| H075 | cttcctgctgcaactgctga | + | |
| H076 | tccaggctggtctccaagtc | ++ | |
| H077 | ggcacacccccattcctttc | + | |
| H078 | gacttccggcaggttaaaca | | |
| H079 | tggcccccgaaagactccct | ++ | |
| H080 | caaatctgttcccacacttc | + | |
| H081 | acactcctctccttcttcca | | |
| H082 | tcctctggctccccacagtc | + | |
| H083 | tgcagcagcgattcatacat | | |

**[Table 6-3]**

| | | | |
|---|---|---|---|
| H084 | cagggtacaggtggtggcat | ++ | |
| H085 | gcgcacacagcgtccggctt | + | |
| H086 | cttcacagcacagcccatgt | | |
| H087 | tgcaggcttcagctggcagt | | |
| H088 | gagtccctgcacgctgttcc | + | |
| H089 | ggaggtcacaggagttgctg | + | |
| H090 | ggcccctgtgcagaactctg | ++ | |
| H091 | ttggctgggcagtgagggct | ++ | |
| H092 | gcccatcgtgcaggtacacg | ++ | |
| H093 | gtccacatcctgacatgagt | + | |
| H094 | atgccattgtagcagtagcc | + | |
| H095 | gctgctcgtgagtctggcag | | |
| H096 | tccccagagtgtgacacact | + | |
| H097 | ggggcaggtttagcacctgg | | |
| H098 | ctctctcaaagcagatccca | + | |
| H099 | aggatcacctgcagaattga | + | |
| H100 | actttgccacagttgccata | + | |
| H101 | tggcaaaggaactcttcgag | | |
| H102 | agcatctctcatctcgcatt | + | |
| H103 | cactggatttttccacattt | | |
| H104 | gccggctggcacctccttga | +++ | |
| H105 | ggcattggtaccaatgactg | + | |
| H106 | atgtttgtttctatggaaac | | |
| H107 | ggcctccttgctgcaggggg | ++ | |
| H108 | ggtcccccggcacagaatcc | | |
| H109 | tcatcgcccaagtacacgtg | ++ | |
| H110 | caagccctgggtccggcatg | ++ | + |
| H111 | acactttgtgcctgcaagca | + | |
| H112 | aggcagatttttccatctgc | | |
| H113 | tattttgacattgacgattc | | |
| H114 | gtgaaccccaaagacactaa | + | |
| H115 | tggcactgcattgcacactc | ++ | |
| H116 | tgttgcacacccctctgccg | | |
| H117 | gcagtggcagttcttcctgt | + | |
| H118 | ggaggtgcccagtgggcctc | ++ | + |
| H119 | agccaaacttgtcacagaag | + | |
| H120 | gctgtctgtgcttcctccaa | | |
| H121 | tctgcttgccggatggggcc | | |
| H122 | ctatggttaaaccttggtta | | |
| H123 | caggatggtcaccagaattc | + | |
| H124 | aatccggcagcaagaagaca | | |
| H125 | tccttttgagataaaccaca | | |

**[Table 6-4]**

| | | | |
|---|---|---|---|
| H126 | cagcagtcgtatcaaggtct | + | |
| H127 | gtggtcttcttatttgtaaa | | |
| H128 | cacaccttagtttttcaatg | | |
| H129 | gggtggccgggaagggcgca | ++ | |
| H130 | tgacagggttggaagccacg | +++ | ++ |
| H130+5 | gagcctgacagggttggaag | | ++ |
| H130+10 | gaggtgagcctgacagggtt | | + |
| H130+15 | tggccgaggtgagcctgaca | | ++ |
| H131 | caaggtggccgaggtgagcc | +++ | ++ |
| H131+5 | ttttccaaggtggccgaggt | | + |
| H131+10 | aggccttttccaaggtggcc | | + |
| H131+15 | tcatcaggccttttccaagg | | |
| H132 | cttcctcatcaggccttttc | ++ | |
| H132+5 | ggcggcttcctcatcaggcc | | |
| H132+10 | aatctggcggcttcctcatc | | |
| H132+15 | gtaggaatctggcggcttcc | | + |
| H132+16 | ggtaggaatctggcggcttc | | |
| H132+17 | gggtaggaatctggcggctt | | ++ |
| H132+18 | tgggtaggaatctggcggct | | + |
| H132+19 | gtgggtaggaatctggcggc | | + |
| H133 | ggtgggtaggaatctggcgg | +++ | +++ |
| H133+1 | cggtgggtaggaatctggcg | | ++ |
| H133+2 | tcggtgggtaggaatctggc | | ++ |
| H133+3 | ttcggtgggtaggaatctgg | | + |
| H133+4 | cttcggtgggtaggaatctg | | ++ |
| H133+5 | ccttcggtgggtaggaatct | | + |
| H133+10 | attgtccttcggtgggtagg | | |
| H133+15 | ctgggattgtccttcggtgg | | |
| H134 | atctcctgggattgtccttc | ++ | + |
| H134+5 | cagcaatctcctgggattgt | | |
| H134+10 | cactgcagcaatctcctggg | | |
| H134+15 | tctgacactgcagcaatctc | | |
| H135 | aacattctgacactgcagca | ++ | + |
| H135+5 | atgtcaacattctgacactg | | |
| H135+10 | tgctgatgtcaacattctga | | |
| H135+15 | gggtctgctgatgtcaacat | | |
| H136 | ttgaggggtctgctgatgtc | +++ | + |
| H136+5 | ggccgttgaggggtctgctg | | + |
| H136+10 | attcaggccgttgaggggtc | | |
| H136+15 | gggacattcaggccgttgag | | + |
| H136+16 | agggacattcaggccgttga | | + |
| H136+17 | gagggacattcaggccgttg | | + |

**[Table 6-5]**

| | | | |
|---|---|---|---|
| H136+18 | tgagggacattcaggccgtt | | ++ |
| H136+19 | ctgagggacattcaggccgt | | +++ |
| H137 | gctgagggacattcaggccg | ++ | ++ |
| H137+1 | ggctgagggacattcaggcc | | ++ |
| H137+2 | gggctgagggacattcaggc | | +++ |
| H137+3 | ggggctgagggacattcagg | | ++ |
| H137+4 | tggggctgagggacattcag | | ++ |
| H137+5 | ctggggctgagggacattca | | |
| H137+10 | gttgactggggctgagggac | | |
| H137+15 | gctgagttgactggggctga | | |
| H138 | cactcgctgagttgactggg | +++ | ++ |
| H138+5 | ggaagcactcgctgagttga | | |
| H138+10 | ggggaggaagcactcgctga | | |
| H138+15 | gtggaggggaggaagcactc | | |
| H139 | gcccggtggaggggaggaag | +++ | + |
| H139+5 | gtggggcccggtggagggga | | |
| H139+10 | tgcacgtggggcccggtgga | | + |
| H139+11 | gtgcacgtggggcccggtgg | | + |
| H139+12 | ggtgcacgtggggcccggtg | | + |
| H139+13 | aggtgcacgtggggcccggt | | + |
| H139+14 | taggtgcacgtggggcccgg | | ++ |
| H139+15 | ctaggtgcacgtggggcccg | | +++ |
| H139+16 | gctaggtgcacgtggggccc | | ++ |
| H139+17 | cgctaggtgcacgtggggcc | | + |
| H139+18 | acgctaggtgcacgtggggc | | ++ |
| H139+19 | gacgctaggtgcacgtgggg | | + |
| H140 | ggacgctaggtgcacgtggg | +++ | + |
| H140+5 | ggcagggacgctaggtgcac | | |
| H140+10 | ggtctggcagggacgctagg | | |
| H140+15 | gcaggggtctggcagggacg | | |
| H141 | ggctggcaggggtctggcag | ++ | |
| H141+5 | ggcttggctggcaggggtct | | |
| H141+10 | gtgcaggcttggctggcagg | | |
| H141+15 | cctaagtgcaggcttggctg | | + |
| H142 | gcctgcctaagtgcaggctt | ++ | ++ |
| H142+5 | cctgggcctgcctaagtgca | | |
| H142+10 | ggtcccctgggcctgcctaa | | |
| H142+15 | ttacaggtcccctgggcctg | | |
| H143 | ttggcttacaggtcccctgg | +++ | + |
| H143+5 | ggggtttggcttacaggtcc | | ++ |
| H143+6 | gggggtttggcttacaggtc | | |
| H143+7 | ggggggtttggcttacaggt | | |

**[Table 6-6]**

| | | | |
|---|---|---|---|
| H143+8 | aggggggtttggcttacagg | | |
| H143+9 | gaggggggtttggcttacag | | + |
| H143+10 | tgaggggggtttggcttaca | | + |
| H143+11 | ctgaggggggtttggcttac | | |
| H143+12 | tctgaggggggtttggctta | | + |
| H143+13 | ttctgaggggggtttggctt | | |
| H143+14 | cttctgaggggggtttggct | | + |
| H143+15 | gcttctgaggggggtttggc | | +++ |
| H144 | cagaggcttctgaggggggt | ++ | + |
| H144+5 | gcaggcagaggcttctgagg | | ++ |
| H144+6 | tgcaggcagaggcttctgag | | ++ |
| H144+7 | ctgcaggcagaggcttctga | | ++ |
| H144+8 | tctgcaggcagaggcttctg | | + |
| H144+9 | atctgcaggcagaggcttct | | + |
| H144+10 | gatctgcaggcagaggcttc | | +++ |
| H144+11 | ggatctgcaggcagaggctt | | ++ |
| H144+12 | aggatctgcaggcagaggct | | +++ |
| H144+13 | gaggatctgcaggcagaggc | | ++ |
| H144+14 | agaggatctgcaggcagagg | | ++ |
| H144+15 | cagaggatctgcaggcagag | | + |
| H144+16 | ccagaggatctgcaggcaga | | ++ |
| H144+17 | gccagaggatctgcaggcag | | +++ |
| H144+18 | ggccagaggatctgcaggca | | +++ |
| H144+19 | tggccagaggatctgcaggc | | +++ |
| H145 | ctggccagaggatctgcagg | +++ | +++ |
| H145+1 | tctggccagaggatctgcag | | +++ |
| H145+2 | ttctggccagaggatctgca | | +++ |
| H145+3 | gttctggccagaggatctgc | | ++ |
| H145+4 | tgttctggccagaggatctg | | ++ |
| H145+5 | ttgttctggccagaggatct | | +++ |
| H145+6 | gttgttctggccagaggatc | | |
| H145+7 | agttgttctggccagaggat | | |
| H145+8 | gagttgttctggccagagga | | |
| H145+9 | cgagttgttctggccagagg | | |
| H145+10 | ccgagttgttctggccagag | | ++ |
| H145+15 | gtgagccgagttgttctggc | | |
| H146 | catgagtgagccgagttgtt | ++ | + |
| H146+5 | caaggcatgagtgagccgag | | |
| H146+10 | ctggccaaggcatgagtgag | | + |
| H146+15 | gggtcctggccaaggcatga | | |
| H147 | tcctggggtcctggccaagg | +++ | ++ |
| H147+5 | cattgtcctggggtcctggc | | |

**[Table 6-7]**

| | | | |
|---|---|---|---|
| H147+10 | tctcccattgtcctggggtc | | |
| H146+15 | cccagtctcccattgtcctg | | |
| H148 | cggagcccagtctcccattg | ++ | + |
| H148+5 | ccaggcggagcccagtctcc | | + |
| H148+10 | gggtgccaggcggagcccag | | ++ |
| H148+15 | ctgaggggtgccaggcggag | | |
| H149 | caggtctgaggggtgccagg | +++ | + |
| H149+5 | tggagcaggtctgaggggtg | | |
| H149+10 | tattgtggagcaggtctgag | | + |
| H149+15 | gtggatattgtggagcaggt | | |
| H150 | ttggtgtggatattgtggag | +++ | + |
| H150+5 | ggcacttggtgtggatattg | | + |
| H150+10 | atctgggcacttggtgtgga | | ++ |
| H150+15 | ggtggatctgggcacttggt | | + |
| H151 | gtgtgggtggatctgggcac | +++ | ++ |
| H152 | cttctcacttaatataggcg | + | |
| H153 | ctgttgaaaaaaggtgtcgg | ++ | |
| H154 | agtgcaaacttctgtcttca | | |
| H155 | tccaactggagctgaaagat | ++ | |
| H156 | taaaagttggtacaaaaaac | + | |
| H157 | ttaaacattaaaaaaaatcc | | |
| H158 | gttcttatagtaatgatgtt | | |
| H159 | actgacggcagtagctcaaa | ++ | |
| H160 | gcaccatagcacagcacagc | ++ | |
| H161 | tacctgtgcaagtagacaga | ++ | |
| H162 | ataaattaataatttacaag | | |
| H163 | cactgtaatcaacattctgc | + | |
| H164 | atgcctactacagcgcactg | ++ | |
| H165 | aaaactcagtgatggtaaaa | + | |
| H166 | aacaagccttcctgccatgg | ++ | |
| H167 | cactaaaatactaaaagcac | | |
| H168 | caagcaggatatttcaagtt | + | |
| H169 | catcctgtccagaatcccat | ++ | |
| H170 | ccttgatcagaaagcaaaca | + | |
| H171 | gggactgctttccaataagg | ++ | |
| H172 | gcacagctgggggtagttgg | +++ | + |
| H173 | agctgcatctggtaccataa | +++ | ++ |
| H174 | attctacttgggatctcttg | | |
| H175 | aatccagaaaatcaactgag | | |
| H176 | ggctctggcctgagatgggg | + | |
| H177 | gcctggacctgaagcccctt | +++ | |
| H178 | ctccctgaaagccaaacaca | + | |

**[Table 6-8]**

| | | | |
|---|---|---|---|
| H179 | gttgtcaaggggcacagggc | ++ | |
| H180 | ccctgggagcctgcctgcca | ++ | |
| H181 | gccagatttctcccaggtgt | ++ | |
| H182 | ccaaagcttcctggccagaa | ++ | |
| H183 | gtctgcaacccaggttctca | ++ | |
| H184 | ggctacaccttaagattcct | + | |
| H185 | tccagtctctatcctggtgt | | |
| H186 | agttctggcttgtctagtgt | | |
| H187 | ggctggtcagctcagggtca | +++ | |
| H188 | ccccttccaaacatgctcac | + | |
| H189 | gccttgagtgacactacaga | + | |
| H190 | tggcatttctatcaagcacc | | |
| H191 | cagcgagaaaaagaagtgct | | |
| H192 | tggcagtgctctagaaagga | | |
| H193 | caagctaaataacctactgg | | |
| H194 | tacagaaacaccacctttcc | | |
| H195 | tgcctgggcagtaggtttct | ++ | |
| H196 | agggaggtggcggtttgcag | +++ | + |
| H197 | tgctcagctccaagcagtat | ++ | |
| H198 | tattacagtttgtggtgatt | | |
| H199 | gtctgaatacaggatcattg | | |
| H200 | cccatggaaagtcctcatct | | |
| H201 | catctgaaaatagttgtggt | | |
| H202 | tagatctggttaatggttca | | |
| H203 | agtaaacagacttgattgac | | |
| H204 | gttaataagttgaaccttgc | | |
| H205 | cataaagagtctgcctaatt | | |
| H206 | ttggttgtagtttttgcaag | | |
| H207 | cccatgaacatcacattcca | | |
| H208 | gatagcagacatgaactata | | |
| H209 | gtccaatatctacgaataat | | |
| H210 | ccccatagagaaggttcttt | ++ | |
| H211 | caagttggaaaaagaggatg | | |
| H212 | cttttaaagattcctgcagc | | |
| H213 | tcagactctgttaaaagcat | | |
| H214 | caagtgtttaagaaataggt | | |
| H215 | gatgctcaacaggtaggttg | | |
| H216 | atttccttatcacattctgt | | |
| H217 | ggaagttgataagcaagttg | +++ | |
| H218 | gccacatctcataatattta | | |
| H219 | ttcaaggggatgctgcccaa | +++ | + |
| H220 | ggcatttgaagagtgaagag | | |

**[Table 6-9]**

| | | | |
|---|---|---|---|
| H221 | gaaacatggctccctagtca | + | |
| H222 | agtcactttaaagaccttgt | | |
| H223 | tttgtatttctcatgccatt | | |
| H224 | ttttaccttatctgagtatt | | |
| H225 | aagacagaggcatcatggca | +++ | |
| H226 | taatgtgaaaaccagtccag | | |
| H227 | aactgttgtcaattgtcttc | | |
| H228 | acactcagagtgaattatgt | | |
| H229 | aaagaaggctttctcataaa | + | |
| H230 | taggaaaactgttgacccca | ++ | |
| H231 | tatttttctgtttcaaagca | | |
| H232 | aaaccaagattcttggtaca | | |
| H233 | agttttgttttctggaaggc | + | |
| H234 | acaccgggaaagtgaaatgc | ++ | |
| H235 | ttgcctagatacagtgggga | ++ | |
| H236 | ccatagtcatgaatactatg | | |
| H237 | gtgtcacgtgtttagtttat | + | |
| H238 | gttcccttttgtgtgtgttt | | |
| H239 | ttggaatgtattagagctgg | + | |
| H240 | acagatgcatgctatacgag | ++ | |
| H241 | acttaataactatagaataa | | |
| H242 | atggctttacattttaaaga | | |
| H243 | cagcagtattattttccagc | + | |
| H244 | cagtaattctgtatgtatct | | |
| H245 | ttaccaagtgtaatcagtta | | |
| H246 | tatgtttggctttagtacaa | | |
| H247 | aacctttttaatagtatata | + | |
| H248 | atgcaccataaaattctgta | | |
| H249 | aaaaagacaatgcccacgta | ++ | |
| H250 | atctaaggatttgggcatct | + | |
| H251 | aggaagggctaacatgccag | +++ | |
| H252 | tcatatcctcttataattgg | | |

**Table 7**

| No. | Sequence | Supression of expression, 50nM | Supression of adipose differentiation 300 nM |
|---|---|---|---|
| 1 | gttgcaagtgtttaagaaat | | |
| 2 | ctggttaatggttcacatct | ++ | ++ |
| 3 | aaacatgctcacggctggtc | ++ | ++ |
| 4 | ggtcctggccaaggcatgag | + | |
| 5 | gtttggcttacaggtcccct | ++ | ++ |
| 6 | gctttacattttaaagaact | | |
| 7 | tagaataaacagatgcatgc | + | |
| 8 | acactcagagtgaattatgt | | |
| 9 | ctgtctgcaacccaggttct | + | |
| 10 | ttgaccccaaaagaaggctt | | |
| 11 | ctacgaataatgatagcaga | | |
| 12 | tgctctagaaaggacagcga | | |
| 13 | atactatgttgcctagatac | | |
| 14 | caagattcttggtacatatt | | |
| 15 | gattctacttgggatctctt | | |
| 16 | aatcagttacagtaattctg | | |
| 17 | ggttcacatctgaaaatagt | | |
| 18 | ttccaaacatgctcacggct | | |
| 19 | tgttgcctagatacagtggg | | |
| 20 | agcacagcacagcactgacg | | |
| 21 | tcaaagcataggaaaactgt | | |
| 22 | gactgctttccaataaggcc | | |
| 23 | gaattatgtaactgttgtca | | |
| 24 | tcttatttgtaaacagcagt | | |
| 25 | atgccagatctaaggatttg | | |
| 26 | atggcattttaccttatctg | | |
| 27 | ttgactagatctggttaatg | | |
| 28 | tcccaagctaaataacctac | | |
| 29 | taaaacactcagagtgaatt | | |
| 30 | agcaggatatttcaagttca | | |
| 31 | tttctatcaagcaccgcctt | | |
| 32 | tcactttaaagaccttgtga | | |
| 33 | tggctctggcctgagatggg | | |
| 34 | acattctgcataaattaata | | |
| 35 | gcacacaccttagtttttca | | |
| 36 | tcaagttcactaaaatacta | | |
| 37 | ccacatctcataatatttag | | |

**Table 8**

| No. | Sequence | 200 nM |
|---|---|---|
| H075-OMe1 | TGCTGcaactgctgaACACC | + |
| H075-OMe2 | AAGTCcttcctgctgCAACT | + |
| H076-OMe1 | GCTGGtctccaagtcCTTCC | ++ |
| H076-OMe2 | CTTTCtccaggctggTCTCC | +++ |
| H077-OMe1 | ACCCCcattcctttcTCCAG | |
| H077-OMe2 | AAACAggcacaccccCATTC | + |

### (Example 22) Effect of antisense compound on the adipocyte proliferation and differentiation

### (1) Cell inoculation

First, human preadipocyte (Human Preadipocytes-sq POIETICS #PT-5020, Sanko Junyaku Co., Ltd.) was suspended in growth medium (#PT-8200, Sanko Junyaku Co., Ltd.), and after adding the suspension to a 96 well culture plate at 1 x 10⁴ cells/well/100 µL, the cells were cultured overnight at 37°C in the presence of 5% CO₂.

### (2) Introduction of antisense oligodeoxynucleotide

The preadipocyte was cultured overnight, and after washing the preadipocyte twice with serum-free DMEM, Opti-MEM was added at 80 µL/well.
Gene transfection reagent Lipofectin was diluted with Opti-MEM to 20 µg/mL, and the dilution was allowed to stand at room temperature for 30 minutes. Next, the antisense oligodeoxynucleotide synthesized as described above was heated at 90°C for 3 minutes, and after quenching on ice, diluted with Opti-MEM to a concentration 10 times higher than the final concentration. Equal amounts of the Lipofectin in Opti-MEM and the antisense oligodeoxynucleotide in Opti-MEM were mixed, and the mixture was incubated at room temperature for 15 minutes and added to 96 well plate at 20 µL/well. After incubating at 37°C for 4 hours in the presence of 5% CO₂, it was used for examination as described below.

### (3) Stimulation for differentiation of preadipocyte

The differentiation medium was prepared by using growth medium (#PT-8200, Sanko Junyaku Co., Ltd.) and supplement kit (PGM Single Quots, #PT-9500, Sanko Junyaku Co., Ltd.) according to the instruction of the kit. Next, the culture supernatant containing the Lipofectin was removed from the 96 well plate prepared in the above (2), and the plate was washed twice with serum-free DMEM, and the differentiation medium prepared as described above was added at 100 µL/well. The plate was then incubated at 37°C for 5 days to 15 days in the presence of 5% CO₂.

### (4) Examination of the proliferation and differentiation

Accumulation of the neutral fat in the adipocyte was determined by fluorometry using Adipo Red stain.
After taking the 96 well plate out of the CO₂ incubator, the plate was allowed to stand until it reached room temperature. The plate was centrifuged at 300 g (1200 rpm) for 10 minutes at room temperature to remove the medium. Next, after gently washing with PBS (SIGMA) at 200 µL/well, PBS was added to the well at 200 µL/well.
Next, Adipo Red Assay Reagent (Adipo Red, Cat. #PT-7009, Lot.3F0497, A Cambrex Company) was added at 5 µL/well, and the plate was allowed to stand at room temperature for at least 10 minutes to thereby stain the neutral fat accumulated in the adipocyte. The fat accumulation was determined by measuring fluorescence intensity in a fluorometer at an excitation wavelength of 485 nm and an emission wavelength of 535 nm.

### (5) Evaluation of the adipocyte differentiation

The suppression rate of the adipocyte differentiation was calculated by converting the fluorescence intensity of the well transfected with the antisense compound in relation to the fluorescence intensity of the well without transfection with the antisense compound which was assumed as 100%. The results are shown in Table 7. The result was rated "+" when the suppression rate was at least 25%, "++" when the suppression rate was at least 50%, and "+++" when the suppression rate was at least 75%.

### (6) Effect of the antisense compound on the adipocyte proliferation and differentiation

Of the antisense compounds described in Table 6, the effect of suppressing the human adipocyte differentiation was examined for those described in Table 9 by the method of Example 21. It was then revealed that the antisense compounds of Table 9 also suppresses the differentiation of the human adipocyte.

**Table 9**

| Sequence name | Score |
|---|---|
| H005 | ++ |
| H130 | +++ |
| H131 | +++ |
| H133+2 | + |
| H135+15 | + |
| H136+19 | + |
| H139+14 | ++ |
| H143+15 | ++ |
| H144+18 | ++ |
| H151 | ++ |

### (Example 23) Examination of RNase H-dependent activity of the antisense compound

For the antisense compounds which showed the activity of suppressing the meltrin α expression in Example 21, antisense compounds having all of the nucleotides in the sequence modified with 2'-methoxy (2'-O-CH₃) was synthesized to prevent recognition by RNase H. These antisense compounds and the original phosphorothioate form were compared for their activity of suppressing the meltrin α expression according to the method of Example 21. It was then found that, of the sequences shown in Table 9, sequence numbers H130, H131, H144+18, and H151 exhibited reduced activity of suppressing the meltrin α expression in the 2'-methoxy modified form compared to the phosphorothioate form, confirming the RNase H dependency of such activity.

### (Example 24) Effect of meltrin α on blood parameters on a high fat diet

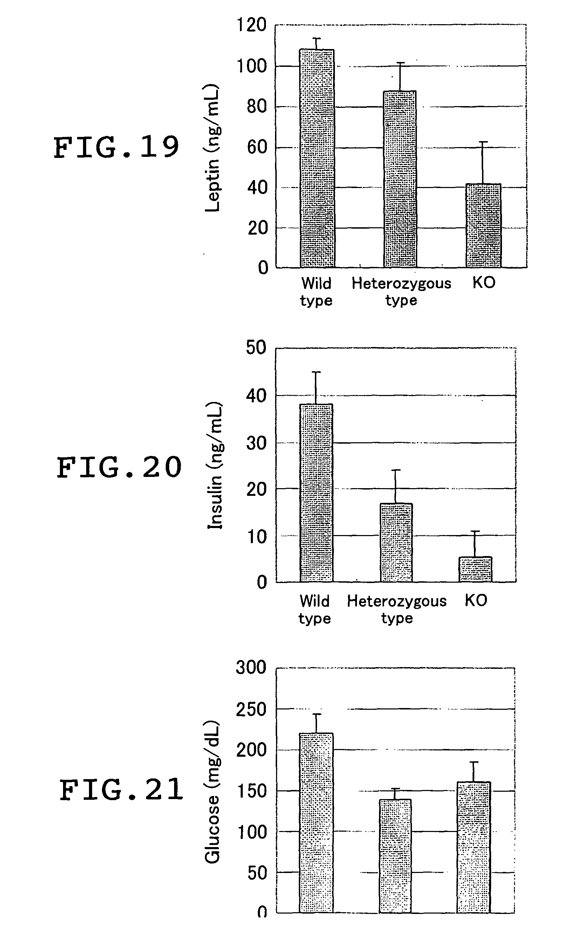

In order to examine the effect of meltrin α on glucose metabolism, meltrin α knockout mice and meltrin α heterozygous mice produced by Kurisaki et al. (Mol. Cell. Biol. 23: 55-61; 2003) and wild-type mice were fed on a high fat diet for 24 weeks, and body weight, and insulin, leptin, and glucose concentrations in blood of the individual mice were measured. Insulin concentration was measured by Levis R insulin - mouse U type (Shibayagi), and leptin concentration was measured by mouse leptin assay kit (Morinaga). Glucose was measured by using glucose CII Test Wako (Wako Pure Chemical Industries, Ltd.). As shown in FIGS. 18 to 21, inhibition of the meltrin α expression resulted, in the case of knockout mice, in the prevention of increase in the body weight as well as blood insulin concentration and blood glucose level on the high fat diet, and in the case of heterozygous mice, in the prevention of increase in the blood insulin concentration and the blood glucose level on the high fat diet stress. These results indicate that, since the wild-type mice and the heterozygous mice are similar in their body weight, the effect on the blood insulin concentration and the blood glucose level is not the simple result of suppressing the increase of the body weight. This in turn indicates that, when the meltrin α is suppressed, not only the development of obesity (antiobesity action) but also development of hyperglycemia and hyperinsulinemia is also suppressed (anti-diabetic action).

### (Example 25) Preventive and therapeutic effects of the meltrin α antagonist on diabetes

### (1) Effect on type 1 diabetes

Streptozotocin (STZ) diabetic rat model which is a type 1 diabetic animal model is used to investigate preventive and therapeutic effect of the meltrin α antagonist on type 1 diabetes. Male SD rat of about 4 week old is intraperitoneally administered with 65 mg/kg of streptozotocin to induce diabetes. The rat which exhibits hyperglycemia, urinary sugar, hyperphagia, and polydipsia after 1 week is used for the following experiment. The thus prepared STZ diabetic rat model is administered with meltrin α antagonist at a dose according to Example 18 for 1 to 15 weeks. Blood is collected from the tail vein after 20 hours of fasting at every week after the start of housing, and the blood glucose level is measured. In addition, glucose tolerance test is conducted from week 0 at every two weeks. More specifically, after 20 hours of fasting, 2 g/kg of glucose is orally administered, and blood is collected from the tail vain before the glucose administration (0 hour), and at 0 hour, 0.5 hour, 1 hour, 1.5 hours, and 2 hours after the administration, respectively to measure the blood glucose level. Prevention in the increase of fasting blood glucose is confirmed from the value of fasting blood glucose in the test period, and/or improvement in the glucose tolerance is confirmed from the results of glucose tolerance test, and the preventive and therapeutic effects of the meltrin α antagonist on type 1 diabetes is thereby demonstrated.

### (2) Effect on type 2 diabetes

As in the case of type 1 diabetes, effect of the meltrin α antagonist is examined by using KKAY diabetic mouse model which is a type 2 diabetic animal model. After preliminarily housing KKAY/TaJcl mice of about 4 week old for 1 week, the meltrin α antagonist is administered to the mice at a dose according to Example 18 for 1 to 15 weeks. Blood is collected from the ocular fundus of the mice having fasted for 5 hours with a blood collection capillary at every week from the start of housing and the blood glucose level is measured. Glucose tolerance test is also conducted at 5 weeks after the start of the housing. More specifically, after 20 hours of fasting, 2 g/kg of glucose is orally administered, and blood is collected from the ocular fundus before the glucose administration (0 hour), and at 0.5 hour, 1 hour, 1.5 hours, and 2 hours after the administration, respectively to measure the blood glucose and blood insulin levels. Prevention in the increase of fasting blood glucose or blood insulin is confirmed from the value of fasting blood glucose in the test period, and/or improvement in the glucose tolerance is confirmed from the results of glucose tolerance test, and the preventive and therapeutic effects of the meltrin α antagonist on type 2 diabetes is thereby demonstrated.

### (Example 26) Amino acid sequencing of variable region of the anti-meltrin α antibody

Amino acid sequences of the variable regions were determined for the antibody having the inhibitory activity for the meltrin α protease activity confirmed in Example 10 (antibody No. 129, clone No. F1196-152-2) and the anti-meltrin α antibodies (F1196-114-2 and F1196-113-1) whose inhibitory activity for the meltrin α protease activity has been confirmed by the procedure similar to that of Example 10. More specifically, total RNA was extracted from the hybridoma producing the desired anti-meltrin α monoclonal antibody by using RNeasy Micro Kit (QIAGEN), and single stranded cDNA was synthesized by SuperScript III First-Strand Synthesis System for RT-PCR kit (Invitrogen). The variable regions were amplified by the PCR using the resulting single stranded cDNA for the template with Mouse Ig-Primer Set (Novagen), and the nucleotide sequences of the heavy chain variable region and the light chain variable region were determined by using the DNA fragment produced by this PCR amplification for the tempelate according to the method normally used in the art. Nucleotide sequences (heavy chain variable region, SEQ ID NOS: 39 to 41; light chain variable region, SEQ ID NOS: 42 to 44) of clone Nos. F1196-113-1, F1196-114-2, and F1196-152-2 and the amino acid sequences encoded by such sequences (heavy chain variable region, SEQ ID NOS: 45 to 47; light chain variable region, SEQ ID NOS: 48 to 52) are shown in Sequence Listing and Table 10. Among these, about 50 nucleotides at the 5' end was unreadable for the light chain of F1196-152-2, and therefore, in the Sequence Listing, sequence of several nucleotides deduced by comparing with the most homologous sequence in the open database (XM_485751) is indicated as "n". The sequence of the CDR region in the amino acid sequence of the variable region is shown in Table 10 according to the definition of Kabat. The 3 hybridomas producing such monoclonal antibodies were internationally deposited on August 2, 2005 to the National Institute of Advanced Industrial Science and Technology (Independent Administrative Institute), International Patent Organism Depositary (IPOD) (Chuo-dairoku, 1-1, Higashi 1-chome, Tsukuba-shi, Ibaraki-ken, Japan) with the Accession Nos. of FERM ABP-10383 [F1196-113-1], FERM ABP-10384 [F1196-114-2], and FERM ABP-10385[F1196-152-2]).

**Table 10**

| Clone No. | H/L | CDR | Sequence (Nucleotide/Amino acid) | Amino acid SEQ ID NO: | Nucleotide SEO ID NO: |
|---|---|---|---|---|---|
| | H | 1 | AGATACTGGATGAGT ArgTyrTrpMETSer | 51 | 52 |
| | | 2 | GAAATTAATCCAGATAGCAGTACGATAAACTATACGCCATCTCTAAAGGAT GluIleAsnProAspSerSerThrIleAsnTyrThrProSerLeuLysAsp | 53 | 54 |
| F1196-113-1 | | 3 | CCGGATAGTTACTATAGGTACTATGCTATGGACTAC ProAspSerTyrTyrArgTyrTyrAlaMETAspTyr | 55 | 56 |
| | L | 1 | AGGGCAAGTCAGGACATTAGCAATTATTTAAAC ArgAlaSerGlnAspIleSerAsnTyrLeuAsn | 57 | 58 |
| | | 2 | TACACATCAAGATTACACTCA TyrThrSerArgLeuHisSer | 59 | 60 |
| | | 3 | CAACAGGGTAATACGCTTCCGTACACG GlnGlnGlyAsnThrLeuProTyrThr | 61 | 62 |
| | H | 1 | ACCTATGGAATGAGC ThrTyrGlyMETSer | 63 | 64 |
| | | 2 | TGGATAAACACCTACTCTGGAGTGCCAACATATGCTGATGACTTCAAGGGA TrpIleAsnThrTyrSerGlyValProThrTyrAlaAspAspPheLysGly | 65 | 66 |
| F1196-114-2 | | 3 | TCCTATTACTACGGTAGTACCTGGGTGGACTAC SerTyrTyrTyrGlySerThrTrpValAspTyr | 67 | 68 |
| | L | 1 | AGATCTAGTCAGAGCATTGTACATAGTAATGGAAACACCTATTTAGAA ArgSerSerGlnSerIleValHisSerAsnGlyAsnThrTyrLeuGlu | 69 | 70 |
| | | 2 | AAAGTTTCCAACCGATTTTCT LysValSerAsnArgPheSer | 71 | 72 |
| | | 3 | TTTCAAGGTTCACATGTTCCGTGGACG PheGlnGlySerHisValProTrpThr | 73 | 74 |
| | H | 1 | GACCATACTATTCAC AspHisThrIleHis | 75 | 76 |
| | | 2 | TATATTCATCCTAGAGATGGTAGTACTAAGTACAATGAGAAGTTCAAGGGC TyrIleHisProArgAspGlySerThrLysTyrAsnGluLysPheLysGly | 77 | 78 |
| F1196-152-2 | | 3 | GGGGGTTTCTACGGCAGTAGTCCCTGGTTTGCTTAC GlyGlyPheTyrGlySerSerProTrpPheAlaTyr | 79 | 80 |
| | L | 1 | AGGTCTAGTAAGAGTCTCCTGCATAGTAATGGCAACACTTACTTGTAT ArgSerSerLysSerLeuLeuHisSerAsnGlyAsnThrTyrLeuTyr | 81 | 82 |
| | | 2 | CGGATGTCCAACCTTGCCTCA ArgMETSerAsnLeuAlaSer | 83 | 84 |
| | | 3 | ATGCAACATCTAGAATATCCGTACACG METGlnHisLeuGluTyrProTyrThr | 85 | 86 |

### (Example 27) Production of mouse - human chimeric antibody by genetic engineering

An antibody (chimeric antibody) in which the V region having the antigen binding activity is derived from the hybridoma antibody, that is, mouse antibody and the C region is from human is produced by the procedure as described below.

### (1) Construction of plasmid expressing mouse - human chimeric antibody

Plasmid expressing the anti-meltrin α antibody mouse - human chimeric antibody is constructed as described below.
First, gene fragment C coding for the heavy chain variable region is prepared, and in the meanwhile, gene fragment D coding for the heavy chain constant region is also prepared. These fragments are ligated in the downstream of EF promoter of the expression vector pEF2cew so that these fragments form fragment C + fragment D to thereby construct the plasmid expressing the heavy chain. Next, DNA fragment E coding for the light chain variable region is prepared, and in the meanwhile, DNA fragment F coding for the human light chain constant region is also prepared. These fragments are ligated in the downstream of EF promoter of the expression vector pEF2cew so that these fragments form fragment E + fragment F to thereby construct the plasmid expressing the light chain.

### (2) Expression of recombinant mouse - human chimeric antibody and confirmation of binding activity for meltrin α

The plasmid expressing the mouse - human chimeric antibody constructed is expressed and purified by the procedure described in Examples 5 and 7. More specifically, COS-1 cells are co-transfected with the plasmid expressing the heavy chain and the plasmid expressing the light chain of the desired mouse - human chimeric antibody, and after incubating at 37°C for 3 days, the culture supernatant is purified on a protein A column. The thus obtained mouse - human chimeric antibody is confirmed for its purity by SDS-PAGE, and then confirmed for its binding ability to the human meltrin α. First, the human meltrin α prepared in Example 5 is diluted with D-PBS to 2 µg/mL, and the dilution is added to an immunoplate (Maxisorp, NUNC) at 50 µL/well. Next, after incubating at 37°C for 1 hour and washing 5 times with ion-exchanged water, the well is blocked by adding 100 µL of D-PBS containing 2% StabilGuard (SurModics) to each well. Next, the purified mouse - human chimeric antibody is added to each well, and after reacting at 37°C for 1 hour, the well is washed 3 times with physiological saline containing 0.05% Tween 20. Peroxidase-labeled anti-human light chain K antibody (DAKO) is diluted 1000 folds with D-PBS containing 10% rabbit serum, and 50 µL of the dilution is added to each well. After reacting at 37°C for 1 hour and washing 5 times as described above, TMB solution (BioFix) is added to each well. After reacting at room temperature for 10 minutes, the reaction is ceased by adding 0.5M sulfuric acid solution, and absorbance at 450 nm is measured with a plate spectrophotometer (Multiscan JX, Dainippon Pharmaceutical Co. Ltd.). As a consequence, the mouse - human chimeric antibody prepared is confirmed to be capable of binding to the human meltrin α.

### (Example 28) Preparation of humanized antibody

### A. Preparation of humanized antibody (Method 1)

### (1) Computer modeling of variable region of humanized F antibody

In order to maintain the high affinity in the humanized antibody, framework residues are selected according to the common method of Queen et al. (Proc.Natl.Acad.Sci.USA 86: 10029, 1989). The human sequence is selected so that the selected sequence has a high framework homology with the anti-human meltrin α mouse monoclonal antibody based on the κ light chain and heavy chain sequence database of Kabat et al. (Sequences of proteins of immunological interest, 5th ed., U.S. Department of Health and Human Services, 1991). Using the computer analysis, necessary alteration of the amino acids in the optimal framework is conducted. More specifically, molecular model of the antibody variable region is constructed by using a protein modeling tool such as computer program ENCAD (Levitt, J. Mol. Biol. 168, 595, 1983), Homology (Accelrys), or FAMS (SGI). The CDR sequence of the antibody is transplanted in the FR of the human Eu antibody molecular model (Stephens et al., Immunology 85 (4), 668-674 (1995)) obtained from the antibody database. By optimization and simulation analysis such as molecular optimization or molecular dynamics calculation, the amino acid from the mouse antibody is substituted at the position where contact between the CDR and the FR is expected to be improved by the amino acid substitution in the FR region where the CDR and the FR are in significant contact in contrast to the original human antibody model on the computer model. The amino acid residue in the FR which is only rarely found at the corresponding position in the database of the human antibody is substituted with the human consensus amino acid of the corresponding position. Since the success or the failure of the amino acid substitution is confirmed by the resulting activity, several types of antibodies with different amino acid substitution are prepared.

### (2) Construction of the humanized antibody

A gene coding for the amino acid sequence including signal peptide, splice donating signal, and restriction sites is constructed based on the sequence selected in Example 26. The constructed gene is prepared so that several kinds of synthetic nucleotides (each having a length of about 80 nucleotides) overlap each other. More specifically, oligo sequences are paired and annealed, and then extended by using a Klenow fragment of the DNA polymerase to produce a double stranded fragment. This fragment is denatured and the resulting single stranded sequences are annealed as described above, and extended by Klenow fragment of the DNA polymerase to produce a double stranded fragment coding for the full length gene. The resulting fragment is amplified by PCR, and after purification, the fragment is cleaved with a restriction enzyme and purified. The purified fragment is ligated to the gene fragment containing from CH1 exon to CH3 exon in the human IgGs constant region gene, and incorporated in the downstream of the EF promoter of the expression vector pEF2cew to construct the plasmid expressing the humanized heavy chain. When the number of amino acids substituted is small, the amino acid mutation may be introduced in the expression plasmid by site-directed mutagenesis. The sequence of the light chain variable region can also be constructed by the procedure as described above.
In order to prepare the transformant producing the antibody, the plasmids of the heavy chain and the light chain are respectively cleaved and linearized with a restriction enzyme, and then transfected into mouse myeloma cell Sp2-O-ag14 (ATCC CRL1581) using Gene Pulser (BIORAD). For example, about 20 µg of the linearized DNA fragment is electropolated into 1 x 10⁷ cells at 360 V and a capacitance of 25 µFD. Next, the cells are inoculated in a 96 well plate, and after incubating for 2 days, D-MEM (Sigma) containing 10% FCS, 1 x HT (Invitrogen), 0.25 mg/ml Xanthine, and 1 µg/ml Mycophenolic acid is added for selection of the cells having the plasmid fragment incorporated therein. After continuing the incubation for another 2 weeks, the antibody in the culture supernatant is analyzed to select the target cell strain producing the humanized anti-meltrin α antibody. More specifically, the antibody in the culture supernatant is allowed to react with the immobilized meltrin α, and the thus bound antibody is detected with peroxidase-labeled anti-human IgGs antibody. The detected antibody-producing cell line which exhibited the binding is incubated in the culture medium containing 10% FCS to confluency, and the medium is then changed to a serum free medium (Hybridoma SFM, Invitrogen). The culture supernatant is collected, and the antibody in the culture supernatant is allowed to bind to protein A (Prosep-A, Millipore), and eluted with 0.1M glycine HCl (pH 3.0). The purified antibody is dialyzed against PBS-(Sigma), and antibody concentration is calculated from the absorbance at 280 nm (1 mg/mL of human antibody shows the absorbance of about 1.3).

### (3) Evaluation of humanized antibody

The binding activity between the humanized antibody and the meltrin α is confirmed according to the method of Example 8. The binding ability of the humanized antibody and the original mouse antibody is measured by Biacore system (BIACORE). More specifically, purified human meltrin α is immobilized on a CM5 chip (BIACORE) according to the manual of Biacore 3000, and then, serial dilution of the antibody is prepared with HBS-EP buffer (BIACORE) for injection of the sample. The thus obtained data is analyzed by the analysis program (BIA Evaluation, BIACORE) of BIACORE to calculate affinity (Kd), and the binding ability of the humanized antibody and that of the original mouse antibody are compared.

### B. Preparation of the humanized antibody (Method 2)

### (1) Preparation of the humanized antibody gene

In order to maintain the transplanted CDR sequence as a suitable active domain structure in the humanized antibody, concurrent transplantation of a sequence in the original FR region is also effective. It is possible to deduce which amino acid is involved in the maintenance of the CDR domain structure from the characteristics (such as hydrophobicity, hydrophilicity, acidity, basicity, and molecular size) of the amino acids in the FR, as well as by the computer aided modeling using a software QUANTA/CHARMm or Modeler (Molecular Simulations Ins.) which works on Silicon Graphics. The human antibody sequences registered in Brookhaven Protein Data Bank (PDB) are searched for the three-dimensional structure of the antibody having a high homology with the VH and the VL region of the anti-meltrin α antibody, and the three-dimensional structure of the anti-meltrin α antibody is deduced from such structure. On the deduced three-dimensional structure, a group of amino acids (first group) in the FR region which is bonded to the CDR of the heavy chain and the light chain by hydrogen bond is selected, and a group of amino acids (second group) in the FR region which is bonded to such group by the hydrogen bond is further selected. In the similar manner, a group of amino acids (first group) in the FR region which is deduced to be bonded to the CDR by energy bonding such as electrostatic interaction or van der Waals force is selected, and a group of amino acids (second group) in the FR region which is deduced to be bonded to such group is further selected. The thus selected groups of amino acids in the FR region are transplanted in the human antibody sequence together with the CDR amino acids. However, when a sequence that would not be found in the amino acids in the variable region of the human antibody sequence obtained, for example, by the classification of Kabat et al. (Sequences of proteins of immunological interest, 5th ed., U.S. Department of Health and Human Services, 1991) or NCBI (National Center for Biotechnology Information) is generated, such amino acid is not transplanted. Based on the thus obtained information, the sequence which is to be transplanted in the VH and the VL of the human antibody sequence is determined and the gene used in producing the humanized antibody is constructed.
The thus constructed gene is amplified by combining a kit from Amersham (Oligonucleotide-directed in vitro mutagenesis system version 2) with PCR; by the amplification using a combination of several synthesized long nucleotides; or by amplifying the gene using the VH or the VL gene of the chimeric antibody for the template with several primers and further obtaining the full length gene fragment by using the amplified gene fragments for the template. The amplified gene fragments are ligated to the gene fragment of the constant region, and incorporated in the downstream of the EF promoter of the expression vector pEF2cew to thereby construct the plasmid expressing the humanized antibody. The thus prepared plasmid is transfected into the cell by the method described in Example 28, A(2) to produce the transformant, and the purified antibody is also prepared in a similar method. In addition, the antibody is evaluated by repeating the procedure of Example 28, A(3).

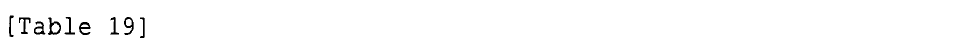

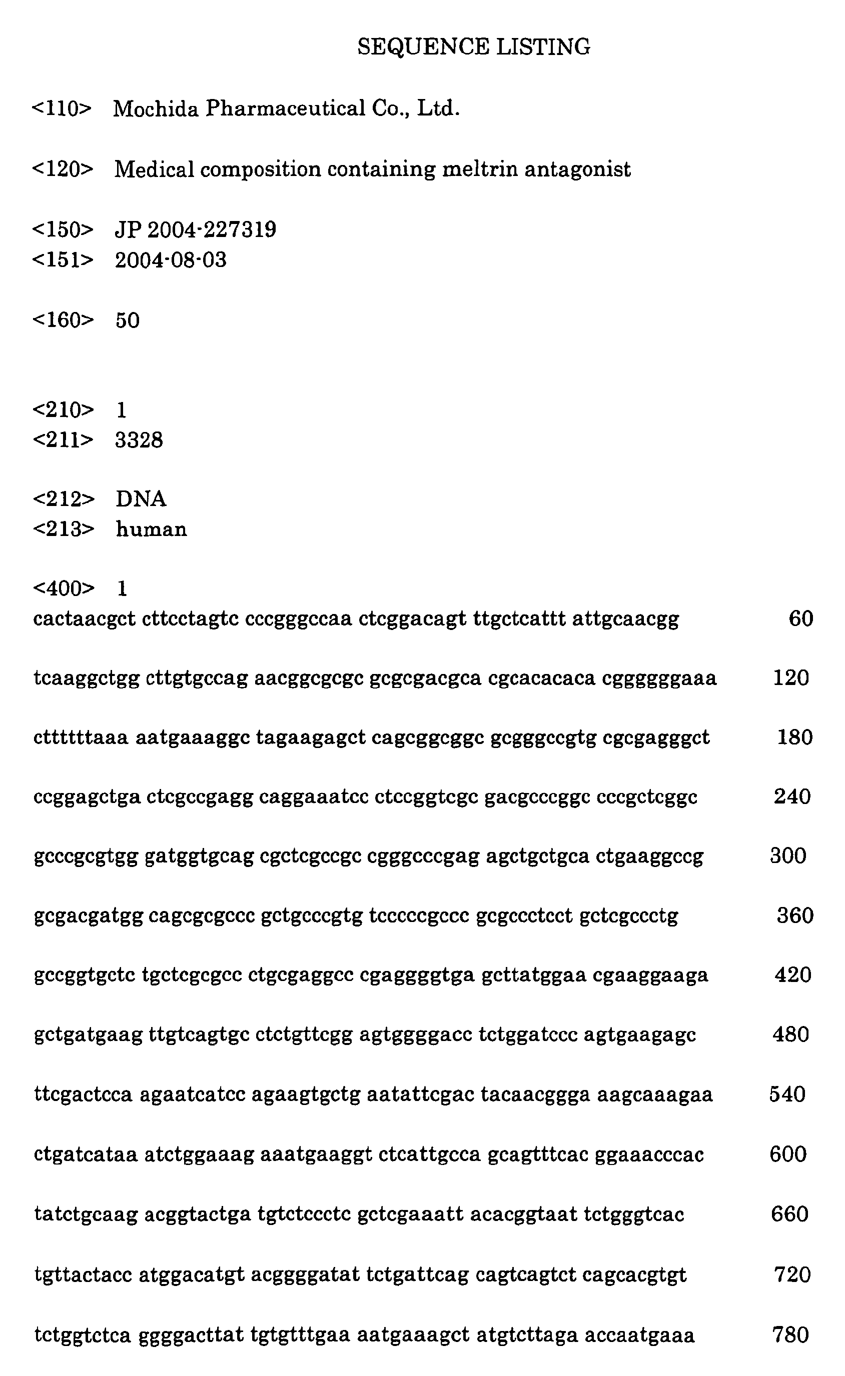

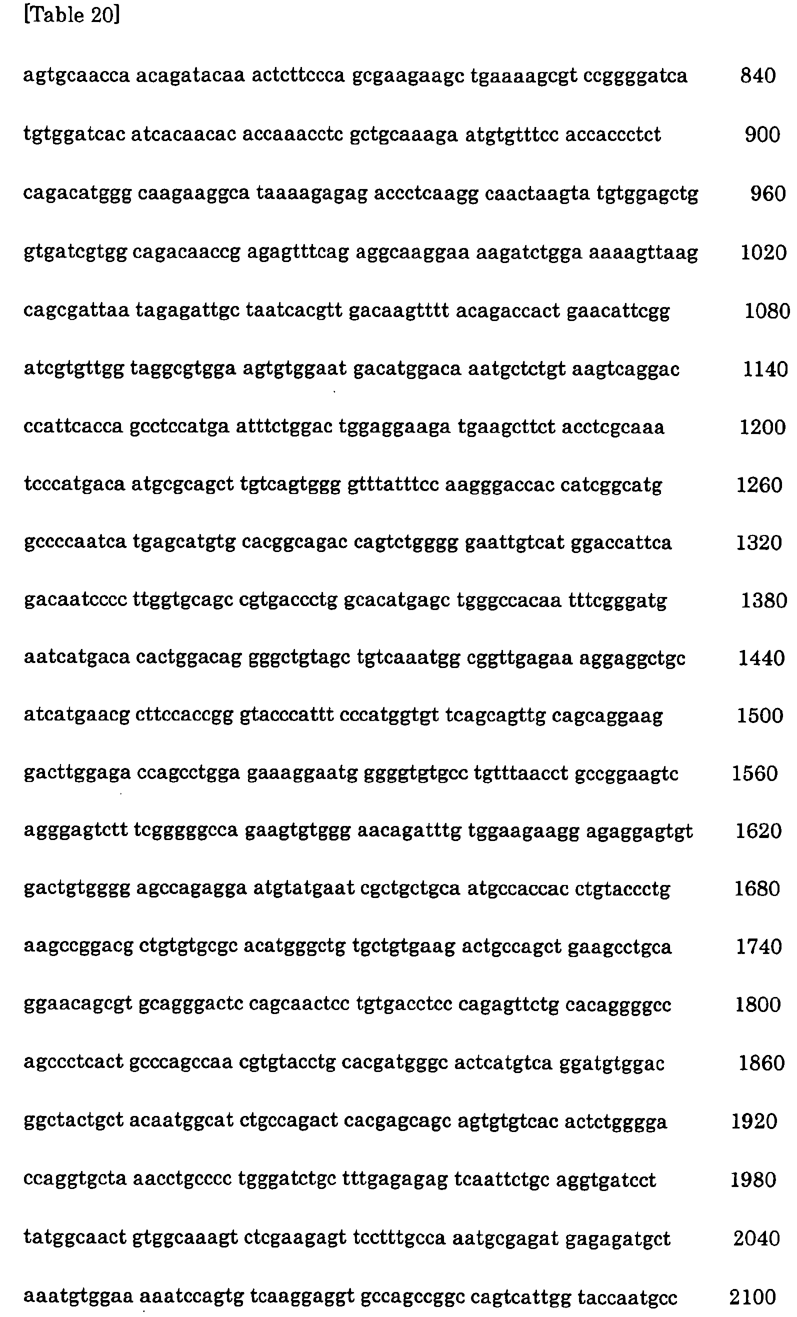

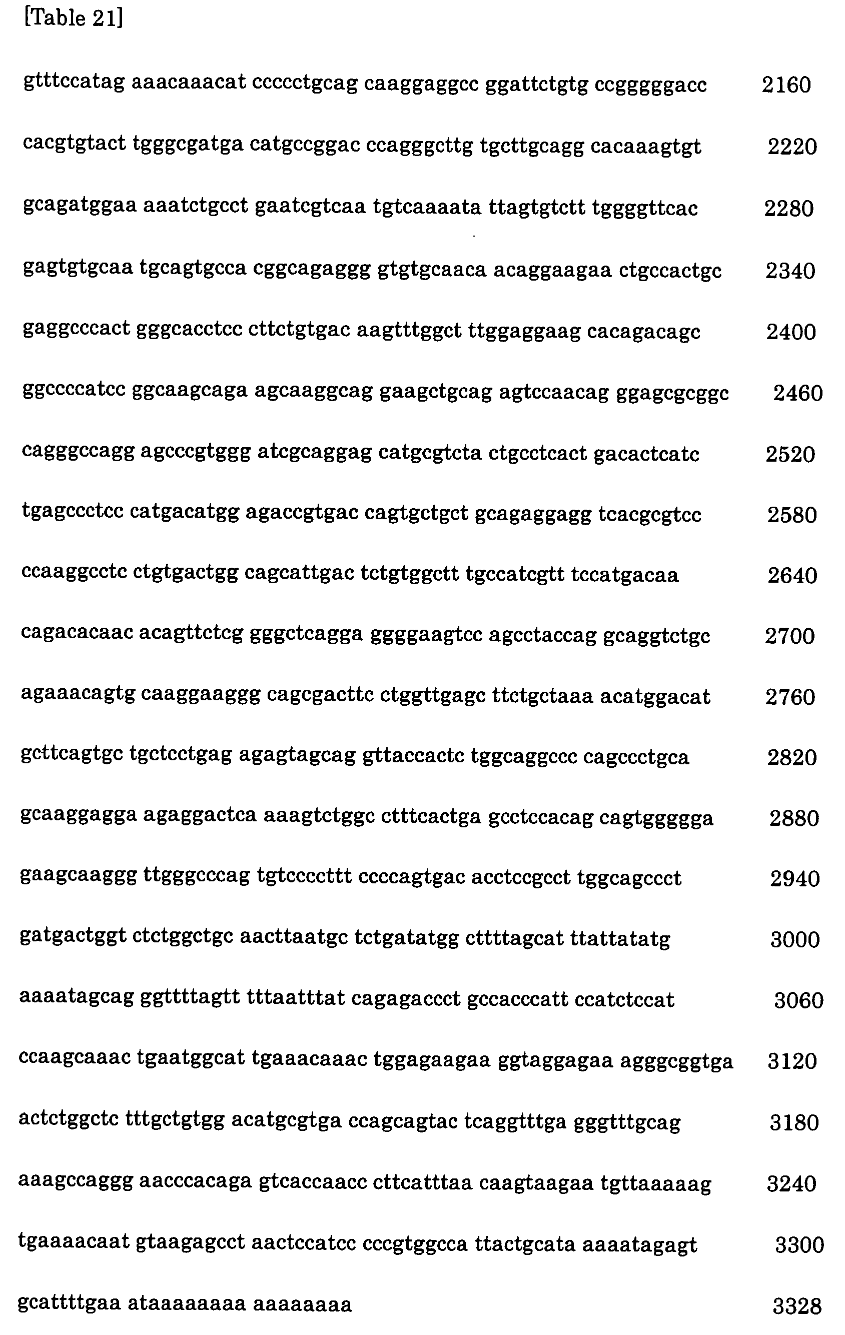

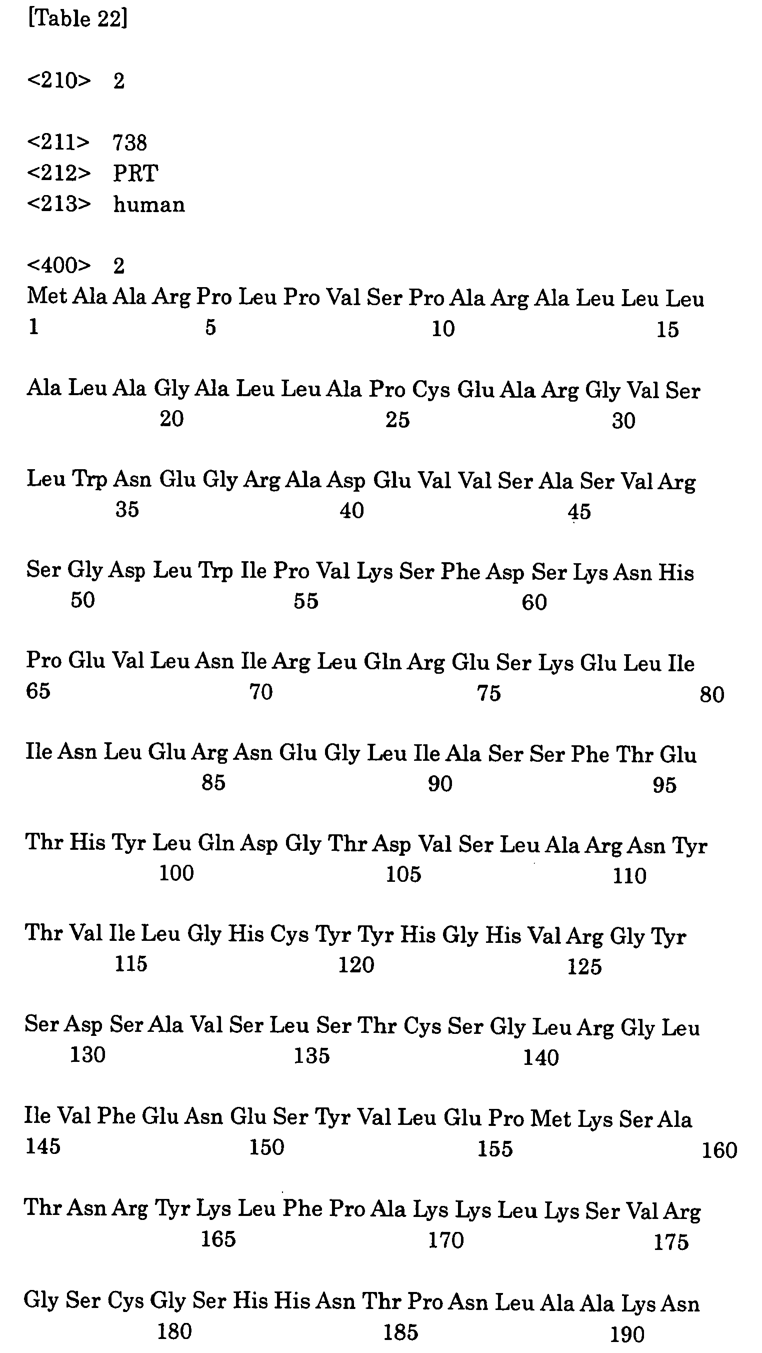

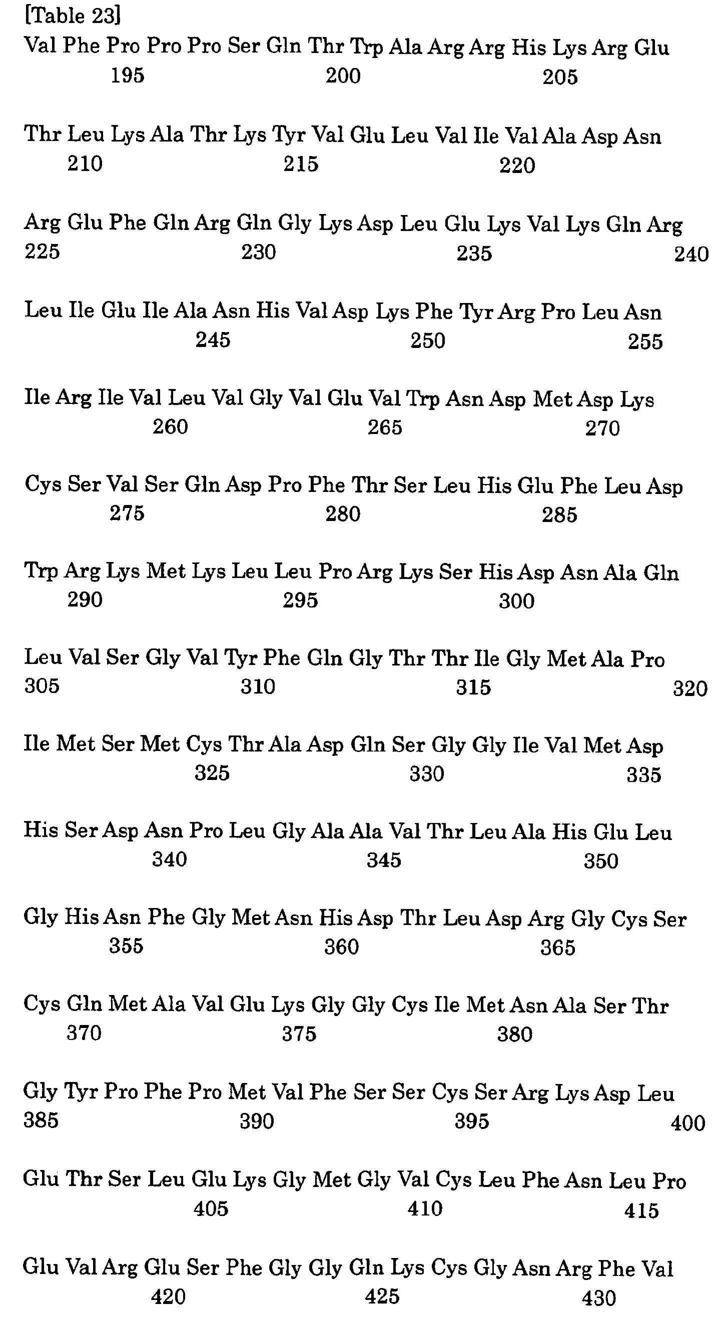

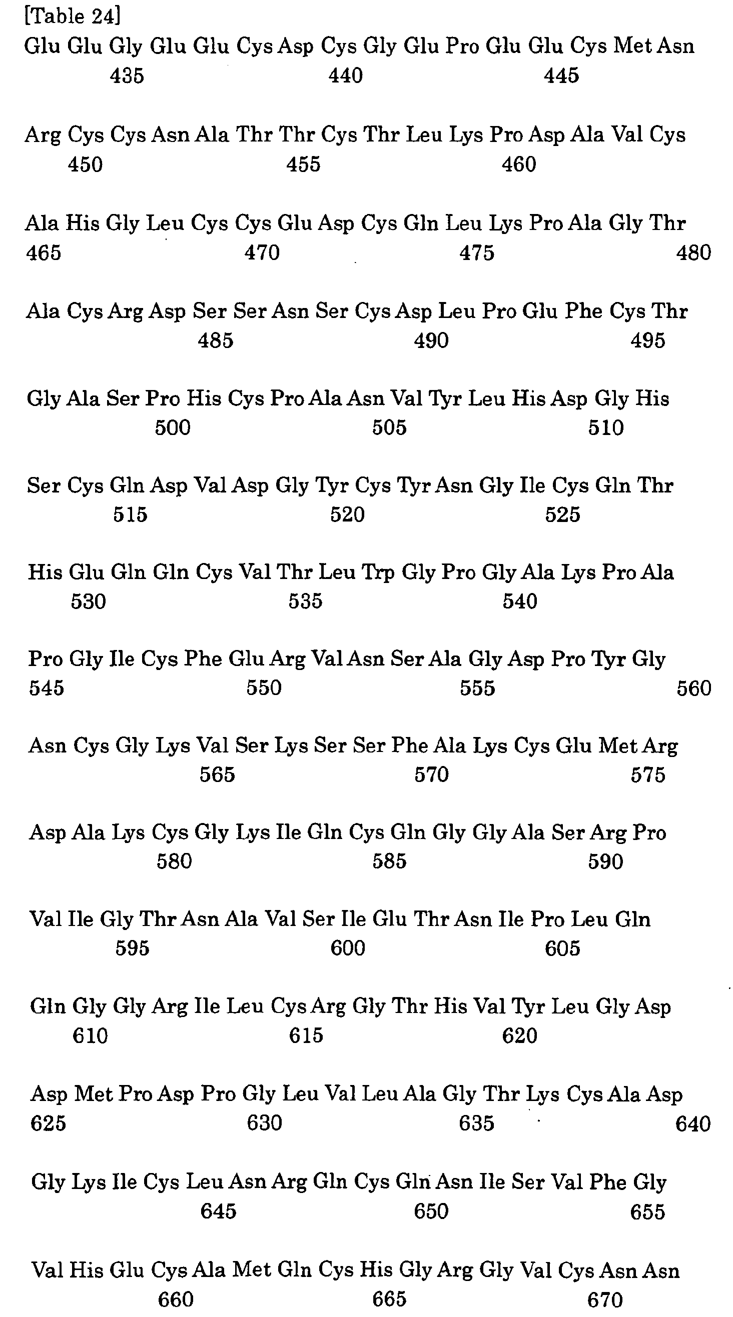

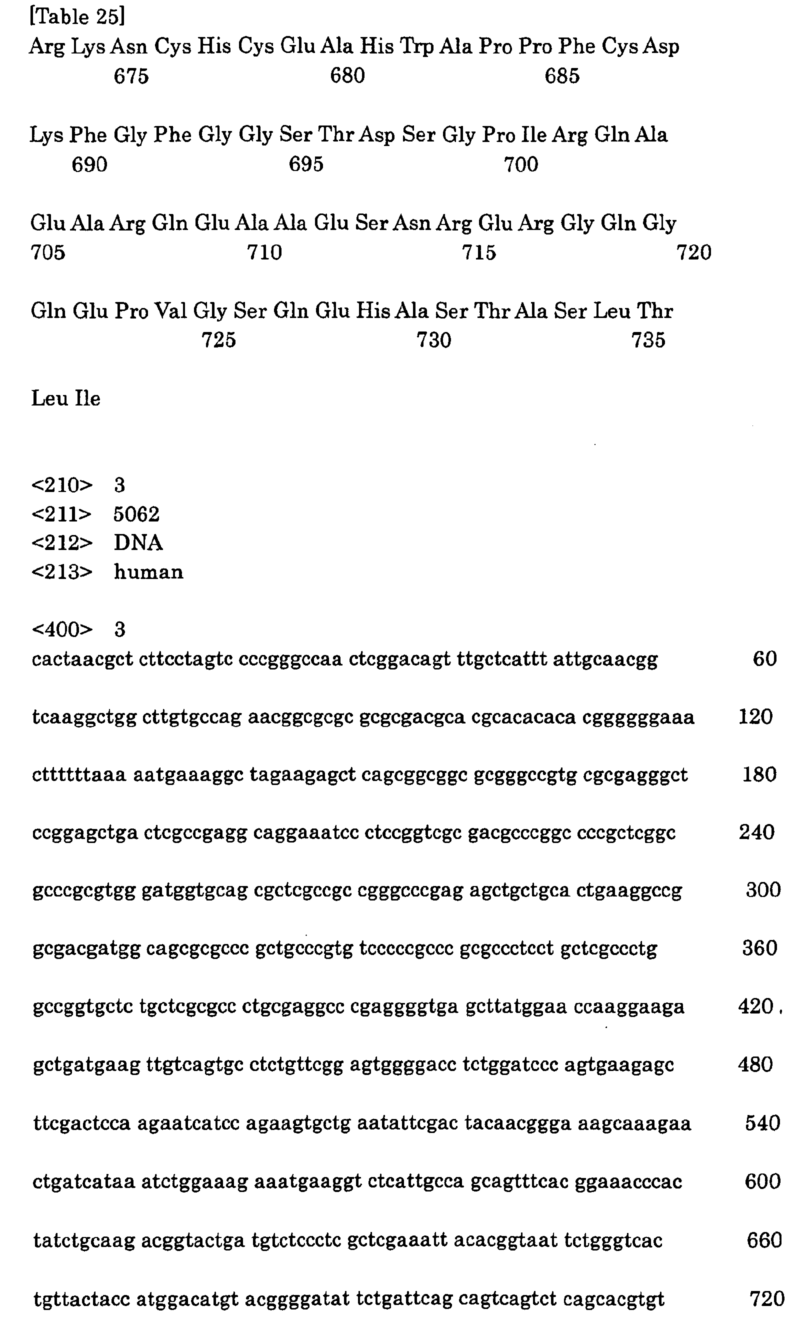

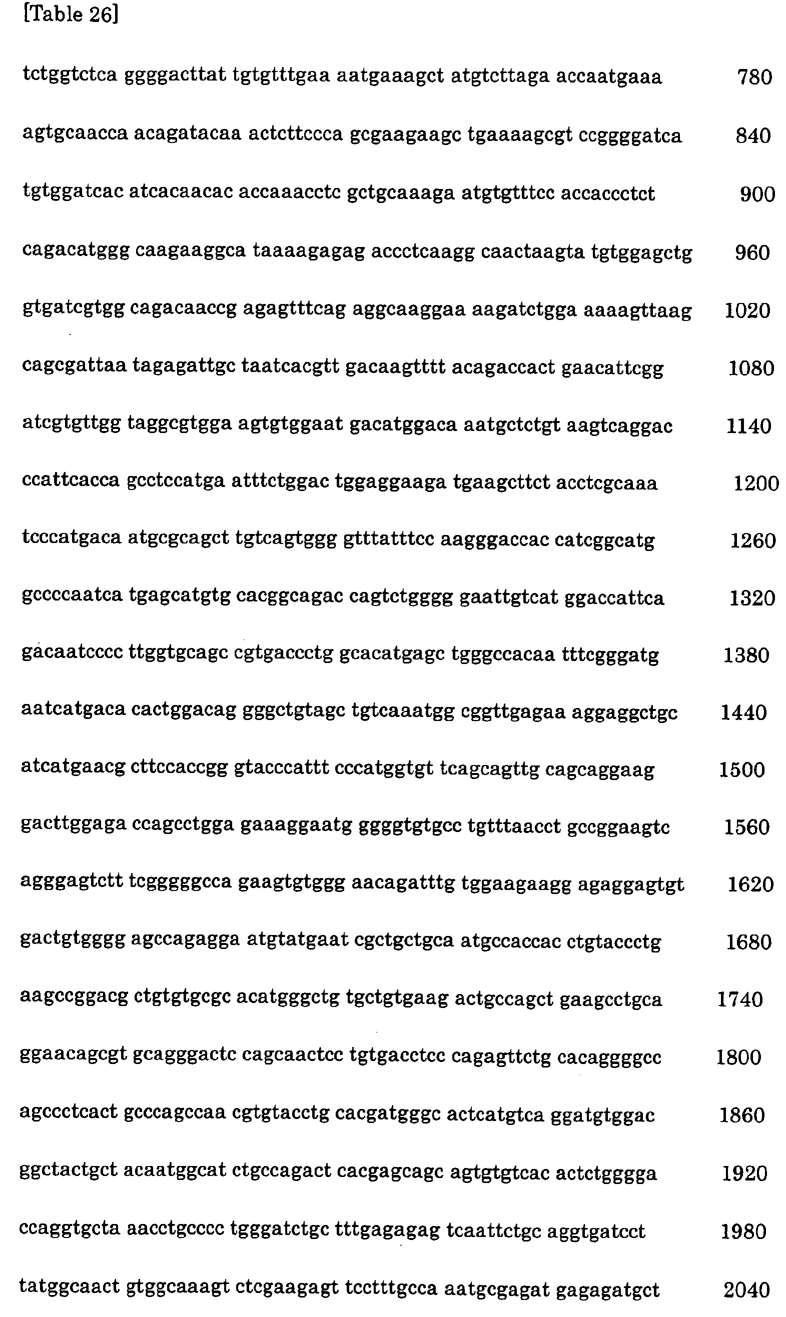

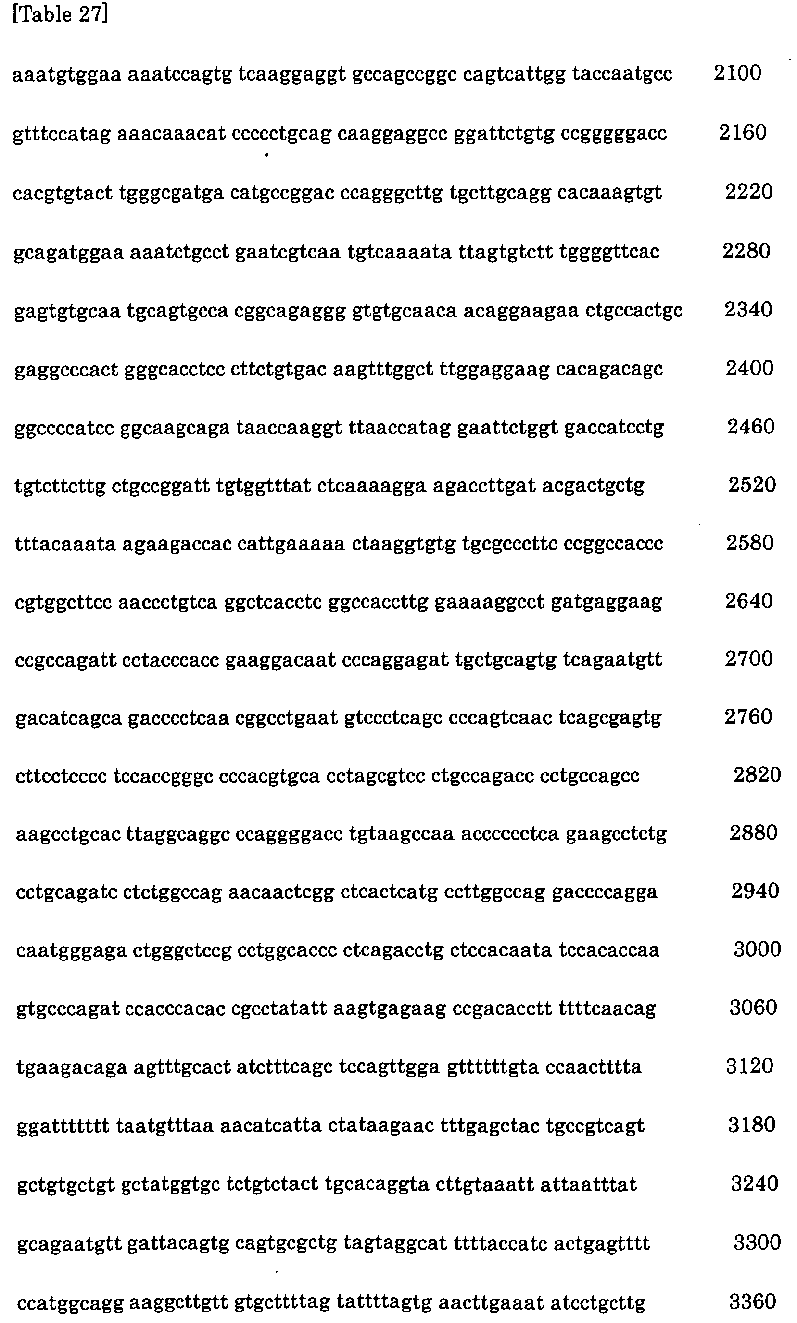

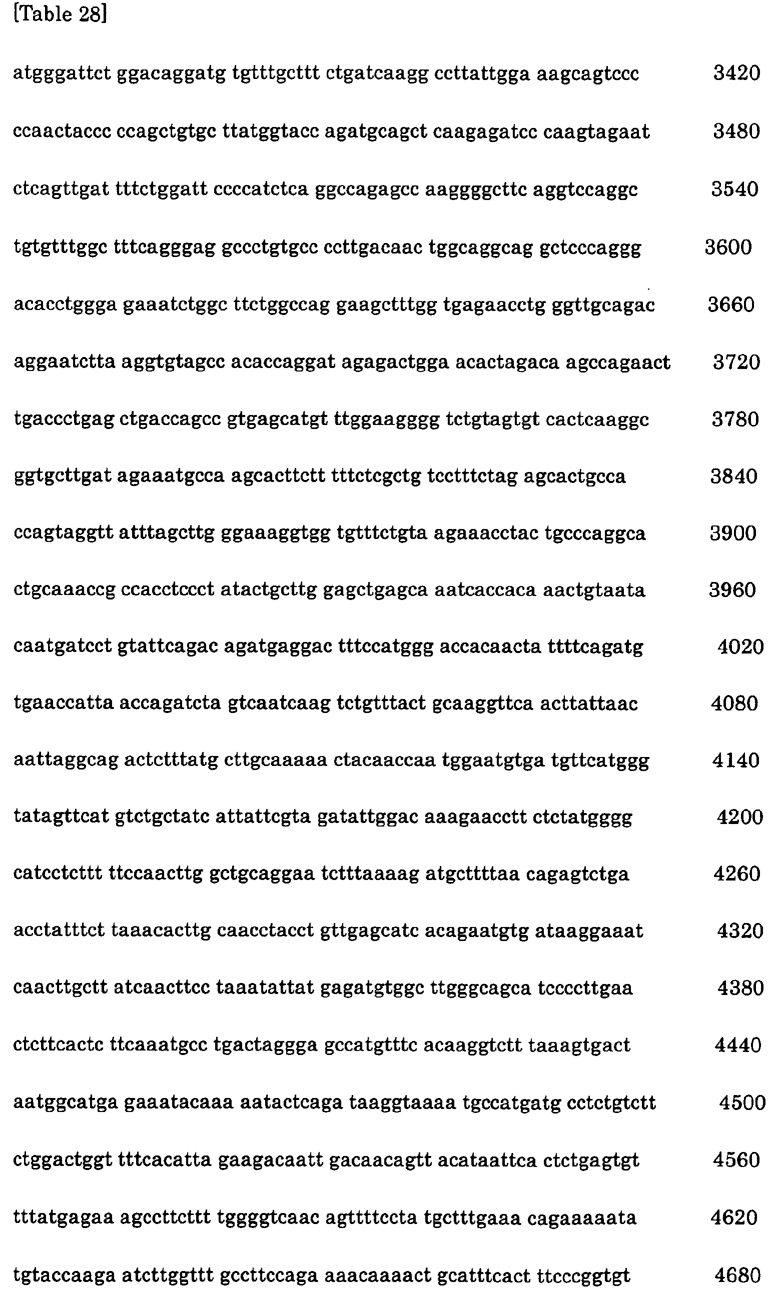

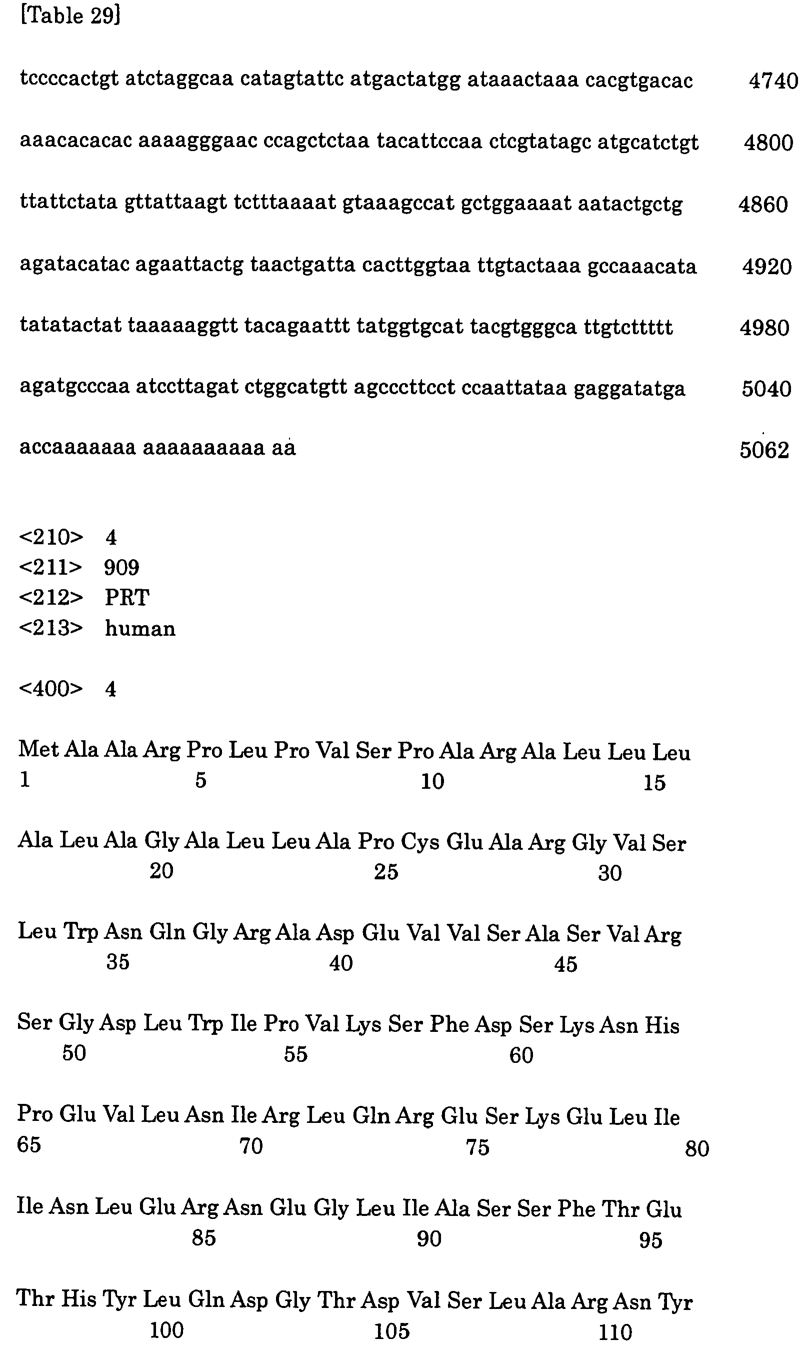

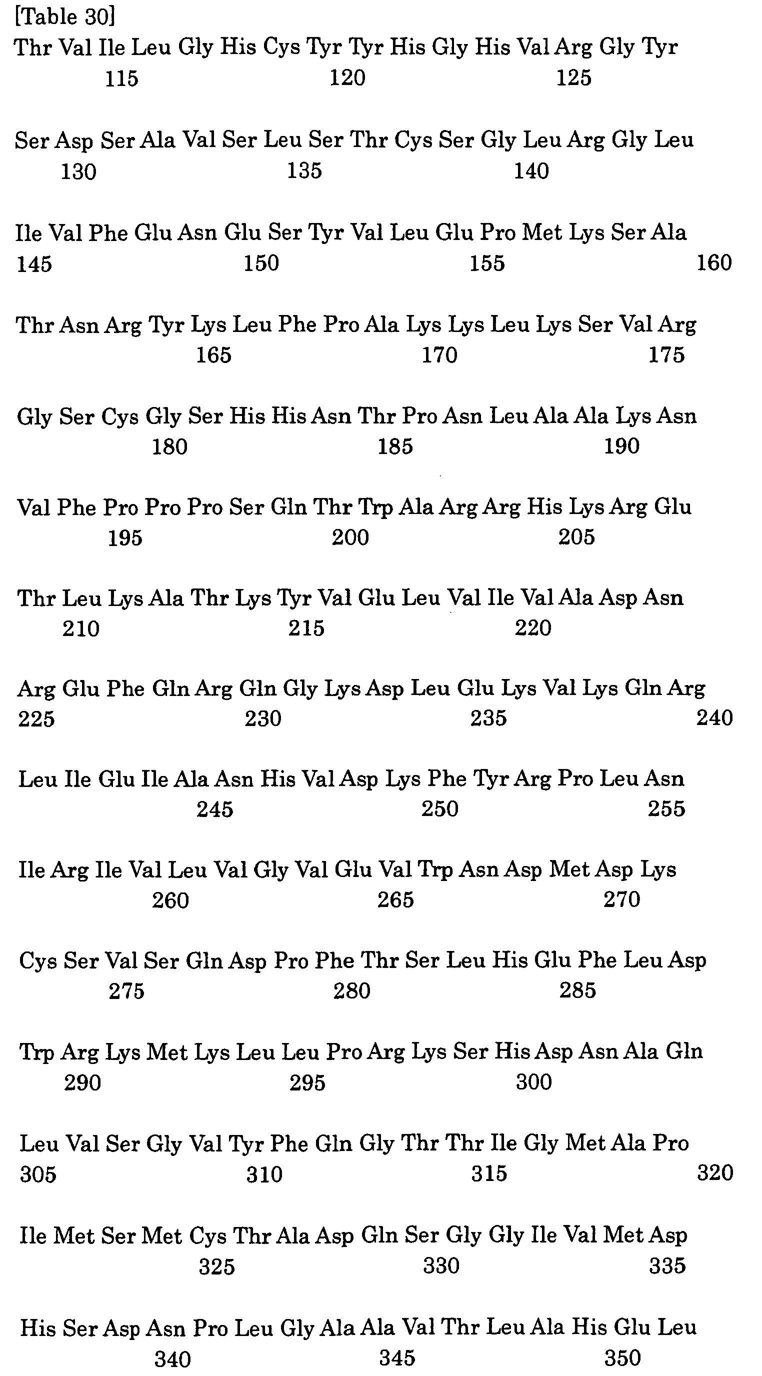

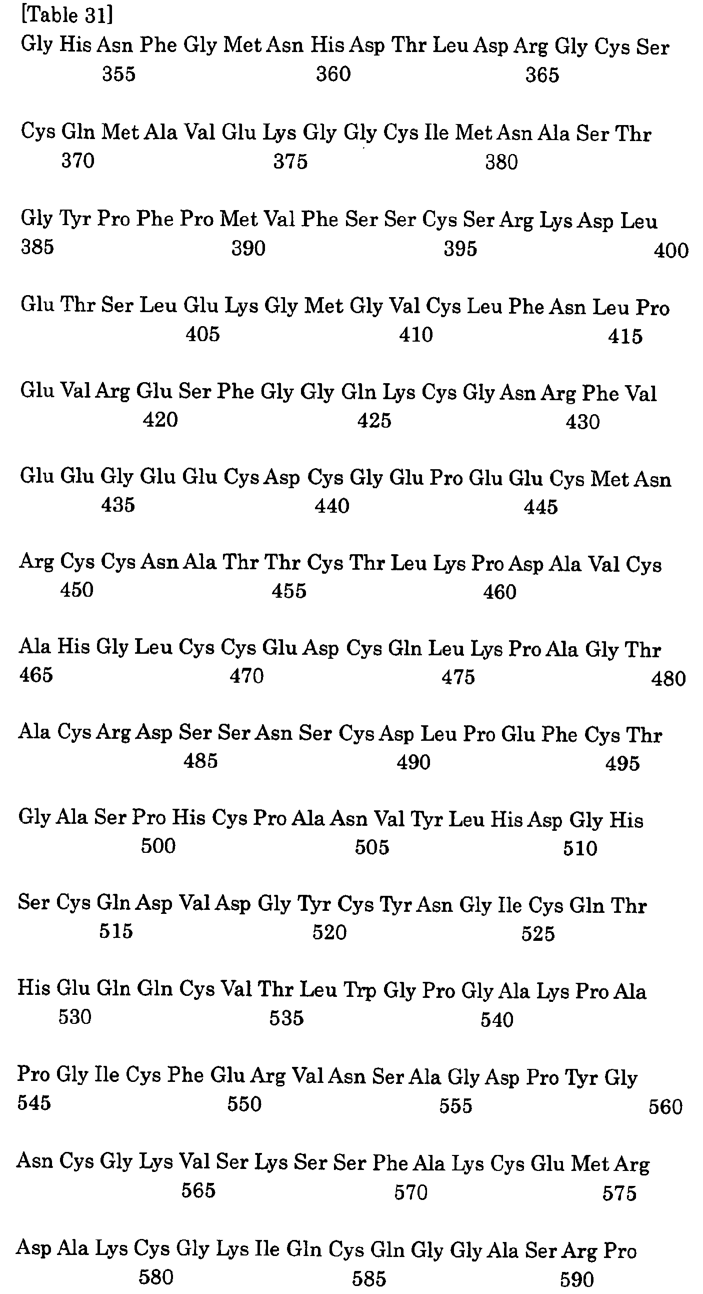

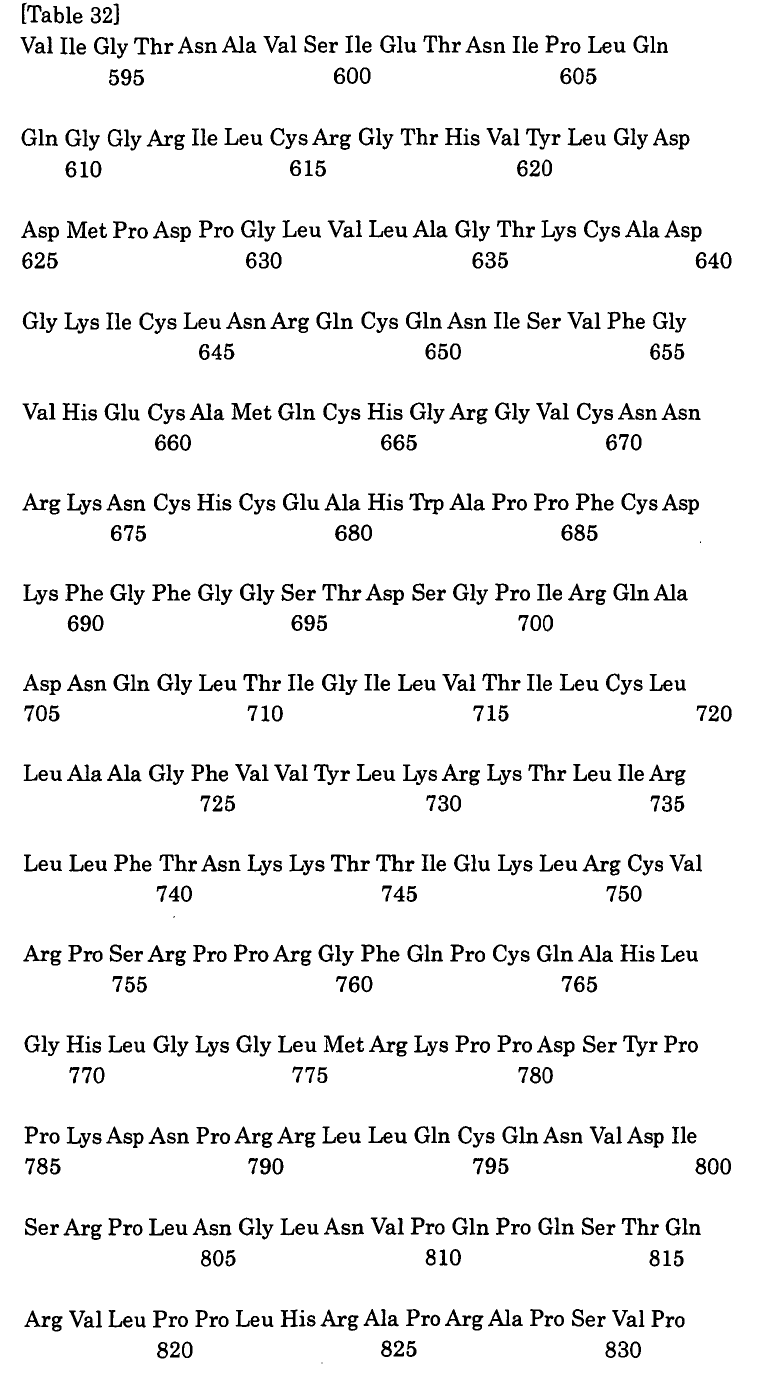

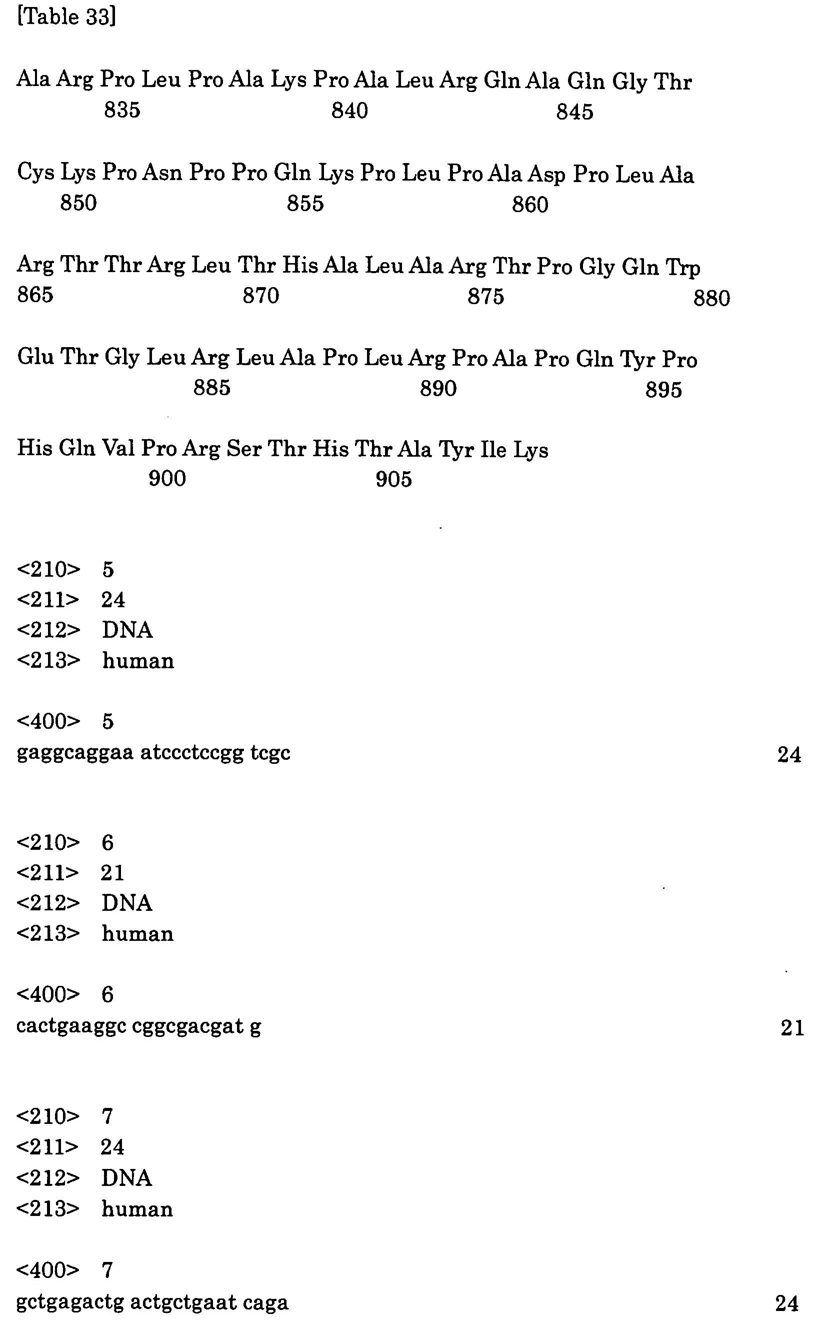

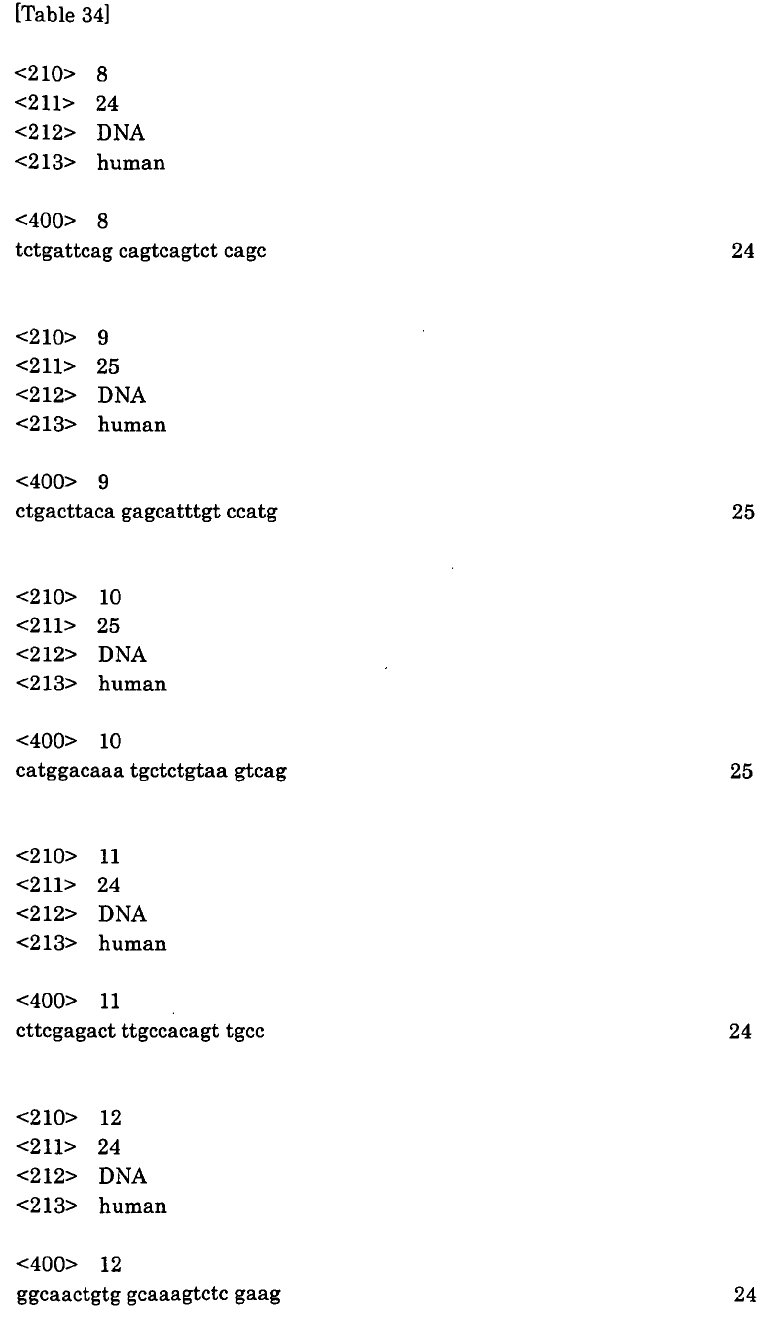

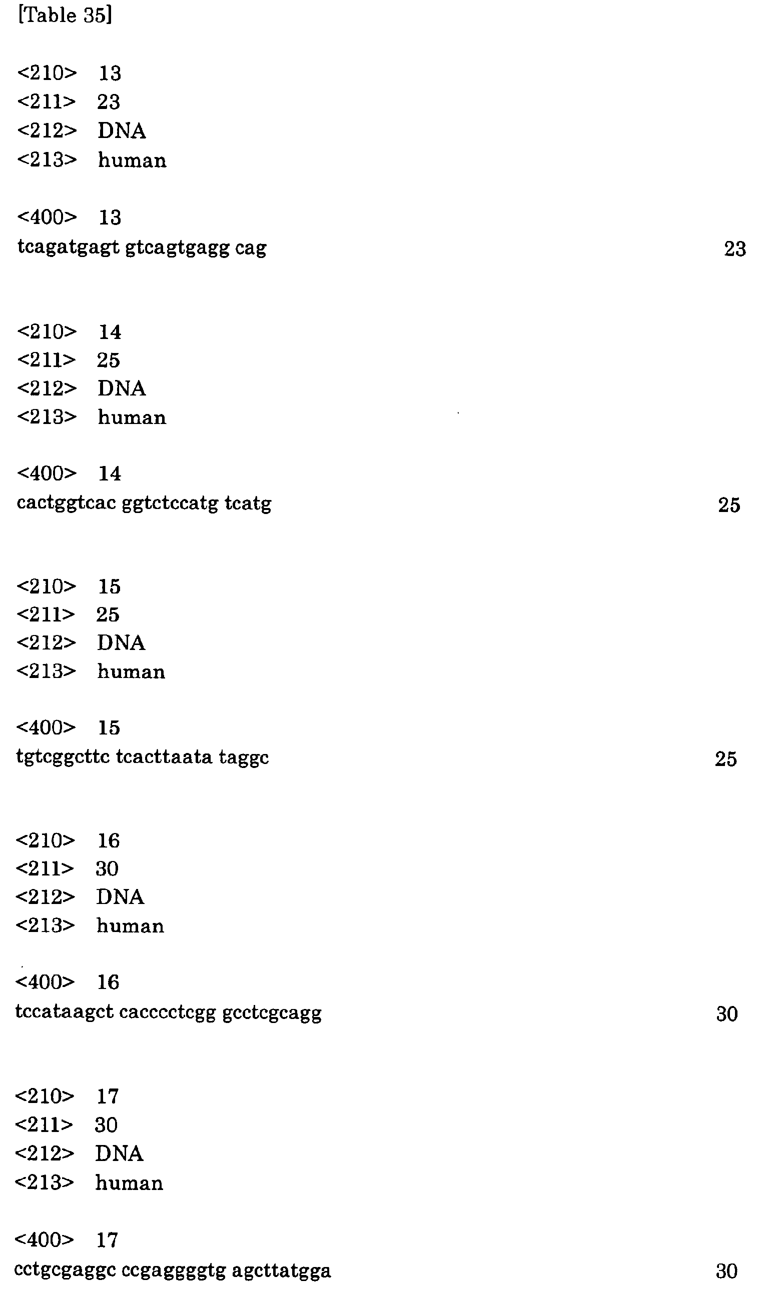

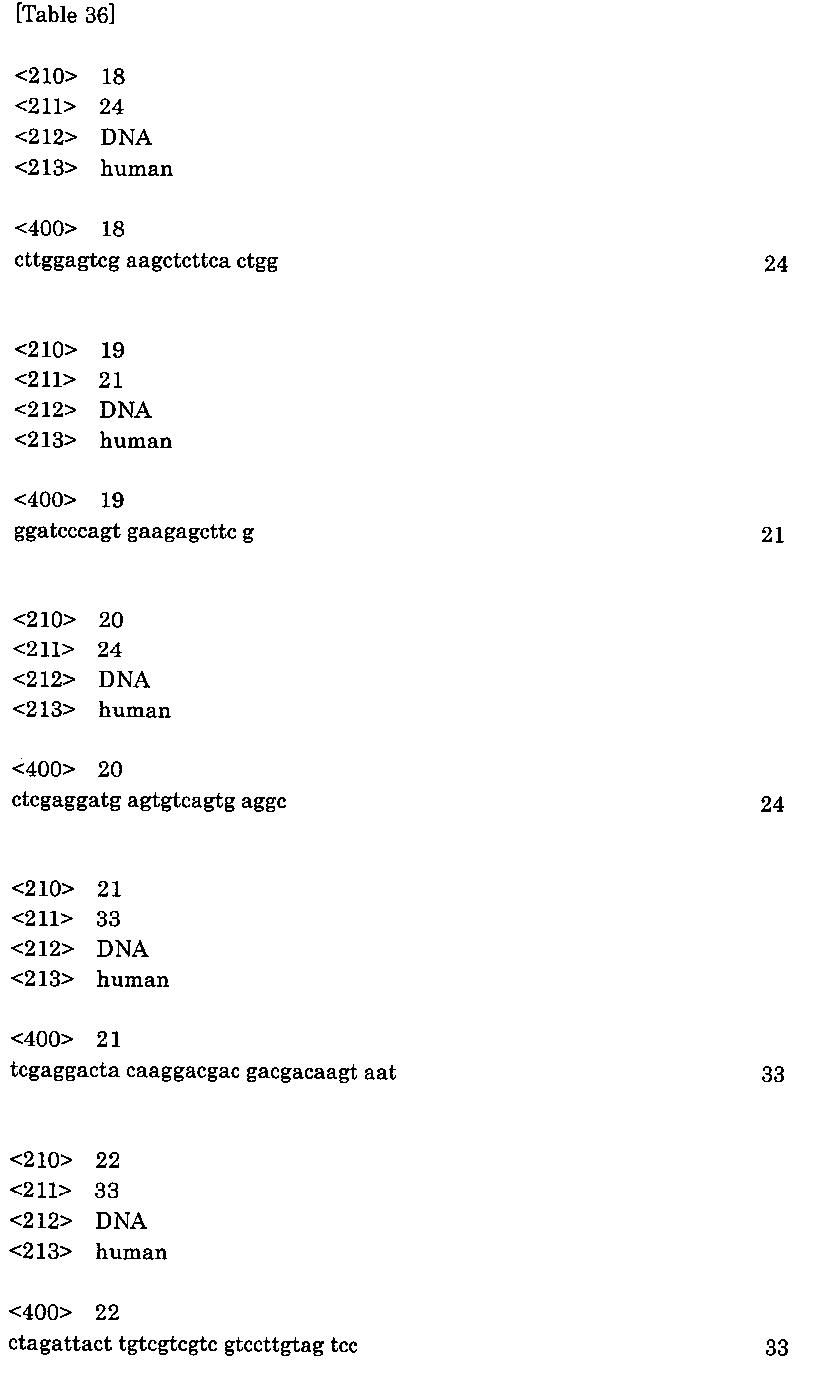

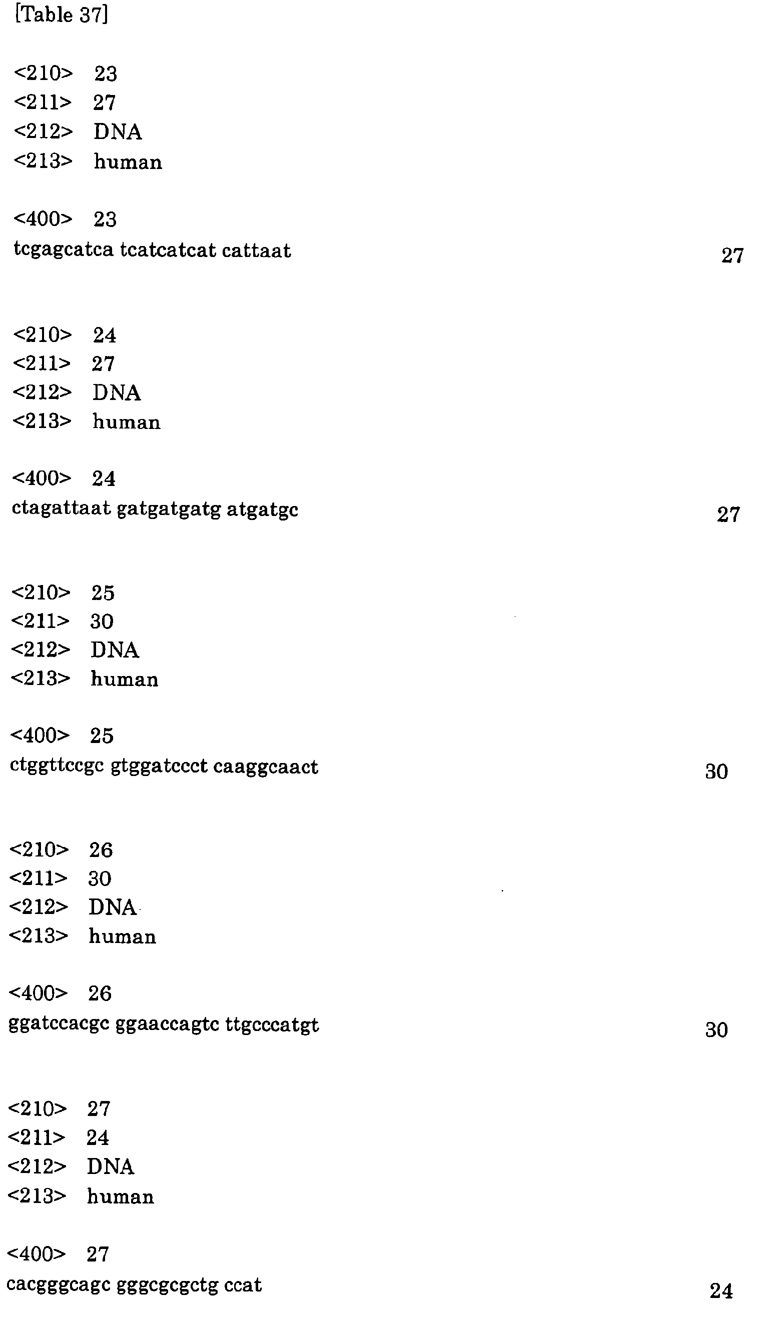

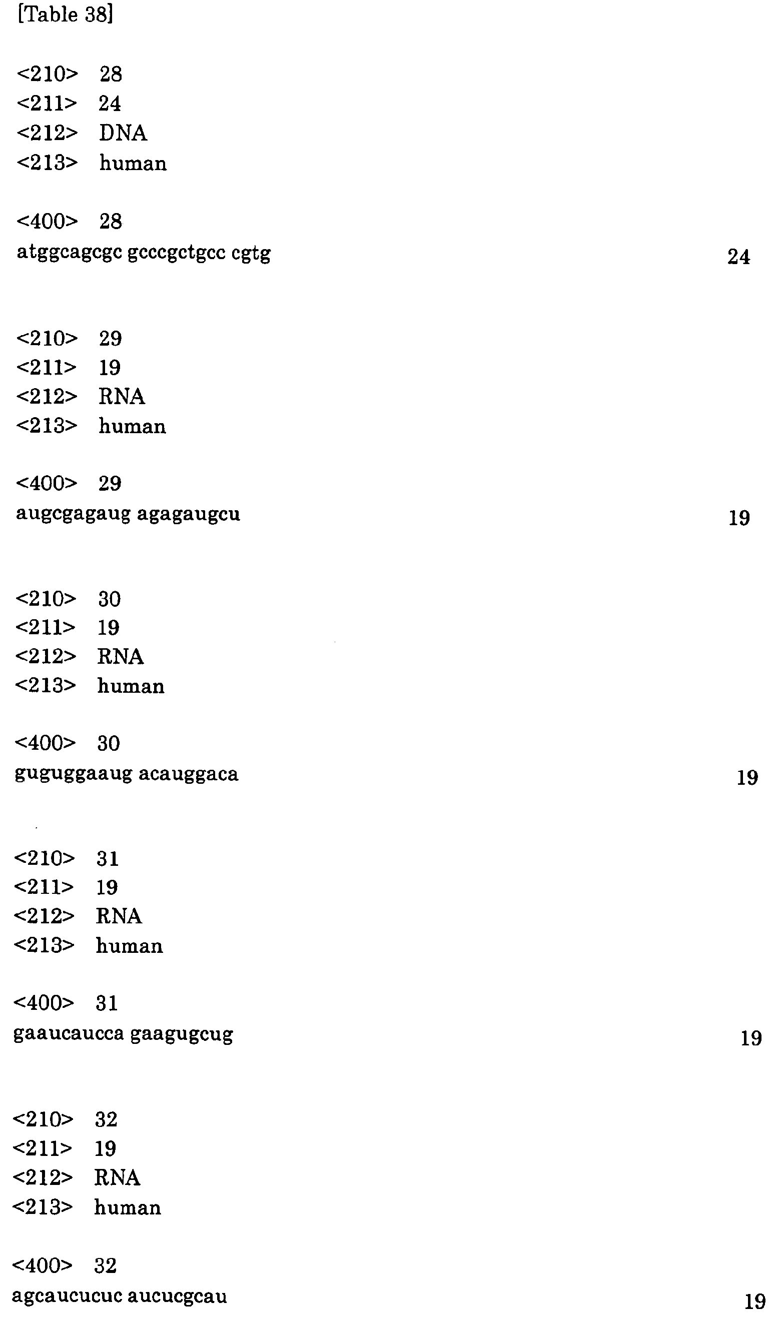

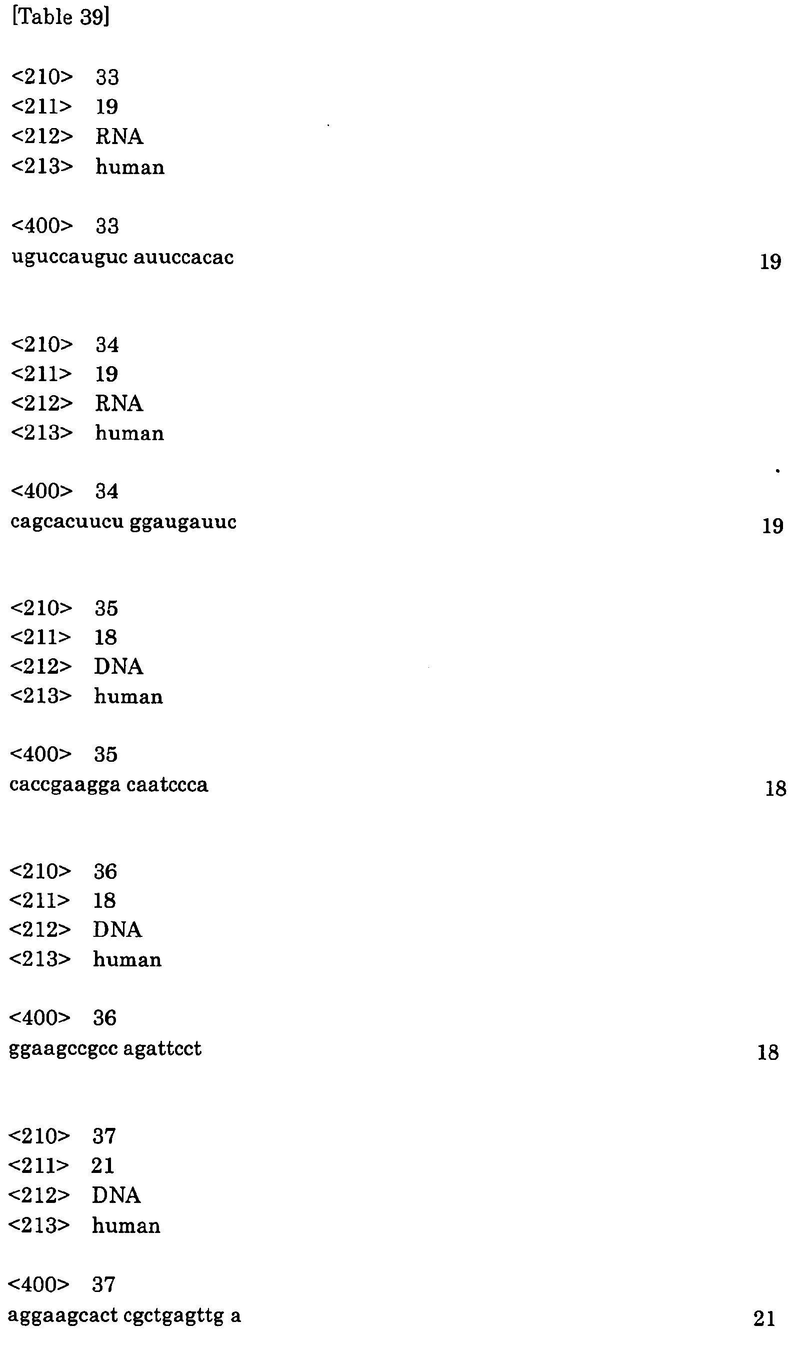

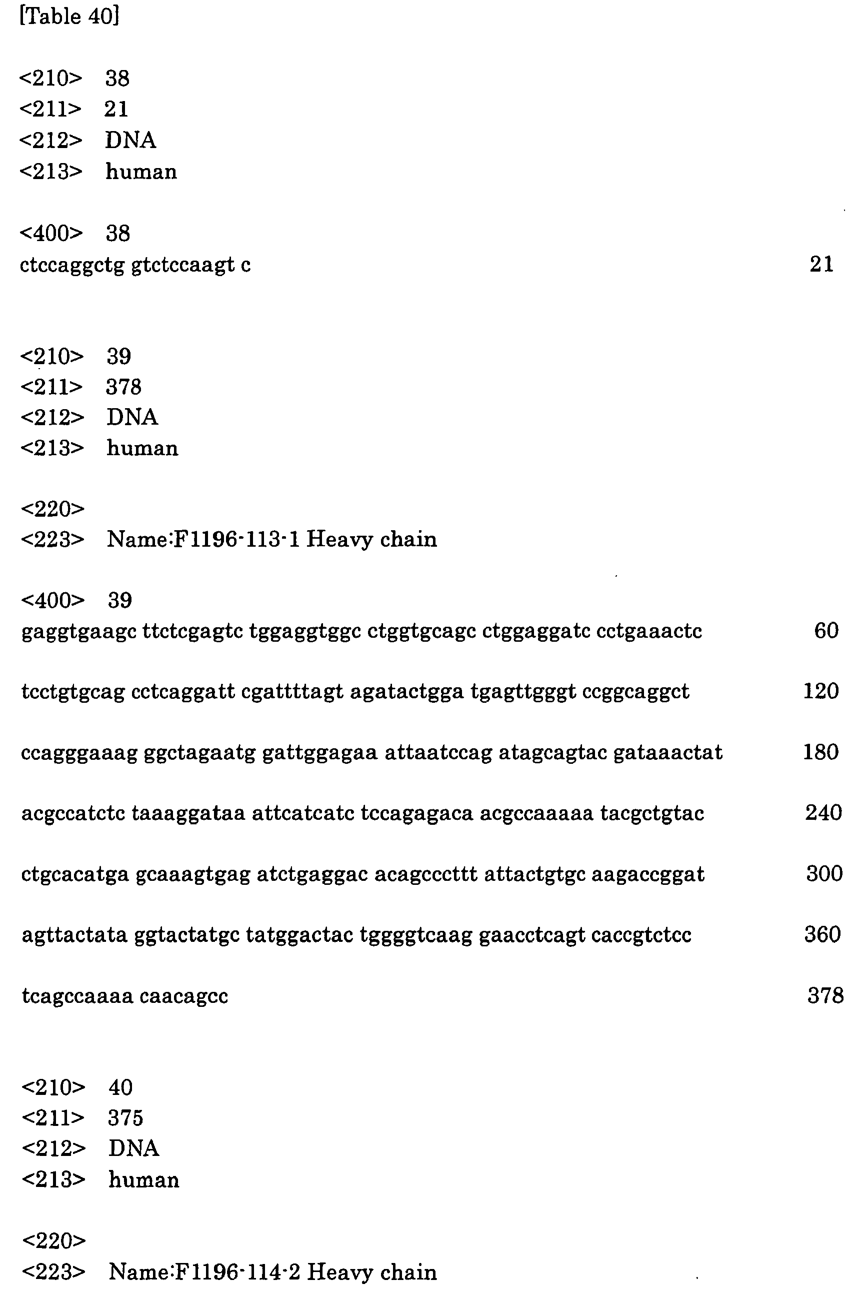

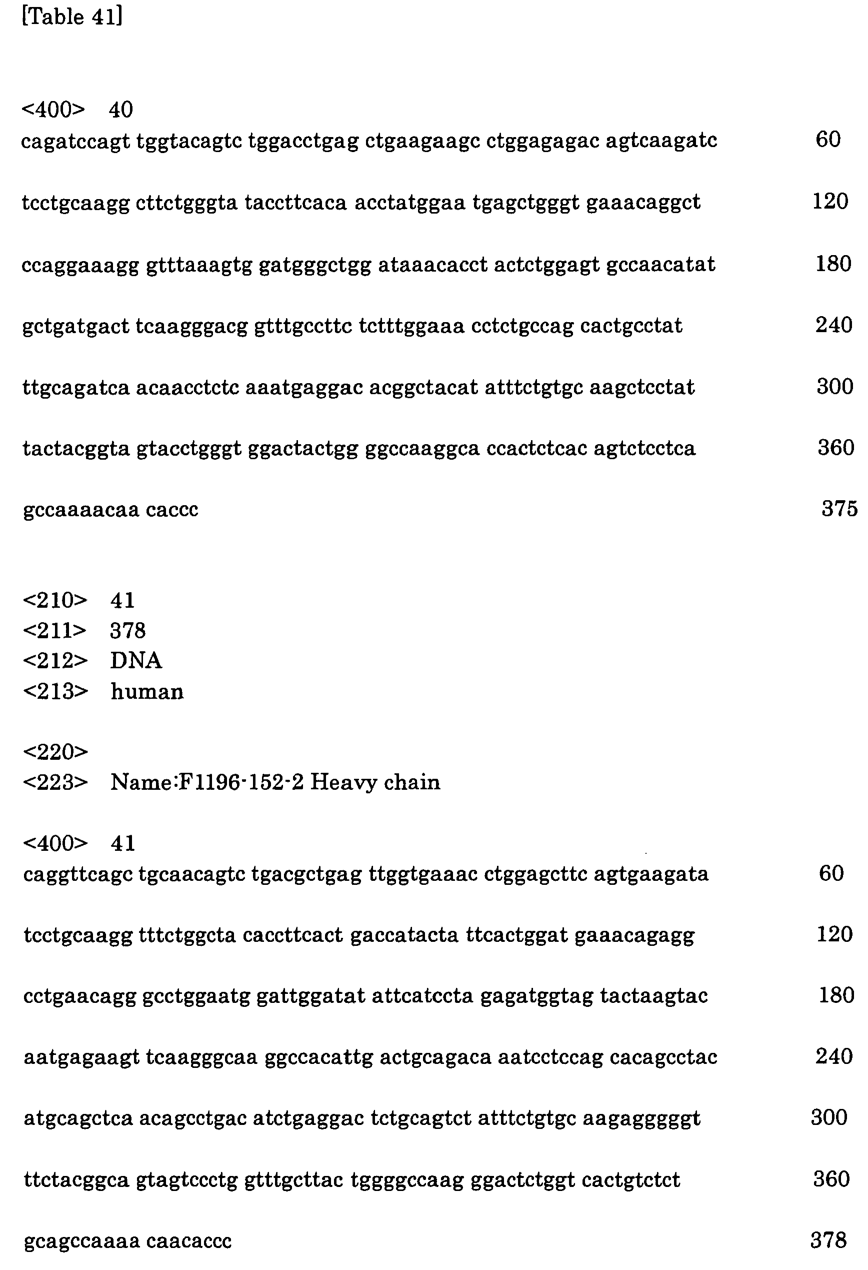

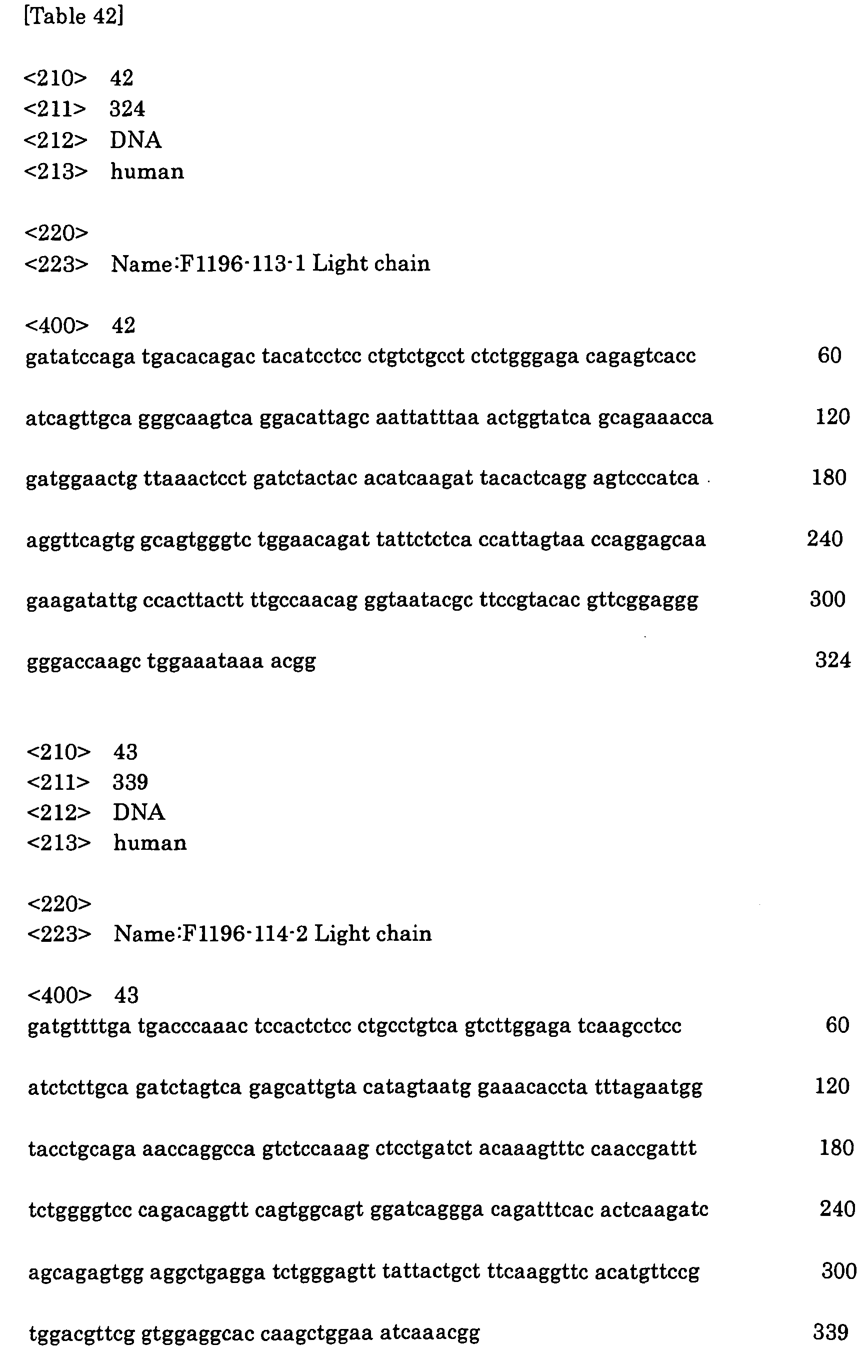

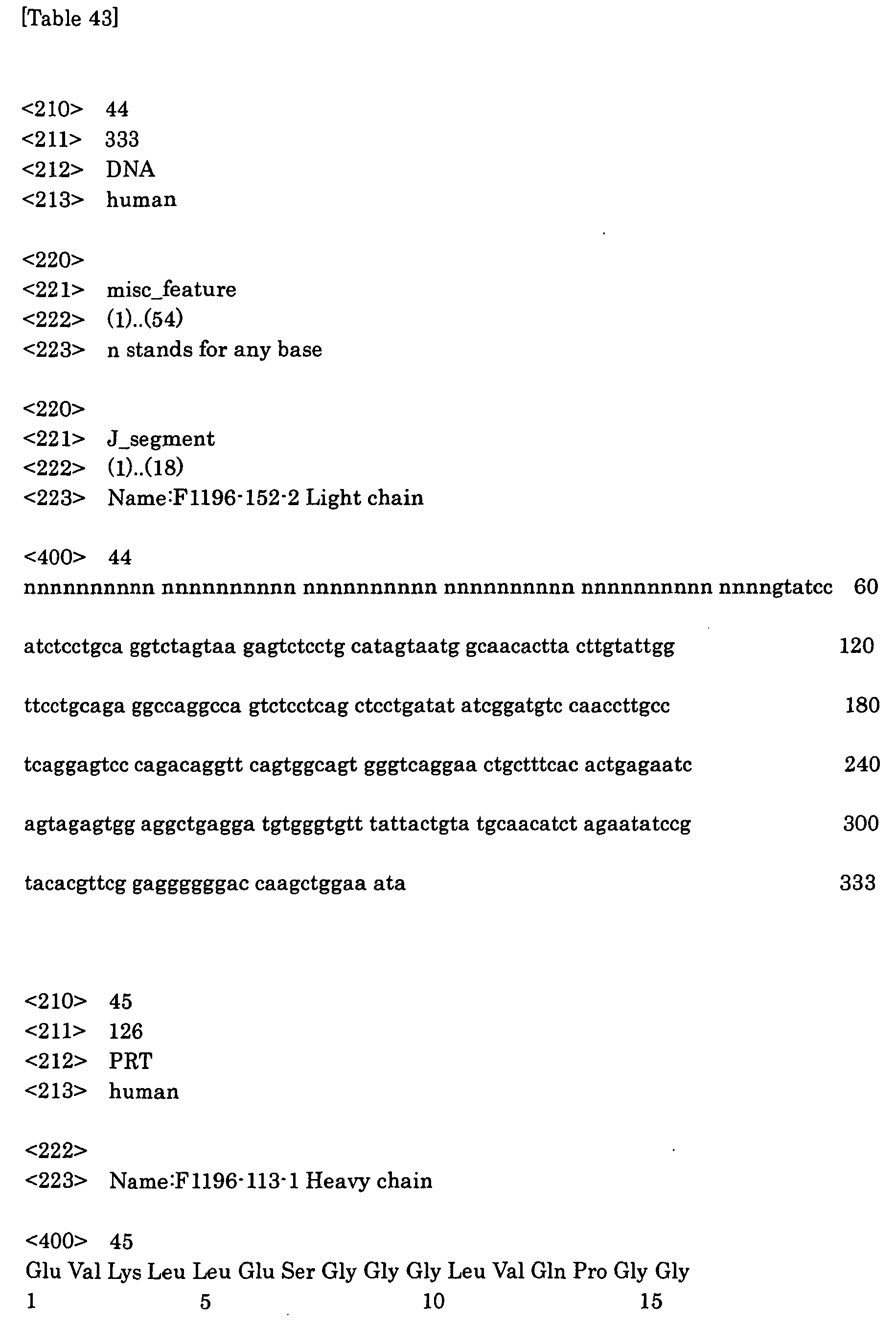

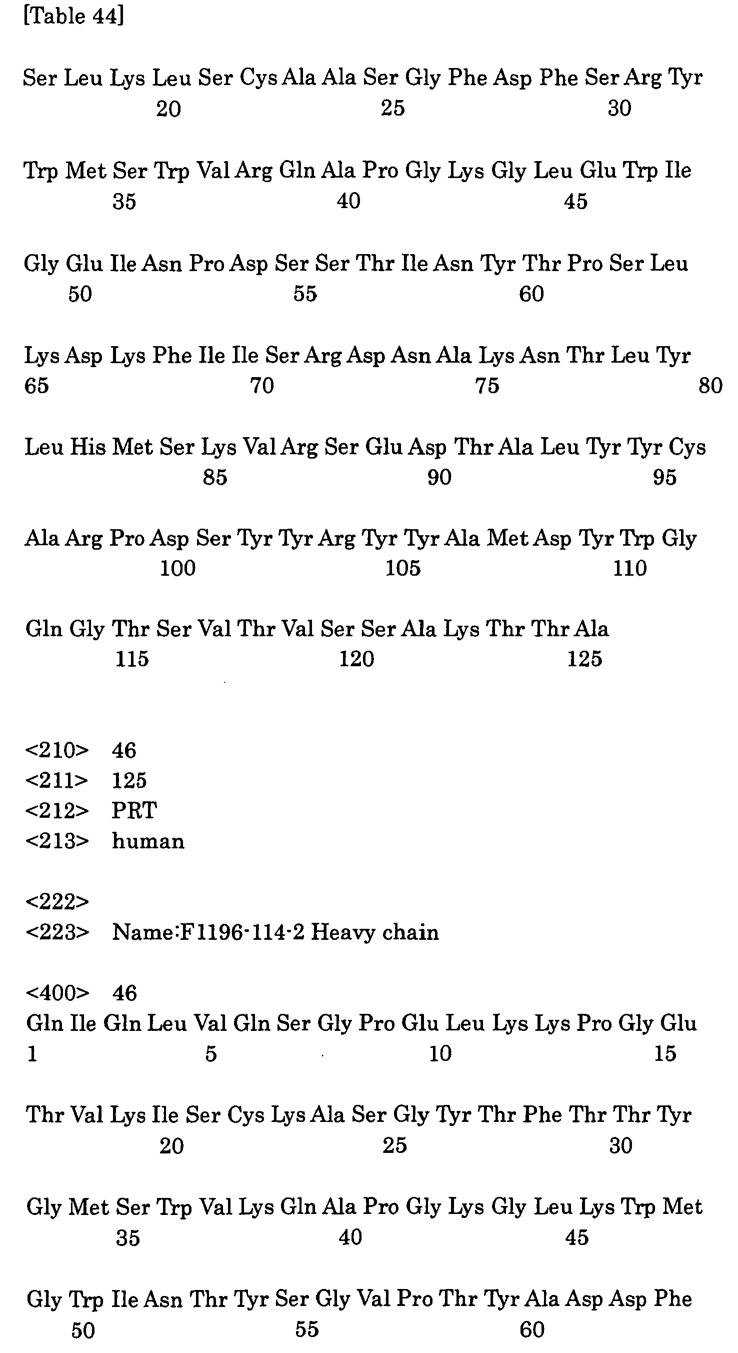

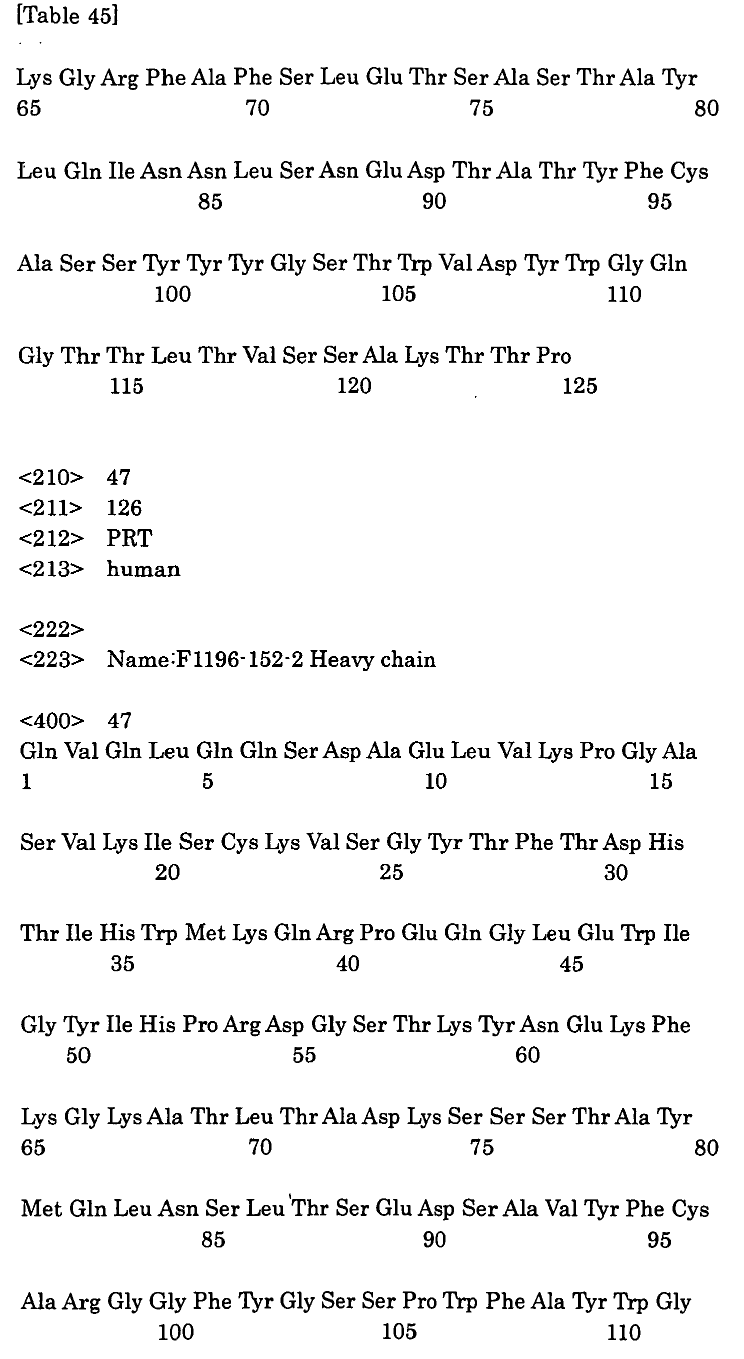

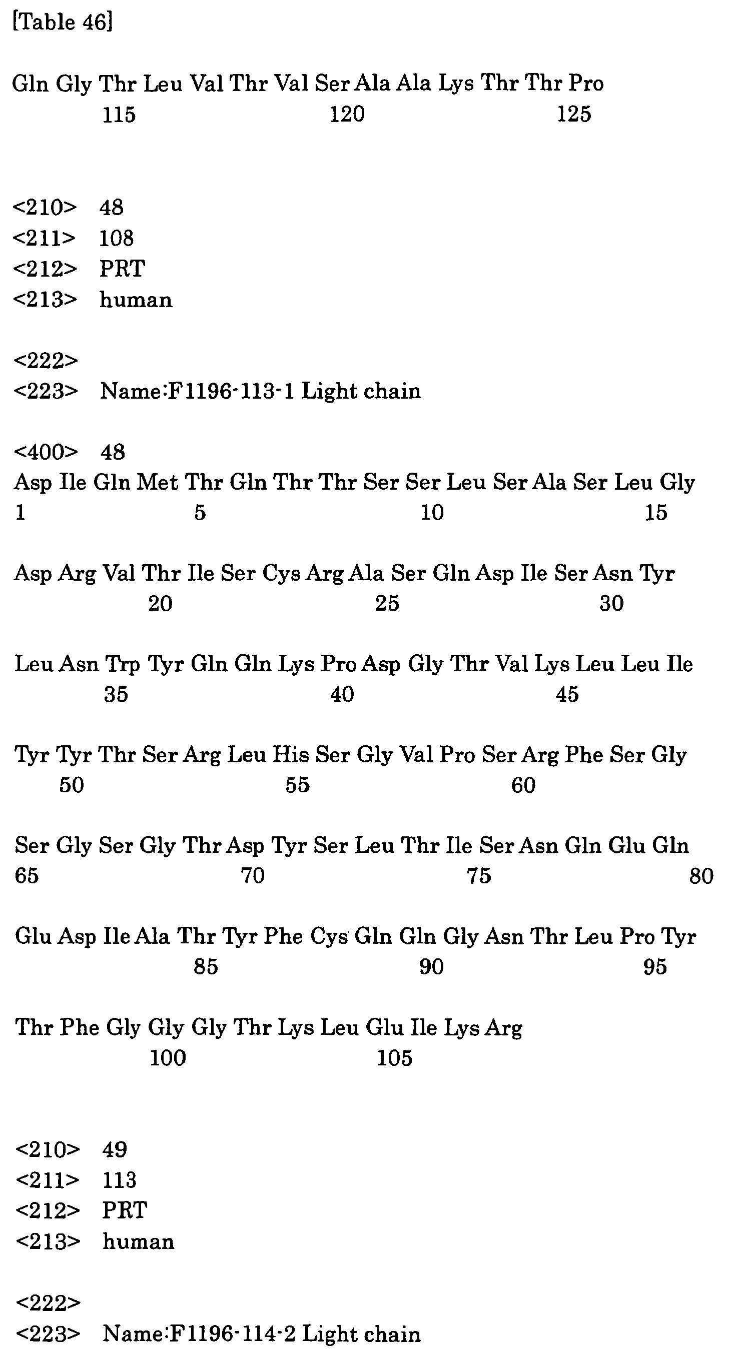

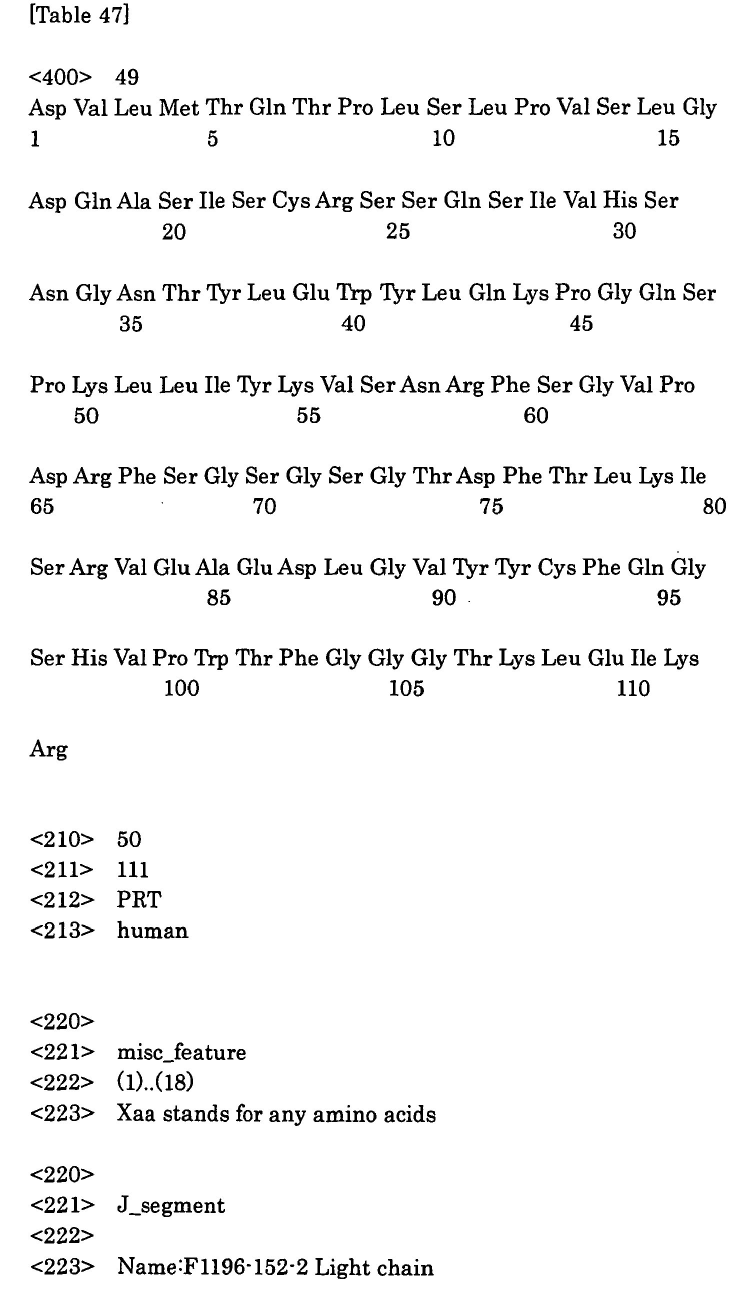

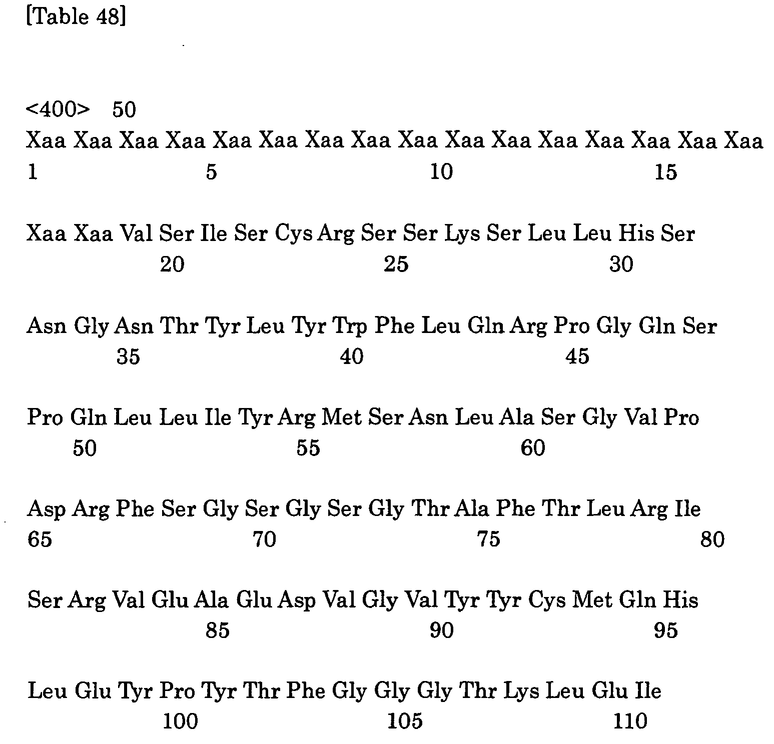

## Claims

1. A preventive or therapeutic agent for obesity, adiposity, or a disease caused by or related to obesity, which contains a meltrin α antagonist as its effective component.

2. The preventive or therapeutic agent according to claim 1 wherein the meltrin α antagonist suppresses expression of meltrin α.

3. The preventive or therapeutic agent according to claim 2 wherein the meltrin α antagonist is an antisense compound for human meltrin α.

4. The preventive or therapeutic agent according to claim 2 wherein the human meltrin α antagonist is siRNA for human meltrin α.

5. The preventive or therapeutic agent according to claim 1 wherein the meltrin α antagonist inhibits activity of meltrin α.

6. The preventive or therapeutic agent according to claim 5 containing an anti-meltrin α antibody capable of recognizing human meltrin α as its effective component.

7. The preventive or therapeutic agent according to claim 6 wherein the antibody is a human antibody.

8. The preventive or therapeutic agent according to claim 6 wherein the antibody is a humanized antibody.

9. The preventive or therapeutic agent according to claim 5 wherein the antagonist is a low molecular weight compound.

10. The preventive or therapeutic agent according to any one of claims 1 to 9 which is administered to a patient having at least one symptom selected from obesity, fatty liver, hyperlipidemia, hyperglycemia, and hyperinsulinemia.

11. The preventive or therapeutic agent according to any one of claims 1 to 10 which exhibits at least one of the following effects of:
(1) suppressing accumulation of white adipocytes;
(2) suppressing proliferation of white adipocytes;
(3) suppressing accumulation of adipocytes in the internal organs;
(4) reducing blood cholesterol;
(5) enhancing energy metabolism; and
(6) suppressing formation and progress of fatty liver and onset of hepatopathy.

12. The preventive or therapeutic agent according to any one of claims 1 to 10 wherein the disease caused by or related to obesity is diabetes.

13. A preventive or therapeutic agent for diabetes containing a meltrin α antagonist as its effective component.

14. An antisense compound for meltrin α against the region of nucleotide Nos. 61 to 360, or 2581 to 3020 in SEQ ID NO: 3 of the Sequence Listing.

15. An antisense compound for meltrin α against the region of nucleotide Nos. 2581 to 2680, 2701 to 2760, 2771 to 2800, 2849 to 2905, or 2971 to 3020 in SEQ ID NO: 3 of the Sequence Listing.

16. An antisense compound for meltrin α containing any one of the nucleotide sequences shown in Tables 6 to 8.

17. The preventive or therapeutic agent according to claim 3 or any one of claims 10 to 12 which contains the antisense compound for meltrin α of any one of claims 13 to 16 as its effective component.

18. An antibody, its active fragment, or a derivative thereof which recognizes human meltrin α, and which has at least one activity selected from enhancement of energy metabolism, inhibition of proliferation of preadipocyte, inhibition of differentiation of adipocyte, and inhibition of metalloprotease activity of the human meltrin α.

19. Heavy chain, its active fragment, or a derivative thereof of an antibody which has amino acid sequences of SEQ ID NOS: 51, 53, and 55; SEQ ID NOS: 63, 65, and 67; or SEQ ID NOS: 75, 77, and 79 as VH CDR1, VH CDR2, and VH CDR3, respectively.

20. Light chain, its active fragment, or a derivative thereof of an antibody which has amino acid sequences of SEQ ID NOS: 57, 59, and 61; SEQ ID NOS: 69, 71, and 73; or SEQ ID NO: 81, 83, and 85 as VL CDR1, VL CDR2, and VL CDR3, respectively.

21. An antibody, its active fragment, or a derivative thereof which has amino acid sequences of SEQ ID NOS: 51, 53, 55, 57, 59, and 61; amino acid sequences of SEQ ID NOS: 63, 65, 67, 69, 71, and 73; or amino acid sequences of SEQ ID NOS: 75, 77, 79, 81, 83, and 85 as VH CDR1, VH CDR2, VH CDR3, VL CDR1, VL CDR2, and VL CDR3, respectively.

22. A polynucleotide containing nucleotide sequence coding for H chain and/or L chain of the antibody of any one of claims 18 to 21 or its active fragment or a derivative thereof.
